(19) Europäisches Patentamt
European Patent Office
Office européen des brevets

(11) Publication number: **0 302 599 A2**

# (12) EUROPEAN PATENT APPLICATION

(21) Application number: 88306197.0

(22) Date of filing: 07.07.88

(51) Int. Cl.4: C07D 493/08 , C07D 493/20 , C07D 497/08 , C07D 493/18 , A01N 43/90 , //(C07D493/08, 317:00,311:00),(C07D493/08, 319:00,311:00),(C07D493/20, 317:00,311:00,311:00), (C07D493/18,317:00,311:00, 303:00),(C07D497/08,327:00, 311:00)

The title of the invention has been amended (Guidelines for Examination in the EPO, A-III, 7.3).

(30) Priority: 08.07.87 NZ 221006
08.07.87 NZ 221007
08.07.87 NZ 221008
08.07.87 NZ 221009

(43) Date of publication of application:
08.02.89 Bulletin 89/06

(84) Designated Contracting States:
AT BE CH DE ES FR GB GR IT LI LU NL SE

(71) Applicant: **HER MAJESTY THE QUEEN IN RIGHT OF NEW ZEALAND DEPARTMENT OF SCIENTIFIC AND INDUSTRIAL RESEARCH CHEMISTRY DIVISON**
Gracefield Road
Lower Hutt(NZ)

(72) Inventor: Furneaux, Richard Hubert
35 Huntingdon Street
Wilton Wellington(NZ)
Inventor: Mason, Jennifer Mary
11 Rakeiora Grove
Petone(NZ)
Inventor: Tyler, Peter Charles
143 Creswick Tce
Northland Wellington(NZ)
Inventor: Blattner, Regine
281 B Muritai Drive
Eastbourne(NZ)
Inventor: Lawson, Kevin Robert
High Style Piddington Lane
Piddington High Wycombe, Bucks HI11 2BB(GB)
Inventor: Chrystal, Ewan James Turner
7 Coombe Pine Crown Wood
Bracknell Berkshire RG12 3TG(GB)
Inventor: Mitchell, Glynn
159 High Street
Iver, Bucks SL0 9OB(GB)
Inventor: Pilkington, Brian Leslie
9 Highway Road
Maidenhead Berkshire SL6 5AE(GB)

(74) Representative: Holmes, Michael John et al
Frank B. Dehn & Co. Imperial House 15-19 Kingsway
London WC2B 6UZ(GB)



Xerox Copy Centre

(54) **Bicyclo-octane and bicyclo-nonane derivatives, processes for their preparation and their use as herbicides.**

(57) Bicyclo-octane and bicyclo-nonane derivatives are described having herbicidal and plant growth regulating properties. The derivatives are based on the general formula

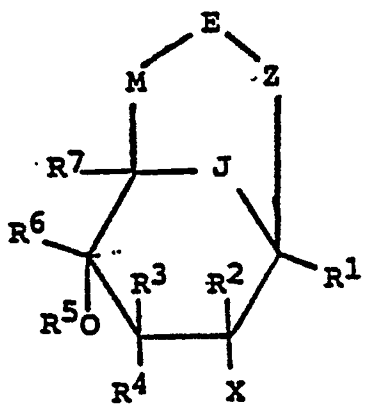

(I)

## Chemical Compounds and Compositions

The present invention relates to bicyclo-octane and -nonane derivatives and their analogues, processes for their production and their use as herbicides and plant growth regulants.

The compounds of the invention have the formula (I)

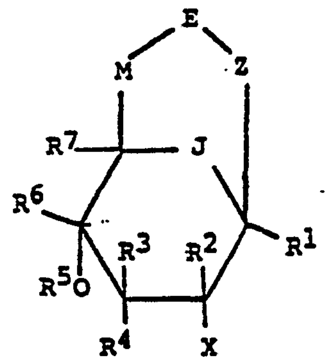

(I)

in which

$R^1$ represents a hydrogen or halogen atom, an O-, N-, P- or S-linked organic or optionally substituted hydrocarbyl group

or $R^1$, together with X, E or Z, may form an optionally substituted fused alicyclic or heterocyclic member;

$R^2$ represents a hydrogen or fluorine atom, a hydroxy group or an optionally substituted hydrocarbyl or O-linked hydrocarbyl group or $R^2$, together with X, may form a ketonic oxygen atom -O- or a group of the formula $-CH_2O-$;

$R^3$ represents a hydrogen or halogen atom, a hydroxy group or an optionally substituted hydrocarbyl or O-linked hydrocarbyl group

or $R^3$, together with $R^4$, may form an optionally substituted hydrocarbyl group;

$R^4$ represents a hydrogen or halogen atom, a hydroxy group or an optionally substituted hydrocarbyl or O-linked hydrocarbyl group

or $R^4$, together with X, may form an oxygen atom;

$R^5$ represents an optionally substituted aralkyl, alicyclicalkyl, heterocyclicalkyl, aryl, alicyclic, heterocyclic, alkyl, alkenyl or alkynyl group

$R^6$ represents a hydrogen atom or an optionally substituted hydrocarbyl group;

$R^7$ represents a hydrogen or halogen atom, an O-, N-, P- or S-linked organic or optionally substituted hydrocarbyl group or together with M or E, $R^7$ may form an optionally substituted fused alicyclic or heterocyclic member;

X represents an O-, N- or S-linked organic group, an optionally substituted hydrocarbyl group, a halogen atom, a hydroxy, amino, alkoxyamino, nitro, cyano, azido, sulpho or phospho group,

M, E and Z independently of each other represent an O or S atom, a sulphinyl, sulphonyl, amino, phosphinico or N-linked organic group, a single bond, or the group $CR^8R^9$,

wherein $R^8$ represents a hydrogen or halogen atom, an O-, N-, P- or S-linked organic or optionally substituted hydrocarbyl group, a hydroxy, amino, alkoxyamino, nitro, cyano, azido, sulpho or phospho group or $R^8$, together with $R^9$, may form an optionally substituted spiroalicyclic or spiroheterocyclic member, an optionally substituted hydrocarbyl group or a double bonded oxygen atom;

and wherein $R^9$ represents a hydrogen or halogen atom, an O-, N-, P- or S-linked organic or optionally substituted hydrocarbyl group, a hydroxy, amino, alkoxyamino, nitro, cyano, azido, sulpho or phospho group or; and

wherein, adjacent M, E or Z groups may be joined by a double bond; and

wherein, two or more of M, E and Z may form an optionally substituted fused alicyclic or heterocyclic ring;

with the proviso that only one of the group M, E or Z may represent a single bond;

J represents an O or S atom, a sulphinyl, sulphonyl, amino, phosphinico or N-linked organic group or an optionally substituted hydrocarbyl group;

and the enantiomers, salts and mixtures thereof;

with the following provisos:

(i) when one of M, E and Z is a single bond and J is an oxygen atom then at least one of the remainder of M, E and Z represents an O or S atom, a sulphinyl, sulphonyl, amino, phosphinico or N-linked organic group or the group $CR^8R^9$, in which at least one of $R^8$ or $R^9$ is not a hydrogen atom or O-linked organic or optionally substituted hydrocarbyl group;

(ii) when -M-E-Z- is $-OCH_2-$, J is an O atom, X and $R^5O$ are the same aralkoxy group, and $R^1$ is hydrogen, then the enantiomer thereof is not included;

(iii) when -M-E-Z- together form the group $-CH_2O-$, J is an oxygen atom, $R^7$ is a hydrogen atom, and $R^5$ is an optionally substituted aralkyl group, then

(a) $R^1$ represents an O-, N-, P- or S-linked organic group; or

(b) $R^1$, together with X, forms an optionally substituted fused alicyclic or heterocyclic member; or

(c) $R^2$ represents a fluorine atom; or

(d) $R^3$ or $R^4$ represents a halogen atom; or

(e) $R^3$, together with $R^4$, forms an optionally substituted hydrocarbyl group; or

(f) $R^4$, together with X, forms an oxygen atom;

(iv) when -M-E-Z-- is $-OCH_2-$, J is an oxygen atom, $R^1$ is a hydrogen atom and X is an optionally substituted aralkoxy group, then

(a) $R^2$ represents a fluorine atom; or

(b) $R^3$ or $R^4$ represents a halogen atom; or

(c) $R^3$, together with $R^4$, forms an optionally substituted hydrocarbyl group; or

(d) $R^4$, together with X, forms an oxygen atom;

(v) when J is O, -M-E-Z- is $-CH_2O-$ and $R^1$ and $R^7$ both represent a hydrogen atom then:

(a) $OR^5$, X and $R^3$ are not the same substituted alkyloxy group;

(b) when $R^3$ is a hydroxy or O-linked organic group, X is a fluorine atom, or a hydroxy, O-linked organic, amino, azido, methyl, acetamido or benzylamino group, $R^5$ is not an unsubstituted $C_1$-$C_4$ alkyl or $C_2$-$C_4$ alkenyl or a tetrahydrofuran-1-methyl group in which each of the 2, 3 and 4 positions is substituted with an O-linked organic or hydroxy group; or a substituted alkyl group where the substituent is O-linked or contains oxygen as the only heteroatom; and

(vi) when -M-E-Z- is $-CR^8R^9O-$, $R^8$ is an exo-ethyl or exo-fluoro group and $R^1$, $R^7$ and $R^9$ are hydrogen, then $OR^5$, X and $R^3$ are not all benzyloxy;

(vii) when -M-E-Z- is $-CH_2O-$, J is an oxygen atom, and $R^1$, $R^2$, $R^3$, $R^6$ and $R^7$ are hydrogen atoms, then $R^5O$, $R^4$ and X are not all methoxy groups;

(viii) when -M-E-Z- is $-C(phenyl)_2O-$, J is an oxygen atom, and $R^1$, $R^2$, $R^4$, $R^6$ and $R^7$ represent hydrogen atoms then $R^5O$, $R^3$ and X are not all methoxy;

(ix) when -M-E-Z- is $-CH_2O-$, $R^5$ is methyl, J is an oxygen atom, $R^1$, $R^3$, $R^4$, $R^6$ and $R^7$ are hydrogen atoms then X and $R^2$ are not jointly an oxygen atom, or not both hydroxy or methoxy groups or X is not hydroxy when $R^2$ is a hydrogen atom.

Whilst the invention excludes in provisos (v) to (ix) compounds known in the art, the prior art does not suggest any herbicidal or plant growth regulating activity for those compounds.

Throughout this specification and claims the six-membered ring bearing the $R^5O$ and X substituents in the structural formulae is given according to the Hawarth projection wherein the ring substituents are indicated as being above or below the plane of the ring. Hydrogen atoms are not indicated. The typical stable conformation of this six membered ring in these compounds is the chair form shown in formula (A)

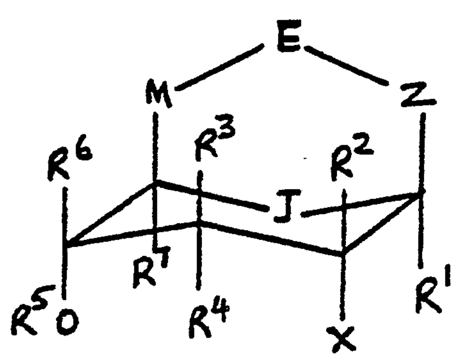

(A)

In some circumstances the chair form may be distorted with substituents X, $R^3$ and $R^5O$ becoming wholly or partially equatorial.

Preferred compounds of formula (I) for the present invention include those compounds wherein

$R^1$ represents a hydrogen atom or an optionally substituted hydrocarbyl group of $C_{1-6}$ carbon atoms;
$R^2$ represents a hydrogen atom;
$R^3$ represents a hydrogen atom;
$R^4$ represents a hydrogen atom;
$R^5$ represents a benzyl, methylbenzyl, benzothienyl, 2-thiophenemethyl, 3-thiophenemethyl, indan-2-yl, tetrahydropyran-2-ylmethyl, ($C_{6-8}$-cycloalkyl)methyl, 2-alkyl-alkyl or cycloalkenemethyl group optionally substituted with up to two substituents independently chosen from chlorine, bromine or fluorine atoms, a methoxy group or a $C_{1-3}$ alkyl group optionally substituted with up to three fluorine atoms;
$R^6$ represents a hydrogen atom;
$R^7$ represents a hydrogen atom or an optionally substituted hydrocarbyl group of $C_{1-6}$ carbon atoms;
M, E or Z
independently represent an O or S atom, an amino, sulphinyl, sulphonyl or $C_{1-6}$ alkylamino group, a single bond, a $C_{1-6}$ alkylene group or the group $-CR^8R^9-$ wherein
$R^8$ represents a hydrogen, fluorine, chlorine or bromine atom, an optionally substituted hydrocarbyl group of $C_{1-6}$ carbon atoms or an alkylthio group;
$R^9$ represents a hydrogen, fluorine, chlorine or bromine atom, an optionally substituted hydrocarbyl group of $C_{1-6}$ carbon atoms, or an alkylthio group;
X represents a $C_{1-6}$ alkoxy group, an optionally substituted hydrocarbyl group of $C_{1-6}$ carbon atoms, a fluorine, chlorine or bromine atom or an azido group;
J represents an O or S atom, an amino or a $C_{1-6}$ alkylamino group or a $C_{1-6}$ alkylene group.

In a form mostly preferred, compounds of formula (I) for the present invention include those compounds wherein:
$R^1$ represents a hydrogen atom or an unbranched $C_{1-6}$ alkyl group optionally substituted in the terminal position with a halogen or methoxy group;
$R^2$ represents a hydrogen atom;
$R^3$ represents a hydrogen atom;
$R^4$ represents a hydrogen atom;
$R^5$ represents a benzyl, methylbenzyl, benzothienyl, 2-thiophenemethyl, 3-thiophenemethyl, indan-2-yl, tetrahydropyran-2-ylmethyl, cyclooctanemethyl, 3-cyclohexenemethyl or 2-($C_{1-2}$ alkyl)-$C_{3-6}$ alkyl cyclohexanemethyl group optionally substituted with up to two substituents independently chosen from chlorine, bromine or fluorine atoms, a methoxy group or a $C_{1-3}$ alkyl group optionally substituted with up to three fluorine atoms;
$R^6$ represents a hydrogen atom;
$R^7$ represents a hydrogen atom or an optionally substituted hydrocarbyl group of $C_{1-6}$ carbon atoms;
M, E or Z
independently represent an O or S atom, an amino or $C_{1-6}$ alkylamino group, a single bond, a $C_{1-6}$ alkylene group or the group $-CR^8R^9-$ wherein
$R^8$ represents a hydrogen, fluorine, chlorine or bromine atom, an optionally substituted hydrocarbyl group of $C_{1-6}$ carbon atoms or an alkylthio group;
$R^9$ represents a hydrogen, fluorine, chlorine or bromine atom, an optionally substituted hydrocarbyl group of $C_{1-6}$ carbon atoms or an alkylthio group;
X represents an ethoxy, methoxy or azido group, a fluorine, chlorine or bromine atom, or an optionally substituted hydrocarbyl group of $C_{1-6}$ carbon atoms;
J represents an O or S atom, an amino or a $C_{1-6}$ alkylamino group or a $C_{1-6}$ alkylene group.

The terms "alkyl" and "alkoxy" except in the case of $R^5$ used herein refer preferably to $C_{1-6}$, especially $C_{1-4}$' alkyl and alkoxy groups, most preferably methyl, methoxy, ethyl, ethoxy, n-propyl, n-propoxy, isopropyl and isopropoxy, and include also such groups substituted by one or more $C_{1-6}$ cycloalkyl groups.

In the case of $R^5$, the alkyl group is preferably a 2-(alkyl)-alkyl group having at least 4 carbon atoms.

The terms "alkenyl" and "alkenyloxy", and "alkynyl" and "alkynyloxy" except in the case of $R^5$ used herein refer preferably to $C_{2-6}$' especially $C_{2-4}$ groups such as allyl, allyloxy, propynyl and propynyloxy groups.

In the case of $R^5$, alkenyl and alkynyl groups are preferably branched at the 2-carbon atom and have at least 4 carbon atoms.

The term "alicyclic group" refers to a carbocyclic group that may be saturated or unsaturated. Examples of such groups which may be mentioned include cyclopropyl, cyclobutyl, cyclopentyl, cyclohexyl, cycloheptyl, 1-cyclohexen-1-yl and 2-cyclohexen-1-yl.

The term "heterocyclic group" refers to an alicyclic group within which one or more of the ring carbon

atoms is replaced by a heteroatom, with a consequent adjustment where necessary, in the number of substituents present to allow for any valence difference between the heteroatom and the replaced carbocyclic carbon atom. A heteroatom may be for example an oxygen, nitrogen or sulphur atom.

Examples of such groups which may be mentioned include tetrahydrofuranyl, tetrahydrothiopyranyl, pyrrolidinyl and piperidyl, each of which may optionally be linked through different carbocyclic carbon atoms.

The term "aryl group" refers to a carbocyclicaryl or heterocyclicaryl group. Examples of such groups which may be mentioned include the phenyl, naphthyl, anthryl, quinolyl, pyridyl, furyl, thienyl, benzimidazolyl, benzothienyl and xanthenyl group.

The terms "alicyclicalkyl group" and "heterocyclicalkyl group" refer to an alkyl group substituted by an alicyclic group or a heterocyclic group, respectively, such groups being defined herein. The alkyl portion of the group may suitably contain up to six carbon atoms, not including carbon atoms which may be present in any substituent groups. These groups may be optionally substituted in the alkyl, alicyclic or heterocyclic portion. Examples of alicyclicalkyl groups which may be mentioned include the cyclohexylmethyl, cyclopentylmethyl, 2-cyclohexen-1-ylmethyl and 3-cyclohexen-1-ylmethyl groups. Examples of heterocyclicalkyl groups which may be mentioned include the tetrahydrofuran-2-ylmethyl, tetrahydropyran-2-ylmethyl, tetrahydropyran-3-ylmethyl and tetrahydrothiopyran-2-ylmethyl groups.

The term "fused alicyclic member" or "fused heterocyclic member" refers to that portion of an alicyclic or heterocyclic group fused to the parent bicyclic structure of formula (I) and represented by specified combinations of substituent groups of formula (I). Examples of such members which may be mentioned include the 3-oxapropan-1,3-diyl and propan-1,3-diyl groups.

The term "aralkyl group" herein refers to a carbocyclicaryl-alkyl- or heterocyclicaryl-alkyl group, which may optionally be substituted in the aryl and/or alkyl portions. The component alkyl and aryl groups may be as defined herein. The aryl and alkyl portions may if desired be linked together via a hereroatom (eg sulphur) The alkyl chain portion may suitably contain up to 6 carbon atoms, not including carbon atoms which may be present in any substituent groups. Examples of such groups which may be mentioned include the benzyl, 2-phenylethyl, phenylthiomethyl and phenylsulphonyl-methyl groups.

Optional substitution on an alicyclic group or member, a heterocyclic group or member, an alkyl, aryl, aralkyl, alicyclicalkyl or heterocyclic group may for example be by one or more alkyl, alkenyl, alkynyl, hydroxy, alkoxy (eg methoxy), acyloxy, carboxy, alkoxycarbonyl, cyano or alkylthio groups, halogen or double bonded oxygen atoms (eg an aldehyde, ketone or epoxide moiety).

Substituents present on the aryl, alicyclic or heterocyclic group or fused alicyclic or heterocyclic member may be linked to form a fused ring system. Examples of groups or members with such fused ring substituents which may be mentioned include the adamantyl, decahydronaphthyl and indan-7-yl groups and the 1,4-epoxy-cyclohexan-1-yl group and its alkyl substituted analogues.

Substituents present on the aryl, alicyclic or heterocyclic portion of an aralkyl, alicyclicalkyl or heterocyclicalkyl group respectively, may be linked to the alkyl portion or to the substituents of the alkyl portion, to form a closed ring between the aryl, alicyclic or heterocyclic portion and the alkyl portion. Examples of groups with such fused ring substituents which may be mentioned include the indan-2-yl group.

The term "amino group" used herein represents the group $-NH_2$. In the case where J, M, E or Z of formula (I) represents an "amino group" the groups -NH- or -N= are included.

The term "O-, N-, P- and S-linked organic groups" herein refers to organic carbon-containing groups which are linked through a C-O-, C-N-, C-P- and C-S- linkage respectively

O-linked organic groups in the above definition of formula (I) include alkoxy, substituted alkoxy [eg, substituted by an epoxy group or by one or more halogen atoms or hydroxy, alkoxy, aryl (eg phenyl, halophenyl, methylphenyl, trifluoromethylphenyl or methoxyphenyl), alkoxycarbonyl or aralkoxycarbonyl groups], alkenyloxy (eg allyloxy), alkynyloxy (eg prop-2-ynyloxy) and acyloxy (eg acetoxy) groups.

N-linked organic groups in the above definition of formula (I) include mono- or di-alkylamino, mono- or di-aralkylamino, trialkylammonium, carboxamido [eg acetamido and 1-alkyl-3-arylureido (eg 1-methyl-3-phenyl- or 1-methyl-3-(3,4-dichlorophenyl)-ureido)], dicarboximido (eg phthalimido) and N-azole (eg N-pyrazolyl, N-triazolyl or N-imidazolyl) groups.

S-linked organic groups in the above definition of formula (I) include alkylthio, alkylsulphinyl and alkylsulphonyl groups and substituted derivatives thereof (eg aralkylthio groups such as the benzylthio group).

P-linked organic groups in the above definition or formula (I) include groups of formulae $-P(O)R^AR^B$, $-P(O)(OR^A)R^B$ and $-P(O)(OH)R^A$ wherein $R^A$ and $R^B$ independently represent optionally substituted hydrocarbyl groups.

The term "hydrocarbyl group" herein refers to a saturated or wholly or partially unsaturated group containing only carbon and hydrogen atoms. Examples of such groups which may be mentioned include alkyl (eg methyl), alkenyl, alkynyl groups (particularly those containing up to 6, especially up to 4, carbon atoms) and carbocyclicaryl and carbocyclicaryl-alkyl groups (particularly the phenyl and phenyl-$C_{1-4}$-alkyl groups).

Optional substitution on a hydrocarbyl group may for example be by one or more hydroxy, alkoxy (eg methoxy), acyloxy, cyano or alkylthio groups, halogen or double bonded oxygen atoms (as in an aldehyde, ketone or epoxide moiety).

The term "halogen atom" or "halo" herein includes fluorine, chlorine, bromine and iodine atoms.

As $R^5$, "substituted alkyl" does not include substituted or unsubstituted aralkyl, heterocyclicalkyl and alicyclicalkyl which are alternative values for $R^5$.

Some of the groups in the compounds encompassed by formula I have acidic or basic character and thus can form salts with acids or bases. In addition other substituents include groups which can form other simple derivatives such as esters and amides which will normally retain the herbicidal or plant growth regulating activity of the parent compound. Such derivatives are encompassed by the invention as are the salts.

The salts may for example be addition salts formed with a suitable acidic or basic group of the compound of formula (I) or for example an alkali metal (eg sodium} salt of a compound in which X represents a sulpho or phospho group, and with compounds of formula (I) containing $R^8$ or $R^9$, where $R^8$ or $R^9$ represents a sulpho or phospho group.

The compounds of formula (I) are capable of optical isomerism. The invention includes within its scope enantiomers of compounds of formula (I) and salts thereof. The invention also includes within its scope all possible isomer mixtures.

In a further aspect, the invention provides herbicidal compositions or plant growth regulating compositions containing compounds of formula (I) as defined above except that compounds of proviso (v) to (ix) are included, together with a suitable carrier.

In a still further aspect, the invention provides a method for preparing compositions as described above, which method comprises admixing a suitable amount of at least one compound of formula (I) as defined above or a salt or enantiomer thereof with a suitable carrier.

Suitable solid carriers are natural and synthetic clays and silicates, for example natural silicas, magnesium silicates, calcium carbonates, magnesium aluminium silicates, aluminium silicates, calcium sulphate, waxes, bitumens or synethetic silicon oxides, calcium or aluminium silicates, polymers such as polyvinyl chloride and styrene polymers or solid fertilisers, for example superphosphates. Suitable solid diluents include for example kaolin, bentonite, kieselguhr, dolomite, calcium carbonate, talc powdered magnesia and Fuller's earth. Examples of suitable fluid carriers are water, alcohols, glycols, ketones, ethers, aromatic hydrocarbons such as benzene, toluene and xylene; petroleum fractions and chlorinated hydrocarbons, including liquefied gases.

The compositions of the invention may contain a surface-active agent which may be ionic (eg cationic or anionic) or non-ionic emulsifying, dispersing or wetting agent. Any of the surface-active agents usually applied in formulating herbicides or pesticides may be used. Examples of suitable surface-active agents are quaternary ammonium compounds such as cetyltrimethylammonium bromide; soaps; salts of aliphatic monoesters of sulphuric acid, eg sodium lauryl sulphate; the sodium or calcium salts of polyacrylic acids and lignin sulphonic acids; the condensation product of fatty acids or aliphatic amines or amides containing at least 12 carbon atoms with ethylene or propylene oxide; fatty acid esters of glycerol, sorbitan, sucrose or pentaerythritol; condensation products of fatty alcohols or alkyl phenols with ethylene oxide; alkali or alkaline earth metal salts, preferably sodium salts, of sulphuric or sulphonic acid esters containing at least 10 carbon atoms; and polymers of ethylene oxide and copolymers of ethylene oxide and propylene oxide. Other nonionic agents are the partial esters derived from long chain fatty acids and hexitol anhydrides, for example sorbitol monolaurate; the condensation products of the said partial esters with ethylene oxide and the lecithins.

The compositions of the invention may for example be formulated as wettable powders, dusts, granules, solutions, emulsifiable concentrates, emulsions, suspension concentrates and aerosols.

The composition may for example contain up to 99% by weight of the herbicidal and/or plant growth regulatory active agent. If desired, stabiliser(s) and/or additives such as defoamers, corrosion inhibitors, antioxidants, penetrants or stickers may be present.

The composition of the invention may contain other ingredients, for example, protective colloids such as gelatin, glue, casein, gums, cellulose ethers, and polyvinyl alcohol; and thixotropic agents eg, bentonites and sodium polyphosphates. Suitable stabilisers include ethylenediaminetetra-acetic acid, urea, and

triphenyl phosphate. As stickers there may be used, for example, non-volatile oils.

The compounds of the invention may be used in admixture with other herbicides and pesticides. In particular they may be mixed with active materials such as:

A. benzo-2,1,3-thiadiazin-4-one-2,2-dioxides such as 3-isopropylbenzo-2,1,3-thiadiazin-4-one-2,2-dioxide (bentazon);

B. hormone herbicides, particularly the phenoxy alkanoic acids such as 4-chloro-2-methylphenoxy acetic acid (MCPA), 2-(2,4-dichlorophenyl)propionic acid (dichloroprop), 2,4,5-trichlorophenoxyacetic acid (2,4,5-T),4-(4-chloro-2-methoxyphenoxy)-butyric acid (MCPB), 2,4-dichloro-phenoxyacetic acid (2,4-D), 4-(2,4-dichlorophenoxy)butyric acid (2,4-DB), 2-(4-chloro-2-methylphenoxy)-propionic acid (mecoprop), and their derivatives (eg, salts, esters and amides);

C. 3-[4-(4-halophenoxy)phenyl]-1,1-dialkylureas such as 3-[4(4-chlorophenoxy)phenyl]-1,1-dimethylurea;

D. dinitrophenols and their derivatives (eg, acetates) such as 2-methyl-4,6-dinitrophenol (DNOC), 2-t-butyl-4,6-dinitrophenol (dinoterb), 2-secbutyl-4,6-dinitrophenol (dinoseb) and its ester, dinoseb acetate;

E. dinitroaniline herbicides such as N′,N′-diethyl-2, 6-dinitro-4-tirluoromethyl-m-phenylenediamine (dinitramine), 2,6-dinitro-N,N-dipropyl-4-trifluoromethylaniline (trifluralin) and 4-methyl-sulphonyl-2,6-dinitro-N,N-dipropylaniline (nitralin);

F. phenylurea herbicides such as N′-(3,4-dichlorophenyl)-N,N-dimethylurea (diuron) and N,N-dimethyl-N′-[3-(trifluoromethyl)phenyl]urea (flumeturon);

G. phenylcarbamoyloxyphenylcarbamates such as 3-[methoxycarbonylamino]phenyl (3-methylphenyl) carbamate (phenmedipham) and 3-[ethoxycarbonylamino]phenyl phenyl-carbamate (desmedipham);

H. 2-Phenylpyridazin-3-ones such as 5-aminio-4-chloro-2-phenylpyridazin-3-one (pyrazon);

I. uracil herbicides such as 3-cyclohexyl-5,6-trimethyleneuracil (lenacil), 5-bromo-3-sec-butyl-6-methyl-uracil (bromacil) and 3-t-butyl-5-chloro-6-methyl-uracil (terbacil);

J. triazine herbicides such as 2-chloro-4-ethylaminio-6-(i-propylamino)-1,3,5-triazine (atrazine), 2-chloro-4,6-di-(ethylamino)-1,3,5-triazine (simazine) and 2-azido-4-(i-propylamino)-6-methylthio-1,3,5-triazine (aziprotyrne);

K. 1-alkoxy-1-alkyl-3-phenylurea herbicides such as 3-(3,4-dichlorophenyl)-1-methoxy-l-methylurea (linuron), 3-(4-chlorphenyl)-1-methoxy-1-methylurea (monolinuron), 3-(4-bromo-4-chlorophenyl)-1-methoxy-1-methylurea (chlorobromuron);

L. thiolcarbamate herbicides such as S-propyl dipropylthiocarbamate (verolate);

M. 1,2,4-triazin-5-one herbicides such as 4-amino-4,5-dihydro-3-methyl-6-phenyl-1,2,4-triazine-5-one (metamitron) and 4-aminio-6-t-butyl-4,5-dihydro-3-methylthio-1,3,4-triazin-5-one (metribuzin);

N. benzoic acid herbicides such as 2,3,6-trichlorobenzoic acid (2,3,6-TBA), 3,6-dichloro-2-methoxy-benzoic acid (dicamba) and 3-amino-2,5-dichlorobenzoic acid (chloramben);

O. anilide herbicides such as N-butoxymethyl-chloro-2′,6′-diethylacetanilide (butachlor), the corresponding N-methoxy compound (alachlor), the corresponding N-i-propyl compound (propachlor), 3′,4′-dichloropropionanilide (propanil) and 2-chloro-N-[pryazol-1-ylmethyl]acet-2′-6′-xylidide (metazachlor);

P. dihalobenzonitrile herbicides such as 2,6-dichlorobenzonitrile (dichlobenil), 3,5-dibromo-4-hydroxybenzonitrile (bromoxynil) and 3,5-diiodo-4-hydroxybenzonitrile (ioxynil);

Q. haloakanoic herbicides such as 2,2-dichloro-propionic acid (dalapon), trichloroacetic acid (TCA) and salts thereof;

R. diphenylether herbicides such as 4-nitrophenyl 2-nitro-4-trifluoromethylphenyl ether (fluorodifen), methyl 5-(2,4-dichlorophenoxy-2-nitrobenzoate (bifenox), 2-nitro-5-(2-chloro-4-trifluoromethylphenoxy) benzoic acid, (acifluorfen) and salts and esters thereof, 2-chloro-4-trifluoromethylphenyl 3-ethoxy-4-nitrophenyl ether (oxyfluorfen) and 5-(2-chloro-4-(trifluoromethyl)phenoxy-N-(methylsulphonyl)-2-nitrobenzamide (fomesafen), 5-(2-chloro-6-fluoro-4-(trifluoromethyl)phenoxy)-N-(ethylsulphonyl)-2-nitrobenzamide (halosafen);

S. phenoxyphenoxyproprionate herbicides such as 2-(4-(4′-trifluoromethylphenoxy)phenoxy)-propionic acid methyl ester (trifop-methyl), 2-(4-((5-trifluoromethyl)-2-(pyridinyl)oxy)phenoxy)-propanoic acid (fluazifop) and esters thereof, 2-(4-((3-chloro-5-trifluoromethyl)-2-(pyridinyl)-oxy)phenoxy)propanoic acid (haloxyfop) and esters thereof, 2-(4-((6-chloro-2-quinoxalinyl)oxy)phenoxy)propanoic acid (xylofop) and esters thereof;

T. cyclohexanedione herbicides such as 2,2-dimethyl-4,6-dioxo-5-(1-((2-propenyloxy)amino)-butylidine)cyclohexanecarboxylic acid (alloxydim) and salts thereof, 2-(1-ethoxyimino)butyl-5-(2-(ethylthio propyl)-3-hydroxy-2-cyclohexen-1-one (sethoxydim), 2-(1-(3-chloroallyloxyimino)butyl)-5-(2-ethylthiopropyl)-3-hydroxycyclohex-2-enone (cloproxydim), 2-(1-ethoxymino)butyl-3-hydroxy-5-thian-3-yl-cyclohex-2-enone-(cycloxydim);

U. sulfonyl urea herbicides such as 2-chloro-N-(4-methoxy-6-methyl-1,3,5-triazin-2-yl)-aminocarbonylbenzenesulphonamide (chlorosulfuron), methyl 2-((((4,6-dimethyl-2-pyrimidinyl}amino)-carbonyl)-amino)sulphonylbenzoic acid (sulfometuron), 2-(((3-(4-methoxy-6-methyl-1,3,5-triazin-2-yl)-carbonyl)-amino)sulphonyl)-benzoic acid (metsulfuron) and esters thereof;

V. imidazolidininone herbicides such as 2-(4,5-dihydro-4-isopropyl-4-methyl-5-oxoimidazol-2-yl)-quinoline-3-carboxylic acid (imazaquin), methyl 6-(4-isopropyl-4-methyl-5-oxo-2-imidazolin-2-yl)-m-toluate and p-toluate isomer (AC 222293);

W. arylanilide herbicides such as 1-methylethyl-N-benzoyl-N-(3-chloro-4-fluorophenyl)-L-alanine (flamprop-isopropyl), ethyl N-benzoyl-N-(3,4-dichlorophenyl)-DL-alaninate (benzoylprop-ethyl), N-(2,4-difluorophenyl)-2-(3-(trifluoromethyl)-phenoxy)-3-pyridinecarboxamide (diflufenican);

X. amino acid herbicides such as N-(phosophonomethyl)-glycine (glyphosate) and DL-homoalanin-4-yl-(methyl)-phosphinic acid (phosophinothricin) and their salts and esters;

Y. organoarsenical herbicides such as monosodium methanearsonate (MSMA); and

Z. miscellaneous herbicides including N,N-dimethyldiphenylacetamide (diphenamid), N-(1-naphthyl)-phthalamic acid (naptalam) and 3-aminio-1,2,4-triazole, 2-ethoxy-2,3-dihydro-3,3-dimethyl-benzofuran methanesulfonate (ethofumesate), 1,4-epoxy-p-menth-2-yl 2-methylbenzyl ether (cinmethylin), 2-(2-chlorophenyl)methyl-4,4-dimethyl-3-isoxazolidinone (dimethazone);

AA. Examples of useful contact herbicides include: bipyridylium herbicides such as those in which the active entity is the 1,1′-dimethyl-4,4′-dipyridylium ion (paraquat) and those in which the active entity is the 1,1′-ethylene-2,2′-dipyridylium ion (diquat).

The complementary herbicide is suitably present in the mixture or composition in an amount such that it is applied at its conventional rate.

The amount of active compound(s) used can vary within a fairly wide range. It depends essentially on the nature of the desired effect. In general, the amounts used are from 0.01 to 10 kg of active compound per ha, preferably from 0.1 to 5 kg of active compound per ha.

The compositions of the invention can be applied either before or after the emergence of the plants. They can also be worked into the soil before sowing.

In addition to their application as herbicides and/or plant growth regulants, certain of the compounds of formula (I) and their enantiomers and salts are valuable as starting materials for the preparation of other active compounds of formula (I) and their enantiomers and salts.

The compounds of formula (I), their intermediates, enantiomers and salts thereof may, for example, be prepared by the following processes:

(a) cyclising a compound of formula (IIa), (IIb), (IIc), (IId), (IIIa), (IIIb), (IIIc) and (IIId)

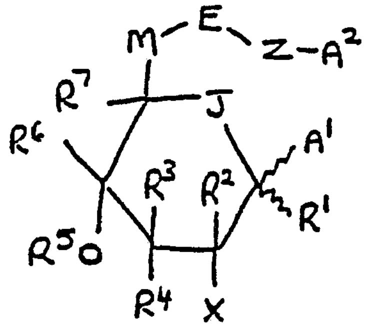

(IIa)

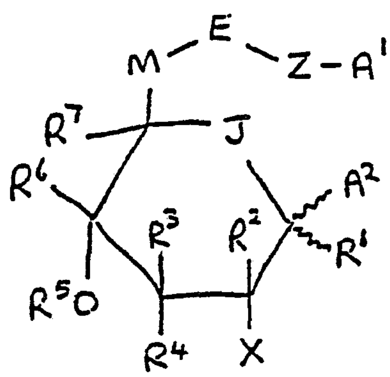

(IIb)

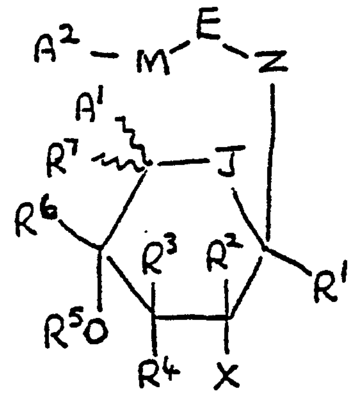

(IIc)

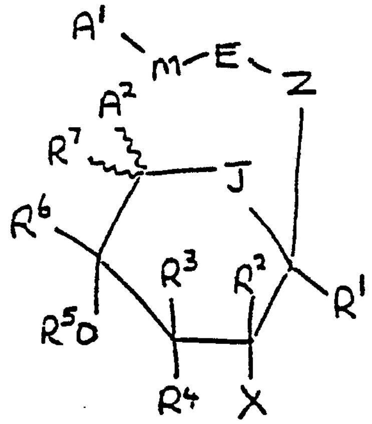

(IId)

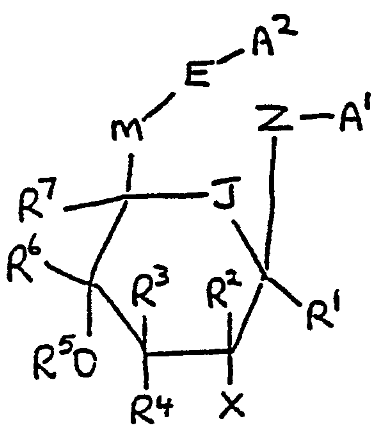

(IIIa)

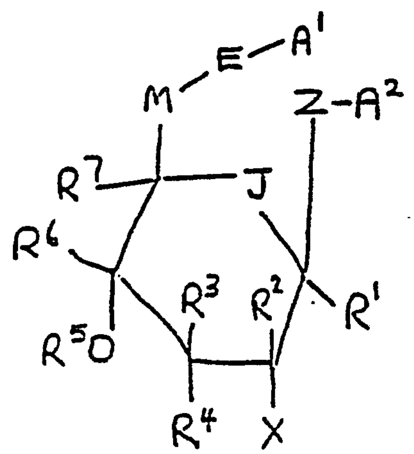

(IIIb)

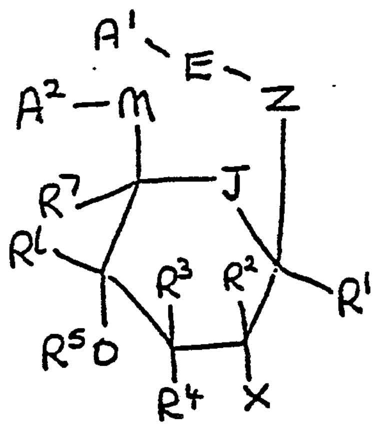

(IIIc)

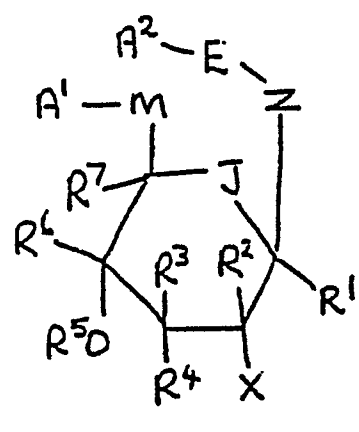

(IIId)

(in which X, $R^1$, $R^2$, $R^3$, $R^4$, $R^5$, $R^6$, $R^7$, M, J, E and Z are as defined above, or represent protected forms thereof,
$A^2$ is a group or atom capable of being split off to leave the atom or group to which it was joined with a negative charge,
and $A^1$ is a group or atom capable of being split off to leave the group or atom to which it was joined with a

positive charge)
or an enantiomer thereof,
and optionally subsequently splitting off or converting any protective groups present to produce the desired compounds.

The term "protected form thereof" used throughout this specification includes the use of temporary deactivating substituents and the use of different substituents which are precursors for the desired group. For example, in this process a protected form of $OR^5$ may be a hydroxy group which is inactive to the reaction conditions but may readily be replaced by the desired group of formula (I). In a further example, a protected form of $R^5$ may be an aralkyl group such as benzyl, which may be removed by hydrogenolysis and replaced by the desired $R^5$ group defined in formula (I), or which may be hydrogenated to yield a desired compound of formula (I) in which $R^5$ represents an optionally substituted alicyclicalkyl group.

Groups or atoms for $A^2$ capable of being split off to leave a negative charge include hydrogen atoms, aralkyl groups eg, benzyl, trityl or diphenylmethyl groups organo-silyl groups, e.g. t-butyl-diphenylsilyl or trimethylsilyl groups, or acyl groups, eg, acetyl or benzoyl groups.

Groups or atoms for $A^1$ capable of being split off to leave a positive charge include halogen atoms, alkoxy groups (eg, methoxy), hydroxy groups, hydroxy groups esterified by strong organic acids such as benzenesulphonic acid, methanesulphonic acid or p-toluenesulphonic acid or activated hydroxy groups, eg benzyloxy, trityloxy, diphenylmethoxy, t-butyl-diphenylsilyloxy or trimethylsilyloxy groups or acyloxy, eg acetoxy or benzoyloxy, groups.

The cyclisation reaction is conveniently carried out in the presence of an acid or base, for example a Lewis acid, a carboxylic acid, strong mineral acid or an organic or inorganic base. Suitable acids include for example hydrogen halide acids, acetic acid, trifluoroacetic acid, or cationic exchange resins in the $H^+$-form. Suitable bases include for example alkali or alkaline earth metal hydroxides, carbonates or bicarbonates.

The reaction is suitably carried out in a solvent. Solvents may for example be chosen, depending on the reactants and conditions employed, from halogenated hydrocarbons, aromatic hydrocarbons such as toluene or xylene, alcohols, ethers or ketones such as acetone and carboxylic acids such as acetic acid, in the presence or absence of water.

The cyclisation may be effected at room temperature or at elevated temperatures.

Compounds of formula (IIa-d) and (IIIa-d), where not known in the art, may be prepared by methods analogous to those known in the art.

For example, compounds of formula (IIa-d) and (IIIa-d) wherein $A^2$ represents an activated hydroxy group may be prepared from the corresponding compound of formula (IIa-d) and (IIIa-d) wherein $A^2$ represents a hydroxy group by conventional methods.

Conditions suitable for the preparation and cyclization of compounds of formula (IIa-d) or (IIIa-d) have been described in the syntheses of 8-oxa-6-thiabicyclo[3.2.1]octane derivatives (M. Akagi et al., Chem.Pharm.Bull.(1963) 11, 58-61; D. Horton and J.D. Wander, Eds., "The carbohydrates", Vo.1B, 2nd Ed. (1980) Academic Press), a 2,9-dioxabicyclo[3.3.1]nonane derivative (A.A. Kandil and K.N. Slessor, J.Org.Chem. (1985) 50, 5649-5655; R.H. Schlessinger et al., J.Am.Chem.Soc. (1985) 107, 1777-1778; R.E. Ireland et al., J.Am.Chem.Soc. (1981) 103, 3205-3207; S.J. Angyal and T.Q. Tran, Can.J.Chem. (1981) 59, 379-383; C.Nuekom et al., J.Am.Chem.Soc. (1986) 108, 5559-5568), a 3,9-dioxabicyclo[3.3.1]nonane derivative (E-F. Fuchs and J. Lehmann, Chem.Ber. (1975) 108, 2254-2260), 9-oxa-2-azabicyclo[3.3.1]nonane derivatives (Y. Nakahara et al., Tetrahedron (1986) 42, 6465), 6-oxa-8-azabicyclo[3.2.1]octane and 6,8-diazabicyclo[3.2.1]octane derivatives (H. Paulsen and K. Todt, Adv.Carbohydr.Chem (1968) 23, 115-232 and references therein).

Compounds of formula (IIIa) wherein the groups $OR^5$ and $EA^2$ represent hydroxy groups may conveniently be prepared by acid cleavage of a corresponding compound containing the partial structure (IV)

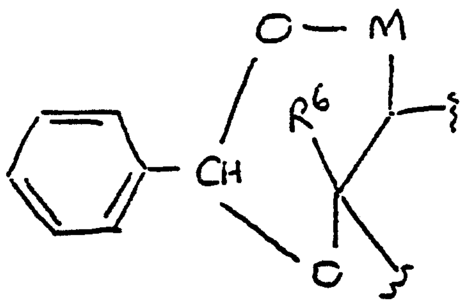

(IV)

(wherein $R^6$ and M are as defined above or represent protected forms thereof) which compounds are known in the literature or may readily be derived therefrom (see eg, Carbohydr. Res. 1980, 86, 158-160).

Compounds of formula (IIa-d) and (IIIa-d) wherein $OR^5$ represents a hydroxy group and $R^6$ represents an optionally substituted hydrocarbyl group may for example be prepared by a Grignard reaction on a corresponding compound containing the partial structure (IVa)

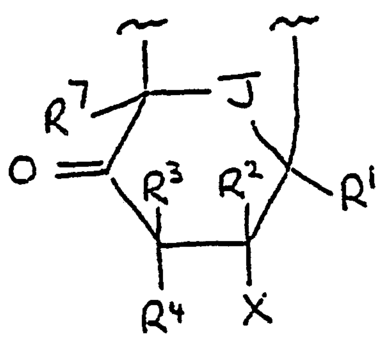

(IVa)

(wherein $R^1$, $R^2$, $R^3$, $R^4$, $R^7$, J and X are as defined above or represent protected forms thereof) using a Grignard reagent of formula (V)

$R''MgHal$ (V)

(wherein $R''$ is an optionally substituted hydrocarbyl group or a protected form thereof and Hal is a halogen atom) and optionally splitting off or converting any protective groups present.

The Grignard reaction normally results in a mixture of the epimers, which may conveniently be resolved into the desired compound of formula (IIa-d) or (IIIa-d) either before or after the cyclisation.

Compounds with the partial structure (IVa) may conveniently be prepared by oxidising a corresponding hydroxy compound, for example using a "Swern" oxidation with dimethyl sulphoxide/oxalyl chloride as the oxidising agent (see A.J. Mancuso and D. Swern, Synthesis (1981) 179).

A compound of formula (IIe)

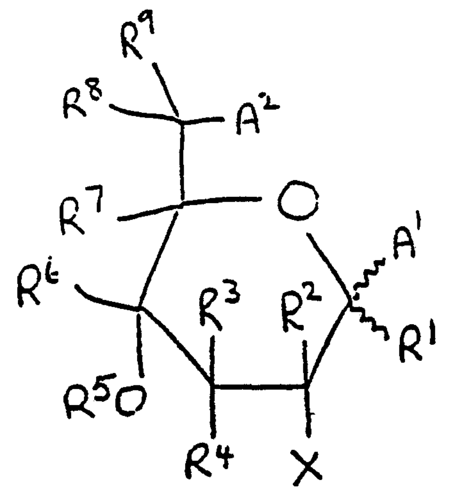

(IIe)

(wherein X, $R^1$, $R^2$, $R^3$, $R^4$, $R^5$ and $R^6$ are as defined above or represent protected forms thereof and wherein $A^2$ represents a hydroxy group, $R^7$ represents an optionally substituted hydrocarbyl group and $R^8$ and $R^9$ represent hydrogen atoms) can be obtained from a compound of formula (I) (wherein -M-E-Z- represents $-CH_2O-$ and $R^1$ and $R^7$ represent hydrogen atoms) by a sequence of six reactions. Conversion of this latter compound of formula (I) to a ketone of formula (VI)

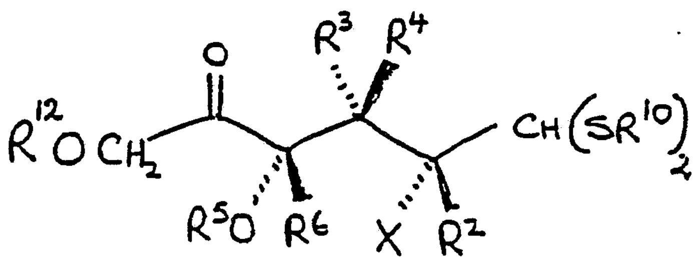

(VI)

[wherein $R^{10}$ represents a hydrocarbyl group (eg, together the two $R^{10}$ groups may represent an ethane-1,2-diyl group), $R^{12}$ represents a hydroxyl protecting group (eg, conveniently a bulky silyl group such as tert-butyldiphenylsilyl) and X, $R^2$, $R^3$, $R^4$, $R^5$ and $R^6$ are as defined above]
may be effected by reaction in turn with: (i) an appropriate thiol or dithiol in the presence of an acidic catalyst, eg, ethane-1,2-dithiol in the presence of boron trifluoride diethyl etherate; (ii) a reagent capable of selectively etherifying a primary hydroxy group such as a trisubstituted-silyl halide in the presence of a base and/or catalyst, conveniently tert-butylchloridiphenylsilane and imidazole in dimethylformamide solution; and (iii) an oxidising agent capable of converting the secondary hydroxyl group to a ketonic group, conveniently using the "Swern" oxidation procedure mentioned above.

Addition of an organometallic reagent, especially a Grignard reagent of formula (V) to a ketone of formula (VI) [Step (iv)] may result in a mixture of tertiary alcohols in which the isomer of formula (VIa)

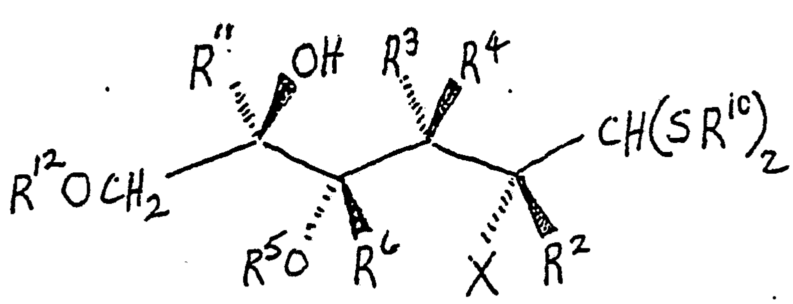

(VIa)

(wherein $R''$, X, $R^2$, $R^3$, $R^4$, $R^5$, $R^6$, $R^{10}$ and $R^{12}$, are as defined above)
may be the predominant product. The desired compound of formula (IIe) may be obtained from this latter acyclic compound by (v) sequential or concommitant removal of its ether protecting group and (vi) methanolytic or hydrolytic cleavage of the dithioacetal moiety using conventional techniques; for example

using tetrabutylammonium fluoride in aqueous acetonitrite to hydrolyze a tert-butyldiphenylsilyl ether and N-bromosuccinimide in methanol to cleave the dithioacetal moiety.

In a second example, a compound of formula (IIe) wherein $A^2$ represents a hydroxy group, $R^8$ represents a hydrogen atom and $R^9$ represents an optionally substituted hydrocarbyl group can be obtained from a related compound of formula (IIe) (wherein $A^2$ represents a hydroxy group and $R^8$ and $R^9$ represent hydrogen atoms) by "Swern" oxidation (see above) to give an aldehyde of formula (IVb)

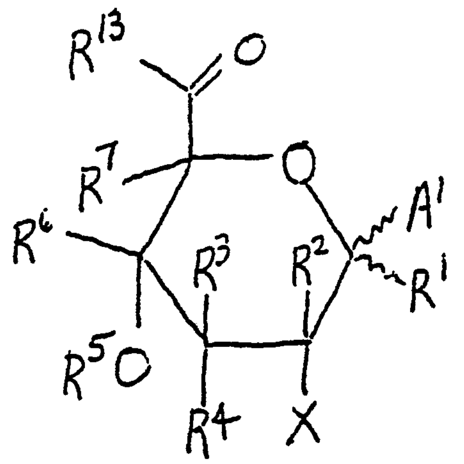

(IVb)

(wherein $R^{13}$ represents a hydrogen atom and $A^1$, X, $R^1$, $R^2$, $R^3$, $R^4$, $R^5$, $R^6$ and $R^7$ are defined as above) which may then be treated with an organometallic reagent, such as a Grignard reagent of formula (V). The predominant product from this reaction sequence normally is the isomer which on cyclization, yields the 7-exo-substituted 6,8-dioxabicylo[3.2.1]octane (an exo-substituent having a syn relationship of the $C_7$ substituent and the 8-oxygen atom). The alternative isomer, if present, may be removed by conventional techniques.

In a third example, a compound of formula (IIe) wherein $A^2$ represents a hydroxy group, $R^8$ represents an optionally substituted hydrocarbyl group, and $R^9$ represents a hydrogen atom can be obtained from a related compound of formula (IIe) (wherein $A^2$ represents a hydroxy group, $R^8$ represents a hydrogen atom and $R^9$ represents an optionally substituted hydrocarbyl group) or from a mixture containing this compound and its epimer, described in the preceding example. Thus oxidation of the latter compound of formula (IIe) (eg, "Swern" oxidation) may result in a ketone of formula (IVb) wherein $R^{13}$ represents an optionally substituted hydrocarbyl group. Stereoselective reduction of this ketone, eg with sodium borohydride, could produce mixture of epimers whereby the predominant product is normally the isomer which on cyclization, yields the 7-endo-substituted 6,8-dioxabicyclo[3.2.1]octane. The alternative isomer, if present, may be separated by chromatography.

In a fourth example, a compound of formula (IIe) wherein $A^2$ represents a hydroxy group and $R^8$ and $R^9$ independently represent optionally substituted hydrocarbyl groups can be obtained from a related compound of formula (IVb) (wherein $R^{13}$ represents an optionally substituted hydrocarbyl group) by treatment with an organometallic reagent, eg a Grignard reagent of formula (V).

Similarly, a compound of formula (IIh)

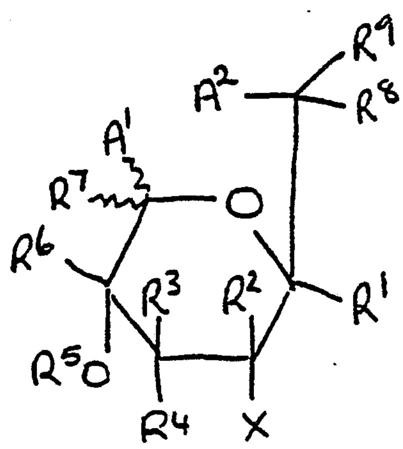

(IIh)

(wherein $R^1$, $R^2$, $R^3$, $R^4$, $R^5$, $R^6$, $R^7$, $R^8$, $R^9$ and X are as herein defined) and wherein $A^2$ represents a hydroxy group, $R^1$ represents an optionally substituted hydrocarbyl group and $R^7$, $R^8$ and $R^9$ represent hydrogen atoms can be obtained from a related compound of formula (I) (wherein -M-E-Z- represent $-OCH_2-$ and $R^1$ and $R^7$ represent hydrogen atoms) by a sequence of six reactions. Conversion of this latter compound of formula (I) to a ketone of formula (VIb)

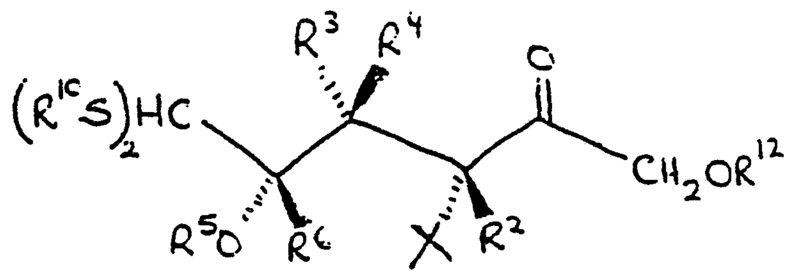

(VIb)

[wherein $R^{10}$ represents a hydrocarbyl group (eg taken together the two $R^{10}$ groups may represent an ethane-1,2-diyl group), $R^{12}$ represents a hydroxyl protecting group (eg conveniently a bulky silyl group such as tertbutyldiphenylsilyl) and X, $R^2$, $R^3$, $R^4$, $R^5$ and $R^6$ are as defined above]

may be effected by reaction in turn with: (i) an appropriate thiol or dithiol in the presence of an acidic catalyst, eg ethane-1,2-dithiol in the presence of boron trifluoride diethyl etherate; (ii) a reagent capable of selectively etherifying a primary hydroxy group such as a trisubstituted-silyl halide in the presence of a base and/or catalyst, conveniently tertbutylchlorodiphenylsilane and imidazole in dimethylformamide solution; and (iii) an oxidising agent capable of converting the secondary hydroxyl group to a ketonic group, conveniently using the "Swern" oxidation procedure mentioned above.

Addition of an organometallic reagent, especially a Grignard reagent of formula (V) [step (iv)] to a ketone of formula (VIb) may result in a mixture of tertiary alcohols in which the isomer of formula (VIc)

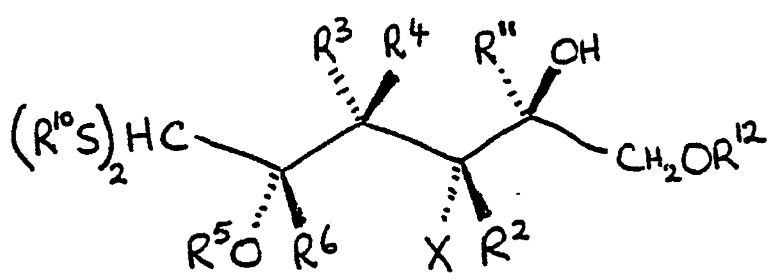

(VIc)

(wherein $R^{11}$, X, $R^2$, $R^3$, $R^4$, $R^5$, $R^6$, $R^{10}$ and $R^{12}$ are as defined above) may be the predominant product. The desired compound of formula (IIh) may be obtained from this latter acyclic compound by (v) sequential or concommitant removal of its ether protecting group and (vi)

methanolytic or hydrolytic cleavage of the dithioacetal moiety using conventional techniques; for example using tetrabutylammonium fluoride in aqueous acetonitrile to hydrolyze a tert-butylidphenylsilyl ether and N-bromosuccinimide in methanol to cleave the dithioacetal moiety.

In a second example a compound of formula (IIh) wherein $A^2$ represents a hydroxy group, $R^8$ represents a hydrogen atom and $R^9$ represents an optionally substituted hydrocarbyl group can be obtained from a related compound of formula (IIh) (wherein $A^2$ represents a hydroxy group and $R^8$ and $R^9$ represent hydrogen atoms) by "Swern"oxidation (see above) to give an aldehyde of formula (IVc).

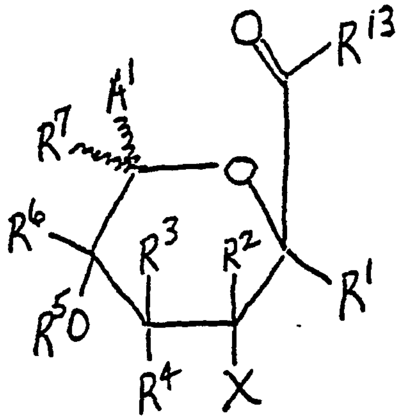

(IVc)

(wherein $R^{13}$ represents a hydrogen atom and $A^1$, X, $R^1$, $R^2$, $R^3$, $R^4$, $R^5$, $R^6$ and $R^7$ are defined as above) which may then be treated with an organometallic reagent, such as a Grignard reagent of formula (V). The predominant product from this reaction sequence normally is the isomer which on cyclization, yields the 7-exo-substituted 6,8-dioxabicyclo[3.2.1]octane (an exo-substituted having a syn relationship of the C-7 substituent and the 8-oxygen atom). The alternative isomer, if present, may be removed by conventional techniques.

In a third example, a compound of formula (IIh) wherein $A^2$ represents a hydroxy group, $R^8$ represents an optionally substituted hydrocarbyl group and $R^9$ represents a hydrogen atom can be obtained from a related compound of formula (IIh) (wherein $A^2$ represents a hydroxy group, $R^8$ represents a hydrogen atom and $R^9$ represents an optionally substituted hydrocarbyl group) or from a mixture containing this compound and its epimer, described in the preceding example. Thus oxidation of the latter compound of formula (IIh) (eg "Swern" oxidation) may result in a ketone of formula (IVc) wherein $R^{13}$ represents an optionally substituted hydrocarbyl group. Stereoselective reduction of this ketone, eg with sodium borohydride, could produce a mixture of epimers whereby the predominant product is normally the isomer which on cyclization, yields the 7-endo-substituted 6,8-dioxabicyclo[3.2.1]octane. The alternative isomer, if present, may be separated by chromatography.

In a fourth example, a compound of formula (IIh) wherein $A^2$ represents a hydroxy group and $R^8$ and $R^9$ independently represent optionally substituted hydrocarbyl groups can be obtained from a related compound of formula (IVc) (wherein $R^{13}$ represents an optionally substituted hydrocarbyl group) by treatment with an organometallic reagent, eg a Grignard reagent of formula (V).

Compounds of formula (IIe) or (IIh) wherein -M-E-Z- represents $-CH_2O-$ or $-OCH_2-$ respectively and $R^1$ and $R^7$ are hydrogen atoms, suitable for introduction of optionally substituted hydrocarbyl groups at $R^8$ and/or $R^9$, may be obtained from compounds of formula (I) (wherein $R^8$ and $R^9$ are hydrogen atoms) by cleavage of the acetal (or ketal) moiety, eg by alcoholysis in the presence of an acid catalyst.

(b) for the preparation of compounds wherein X, $R^1$, $R^2$, $R^3$, $R^4$, $R^5$, and $R^6$, $R^7$, J, M, E and Z are defined as above:

reacting a compound of formula (VII)

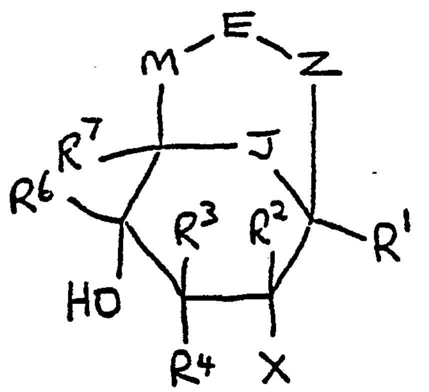

(VII)

or a corresponding alkali metal alkoxide
(wherein X, $R^1$, $R^2$, $R^3$, $R^4$, $R^6$, $R^7$, J, M, E and Z are as defined above or represent protected forms thereof) or an enantiomer thereof,
with a compound serving to introduce the group $R^5$ in place of the hydroxy hydrogen atom and optionally subsequently splitting off or converting any protective groups present to produce the desired compound.

The introduction of the group $R^5$ may conveniently be carried out using a reagent of formula (VIII)
$R^5A^1$ (VIII)
(wherein $R^5$ and $A^1$ are defined as above).

The group $A^1$ may for example include a halogen atom, a p-toluene-sulphonyloxy, trifluoromethanesulphonyloxy or diazonium group.

The reaction may be carried out in an organic solvent such as tetrahydrofuran, dimethylsulphoxide or dimethylformamide.

The reaction may be performed in the presence of a base such as, for example, barium oxide, potassium hydroxide, sodium hydroxide or silver oxide. A quaternary ammonium salt catalyst such as tetrabutylammonium bromide or iodine may be employed, under suitable conditions as reported by Czernecki et al. (Tetrahedron Lett. (1976) 3535-6).

Compounds of formula (VII), where not known in the art, may be prepared by methods analogous to those known in the art, or may be prepared from an analogous compound of formula (VII) wherein $R^5$ for example is a benzyl group by debenzylation (eg, by catalytic hydrogenolysis).

Compounds of formula (VII′), which corresponds to formula (VII),
wherein -M-E-Z- represents $-OCH_2-$, X represents an O-linked organic group and $R^1$, $R^2$, $R^3$, $R^4$, $R^6$ and $R^7$ represent hydrogen atoms,
may be prepared by reaction of a suitably protected form of 1,6-anhydro-2,3-0-benzylidene-$\beta$-L-allopyranose (IIf)

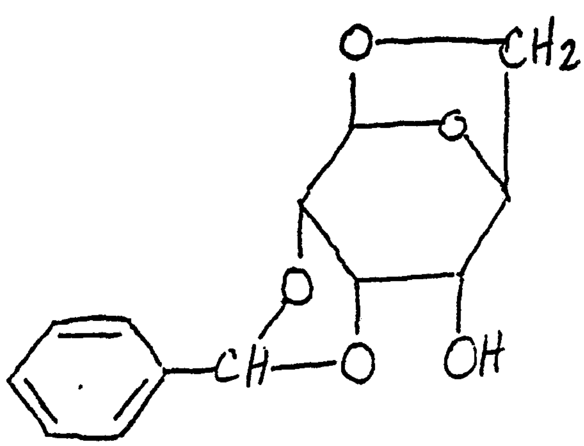

(IIf)

with for example N-bromosuccinimide, and subsequently debrominating a protected form of the compound of formula (IIg)

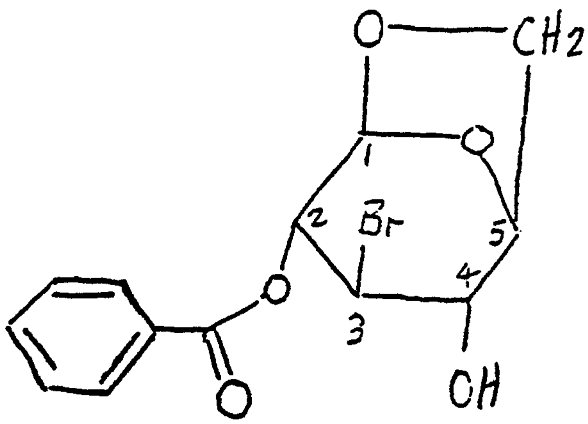

(IIg)

formed and optionally replacing the 2-0-substituent, by conventional methods. The compound of formula (IIf) may be readily prepared from L-gulose (Methods Carbohydr. Chem. (1963), 2, 65) by heating L-gulose with water in the presence of a strong acid resin to form 1,6-anhydro- β -L-gulopyranose, which is then reacted with benzaldehyde dimethyl acetal under acidic conditions and the remaining free 4-hydroxy group is inverted via oxidation to a ketone intermediate and subsequently reduced.

Compounds of formula (VII''), which corresponds to formula (VII), wherein -M-E-Z- is $-CH_2O-$, X is an O-linked organic group and $R^1$, $R^2$, $R^3$, $R^4$, $R^6$ and $R^7$ represent hydrogen atoms,
may be prepared by a similar process starting from D-gulose.

Alkali metal alkoxides corresponding to compounds of formula (VII) may be prepared by conventional methods such as reaction of an alcohol of formula (VII) with an alkali metal hydride (eg, sodium hydride) in a suitable solvent.

Enantiomers of compounds of formula VII' and VII'' , as defined above, can also be obtained in this way starting from D- or L-gulose respectively (see for example E.P. 0229034A).

(c) for the preparation of compounds wherein X represents an O-linked organic group and/or wherein $R^3$ and/or $R^4$ represent an optionally substituted O-linked hydrocarbyl group and $R^1$, $R^2$, $R^5$, $R^6$, $R^7$, J, M, E and Z and the remainder of X, $R^3$ and $R^4$ are as defined above:
reacting a corresponding compound of formula (I) wherein X and/or $R^3$ and/or $R^4$ represent a hydroxy group, and the remainder of X, $R^1$, $R^2$, $R^3$, $R^4$, $R^5$, $R^6$, $R^7$, J, M, E and Z are as defined above or represent protected forms thereof,
or an enantiomer thereof,
or a corresponding alkali metal alkoxide thereof,
with a compound serving to introduce respectively an O-linked organic group at X and/or an optionally substituted O-linked hydrocarbyl group at $R^3$ and/or $R^4$ and optionally subsequently splitting off or converting any protective groups present, to produce the desired compound.

Reagents serving to introduce the O-linked groups at X and/or an optionally substituted hydrocarbyl group at $R^3$ and/or $R^4$ include, for example, organic halides (eg, alkyl or aralkyl halides such as methyl iodide, benzyl bromide or ethyl iodide). Where an organic halide reagent is used, the reaction may be carried out under conditions analogous to those described for reaction (b) above.

The compound of formula (I) used as starting material may for example be prepared by methods (d), (g), (h) or (n)

(d) for the preparation of compounds wherein X represents a hydroxy group and $R^2$ represents a hydrogen atom:
reducing a compound of formula (I) wherein X together with the group $R^2$ represents a ketonic oxygen atom, and $R^1$, $R^3$, $R^4$, $R^5$, $R^6$, $R^7$, J, M, E and Z are as defined above or represent protected forms thereof,
or an enantiomer thereof,
and optionally subsequently splitting off or converting any protective groups present, to produce the desired compound.

Reducing agents which may conveniently be employed include hydride reducing agents such as lithium aluminium hydride, sodium borohydride, lithium triethylborohydride, lithium tri-sec-butylborohydride or

diisobutyaluminium hydride (advantageously in the presence of lithium bromide). The reduction product may contain a pair of epimers, in which case they may be separated by conventional techniques such as fractional crystallisation or chromatography. By selecting suitable reaction conditions the yield of the desired isomer may be maximised.

The reaction may suitably be carried out in an aqueous or non-aqueous solvent, (eg, an alcohol, water or mixture); the choice of solvent depends on the reactants present, for example when lithium aluminium hydride is used as reducing agent, the solvent will be non-aqueous.

(e) for the preparation of compounds wherein X together with the group $R^2$ represents a ketonic oxygen atom and the groups $R^3$ and $R^4$ represent hydrogen atoms and $R^5$ is defined as above:
reaction of an enone of formula (IX)

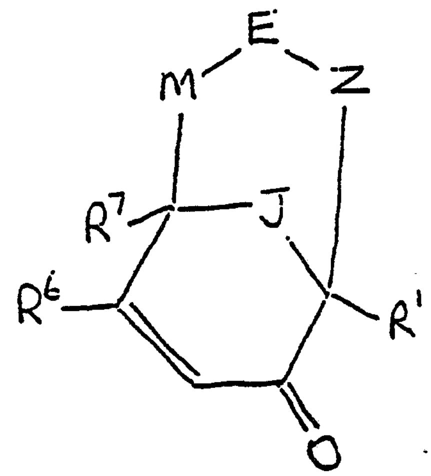

(IX)

with an alcohol of formula (X)
$R^5OH$ (X)
(wherein $R^5$ is defined as above)

The reaction is a Michael addition reaction which may conveniently be carried out in a conventional manner in the presence of an acid or base catalyst. Suitable acidic catalysts include protic acids (eg, sulphuric, hydrochloric, p-toluenesulphonic, trifluoromethanesulphonic or preferably trifluoroacetic acids) or Lewis acids (eg, $BF_3.(C_2H_5)_2O$). Suitable base catalysts include alkali metal alkoxides prepared from alkali metals or hydrides (eg, from sodium metal, sodium hydride or potassium hydride) by reaction with an alcohol, tertiary amines (eg, triethylamine) or solid bases (eg, alumina).

Where a base catalyst is employed, the alcohol of formula (X) may first be diluted with water to increase the reaction rate, as reported in Shafizadeh et al. (Carbohydr. Res. (1979) 71, 169-191).

The reaction conditions may be controlled to minimise unwanted side reactions. For example, under basic conditions the enone starting material or its derivatives may undergo base-catalysed oligomerisation.

Under acidic conditions the product may rapidly react further with the alcohol of formula (X) to form a corresponding ketal of formula (I) wherein both the X and $R^2$ groups are the same as the moiety $R^5O$. The reaction may be partially controlled to lead to a mixture of said ketal product and alcohols wherein X or $R^2$ is a hydroxy group. Such control may suitably be effected by the addition of a reducing agent such as sodium borohydride to the reaction mixture while the keto product predominates, so promoting the reduction by (d) above in preference to the ketal formation.

In practice a mixture of the isomeric alcohols and the ketal may result.

Alternatively, the compound of formula (I) initially formed, wherein X together with the group $R^2$ represents a ketonic oxygen atom and the groups $R^3$ and $R^4$ represent hydrogen atoms, solated and subsequently reacted under acidic conditions with a different alcohol, of formula (XI)
$R''OH$ (XI)
(wherein $R''$ represents an optionally substituted hydrocarbyl group),
to give a different ketal of formula (I) wherein X and $R^2$, which are the same, each represents an optionally substituted 0-linked hydrocarbyl group and the groups $R^3$ and $R^4$ represent hydrogen atoms.

The keto compound formed in the principal reaction may, if desired, be subsequently treated with a Grignard reagent to introduce a hydrocarbyl group at that position. Normally a mixture of isomers will be

produced that may be separated by chromatography. For example, methylmagnesium bromide may be used as a reagent under standard Grignard conditions to produce a mixture of componds of formula (I) in which X is a hydroxy group and $R^2$ is a methyl group, and in which X is a methyl group and $R^2$ is a hydroxy group.

Enones of formula (IX) may be obtained by known techniques; example which may be mentioned include 9-oxabicyclo[3.3.1]nona-3,6-dien-2-one (P.Buchs and C. Ganter, Helve. Chim. Acta (1980) 63, 970-986), the racemic and (+)-forms of bicyclo[3.2.1]oct-3-en-2-one (H.L. Goering et al., J.Am.Chem, Soc. (1961) 83, 1391-1397), bicyclo [3.3.1]non-3-en-2-one (J.P. Schaefer et al., J.Org.Chem. (1967) 32, 1372-1378), various 1- and/or 3- and/or 4-substituted 8-alkyl-2,9-dioxabicyclo[3.3.1]non-7-en-6-ones (K. Mori and H. Kisida, Tetrahedron (1986) 42, 5281-5290; R.E. Ireland et al., J.Am.Chem.Soc (1981) 103, 3205-3207; P. De Shong et al., J.Am.Chem.Soc. (1985) 107, 5219-5224, Tetrahedron Lett. (1986) 27, 2091-2094 and (1982) 23, 2243-2246; S.F. Martin et al., J.org.chem. (1984) 49, 2513), R.K. Boeckmann et al., J.Am.Chem.Soc. (1986) 108 5549-5559; R.H. Schlessinger et al., J.Am.Chem.Soc (1985) 107, 1777-1778; F.E. Ziegler and R.T. Wester, Tetrahedron Lett. (1984) 25, 617-620), various N- and/or 1- and/or 6- and/or 7-substituted 8-azabicyclo[3.2.1]oct-3-en-2-ones (A.R. Katritsky et al., J.Chem.Soc., Perkn Trans. 1 (1979) 1525-1535, J.Chem. Res., Synop. (1981) 208-209, and papers in the same series; M. Hamaguchi et al., J.Chem.Soc., Chem.Commum. (1982) 262-263) and levoglucosenone (Halpern et al. J.Org.Chem. (1973) 38, 204-209 or US Patent No. 3926947).

f) for the preparation of compounds wherein X, $R^1$, $R^2$, $R^5$, $R^6$, $R^7$, J, M, E and Z are as defined above and $R^3$ and $R^4$ represent hydrogen atoms:
reacting a compound of formula (XII)

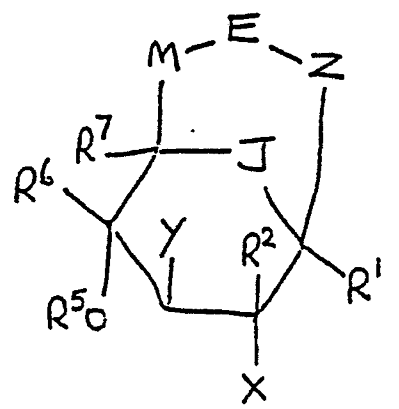

(XII)

(in which X, $R^1$, $R^2$, $R^5$, $R^6$, $R^7$, J, M, E and Z are defined as above, or represent protected forms thereof, and Y represents the group OC(S)Q; wherein Q may be, for example, thiomethyl, phenoxy, imidazol-1-yl or other suitable moiety or represents a protected form thereof),
or an enantiomer thereof,
with trialkyltin hydride, (eg, tributyltin hydride)
and optionally subsequently splitting off or converting any protective groups to produce the desired compounds.

The reaction may conveniently be carried out under reflux in an organic solvent such as toluene, and can be catalysed by a radical initiator or by irradiation.

The compound of formula (XII) may for example be prepared from a corresponding compound of formula (XII) wherein Y represents a hydroxy group and X, $R^1$, $R^2$, $R^5$, $R^6$, $R^7$, M, E and Z are defined as above using known methods described for example in J.Am.Chem.Soc. (1983) 105, 4059-4065. Such a compound of formula (XII) may for example, be prepared as described in reaction (g), (h) or (j) below.

(g) for the preparation of compounds wherein $R^1$, $R^5$, $R^7$, J, M, E and Z are as defined above, $R^2$, $R^4$ and $R^6$ represent hydrogen atoms, $R^3$ represents a hydroxy group and X is the same as the group $OR^5$: diether formation involving the 1- and 3-oxygen atoms of the 1,2,3-triol (XIII)

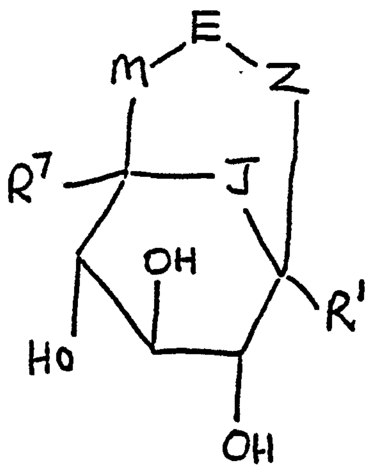

(XIII)

Compounds of formula (XIII) might for example be anhydrosugars or their derivatives.

Suitable reaction conditions are reported in Can.J.Chem. (1982) 60, 299-303. For example, the reaction may be effected in the presence of a base such as barium oxide, using benzyl bromide as a reagent, in a solvent such as dimethylformamide.

(h) for the preparation of compounds wherein $R^3$ represents a hydroxy group, X represents an O-linked organic group, an optionally substituted hydrocarbyl group, a halogen atom, or a hydroxy, amino, cyano or azido group, $R^2$ and $R^4$ represent hydrogen atoms, and $R^1$, $R^5$, $R^6$, $R^7$, M, E, Z and J are as defined above: reacting an epoxide of formula (XIV)

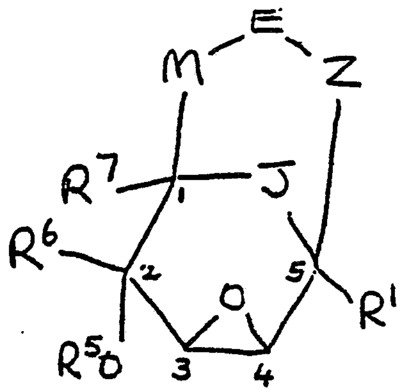

(XIV)

wherein $R^1$, $R^5$, $R^6$, $R^7$, J, M, E and Z are as defined for formula (I) or represent protected forms thereof) or an enantiomer thereof,
with a compound serving to selectively introduce the group X as defined above at the 4-position,
and optionally subsequently splitting off or converting any protective groups present to produce the desired compound.

Where X represents an O-linked organic group, the compound serving to introduce said group at the 4-position will preferably be an organic alcohol, eg, methanol.

Where X represents an optionally substituted hydrocarbyl group, the compound serving to introduce said group at the 4-position will preferably be an organometallic derivative of said hydrocarbyl group. Regioselectivity may be achieved by the use of particular known reaction conditions (see, for example, Tetrahedron Lett. (1981) 22, 4315-8 or Tetrahedron Lett. (1983) 24, 5823-6).

Where X represents a halogen atom, the compound serving to introduce said group at the 4-position will preferably be a halide salt, eg, potassium bifluoride, tetrabutylammonium bromide, diethylaluminium chloride or magnesium iodide. Regioselectivity may be achieved by the use of particular known reaction conditions (see, for example, J.Chem.Soc.,Chem.Commun. (1969) 77).

Where X represents a hydroxy group, the compound serving to introduce said group at the 4-position will preferably be a strong base resin or an alkali metal hydroxide, eg, sodium hydroxide or potassium

hydroxide. Regioselectivity may be achieved by the use of particular known reaction conditions (see, for example, J.Chem.Soc.C (1971) 3143-3155).

Where X represents an amino group, the compound serving to introduce said group at the 4-position will preferably be aqueous ammonia. Regioselectivity may be achieved by the use of particular known reaction conditions (see, for example, Carbohydr. Res. (1973) 29, 99-111).

Where X represents a cyano group, the compound serving to introduce said group at the 4-position will preferably be a cyanide compound. Organo-metallic cyanide salts such as diethylaluminium cyanide have been found particularly suitable.

Where X represents an azido group, the compound serving to introduce said group at the 4-position will preferably be sodium or tetraalkyl-ammonium azide. Regioselectivity may be achieved by the use of particular known reaction conditions (see, for example, Tetrahedron Lett. (1975) 1493-4).

The reaction generally may conveniently be carried out in a solvent such as, for example, dry tetrahydrofuran, methanol, ethanol, dioxane, ethylene glycol, water and their mixtures.

Where an organic alcohol is used as reagent, however, this may be present in excess to provide the solvent in addition to the reagent.

The epoxide of formula (XIV) may be prepared by ring-closure of a tosylate compound of formula (XV)

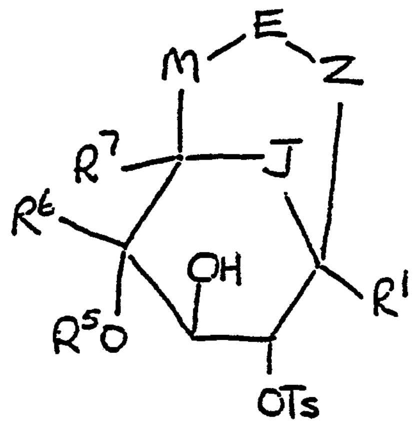

(XV)

(wherein $R^1$, $R^5$, $R^6$, $R^7$, J, M, E and Z are as defined above)
by methodology described in the literature, eg, Collect.Czech.Chem.Commun. (1971) 36, 2216-2225 and Carbohydr. Res. (1972) 25, 11-21.

(i) for the preparation of compounds wherein $R^2$ represents a hydrogen atom and X represents an S-linked organic group or an azido group and $R^1$, $R^3$, $R^4$, $R^5$, $R^6$, $R^7$, M, E, Z and J are as defined above: reacting a compound of formula (XVI)

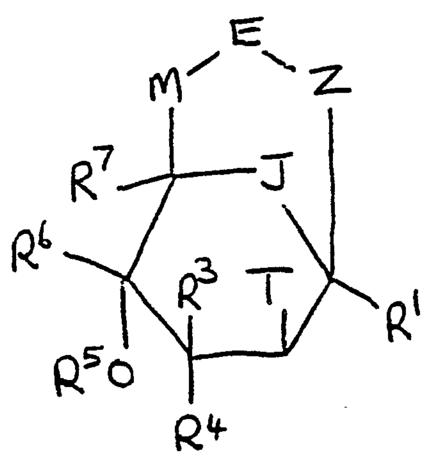

(XVI)

(wherein $R^1$, $R^3$, $R^4$, $R^5$, $R^6$, $R^7$, J, M, E and Z are as defined above or represent protected forms thereof, and T represents a nucleophilically exchangeable group (eg, an organic sulphonyloxy group such as a methanesulphonyloxy, p-toluenesulphonyloxy, trifluoromethanesulphonyloxy or (N-imidazole)sulphonyloxy

group))
with an organic thiol of formula (XVIII)
$R^1SH$ (XVII)
(wherein $R^1$ represents an organic group)
or a reactive derivative thereof,
or with sodium azide,
and optionally subsequently splitting off or converting protective groups to produce the desired compound.

Suitable reactive derivatives of the compound of formula (XX) include, for example alkali metal thioalkoxides (eg, lithium thioalkoxide).

The reaction may conveniently be carried out in an organic solvent such as dimethylsulphoxide or dimethylformamide. The reaction temperature will typically depend on the nature of the starting material. For example, where T represents a trifluoromethanesulphonyloxy group the reaction may suitably be carried out at or below about room temperature; where T represents a p-toluenesulphonyloxy group the reaction may be carried out at an elevated temperature (eg, around 150 °C).

Compounds of formula (XVI) may be prepared by conventional methods from the corresponding alcohols by reaction with the appropriate sulphonyl anhydride or chloride.

(j) for the preparation of compounds whwewin X represents a mono- or di-alkylamino, momo- or di-aralkylamino, trialkylammonium, carboxamido or dicarboximido group and $R^1$, $R^2$, $R^3$, $R^4$, $R^5$, $R^6$, $R^7$, J, M, E and Z are defined as above:
reacting a compound of formula (I), wherein X represents an amino group and $R^1$, $R^2$, $R^3$, $R^4$, $R^5$, $R^6$, $R^7$, J, M, E and Z are defined as above or represent protected forms thereof, with a compound capable of converting said amino group to a mono- or dialkylamino, mono- or di-aralkylamino, trialkylammonium, carboxamido or dicarboximido group,
and optionally subsequently splitting off or converting any protective groups present to produce the desired compound.

Where the group to be formed is a carboxamido or dicarboximido group, the reaction may suitably be carried out using an acyl anhydride or, where not too reactive, an acyl halide, under conventional acylating conditions. For example, to form an acetamido or trifluoroacetamido group, acetic anhydride or trifluoroacetic anhydride in a suitable solvent such as methanol or pyridine may be employed.

Compounds of formula (I) wherein X represents an amino group and $R^1$, $R^2$, $R^3$, $R^4$, $R^5$, $R^6$, $R^7$, J, M, E and Z are as defined above, used as starting materials in the above reaction may be prepared according to processes (h) or (l), for example, either by a separate previous reaction or in situ with the above reaction mixture.

(k) for the preparation of compounds wherein X represents a carboxamido or dicarboxamido group and $R^1$, $R^2$, $R^3$, $R^4$, $R^5$, $R^6$, $R^7$, J, M, E and Z are defined as above:
reacting a compound of formula (I) wherein X represents an azido group and $R^1$, $R^2$, $R^3$, $R^4$, $R^5$, $R^6$, $R^7$, J, M, E and Z are defined as above or represent protected forms thereof,
or an activated derivative thereof,
with an anhydride reagent corresponding to the desired carboxamido or dicarboximido group,
and optionally subsequently splitting off or converting any protective groups to produce the desired compound.

Azide-activating agents which may be employed include for example triphenylphosphine/tetrabutylammonium cyanide (see Tetrahedron Lett. (1984) 25, 4841).

To form a phthalimido group for example, phthalic anhydride may be used in a benzene solvent and the reaction may be carried out at elevated temperature.

The azide starting material may conveniently be obtained using reaction (h) above.

(l) for the preparation of compounds wherein X represents an amino group and $R^1$, $R^2$, $R^3$, $R^4$, $R^5$, $R^6$, $R^7$, J, M, E and Z are defined as above:
reducing a corresponding azido compound of formula (I) wherein X represents an azido group, and $R^1$, $R^2$, $R^3$, $R^4$, $R^5$, $R^6$, $R^7$, J, M, E and Z are as defined above
or represent protected forms thereof,
and optionally subsequently splitting off or converting any protective groups to produce the desired

compound.

The reduction may conveniently be carried out using lithium aluminium hydride in an organic solvent such as diethyl ether.

The azido compound of formula (I) used as starting material may be prepared for example using processes (h) or (i) above.

(m) for the preparation of compounds wherein X together with the group $R^2$ represents a group of formula $-CH_2O-$ and $R^1$, $R^3$, $R^4$, $R^5$, $R^6$, $R^7$, J, M, E and Z are defined as above:
reacting a corresponding compound of formula (I) wherein X together with a group $R^2$ represents a ketonic oxygen atom and $R^1$, $R^3$, $R^4$, $R^5$, $R^6$, $R^7$, J, M, E and Z as above or represent protected forms thereof, with diazomethane,
and optionally subsequently splitting off or converting any protective groups to produce the desired compound.

(n) for the preparation of compounds wherein $R^3$ is a hydrogen atom and $R^4$ represents a hydroxy group, and X, $R^1$, $R^2$, $R^5$, $R^6$, $R^7$, J, M, E and Z are defined as above:
reducing a compound of formula (XVIII)

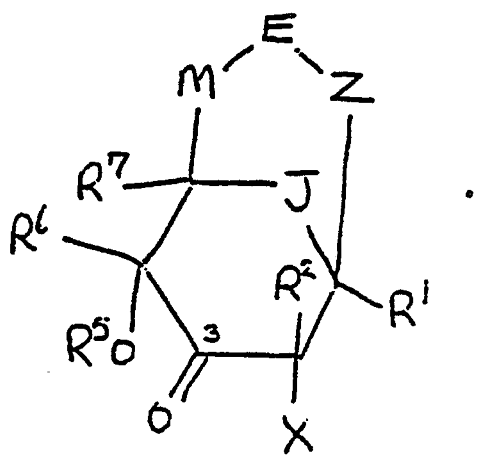

(XVIII)

(wherein X, $R^1$, $R^2$, $R^5$, $R^6$, $R^7$, J, M, E and Z are defined as above or represent protected forms thereof) with a reducing agent capable of selectively forming the desired reduction product with the appropriate overall inversion of stereochemistry at C-3,
and optionally subsequently splitting off or converting any protective groups to produce the desired compound.

As reducing agent sodium borohydride may be particularly mentioned. The reaction may conveniently be carried out in an alcoholic solvent at reduced temperature.

The compound of formula (XVIII) used as starting material may conveniently be prepared by oxidising the corresponding compound of formula (I) wherein $R^3$ is a hydroxy group and $R^4$ is a hydrogen atom, for example using pyridinium dichromate as an oxidant. This later compound of formula (I) may be obtained, for example, using reactions (g) or (h) above.

(o) for the preparation of compounds wherein $R^1$ represents an optionally substituted hydrocarbyl group, Z and J independently represent an O or S atom, an amino or an N-linked organic group and X, $R^2$, $R^3$, $R^4$, $R^5$, $R^6$, $R^7$, M and E are defined as above:
ring-closing a -JH and -ZH protected form of a compound of formula (XIX)

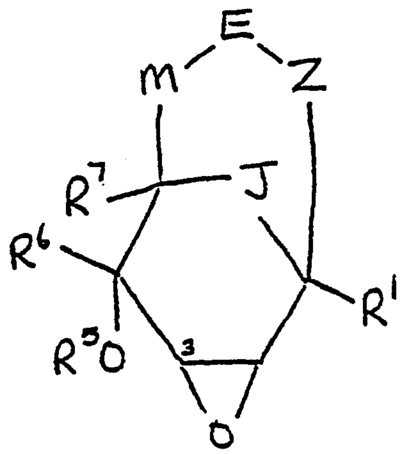

(XIX)

(wherein Z and J independently represent an O or S atom, an amino or an N-linked organic group and X, $R^2$, $R^3$, $R^4$, $R^5$, $R^6$, $R^7$, M and E are defined as above or represent protected forms thereof, and $R''$ represents an optionally substituted hydrocarbyl group)
and optionally subsequently splitting off or converting any protective groups present to produce the desired compound.

It will be appreciated that the stereochemistry of the starting compound of formula (XIX) is important. This compound may for example be prepared by cleavage of a derivative of formula (XX)

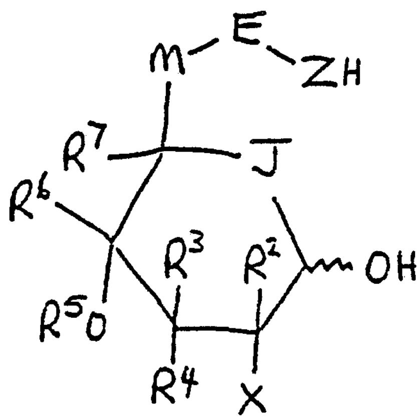

(XX)

(wherein X, $R^2$, $R^3$, $R^4$, $R^5$, $R^6$, $R^7$, M, E, Z and J are as defined for formula (XIX) above or represent protected forms thereof),
either with a Grignard reagent of formula (V) (wherein Hal and $R''$ are defined as above) and subsequent protection of the -JH and -ZH groups and oxidation of the -CH(OH)$R''$ terminal group obtained,
or by reductive cleavage with eg, a borohydride reducing agent, and subsequent protection of the -JH and -ZH groups to form initially a -JH and -ZH protected form of a compound of formula (XXI)

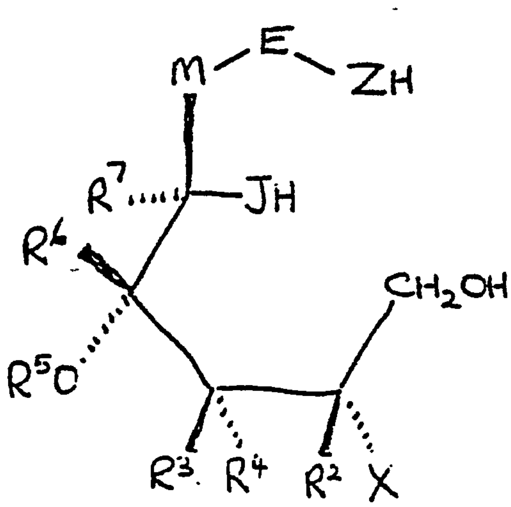

(XXI)

(wherein X, $R^2$, $R^3$, $R^4$, $R^5$, $R^6$, $R^7$, M, E, Z and J are defined as for formula (XIX) above, which is subsequently oxidised to form a -JH and -ZH protected form of an aldehyde compound of formula (XXII)

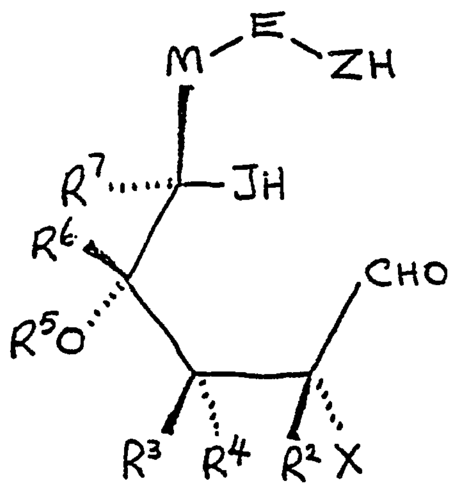

(XXII)

The oxidised compound of formula (XXII) may then preferably be subjected to reaction wih an organometllic reagent or a Grignard reaction with the reagent of formula (V) to introduce the optionally substituted hydrocarbyl group to form a terminal -CH(OH)$R''$ grouping, the alcohol compound formed being then subjected to a second oxidation to produce the desired starting compound of formula (XIX).

The -JH and -ZH groups in the compounds of formula (XIX), (XXI) and (XXII) must be protected during the oxidations, most conveniently by acetal formation, eg with acetone.

The compound formula (XX) may suitably be prepared from a compound of formula (I) wherein $R^1$ is a hydrogen atom by reaction with acetic anhydride under acidic conditions to cleave the C-Z axial bond and generate a diacetate of formula (XXIII)

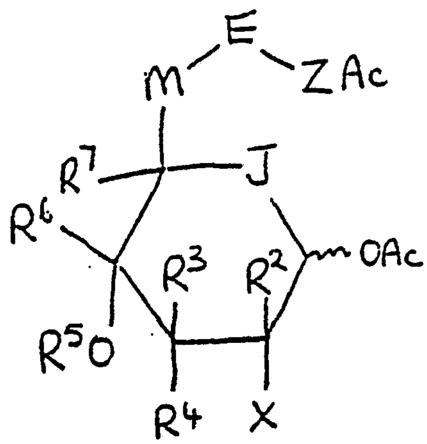

(XXIII)

followed by conventional deacetylation.

(p) for the preparation of compounds wherein $R^7$ ,repsents an optionally substituted hydrocarbyl group, M and J independently represent an O or S atom, an amino or an N-linked organic group and X, $R^1$, $R^2$, $R^3$, $R^4$, $R^5$, $R^6$, Z and E are defined as above:
ring-closing a -JH and -MH protected form of a compound of formula (XXIV)

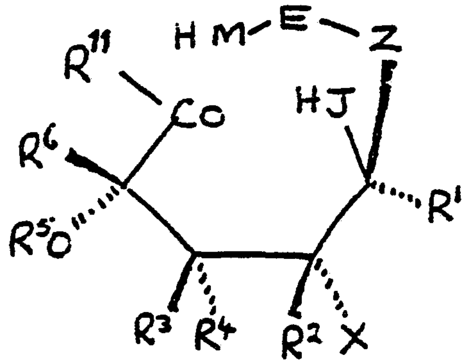

(XXIV)

(wherein M and J independently represent an O or S atom, an amino or an N-linked organic group and X, $R^1$, $R^2$, $R^3$, $R^4$, $R^5$, $R^6$, Z and E are defined as above or represent protected forms thereof,
and $R''$ represents an opticnally substituted hydrocarbyl group)
and optionally subsequently splitting off or converting any protective groups present to produce the desired compound.

It will be appreciated that the stereochemistry of the starting compound of formula (XXIV) is important. This compound may for example be prepared by cleavage of a derivative of formula (XXV)

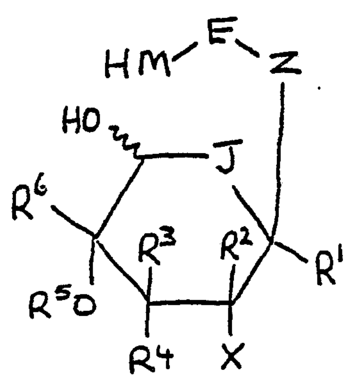

(XXV)

(wherein X, $R^1$, $R^2$, $R^3$, $R^4$, $R^5$, $R^6$, M, E, Z and J are as defined for formula (XXIV) above or represent

protected forms thereof),
either with a Grignard reagent of formula (V) (wherein Hal and R″ are defined as above) and subsequent protection of the -JH and -MH groups and oxidation of the -CH(OH)R″ terminal group obtained,
or by reductive cleavage with eg, a borohydride reducing agent, and subsequent protection of the -JH and -MH groups to form initially a -JH and -MH protected form of a compound of formula (XXVI)

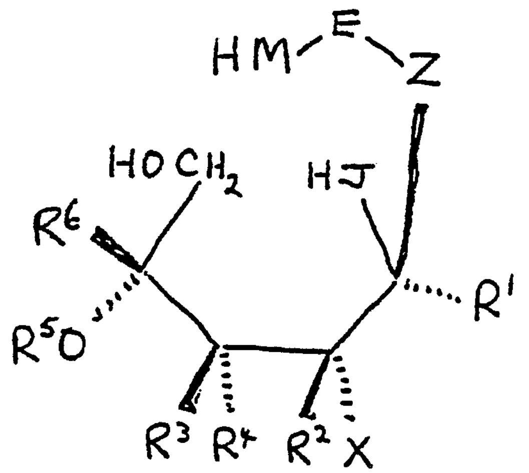

(XXVI)

(wherein X, $R^1$, $R^2$, $R^3$, $R^4$, $R^5$, $R^6$, M, E, Z and J are defined as for formula (XXVI above,
which is subsequently oxidised to form a -JH and -MH protected form of an aldehyde compound of formula (XXVII)

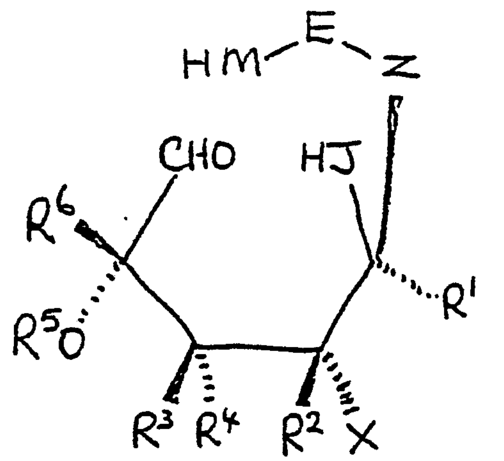

(XXVII)

The oxidised compound of formula (XXVII) may then preferably be subjected to a Grignard reaction with the reagent of formula (V) to introduce the optionally substituted hydrocarbyl group to form a terminal -CH(OH)R″ grouping, the alcohol compound formed being then subjected to a second oxidation to produce the desired starting compound of formula (XXVI).

The -JH and -MH groups in the compounds of formula (XXIV), (XXVI) and (XXVII) must be protected during the oxidations, most conveniently by acetal formation, eg with acetone.

The compound of formula (XXV) may suitably be prepared from a compound of formula (I) wherein $R^7$ is a hydrogen atom by reaction with acetic anhydride under acidic conditions to cleave the C-M axial bond and generate a diacetate of formula (XXVIII)

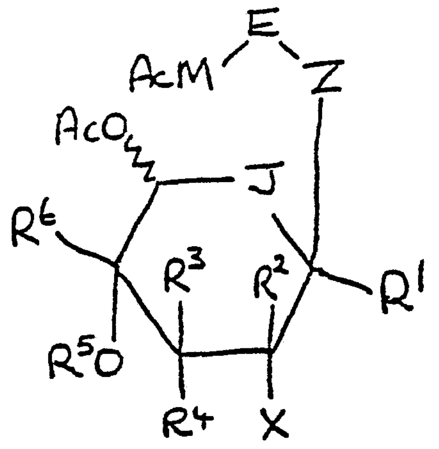

(XXVIII)

followed by conventional deacylation.

(q) for the preparation of compounds of formula (I) wherein X represents a hydroxy group, $R^2$, $R^3$ and $R^4$ represent hydrogen atoms and $R^1$, $R^5$, $R^6$, $R^7$, J, M, E and Z are as defined above: reacting a compound of formula (XXIX)

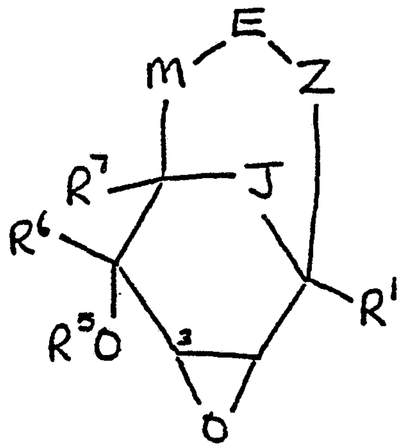

(XXIX)

(wherein $R^1$, $R^5$, $R^6$, $R^7$, J, M, E and Z are as defined above) with a reducing agent (eg, lithium aluminium hydride) capable of specifically or selectively cleaving the epoxide at the 3-position.

(r) for the preparation of compounds of formula (I) wherein X and $R^4$ together represent an oxygen atom, $R^2$ and $R^3$ represent hydrogen atoms and $R^1$, $R^5$, $R^6$, $R^7$, J, M, E and Z are as defined above: reacting an allylic alcohol of formula (XXX)

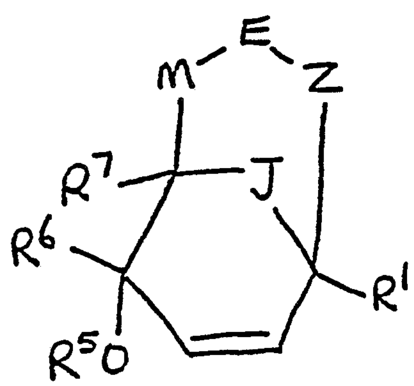

(XXX)

(wherein $R^1$, $R^5$, $R^6$, $R^7$, J, M, E and Z are defined as above)
with a reagent capable of stereoselectively epoxidizing the double bond (eg, 3-chloroperoxybenzoic acid). A compound of formula (XXX) may be obtained from an analogous compound of formula (XXX) in which $R^5$ represents a hydrogen atom, by a method analogous to those described for reaction (b) above. This sequence of introduction of the desired $R^5$ group and epoxidation may be carried out in the reverse order. In either order, the desired isomer can be obtained as the predominant product accompanied in some instances by lesser amounts of the epimeric epoxide from which it can be separated by conventional techniques.

Certain allylic alcohols of formula (XXX) wherein $R^5$ represents a hydrogen atom are known; examples which may be mentioned include a 4,7,7-trimethyl-6-oxabicyclo[3.2.1]oct-2-en-4-ol (D. Mrozinska and K. Piatkowski, Pol. J. Chem. (1980) 54, 693-702), 9-oxabicyclo[3.3.1]nona-3,6-dien-2-ol (R.E. Portman and C. Ganter, Helv. Chim. Acta (1973) 56, 1962-1985), bicyclo[3.2.1]oct-3-en-2-ol (H.S. Goering and S.S. Kramer, J. Org. Chem. (1981) 46, 4605-4608) and a 1,3,4,8-tetrasubstituted 2,9-dioxabicyclo[3.3.1]non-6-en-8-ol (R.H. Schlessinger et al., J. Am. Chem. Soc. (1985) 107, 1777-1778).
The compound of formula (XXX) in which $R^5$ represents a hydrogen atom may be obtained by treatment of an epoxide of formula (XXXI)

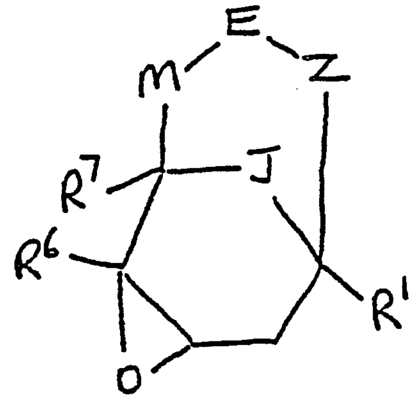

(XXXI)

(wherein $R^1$, $R^6$, $R^7$, J, M, E and Z are as defined above)
with a strong base [eg, n-butyl lithium or preferably lithium bis(trimethylsilyl)amide] as described in the literature (eg, U.P. Singh and R.K. Brown, Can. J. Chem., (1971) 49, 3342).

Epoxides of formula (XXXI) may be obtained by known techniques; examples which may be mentioned include various N-substituted 3-oxa-l0-azabicyclo[4.3.1.$0^{2,4}$]dec-7-enes, 3-10-dioxabicyclo[4.3.1.$0^{2,4}$]dec-7-ene and related derivatives (R.E. Portman and C. Ganter, Helv. Chim. Acta (1973) 56, 1962-1985 and 1991-2007) and 7-acetoxy-3,10-dioxabicyclo[4.3.1.$0^{2,4}$]decane (R.O. Duthaler et al., Helv. Chim. Acta (1972) 55, 1809-1828).

The compound of formula (XXXI) in which $R^1$ and $R^7$ represent hydrogen atoms, J represents oxygen and -M-E-Z- represents $-OCH_2-$ can be prepared as a racemate from acrolein dimer (F. Sweet and R.K. Brown, Can.J.Chem. (1968) 46, 2289). Modification of this latter route may be used to prepare resolved material of the absolute stereochemistry shown for formula (XXXI) (H. Komada, M. Okada, and H. Sumitomo, Macromolecules (1979), 12, 5-9; M. Okada, H. Sumitomo, and A. Sumi, Polym. Bull. (1982) 7, 431-437).

Examples of alkenes which may be stereoselectively epoxidized under standard conditions (see for example F. Sweet and R.K. Brown, Can.J.Chem. (1968) 46, 2289) to give epoxides of formula (XXXI) include variously substituted 8-alkyl-2,9-dioxabicyclo[3.3.1]non-7-enes (P. De Shong et al., Tetrahedron Lett. (1986) 27, 2091-2094; R.H. Schlessinger et al., J.Am.chem.soc. (1985) 107, 1777-1778), a 7,7′-disubstituted 6-oxabicyclo[3.2.1]oct-2-ene (A.A. Vasil'ev et al., Zh. Org. Khim. (1984) 20, 1007-1012), variously substituted 9-oxa- and 9-azabicyclo[3.3.1]non-2-enes (R.E. Portmann and C. Ganter, Helv. Chim. Acta (1973) 56, 1962-1985 and 1991-2007), 1,4,6-trisubstituted 2,9-dioxabicyclo[3.3.1]non-6-enes (R.E. Ireland et al., J. Am. Chem. Soc. (1985) 107, 3271-3278), 3-hydroxymethyl-2,4-dioxabicyclo[3.3.1]non-6-ene and its mesylate (R.B. Woodward, Ger. 2,305,081-2,305,085 (16-8-1973)), variously substituted 2-methyl-6-oxabicyclo[3.2.1]-oct-2-enes (R. Kaiser and D. Lamparsky, Helv. Chim. Acta (1978) 61, 373-382 and 383-386) and 7-trichloromethyl-6-oxabicyclo[3.2.1]oct-3-ene (M.J. Begley et al., J. Chem. Soc., Perkin Trans. 1 (1981) 1112-1118).

(s) for the preparation of compounds of formula (I) wherein $R^1$ represents a hydrogen atom, -M-E-Z- represents an $-OCH_2-$ group and X, $R^2$, $R^3$, $R^4$, $R^5$, $R^6$ and $R^7$ are as defined above:
ring-closing of a vic-dihydroxy compound of formula (XXXII)

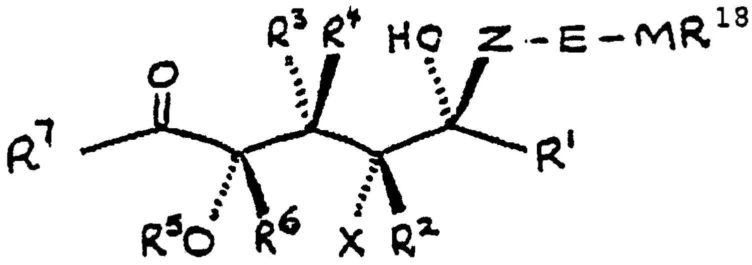

(XXXII)

(wherein X, $R^2$, $R^3$, $R^4$, $R^5$, $R^6$ and $R^7$ are as defined above or represent protected forms thereof)
and optionally subsequently splitting off or converting any protective group present to produce the desired compound.

It will be appreciated that the stereochemistry of the starting compound of formula (XXXII) is important. This compound may be prepared by the following sequence of reactions. Reacting a 6,8-dioxabicyclo-[3.2.1]octane derivative of formula (XXXIII)

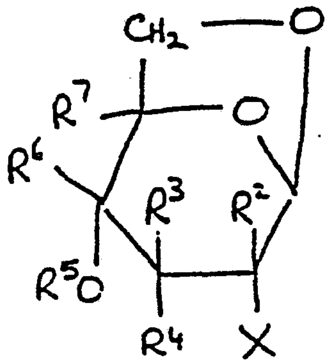

(XXXIII)

(wherein X, $R^2$, $R^3$, $R^4$, $R^5$, $R^6$ and $R^7$ are as defined above or represent protected forms thereof)
with acetic anhydride under acid catalyst (eg, boron trifluoride diethyl etherate), and subsequently removing the O-acetyl groups by conventional deacetylation. Next, reducing the resulting lactol (eg, with sodium borohydride) will form a triol of formula (XXXIV)

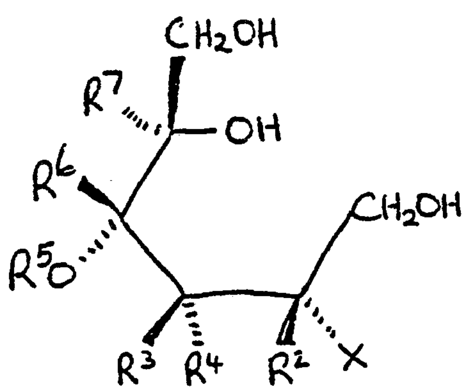

(XXXIV)

(wherein X, $R^2$, $R^3$, $R^4$, $R^5$, $R^6$ and $R^7$ are as defined above)

the vicinal diol moiety of which is protected most conveniently by acetal or ketal formation (eg, with acetone) and the primary alcohol function of which is then oxidised (eg, using a "Swern" oxidation, see above) provides an aldehydic compound of formula (XXXV)

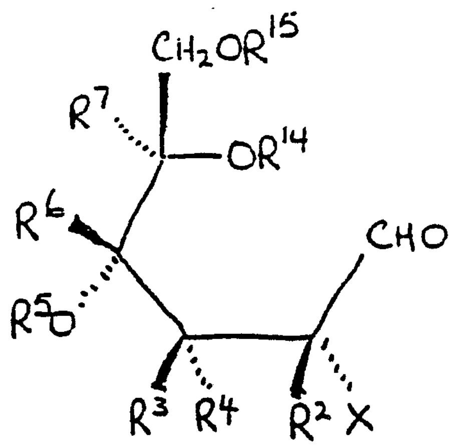

(XXXV)

(wherein X, $R^2$, $R^3$, $R^4$, $R^5$, $R^6$ and $R^7$ are as defined above and $R^{14}$ and $R^{15}$ together may represent an acetal or ketal protecting group thereof).

The addition of the lithium salt of 1,3-dithiane to an aldehyde of formula (XXXV) provides a mixture of epimeric alcohols, from which isomers of formula (XXXVI)

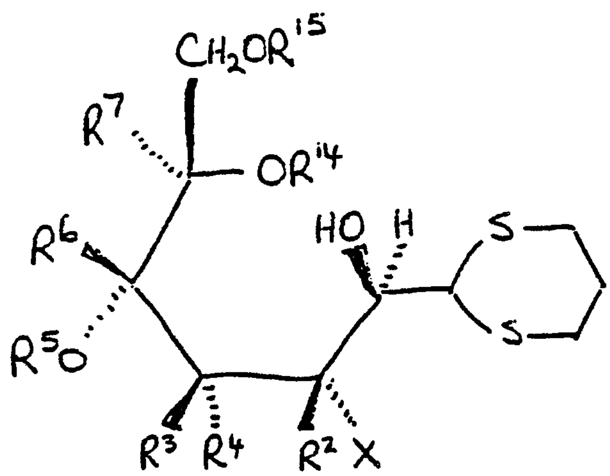

(XXXVI)

(wherein X, $R^2$, $R^3$, $R^4$, $R^5$, $R^6$, $R^7$, $R^{14}$ and $R^{15}$ are as defined above)
with the required (R)-configuration at the new chiral centre, can be isolated by conventional techniques (eg, flash chromatography).

The appropriate isomer can be converted to a vic-dihydroxy compound of formula (XXXVII)

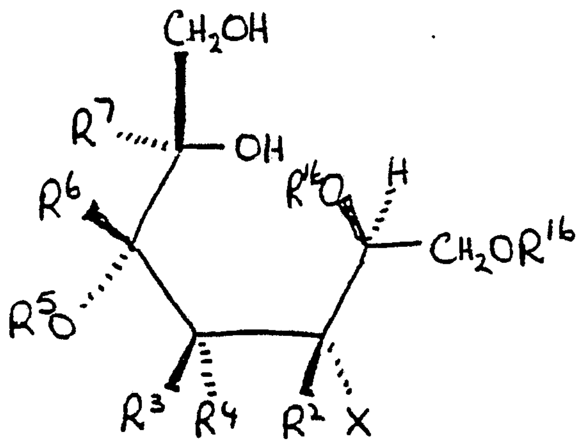

(XXXVII)

(wherein X, $R^2$, $R^3$, $R^4$, $R^5$, $R^6$ and $R^7$ are as defined above, and $R^{16}$ may be an ester or ether protecting group)
by hydrolytic cleavage of the dithiane protecting group, eg using mercury (II) salts or N-bromosuccinimide as reagents, reduction of the resulting aldehyde, eg with sodium borohydride, esterification or etherification of the free diol moiety, eg by acetylation, and finally deprotection of the acetal or ketal protected diol moiety, eg by mild acidic hydrolysis.

The desired compounds of formula (XXXII) can be obtained from compounds of formula (XXXVII) by sequential oxidative cleavage of the vicinal diol moiety (eg, with sodium periodate or lead tetracetate) and removal of the $R^{16}$ protecting groups (eg, by conventional deacetylation in the case that $R^{16}$ represents an acetyl group). Alternatively, desired compounds of formula (XXXII) can be obtained from compounds of formula (XXXVII) wherein $R^7$ represents a hydrogen atom by sequential selective protection (eg, with a bulky silyl group) of the primary alcohol function and oxidation of the secondary alcohol function (eg, by Swern oxidation, see above)

(t) ring closing of a compound of the fomula (XXXVIII) or formula (XXVIIIa)

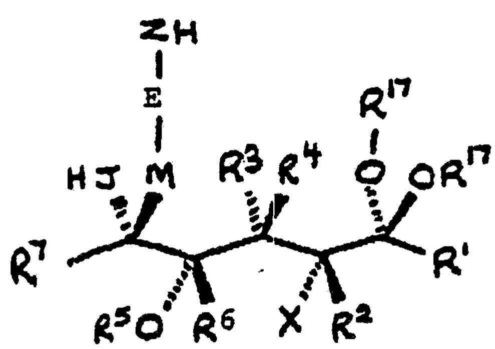

(XXXVIII)

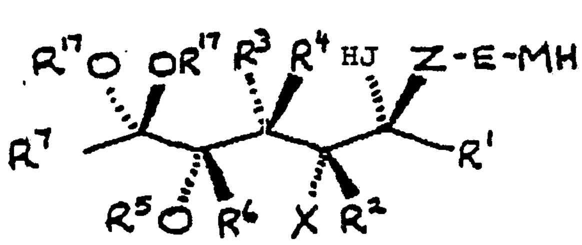

(XXXVIIIa)

(wherein $R^2$, $R^2$, $R^3$, $R^4$, $R^5$, $R^6$, $R^7$, X, J, M, E and Z are as defined under formula (I) and $R^{17}$ is an alkyl group)
or a protected form thereof.

The ring closure may advantageously be conducted by treatment with an aqueous strong acid solution such as strong acetic acid or trifluoroacetic acid with gentle heating if required.

Compounds of formula (XXXVIII) and (XXXVIIIa) can be obtained by known methods (e.g. see K. Narasaka and F. Pai, Tetrahedron (1984) 40, 2233-2238) or by reaction of a compound of formula (XXXIX)

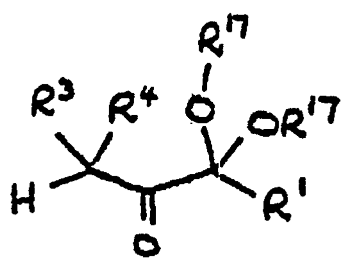

(XXXIX)

(wherein $R^3$ and $R^4$ are as defined above and $R^{17}$ is an alkyl group)
with an appropriate protected form of a compound of formula (XXXX) or (XXXXa)

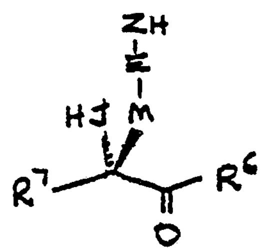

(XXXX)

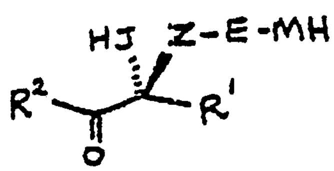

(XXXXa)

(wherein $R^1$, $R^2$, $R^6$, $R^7$, M, E, Z and J are as defined above) followed in an appropriate order by conversion of the ketonic function to an hydroxyl group and optionally converting any free hydroxyl groups to a protected form thereof or to an ether corresponding to $R^5$ and/or X.

(u) for the preparation of copounds wherein $R^1$ represents an optionally substituted hydrocarbyl group: selectively reacting a compound of the formula (XXXXI)

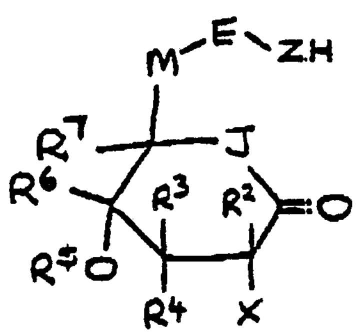

(XXXXI)

(wherein $R^2$, $R^3$, $R^4$, $R^5$, $R^6$, $R^7$, J, X, M, E and Z are as defined under formula I)
or a protected form thereof
with one mole equivalent of an organometallic reagent, advantageously a Grignard reagent, and simultaneously or subsequently ring closing the compound thus obtained and then optionally splitting off or converting any protective groups present. The reaction with the organometallic reagent can be conducted advantageously at low temperatures which facilitates a wide selection of organometallic reagents containing

optionally substituted hydrocarbyl groups which tend to be thermally unstable.

(v) for the preparation of compounds wherein $R^1$ together with X, Z or E represents a fused alicyclic or heterocyclic member:
covalently linking pre-existing groups for $R^1$ and X, Z or E in a similar manner to cyclisation of compounds of formula IIa-IIId described above, the atoms being linked having substituents $A_1$ and $A_2$ as defined above.

(w) for the preparation of compounds wherein $R^3$ is a fluorine atom:
reacting a corresponding compound of formula (I) in which $R^3$ is hydrogen and $R^4$ is a group or atom capable of being split off to leave the group or atom to which it was joined with a positive charge, e.g. where $R^3$ together with $R^4$ is an epoxide,
with a suitable fluoride source.

(x) for the preparation of compounds in which -M-E-Z- is a bridge containing two or more carbon atoms:
ring expanding a corresponding compound of formula (I) containing one less carbon by conventional ring expansion methods, advantageously by opening the bridge, forming a cyano derivative at the position where the new carbon is to be present and then ring closing in known manner.

(y) for the preparation of compounds wherein $R^1$ is an optionally substituted hydrocarbyl group, J is O, Z is $-CR^8R^9-$, -M-E- or -M- is an O or S atom, or a sulphinyl, sulphonyl, amino, phosphinico or N-linked organic group:
ring closing of a compound of formula (XXXXII)

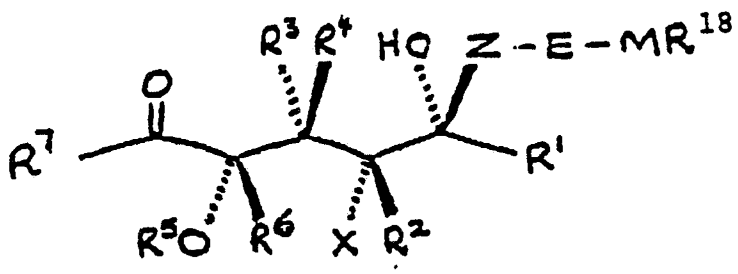

(XXXXII)

(wherein $R^2$, $R^3$, $R^4$, $R^5$, $R^6$, $R^7$ and X are as defined under formula (I), $R^1$ is an optionally substituted hydrocarbyl group, J is an oxygen atom, Z is $-CR^8R^9-$ , -M-E- or -M- is an O or S atom, or a sulphinyl, sulphonyl amino, phosphinico or N-linked organic group)
or a protected form thereof, e.g. by the methods described above for ring closing compounds of formulae IIa-IIId.

The compound of formula (XXXXII) can be obtained by sequential addition of one mole equivalent of an organometallic reagent of formula (XXXXIII)
Met-Z-E-M($R^{18}$) (XXXXIII)
(wherein $R^{18}$ is a suitable protecting group and Met is a suitable metal)
and a Grignard reagent of formula (V) as defined above
to a lactone of formula (XXXXI) wherein $R^1$, -M-E-Z- and J are as defined for formula (XXXXII) to give a compound of formula (XXXXIV)

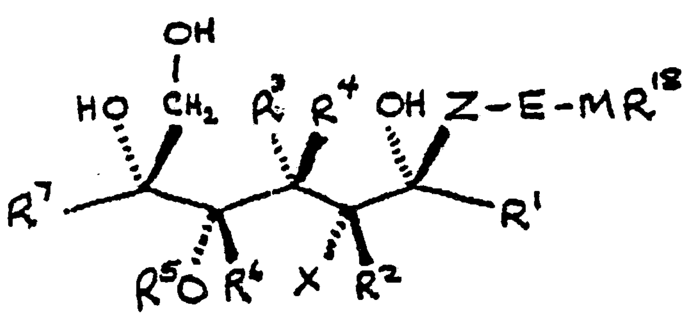

(XXXXIV)

(wherein $R^2$, $R^3$, $R^4$, $R^5$, $R^6$, $R^7$ and X are as defined under formula (I), $R^1$ is an optionally substituted hydrocarbyl group, J is O atom, Z is $-CR^8R^9-$, -M-E- or -M- is an O or S atom, or a sulphinyl, sulphonyl,

amino, phosphinico or N-linked organic group)
which is then subjected to oxidative vic-diol cleavage, e.g. by a periodate reagent.

Compounds of formula (XXXXIV) will be obtained as a mixture of isomers from which the desired isomer of formula (XXXXIV) can be isolated by conventional means.

The sequence of addition of reagents of formulae (XXXXIII) and (V) may be reversed to maximise the preparation of the desired isomer.

(z) for the preparation of salts of compounds of formula (I) and enantiomers thereof:
reacting a compound of formula (I) or enantiomer thereof, initially obtained, with an acid or base, optionally in the presence of a suitable solvent, to form the desired salt. The reaction may be carried out at or around room temperature, and/or under conventional reaction conditions.

(aa) converting compounds of formula (I) to other compounds of formula (I) by conventional methods or by methods illustrated in the Examples.

Protective groups (as herein defined) which may be employed in any of the above reactions include those well known to persons skilled in the art. Appropriate methods for splitting off or converting said protective groups at any desired point of the reaction sequence will also be known in the art.

Certain of the above processes, where newly applied in the preparation of compounds of formula (I) and their enantiomers and salts, constitute further features of the present invention.

Certain of the compounds of formulae (IIa-h), (IIIa-d), (IVa), (IVc), (VI), (VIa), (VIc), (VII), (IX), (XII) to (XVI), (XVIII) to (XXVIII) and (XXX) to (XXXXII) and (XXXXIV) useful as starting materials in the above reactions are new, and along with the processes described herein for their preparation, constitute still further features of the present invention.

Preferred compounds of the invention in view of their good activity are compounds identified hereinafter in Example Nos 1.2, 1.3, 1.4, 1.6, 1.7, 1.10, 1.11, 1.15, 1.16, 1.18, 1.23, 1.24, 1.25, 3.1, 3.3, 3.4, 4.2-4.7, 5.1, 5.3, 5.6, 6.1-6.5, 7.1, 7.2, 12-20, 26, 30-35, 37, 40-45, 47.1-47.9, 47.11, 48, 50, 51, 53, 54, 56.

The compositions according to the present invention may be used especially as weed-killers. Certain of the compositions may act as total herbicides while others may act as selective herbicides. Certain compositions may also be used as plant-growth regulators.

The compositions according to the present invention may be used, for example, to control or eliminate grass weeds, broad-leafed weeds or sedge weeds.

The compositions of the present invention may be particularly effective against monocotyledon weeds. However, the use of compositions according to the invention is in no way restricted to these groups of plants.

The compositions of the invention may be used both as pre-emergence and post-emergence herbicides. Pre-emergence herbicides are usually used to treat the soil in which a crop is to be planted, by application before or during seeding, or after seeding and before the crop emerges. Post-emergence herbicides are applied after the crop plants have emerged from the soil. Certain of the compositions may be used as general (ie, non-selective) herbicides whereas others may be used as selective herbicides for control of weeds in particular crops.

A number of the compositions show selective herbicidal activity when applied pre-emergence to soya, rape, maize and sugar-beet, or post-emergence to cotton, rape, sugar-beet, and rice. Pre-emergence application may also be appropriate in cotton at lower rates of application.

The present invention also provides a method of combatting weeds which comprises applying to the weeds, or to a habitat thereof, a composition of the present invention.

A range of compounds have been tested with regard to their herbicidal and/or plant growth regulatory activity, as will now be described. For convenience the compounds are referred to simply by the number of the Example (see below) in which the preparation of the compound is specifically described.

## GLASS HOUSE TESTS FOR HERBICIDAL ACTIVITY

The compounds were applied to a broad range of crop and monocot and dicot weed species using a travelling boom track sprayer at a volume equivalent to 1000 $l.ha^{-1}$ and at varying concentrations corresponding to the given application rates to test for both pre-emergence and post-emergence activity. Assessments were made for pre-emergence activity 20 days after direct spraying onto the seed, the seed having been covered with a compost for that period. Assessments were also made in some cases for post-emergence activity 13 days after spraying onto young plants.

The assessment scale used was as follows: 0 = 0 - 10% damage
1 = 11 - 25% damage
2 = 26 - 50% damage
3 = 51 - 80% damage
4 = 81 - 95% damage
5 = 96 - 100% damage

The results are shown in Table 1.

Abbreviations used for Plants in Table 1

| | | | | |
|---|---|---|---|---|
| - | Lt | - | Lettuce | |
| | Sb | - | Sugar-beet | A= Crops |
| | Rp | - | Rape | B= Dicot Weeds |
| | Ct | - | Cotton | C= Monocot Weeds |
| A | Sy | - | Soybean | |
| | Mz | - | Maize | |
| | Ww | - | Winter Wheat | |
| | Rc | - | Rice | |
| - | To | - | Tomato | |
| - | Bd | - | Bidens pilosa | |
| | Ip | - | Ipomoea purpurea | |
| | Am | - | Amaranthus retroflexus | |
| | Pi | - | Polygonum aviculare | |
| | Ca | - | Chenopodium album | |
| B | Ga | - | Galium aparine | |
| | Xa | - | Xanthium spinosum | |
| | Xs | - | Xanthium strumarium | |
| | Ab | - | Abutilon theophrasti | |
| - | Co | - | Cassia obtusifolia | |
| - | Av | - | Avena fatua | |
| | Dg | - | Digitaria sanguinalis | |
| | Al | - | Alopecurus myosuroides | |
| | St | - | Setaria viridis | |
| C | Ec | - | Echinochloa crus-galli | |
| | Sh | - | Sorghum halepense | |
| | Ag | - | Agropyron repens | |
| - | Cn | - | Cyperus spp, either Cyperus rotundus or Cyperus esculentus | |

TABLE 1

| COMPOUND (EXAMPLE NO. | RATE OF APPLICATION kg/ha | PRE- OR POST-EMERGENCE APPLICATION | TEST PLANTS Sb | Rp | Ct | Sy | Mz | Ww | Rc | Bd | Ip | Am | Pl | Ca | Ga | Xa | Xs | Ab | Co | Av | Dg | Al | St | Ec | Sh | Ag | Cn |
|---|---|---|---|---|---|---|---|---|---|---|---|---|---|---|---|---|---|---|---|---|---|---|---|---|---|---|---|
| 1.1 | 1.25 | Pre | | | | | | | | | 1 | | 4 | 4 | | 1 | | 2 | 3 | 3 | | 5 | 5 | | 3 | | 2 |
| | 1.25 | Post | | | | | | | | | 2 | | 3 | 2 | | | 0 | 1 | 1 | 0 | | 0 | 1 | | 1 | | 0 |
| 1.2 | 1.25 | Pre | 4 | 2 | 3 | 4 | 4 | 5 | 5 | 3 | 0 | 3 | 3 | 3 | 5 | 1 | - | 3 | 3 | 5 | 5 | 5 | 5 | 5 | 4 | 5 | 4 |
| | 1.25 | Post | 2 | 1 | 1 | 2 | 3 | 3 | 2 | 1 | 1 | 3 | 3 | 3 | 2 | - | 0 | 3 | 0 | 3 | 3 | 3 | 3 | 5 | 3 | 3 | 1 |
| 1.3 | 1.25 | Pre | 4 | 3 | 4 | 4 | 4 | 4 | 5 | 4 | 0 | 3 | 3 | 3 | 5 | 3 | - | 4 | 3 | 5 | 5 | 5 | 5 | 5 | 5 | 5 | 5 |
| | 1.25 | Post | 3 | 2 | 2 | 4 | 4 | 4 | 3 | 4 | 3 | 4 | 4 | 4 | 1 | - | 2 | 2 | 1 | 4 | 5 | 4 | 4 | 5 | 4 | 3 | 2 |
| 1.4 | 1.0 | Pre | 4 | 3 | 4 | 4 | 5 | 4 | 4 | 4 | 0 | 4 | 4 | 4 | 5 | 3 | - | 4 | 4 | 4 | 5 | 5 | 5 | 5 | 4 | 5 | 5 |
| | 1.0 | Post | 2 | 1 | 3 | 3 | 3 | 2 | 1 | 2 | 2 | 3 | 4 | 3 | 2 | - | 0 | 3 | 3 | 0 | 1 | 2 | 4 | 4 | 2 | 2 | 0 |
| 1.5 | 1.25 | Pre | 0 | 0 | 0 | 2 | 0 | 0 | 0 | 1 | 0 | 2 | 2 | 2 | 3 | 0 | - | 0 | 0 | 0 | 2 | 0 | 0 | 3 | 0 | 0 | 0 |
| | 1.25 | Post | 0 | 0 | 0 | 2 | 0 | 0 | 0 | 2 | 1 | 1 | 0 | 2 | 0 | - | 0 | 2 | 1 | 0 | 0 | 0 | 0 | 0 | 0 | 0 | 0 |
| 1.6 | 1.25 | Pre | 4 | 4 | 3 | 4 | 4 | 5 | 5 | 3 | 2 | 0 | 4 | 1 | 5 | 1 | - | 4 | 3 | 5 | 5 | 5 | 5 | 5 | 5 | - | 5 |
| | 1.25 | Post | | | | | | | | | 2 | | 2 | 3 | | | 1 | 2 | 0 | 1 | | 3 | 3 | | 1 | | 0 |

TABLE 1

| COMPOUND (EXAMPLE NO. | RATE OF APPLICATION kg/ha | PRE- OR POST-EMERGENCE APPLICATION | TEST PLANTS | | | | | | | | | | | | | | | | | | | | | | | | |
|---|---|---|---|---|---|---|---|---|---|---|---|---|---|---|---|---|---|---|---|---|---|---|---|---|---|---|---|
| | | | Sb | Rp | Ct | Sy | Mz | Ww | Rc | Bd | Ip | Am | Pl | Ca | Ga | Xa | Xs | Ab | Co | Av | Dg | Al | St | Ec | Sh | Ag | Cn |
| 1.7 | 1.25 | Pre | 4 | 2 | 3 | 4 | 4 | 5 | 5 | 4 | 0 | 1 | 3 | 2 | 4 | 2 | - | 3 | 0 | 5 | 5 | 5 | 5 | 5 | 5 | - | 5 |
| | 1.25 | Post | | | | | | | | | 1 | | 3 | 2 | | | 1 | 2 | 0 | 2 | | 3 | 2 | | 1 | | 0 |
| 1.8 | 1.25 | Pre | | | | | | | | | 0 | | 2 | 3 | 0 | 0 | | 0 | | 0 | | 0 | 0 | | 1 | | 0 |
| | 1.25 | Post | | | | | | | | | 0 | | 0 | 2 | | | 0 | 0 | 0 | 0 | | 0 | 1 | | 0 | | 0 |
| 1.9 | 1.25 | Pre | | | | | | | | | 3 | | 2 | 3 | 3 | 0 | | 0 | 1 | 0 | | 3 | 4 | | | | |
| | 1.25 | Post | | | | | | | | | 0 | | 0 | 0 | 0 | | 0 | 0 | 1 | 0 | | 0 | 1 | | 2 | | 0 |
| 1.10 | 1.25 | Pre | 4 | 4 | 4 | 4 | 4 | 5 | 5 | 4 | 4 | - | 4 | 4 | 5 | 4 | - | 4 | 4 | 5 | 5 | 5 | 5 | 5 | 5 | 5 | 5 |
| | 1.25 | Post | 1 | 1 | 1 | 3 | 3 | 4 | 1 | 3 | 3 | 4 | 3 | 3 | 2 | - | 2 | 3 | 1 | 4 | 4 | 3 | 4 | 4 | 3 | 3 | 2 |
| 1.11 | 1.25 | Pre | 3 | 3 | 4 | 3 | 3 | 2 | 5 | 3 | 2 | - | 3 | 4 | 4 | 2 | - | 4 | 1 | 2 | 5 | 1 | 5 | 5 | 4 | 4 | 4 |
| | 1.25 | Post | 1 | 0 | 1 | 3 | 3 | 0 | 0 | 3 | 1 | 2 | 0 | 3 | 0 | - | 2 | 2 | 1 | 0 | 3 | 0 | 2 | 2 | 0 | 0 | 0 |
| 1.12 | 1.25 | Pre | 3 | 0 | 1 | 0 | 0 | 0 | 0 | 0 | 2 | - | 1 | 1 | 3 | 0 | - | 5 | 2 | 0 | 1 | 0 | 0 | 4 | 0 | 0 | 0 |
| | 1.25 | Post | 1 | 1 | 0 | 2 | 1 | 0 | 0 | 2 | 0 | 0 | 2 | 2 | 0 | - | 0 | 2 | 0 | 0 | 0 | 1 | 0 | 0 | 0 | 0 | 0 |

TABLE 1

| COMPOUND (EXAMPLE NO. | RATE OF APPLICATION kg/ha | PRE- OR POST-EMERGENCE APPLICATION | TEST PLANTS | | | | | | | | | | | | | | | | | | | | | | | | |
|---|---|---|---|---|---|---|---|---|---|---|---|---|---|---|---|---|---|---|---|---|---|---|---|---|---|---|---|
| | | | Sb | Rp | Ct | Sy | Mz | Ww | Rc | Bd | Ip | Am | Pl | Ca | Ga | Xa | Xs | Ab | Co | Av | Dg | Al | St | Ec | Sh | Ag | Cn |
| 1.13 | 1.25 | Pre | 2 | 0 | 0 | 0 | 0 | 0 | 0 | 0 | 0 | 1 | 2 | 4 | 2 | 0 | | 0 | 0 | 0 | 4 | 0 | 2 | 4 | 0 | | 0 |
| | 1.25 | Post | 0 | 0 | 1 | 1 | 0 | 0 | 0 | 1 | 0 | 0 | 0 | 2 | 0 | | 1 | 0 | 1 | 0 | 0 | 0 | 0 | 0 | 0 | 0 | 0 |
| 1.14 | 1.25 | Pre | 0 | 0 | 1 | 0 | 0 | 0 | 0 | 0 | 0 | 0 | 0 | 0 | 0 | 0 | | 0 | 0 | 0 | 2 | 0 | 0 | 0 | 3 | | 0 |
| | 1.25 | Post | 0 | | 0 | 1 | 1 | 0 | 1 | 1 | 0 | 0 | 0 | 0 | 0 | | 1 | 1 | 0 | 0 | 0 | 0 | 0 | 0 | 0 | 0 | 0 |
| 1.15 | 1.25 | Pre | 4 | 3 | 4 | 4 | 4 | 5 | 5 | 4 | 1 | 4 | 4 | 4 | 5 | 4 | - | 3 | 4 | 5 | 5 | 5 | 5 | 5 | - | - | 5 |
| | 1.25 | Post | 0 | 1 | 1 | 2 | 2 | 1 | 1 | 3 | 1 | 3 | 3 | 3 | 2 | - | 1 | 2 | 0 | 3 | 4 | 3 | 4 | 3 | 0 | 1 | 0 |
| 1.16 | 1.25 | Pre | 4 | 2 | 3 | 4 | 3 | 3 | 4 | 3 | 0 | 4 | 4 | 4 | 4 | 3 | - | 4 | 3 | 4 | 5 | 4 | 4 | 5 | 3 | - | 3 |
| | 1.25 | Post | 1 | 2 | 1 | 3 | 1 | 2 | 1 | 2 | 1 | 3 | 3 | 3 | 0 | - | 2 | 3 | 1 | 1 | 3 | 2 | 2 | 2 | 1 | 0 | 0 |
| 1.17 | 1.25 | Pre | 3 | 1 | 3 | 3 | 1 | 1 | 1 | 1 | 1 | 4 | 4 | 3 | 3 | 1 | - | 2 | 3 | 3 | 4 | 3 | 3 | 4 | 3 | - | 1 |
| | 1.25 | Post | 1 | 2 | 0 | 3 | 1 | 1 | 0 | 2 | 0 | 2 | 2 | 2 | 1 | - | 1 | 0 | 0 | 1 | 1 | 1 | 1 | 0 | 0 | 0 | 0 |
| 1.18 | 1.25 | Pre | 3 | 1 | 2 | 3 | 2 | 2 | 1 | 2 | 0 | 4 | 4 | 4 | 4 | 2 | | 3 | 2 | 2 | 5 | 3 | 4 | 5 | 4 | - | 2 |
| | 1.25 | Post | 0 | 1 | 1 | 1 | 1 | 0 | 1 | 3 | 2 | 2 | 3 | 3 | 1 | - | 1 | 1 | 0 | 0 | 0 | 0 | 0 | 1 | 1 | 0 | 0 |

TABLE 1

| COMPOUND (EXAMPLE NO. | RATE OF APPLICATION kg/ha | PRE- OR POST-EMERGENCE APPLICATION | TEST PLANTS | | | | | | | | | | | | | | | | | | | | | | | | |
|---|---|---|---|---|---|---|---|---|---|---|---|---|---|---|---|---|---|---|---|---|---|---|---|---|---|---|---|
| | | | Sb | Rp | Ct | Sy | Mz | Ww | Rc | Bd | Ip | Am | Pl | Ca | Ga | Xa | Xs | Ab | Co | Av | Dg | Al | St | Ec | Sh | Ag | Cn |
| 1.19 | 1.25 | Pre | 2 | 1 | 0 | 2 | 0 | 0 | 0 | 0 | 0 | 2 | 4 | 4 | 2 | 2 | - | 0 | 0 | 0 | 3 | 0 | 2 | 4 | 2 | - | 0 |
| | 1.25 | Post | 0 | 2 | 0 | 2 | 0 | 0 | 0 | 1 | 0 | 1 | 0 | 1 | 2 | - | 1 | 1 | 0 | 0 | 0 | 1 | 0 | 0 | 0 | 0 | 0 |
| 1.20 | 1.25 | Pre | 1 | 0 | 0 | 0 | 1 | 0 | 0 | 0 | 0 | 0 | 1 | 0 | 1 | 0 | - | 0 | 0 | 1 | 0 | 0 | 2 | 3 | 0 | - | 0 |
| | 1.25 | Post | 2 | 2 | 1 | 3 | 1 | 1 | 1 | 2 | 0 | 3 | 1 | 3 | 0 | - | 1 | 1 | 1 | 1 | 1 | 0 | 3 | 1 | 0 | 1 | 0 |
| 1.21 | 1.25 | Pre | | | | | | | | | 0 | | 3 | 3 | | 2 | | 3 | 2 | 0 | | 1 | 0 | | 0 | | 1 |
| | 1.25 | Post | | | | | | | | | 0 | | 1 | 1 | 0 | | 2 | 0 | 2 | 1 | | 1 | 0 | | 1 | | 0 |
| 1.22 | 1.25 | Pre | | | | | | | | | 0 | | 1 | 3 | 1 | 0 | | 1 | 2 | 3 | | 2 | 4 | | 2 | | 3 |
| | 1.25 | Post | | | | | | | | | 0 | | 0 | 1 | 0 | | 0 | 0 | 0 | 0 | | 0 | 1 | | 0 | | 0 |
| 1.23 | 1.25 | Pre | 4 | 3 | 2 | 4 | 4 | 5 | 5 | 4 | 2 | 0 | 4 | 1 | 4 | 2 | - | 4 | 4 | 5 | 5 | 5 | 5 | 5 | 5 | - | 5 |
| | 1.25 | Post | | | | | | | | | 0 | | 2 | 3 | | | 0 | 2 | 0 | 2 | | 2 | 2 | | 1 | | 0 |
| 1.24 | 1.25 | Pre | 3 | 2 | 1 | 4 | 3 | 3 | 4 | 0 | 0 | 1 | 2 | 1 | 4 | 3 | - | 3 | 1 | 3 | 5 | 2 | 4 | 4 | 4 | - | 5 |
| | 1.25 | Post | | | | | | | | | 1 | | 0 | 3 | | | 1 | 2 | 0 | 0 | | 0 | 3 | | 3 | | 0 |

**TABLE 1**

| COMPOUND (EXAMPLE NO. | RATE OF APPLICATION kg/ha | PRE- OR POST-EMERGENCE APPLICATION | TEST PLANTS | | | | | | | | | | | | | | | | | | | | | | | | |
|---|---|---|---|---|---|---|---|---|---|---|---|---|---|---|---|---|---|---|---|---|---|---|---|---|---|---|---|
| | | | Sb | Rp | Ct | Sy | Mz | Ww | Rc | Bd | Ip | Am | Pl | Ca | Ga | Xa | Xs | Ab | Co | Av | Dg | Al | St | Ec | Sh | Ag | Cn |
| 1.25 | 1.25 | Pre | 0 | 2 | 3 | 4 | 3 | 1 | 0 | 0 | 0 | 0 | 0 | 2 | 2 | 0 | - | 1 | 0 | 1 | 5 | 1 | 5 | 5 | 4 | 5 | 4 |
| | 1.25 | Post | 0 | 0 | 0 | 3 | 1 | 0 | 0 | 1 | 1 | 0 | 0 | 0 | 0 | - | 0 | 0 | 0 | 0 | 0 | 0 | 0 | 0 | 0 | 0 | 0 |
| 2 | 1.25 | Pre | | | | | | | | | 0 | | 0 | 0 | 0 | 0 | | 0 | 0 | 0 | | 0 | 0 | | 0 | | 0 |
| | 1.25 | Post | | | | | | | | | 2 | | 1 | 2 | 0 | | 0 | 0 | 1 | 0 | | 0 | 0 | | 1 | | 0 |
| 3.1 | 1.25 | Pre | 4 | 4 | 4 | 4 | 4 | 5 | 5 | 5 | 3 | 4 | 4 | 4 | 5 | 4 | - | 4 | 4 | 5 | 5 | 5 | 5 | 5 | 5 | 5 | 5 |
| | 1.25 | Post | 4 | 2 | 3 | 4 | 4 | 4 | 2 | 4 | 2 | 4 | 4 | 4 | 3 | - | 3 | 3 | 3 | 4 | 4 | 4 | 5 | 5 | 5 | 4 | 3 |
| 3.2 | 1.25 | Pre | | | | | | | | | 0 | | | 1 | 3 | 0 | - | 0 | 0 | 0 | | 3 | 3 | | 0 | | 0 |
| | 1.25 | Post | | | | | | | | | 1 | | | 2 | 2 | - | - | 0 | 0 | 0 | | 0 | 2 | | 1 | | 1 |
| 3.3 | 1.25 | Pre | 1 | 1 | 2 | 1 | 2 | 3 | 5 | 0 | 0 | 0 | 1 | 3 | 4 | 2 | - | 3 | 1 | 2 | 4 | 2 | 3 | 4 | 4 | 5 | 4 |
| | 1.25 | Post | 0 | 0 | 0 | 2 | 0 | 0 | 0 | 2 | 1 | 1 | 0 | 3 | 1 | - | 0 | 3 | 3 | 0 | 1 | 0 | 3 | 3 | 1 | 0 | 0 |
| 3.4 | 1.25 | Pre | 3 | 2 | 4 | 4 | 5 | 5 | 5 | 4 | 4 | 4 | 4 | 3 | 5 | 4 | - | 4 | 3 | 5 | 5 | 5 | 5 | 5 | 5 | 5 | 4 |
| | 1.25 | Post | 0 | 0 | 3 | 3 | 3 | 4 | 2 | 3 | 3 | 1 | 3 | 2 | 2 | - | 0 | 4 | 2 | 4 | 4 | 3 | 4 | 4 | 2 | 3 | 1 |

98

TABLE 1

| COMPOUND (EXAMPLE NO. | RATE OF APPLICATION kg/ha | PRE- OR POST-EMERGENCE APPLICATION | TEST PLANTS | | | | | | | | | | | | | | | | | | | | | | | | |
|---|---|---|---|---|---|---|---|---|---|---|---|---|---|---|---|---|---|---|---|---|---|---|---|---|---|---|---|
| | | | Sb | Rp | Ct | Sy | Mz | Ww | Rc | Bd | Ip | Am | Pl | Ca | Ga | Xa | Xs | Ab | Co | Av | Dg | Al | St | Ec | Sh | Ag | Cn |
| 4.2 | 1.25 | Pre | 4 | 2 | 3 | 4 | 4 | 4 | 5 | 4 | 0 | 4 | 4 | 4 | 4 | 1 | - | 4 | 0 | 4 | 5 | 5 | 5 | 5 | 5 | - | 5 |
| | 1.25 | Post | 3 | 1 | 2 | 3 | 2 | 0 | 0 | 2 | 2 | 2 | 3 | 3 | 2 | - | 1 | 2 | 1 | 3 | 2 | 3 | 4 | 3 | 3 | 2 | 0 |
| 4.3 | 1.25 | Pre | 4 | 3 | 2 | 4 | 4 | 4 | 4 | 1 | 0 | 4 | 4 | 4 | 4 | 1 | - | 4 | 0 | 3 | 5 | 4 | 5 | 5 | 5 | - | 5 |
| | 1.25 | Post | 2 | 0 | 1 | 3 | 1 | 1 | 1 | 3 | 3 | 1 | 0 | 3 | 0 | - | 0 | 3 | 1 | 0 | 2 | 2 | 3 | 3 | 2 | 2 | 0 |
| 4.4 | 1.25 | Pre | 4 | 4 | 2 | 4 | 4 | 4 | 5 | 4 | 1 | 4 | 4 | 4 | 5 | 1 | - | 4 | 0 | 5 | 5 | 5 | 5 | 5 | 5 | - | 5 |
| | 1.25 | Post | 3 | 2 | 2 | 3 | 4 | 2 | 0 | 2 | 3 | 1 | 3 | 3 | 0 | - | 1 | 3 | 2 | 3 | 3 | 3 | 4 | 4 | 2 | 2 | 1 |
| 4.5 | 1.25 | Pre | 0 | 1 | 1 | 4 | 4 | 5 | 5 | 0 | 1 | 1 | 3 | 4 | 4 | 0 | - | 4 | 0 | 4 | 5 | 4 | 5 | 5 | 5 | - | 5 |
| | 1.25 | Post | 1 | 2 | 2 | 3 | 4 | 4 | 1 | 3 | 3 | 0 | 0 | 3 | 1 | - | 2 | 3 | 1 | 3 | 4 | 3 | 4 | 5 | 4 | 3 | 0 |
| 4.7 | 1.25 | Pre | 4 | 4 | 4 | 4 | 4 | 5 | 5 | 4 | 4 | 3 | 4 | 4 | 5 | 3 | - | 4 | 0 | 5 | 5 | 5 | 5 | 5 | 5 | - | 5 |
| | 1.25 | Post | 3 | 2 | 2 | 3 | 4 | 3 | 1 | 3 | 3 | 3 | 3 | 3 | 3 | - | 2 | 3 | 3 | 3 | 4 | 3 | 4 | 4 | 4 | 3 | 0 |

**TABLE 1**

| COMPOUND (EXAMPLE NO. | RATE OF APPLICATION kg/ha | PRE- OR POST-EMERGENCE APPLICATION | TEST PLANTS | | | | | | | | | | | | | | | | | | | | | | | | |
|---|---|---|---|---|---|---|---|---|---|---|---|---|---|---|---|---|---|---|---|---|---|---|---|---|---|---|---|
| | | | Sb | Rp | Ct | Sy | Mz | Ww | Rc | Bd | Ip | Am | Pl | Ca | Ga | Xa | Xs | Ab | Co | Av | Dg | Al | St | Ec | Sh | Ag | Cn |
| 5.1 | 1.25 | Pre | 3 | 1 | 3 | 3 | 4 | 5 | 5 | 2 | 1 | 3 | 3 | 3 | 4 | 2 | - | 3 | 3 | 4 | 5 | 5 | 5 | 5 | - | - | 4 |
| | 1.25 | Post | 1 | 1 | 1 | 3 | 3 | 3 | 1 | 3 | 2 | 1 | 0 | 2 | 1 | - | 1 | 2 | 0 | 3 | 3 | 3 | 4 | 3 | 0 | 3 | 0 |
| 5.2 | 1.25 | Pre | 0 | 0 | 2 | 2 | 3 | 4 | 5 | 1 | 0 | 2 | 2 | 3 | 3 | 0 | - | 3 | 1 | 3 | 5 | 5 | 4 | 4 | - | - | 3 |
| | 1.25 | Post | 0 | 1 | 0 | 2 | 2 | 0 | 0 | 2 | 1 | 0 | 0 | 1 | 0 | - | 0 | 1 | 0 | 0 | 2 | 0 | 3 | 1 | 0 | 0 | 0 |
| 5.3 | 1.25 | Pre | 3 | 1 | 3 | 3 | 5 | 5 | 5 | 2 | 0 | 4 | 3 | 3 | 4 | 2 | - | 3 | 3 | 5 | 5 | 5 | 5 | 5 | - | - | 5 |
| | 1.25 | Post | 0 | 1 | 2 | 3 | 3 | 3 | 0 | 1 | 1 | 0 | 0 | 2 | 0 | - | 1 | 2 | 0 | 2 | 4 | 3 | 3 | 2 | 1 | 3 | 0 |
| 4.6 | 1.25 | Pre | 3 | 2 | 1 | 4 | 5 | 5 | 5 | 2 | 1 | 1 | 4 | 4 | 5 | 1 | 2 | 4 | 1 | 5 | 5 | 5 | 5 | 5 | 5 | | 5 |
| | 1.25 | Post | 1 | 1 | 1 | 2 | 3 | 4 | 1 | 1 | 1 | 0 | 0 | 2 | 0 | | 1 | 2 | 0 | 3 | 3 | 2 | 3 | 4 | 1 | 4 | 2 |
| 6.1 | 1.25 | Pre | 3 | 2 | 3 | 4 | 4 | 5 | 5 | 4 | 3 | 1 | 4 | 3 | 5 | 2 | | 3 | 2 | 5 | 5 | 5 | 5 | 5 | 5 | | 5 |
| | 1.25 | Post | 1 | 0 | 2 | 3 | 4 | 3 | 2 | 2 | 2 | 1 | 2 | 2 | 0 | | 1 | 3 | 1 | 3 | 0 | 1 | 3 | 4 | 3 | 3 | 0 |
| 6.2 | 1.25 | Pre | 3 | 2 | 2 | 3 | 5 | 5 | 5 | 4 | 2 | 1 | 4 | 3 | 5 | 3 | | 3 | 1 | 5 | 5 | 4 | 5 | 5 | 5 | | 5 |
| | 1.25 | Post | 1 | 1 | 2 | 2 | 4 | 3 | 3 | 2 | 2 | 1 | 1 | 3 | 0 | | 1 | 4 | 0 | 3 | 3 | 3 | 2 | 3 | 4 | 4 | 1 |

TABLE 1

| COMPOUND (EXAMPLE NO. | RATE OF APPLICATION kg/ha | PRE- OR POST-EMERGENCE APPLICATION | TEST PLANTS | | | | | | | | | | | | | | | | | | | | | | | | |
|---|---|---|---|---|---|---|---|---|---|---|---|---|---|---|---|---|---|---|---|---|---|---|---|---|---|---|---|
| | | | Sb | Rp | Ct | Sy | Mz | Ww | Rc | Bd | Ip | Am | Pl | Ca | Ga | Xa | Xs | Ab | Co | Av | Dg | Al | St | Ec | Sh | Ag | Cn |
| 6.3 | 1.25 | Pre | 2 | 0 | 1 | 0 | 4 | 5 | 5 | 2 | 0 | 0 | 3 | 1 | 4 | 1 | | 3 | 1 | 5 | 5 | 5 | 4 | 5 | 4 | | 5 |
| | 1.25 | Post | 0 | 1 | 1 | 3 | 4 | 4 | 1 | 3 | 2 | 2 | 1 | 3 | 1 | | 1 | 3 | 1 | 3 | 3 | 1 | 2 | 4 | 3 | 4 | 0 |
| 6.4 | 1.25 | Pre | 3 | 1 | 3 | 3 | 4 | 4 | 5 | 3 | 1 | 0 | 4 | 3 | 4 | 1 | | 3 | 1 | 5 | 4 | 5 | 5 | 5 | 4 | | 4 |
| | 1.25 | Post | 0 | 0 | 1 | 3 | 3 | 0 | 0 | 1 | 1 | 1 | 0 | 1 | 0 | | 1 | 1 | 0 | 0 | 0 | 0 | 1 | 4 | 2 | 2 | 1 |
| 6.5 | 1.25 | Pre | 0 | 0 | 0 | 1 | 4 | 4 | 5 | 1 | 1 | 0 | 2 | 2 | 2 | 1 | | 3 | 0 | 3 | 4 | 4 | 4 | 4 | 4 | | 3 |
| | 1.25 | Post | 0 | 0 | 0 | 3 | 1 | 2 | 1 | 2 | 2 | 1 | 0 | 1 | 0 | | 1 | 1 | 1 | 0 | 0 | 0 | 1 | 4 | 1 | 2 | 0 |
| 12 | 1.25 | Pre | 4 | 0 | 4 | 4 | 4 | 5 | 5 | 4 | 3 | 4 | 4 | 4 | 4 | 4 | - | 4 | 4 | 4 | 5 | 5 | 5 | 5 | 4 | 5 | 5 |
| | 1.25 | Post | 0 | 1 | 2 | 3 | 4 | 4 | 0 | 3 | 3 | 1 | 1 | 4 | 2 | - | 0 | 4 | 0 | 2 | 3 | 3 | 4 | 4 | 3 | 3 | 0 |

**TABLE 1**

| COMPOUND (EXAMPLE NO. | RATE OF APPLICATION kg/ha | PRE- OR POST-EMERGENCE APPLICATION | TEST PLANTS | | | | | | | | | | | | | | | | | | | | | | | | |
|---|---|---|---|---|---|---|---|---|---|---|---|---|---|---|---|---|---|---|---|---|---|---|---|---|---|---|---|
| | | | Sb | Rp | Ct | Sy | Mz | Ww | Rc | Bd | Ip | Am | Pl | Ca | Ga | Xa | Xs | Ab | Co | Av | Dg | Al | St | Ec | Sh | Ag | Cn |
| 13 | 1.25 | Pre | 4 | 4 | 4 | 4 | 5 | 5 | 5 | 4 | 3 | 4 | 4 | 4 | 5 | 4 | - | 3 | - | 5 | 5 | 5 | 5 | 5 | 5 | 5 | 5 |
| | 1.25 | Post | 1 | 1 | 3 | 2 | 3 | 3 | 2 | 2 | 1 | 3 | 3 | 3 | 2 | - | 2 | 3 | 1 | 2 | 3 | 2 | 4 | 4 | 3 | 2 | 3 |
| 14 | 1.25 | Pre | 4 | 3 | 4 | 4 | 5 | 5 | 5 | 4 | 3 | 3 | 4 | 3 | 5 | 3 | - | 3 | - | 5 | 5 | 5 | 5 | 5 | 5 | 5 | 5 |
| | 1.25 | Post | 1 | 1 | 1 | 2 | 4 | 3 | 1 | 3 | 2 | 2 | 3 | 3 | 2 | - | 2 | 3 | 2 | 3 | 3 | 3 | 4 | 4 | 3 | 3 | 2 |
| 15 | 0.25 | Pre | 3 | 2 | 3 | 4 | 4 | 5 | 5 | 4 | 0 | 4 | 4 | 4 | 5 | 3 | - | 3 | 4 | 5 | 5 | 5 | 5 | 5 | 4 | 5 | 5 |
| | 0.25 | Post | 2 | 2 | 1 | 2 | 3 | 1 | 0 | 3 | 2 | 1 | 3 | 3 | 0 | - | 0 | 1 | 1 | 2 | 2 | 2 | 2 | 3 | 0 | 2 | 0 |
| 16 | 0.8 | Pre | 4 | 4 | 4 | 4 | 5 | 5 | 5 | 4 | 2 | 4 | 4 | 4 | 5 | 4 | - | 4 | 4 | 5 | 5 | 5 | 5 | 5 | 5 | 5 | 5 |
| | | Post | | | | | | | | | | | | | | | | | | | | | | | | | |
| 17 | 1.25 | Pre | 4 | 3 | 3 | 4 | 5 | 5 | 5 | 4 | 0 | 4 | 4 | 4 | 5 | 4 | - | 3 | 3 | 5 | 5 | 5 | 5 | 5 | 5 | 5 | 5 |
| | 1.25 | Post | 2 | 1 | 1 | 3 | 3 | 3 | 2 | 3 | 2 | 3 | 3 | 3 | 3 | - | 2 | 3 | 1 | 4 | 3 | 4 | 4 | 5 | 3 | 3 | 2 |
| 18 | 0.9 | Pre | 4 | 1 | 2 | 3 | 4 | 5 | 5 | 0 | 0 | 3 | 4 | 4 | 4 | 0 | | 4 | 3 | 5 | 5 | 5 | 5 | 5 | 4 | | 5 |
| | 0.9 | Post | 0 | 1 | 1 | 3 | 0 | 0 | 0 | 4 | 2 | 0 | 0 | 1 | 0 | | 2 | 2 | 0 | 0 | 0 | 0 | 1 | 0 | 0 | 0 | 0 |

**TABLE 1**

| COMPOUND (EXAMPLE NO. | RATE OF APPLICATION kg/ha | PRE- OR POST-EMERGENCE APPLICATION | TEST PLANTS | | | | | | | | | | | | | | | | | | | | | | | | |
|---|---|---|---|---|---|---|---|---|---|---|---|---|---|---|---|---|---|---|---|---|---|---|---|---|---|---|---|
| | | | Sb | Rp | Ct | Sy | Mz | Ww | Rc | Bd | Ip | Am | Pl | Ca | Ga | Xa | Xs | Ab | Co | Av | Dg | Al | St | Ec | Sh | Ag | Cn |
| 19 | 1.25 | Pre | 4 | 1 | 4 | 3 | 5 | 5 | 5 | 3 | 3 | 3 | 4 | 3 | 5 | 3 | - | 4 | 3 | 5 | 5 | 5 | 5 | 5 | 5 | - | 5 |
| | 1.25 | Post | 1 | 1 | 3 | 3 | 4 | 3 | 2 | 3 | 3 | 3 | 2 | 3 | 2 | - | 3 | 4 | 1 | 3 | 5 | 4 | 5 | 5 | 5 | 3 | 0 |
| 20 | 1.25 | Pre | 3 | 0 | - | 4 | 4 | 3 | 4 | 4 | 0 | 0 | 4 | 4 | 4 | 0 | - | 4 | 1 | 3 | 5 | 4 | 5 | 5 | 3 | - | 4 |
| | 1.25 | Post | 2 | 1 | 1 | 2 | 0 | 0 | 0 | 2 | 2 | 0 | 1 | 1 | 0 | - | 1 | 3 | 2 | 0 | 0 | 0 | 1 | 1 | 0 | 0 | 0 |
| 26 | 1.25 | Pre | 0 | 0 | 0 | 4 | 0 | 3 | 0 | 1 | 0 | 1 | 4 | 4 | 4 | 0 | | 4 | 0 | 0 | 5 | 3 | 5 | 5 | 1 | | 4 |
| | 1.25 | Post | 0 | 2 | 0 | 3 | 0 | 0 | 0 | 1 | 2 | 0 | 0 | 1 | 0 | | 0 | 3 | 1 | 0 | 0 | 0 | 0 | 0 | 0 | 0 | 0 |
| 29 Title Compound B | 0.25 | Pre | 0 | 2 | 0 | 0 | 0 | 0 | 1 | 1 | 0 | 1 | 0 | 3 | - | 0 | - | 3 | 0 | 0 | 0 | 0 | 0 | 0 | 0 | 0 | 0 |
| | 0.25 | Post | 0 | 0 | 0 | 0 | 0 | 0 | 0 | 0 | 0 | 0 | 0 | 0 | 0 | - | 0 | 0 | 0 | 0 | 1 | 0 | 0 | 0 | 0 | 0 | 0 |
| 29 | 1.25 | Pre | | | | | | | | | 0 | | 4 | 4 | 4 | 0 | | 3 | 3 | 3 | | 4 | 5 | | 4 | | 4 |
| | 1.25 | Post | | | | | | | | | 0 | | 0 | 2 | 0 | 0 | | 2 | 0 | 0 | | 3 | 0 | | 0 | | 0 |
| 30 | 1.02 | Pre | 4 | 4 | 4 | 4 | 5 | 5 | 5 | 5 | 4 | 4 | 4 | 4 | 5 | 4 | | 5 | 4 | 5 | 5 | 5 | 5 | 5 | 5 | 5 | 5 |
| | 1.02 | Post | 3 | 3 | 4 | 4 | 5 | 4 | 4 | 4 | 3 | 3 | 4 | 4 | 3 | - | 3 | 4 | 3 | 4 | 4 | 4 | 5 | 5 | 5 | 4 | 3 |

**TABLE 1**

| COMPOUND (EXAMPLE NO. | RATE OF APPLICATION kg/ha | PRE- OR POST-EMERGENCE APPLICATION | TEST PLANTS | | | | | | | | | | | | | | | | | | | | | | | | |
|---|---|---|---|---|---|---|---|---|---|---|---|---|---|---|---|---|---|---|---|---|---|---|---|---|---|---|---|
| | | | Sb | Rp | Ct | Sy | Mz | Ww | Rc | Bd | Ip | Am | Pl | Ca | Ga | Xa | Xs | Ab | Co | Av | Dg | Al | St | Ec | Sh | Ag | Cn |
| 31 | 1.25 | Pre | 4 | 3 | 3 | 4 | 4 | 5 | 5 | 4 | 1 | 1 | 4 | 4 | 5 | 2 | - | 4 | 1 | 5 | 5 | 5 | 5 | 5 | 5 | - | 5 |
| | 1.25 | Post | 0 | 1 | 0 | 4 | 4 | 1 | 0 | 1 | 1 | 2 | 0 | 2 | 0 | - | 1 | 1 | 0 | 2 | 1 | 2 | 3 | 3 | 1 | 1 | 2 |
| 32 | 1.25 | Pre | 4 | 3 | 2 | 4 | 5 | 5 | 5 | 4 | 4 | 4 | 4 | 4 | 5 | 4 | - | 4 | 2 | 5 | 5 | 5 | 5 | 5 | 5 | - | 5 |
| | 1.25 | Post | 3 | 3 | 1 | 4 | 4 | 2 | 1 | 3 | 1 | 1 | 1 | 4 | 0 | - | 3 | 1 | 4 | 2 | 3 | 2 | 4 | 4 | 2 | 3 | 1 |
| 33 | 1.25 | Pre | 4 | 3 | 0 | 3 | 4 | 5 | 5 | 4 | 2 | 1 | 4 | 4 | 5 | 0 | - | 4 | 0 | 5 | 5 | 5 | 5 | 5 | 5 | - | 5 |
| | 1.25 | Post | 1 | 1 | 2 | 4 | 4 | 1 | 0 | 1 | 2 | 1 | 1 | 3 | 0 | - | 1 | 3 | 1 | 2 | 1 | 2 | 3 | 3 | 1 | 2 | 0 |
| 34 | 1.25 | Pre | 0 | 1 | 1 | 3 | 4 | 5 | 4 | 3 | 0 | 0 | 2 | 3 | 3 | 0 | - | 4 | 1 | 4 | 5 | 4 | 5 | 5 | 4 | - | 5 |
| | 1.25 | Post | 1 | 2 | 3 | 3 | 2 | 0 | 0 | 2 | 3 | 0 | 0 | 2 | 2 | - | 2 | 3 | 2 | 0 | 2 | 1 | 2 | 1 | 4 | 1 | 0 |
| 35 | 1.25 | Pre | 4 | 0 | 0 | 4 | 4 | 4 | 1 | 4 | 0 | 0 | 4 | 4 | 2 | 0 | | 4 | 0 | 3 | 5 | 4 | 5 | 5 | 4 | | 4 |
| | 1.25 | Post | 0 | 0 | 0 | 3 | 0 | 0 | 0 | 1 | 1 | 0 | 0 | 2 | 0 | | 0 | 1 | 0 | 0 | 0 | 0 | 0 | 1 | 0 | 0 | 0 |
| 37 | 1.25 | Pre | 4 | 2 | 3 | 4 | 5 | 5 | 5 | 3 | 3 | 3 | 3 | 3 | 5 | 3 | | 3 | 3 | 5 | 5 | 5 | 5 | 5 | 4 | | 5 |
| | 1.25 | Post | | | | | | | | | | | | | | | | | | | | | | | | | |

TABLE 1

| COMPOUND (EXAMPLE NO. | RATE OF APPLICATION kg/ha | PRE- OR POST-EMERGENCE APPLICATION | TEST PLANTS | | | | | | | | | | | | | | | | | | | | | | | | |
|---|---|---|---|---|---|---|---|---|---|---|---|---|---|---|---|---|---|---|---|---|---|---|---|---|---|---|---|
| | | | Sb | Rp | Ct | Sy | Mz | Ww | Rc | Bd | Ip | Am | Pl | Ca | Ga | Xa | Xs | Ab | Co | Av | Dg | Al | St | Ec | Sh | Ag | Cn |
| 40 | 1.25 | Pre | 4 | 4 | 4 | 4 | 5 | 5 | 5 | 4 | 4 | - | 4 | 3 | 5 | 4 | - | 4 | 3 | 4 | 5 | 5 | 5 | 5 | 5 | - | 5 |
| | 1.25 | Post | 3 | 2 | 3 | 3 | 4 | 2 | 2 | 3 | 3 | 2 | 3 | 3 | 2 | - | 3 | 4 | 3 | 3 | 4 | 3 | 4 | 4 | 4 | 3 | 2 |
| 41 | 0.25 | Pre | 4 | 4 | 4 | 4 | 4 | 4 | 4 | 4 | 0 | 4 | 4 | 4 | 4 | 4 | - | 4 | 4 | 4 | 5 | 5 | 5 | 5 | 4 | 5 | 5 |
| | 0.25 | Post | 1 | 0 | 1 | 3 | 3 | 3 | 0 | 4 | 1 | 3 | 3 | 4 | 3 | - | 1 | 3 | 0 | 2 | 1 | 2 | 4 | 3 | 3 | 2 | 0 |
| 42 | 0.25 | Pre | 4 | 2 | 2 | 4 | 5 | 5 | 5 | 4 | 0 | 4 | 4 | 4 | 4 | 4 | | 4 | 4 | 5 | 5 | 5 | 5 | 5 | 5 | | 5 |
| | 0.25 | Post | 2 | 0 | 1 | 3 | 4 | 3 | 0 | 3 | 2 | 2 | 1 | 4 | 2 | | 1 | 3 | 0 | 2 | 3 | 2 | 3 | 3 | 4 | 2 | 2 |
| 43 | 0.25 | Pre | 4 | 4 | 4 | 4 | 4 | 5 | 5 | 4 | 0 | 4 | 4 | 4 | - | 2 | - | 4 | 3 | 5 | 5 | 5 | 5 | 5 | 4 | 5 | 5 |
| | 0.25 | Post | 1 | 1 | 2 | 3 | 3 | 3 | 1 | 4 | 0 | 3 | 3 | 4 | 3 | - | 1 | 4 | 2 | 4 | 4 | 3 | 4 | 3 | 4 | 4 | 2 |
| 44 | 0.25 | Pre | 4 | 2 | 2 | 3 | 5 | 5 | 5 | 4 | 0 | 4 | 4 | 4 | 4 | 2 | | 4 | 4 | 5 | 5 | 5 | 5 | 5 | 4 | | 5 |
| | 0.25 | Post | 1 | 0 | 1 | 2 | 4 | 1 | 1 | 2 | 2 | 0 | 1 | 4 | 0 | | 1 | 2 | 0 | 2 | 0 | 3 | 2 | 3 | 1 | 0 | 0 |
| 45 | 0.25 | Pre | 4 | 3 | 3 | 3 | 4 | 5 | 4 | 1 | 1 | 0 | 2 | 2 | 2 | 0 | | 0 | 4 | 5 | 5 | 5 | 5 | 5 | 4 | | 5 |
| | 0.25 | Post | 0 | 1 | 0 | 4 | 3 | 0 | 0 | 2 | 1 | 3 | 0 | 4 | 0 | | 1 | 0 | 0 | 0 | 0 | 0 | 3 | 3 | 1 | 0 | 0 |

**TABLE 1**

| COMPOUND (EXAMPLE NO. | RATE OF APPLICATION kg/ha | PRE- OR POST-EMERGENCE APPLICATION | TEST PLANTS Sb | Rp | Ct | Sy | Mz | Ww | Rc | Bd | Ip | Am | Pl | Ca | Ga | Xa | Xs | Ab | Co | Av | Dg | Al | St | Ec | Sh | Ag | Cn |
|---|---|---|---|---|---|---|---|---|---|---|---|---|---|---|---|---|---|---|---|---|---|---|---|---|---|---|---|
| 47.1 | 1.25 | Pre | 4 | 4 | 4 | 4 | 5 | 5 | 5 | 4 | 4 | - | 4 | 3 | 5 | 4 | - | 4 | 3 | 4 | 5 | 4 | 5 | 5 | 5 | - | 5 |
| | 1.25 | Post | 3 | 3 | 4 | 3 | 4 | 3 | 3 | 3 | 3 | 3 | 3 | 3 | 3 | - | 3 | 4 | 3 | 3 | 4 | 3 | 4 | 4 | 5 | 3 | 3 |
| 47.2 | 1.25 | Pre | 4 | 4 | 4 | 4 | 5 | 5 | 5 | 4 | 3 | 3 | 4 | 4 | 5 | 4 | - | 4 | 3 | 4 | 5 | 5 | 5 | 5 | 5 | - | 5 |
| | 1.25 | Post | 3 | 3 | 2 | 4 | 3 | 2 | 2 | 3 | 3 | 2 | 3 | 3 | 1 | - | 3 | 4 | 3 | 4 | 4 | 4 | 4 | 4 | 5 | 3 | 2 |
| 47.3 | 1.25 | Pre | 4 | 4 | 4 | 4 | 5 | 5 | 5 | 4 | 3 | - | 3 | 2 | 5 | 3 | - | 4 | 3 | 4 | 5 | 4 | 5 | 5 | 5 | - | 5 |
| | 1.25 | Post | 3 | 2 | 2 | 3 | 3 | 3 | 1 | 3 | 3 | 3 | 2 | 3 | 1 | - | 2 | 3 | 3 | 3 | 4 | 3 | 4 | 3 | 4 | 3 | 2 |
| 47.4 | 1.25 | Pre | 3 | 1 | 2 | 0 | 4 | 4 | 5 | 3 | 0 | 0 | 2 | 3 | 5 | 0 | - | 4 | 0 | 4 | 4 | 4 | 4 | 4 | 2 | - | - |
| | 1.25 | Post | 0 | 1 | 0 | 3 | 2 | 0 | 0 | 1 | 0 | 0 | 0 | 2 | 0 | - | 1 | 0 | 3 | 0 | 0 | 0 | 0 | 0 | 0 | 0 | 0 |
| 47.5 | 1.25 | Pre | 4 | 4 | 0 | 5 | 5 | 5 | 5 | 4 | 1 | 3 | 4 | 2 | 5 | 4 | - | 4 | 1 | 5 | 5 | 5 | 5 | 5 | 5 | - | - |
| | 1.25 | Post | 0 | 4 | 0 | 3 | 4 | 3 | 0 | 3 | 2 | 1 | 1 | 4 | 0 | - | 3 | 2 | 3 | 2 | 4 | 2 | 3 | 3 | 3 | 1 | 1 |
| 47.6 | 1.25 | Pre | 4 | 4 | 0 | 4 | 5 | 5 | 5 | 4 | 1 | 0 | 3 | 2 | 5 | 4 | - | 4 | 0 | 5 | 5 | 5 | 5 | 5 | 5 | - | - |
| | 1.25 | Post | 0 | 2 | 1 | 4 | 4 | 2 | 0 | 4 | 2 | 1 | 1 | 3 | 0 | - | 4 | 3 | 2 | 2 | 4 | 2 | 2 | 4 | 2 | 1 | 1 |

**TABLE 1**

| COMPOUND (EXAMPLE NO. | RATE OF APPLICATION kg/ha | PRE- OR POST-EMERGENCE APPLICATION | TEST PLANTS | | | | | | | | | | | | | | | | | | | | | | | | |
|---|---|---|---|---|---|---|---|---|---|---|---|---|---|---|---|---|---|---|---|---|---|---|---|---|---|---|---|
| | | | Sb | Rp | Ct | Sy | Mz | Ww | Rc | Bd | Ip | Am | Pl | Ca | Ga | Xa | Xs | Ab | Co | Av | Dg | Al | St | Ec | Sh | Ag | Cn |
| 47.7 | 1.25 | Pre | 3 | 3 | 3 | 4 | 4 | 5 | 5 | 3 | 0 | 3 | 3 | 3 | 4 | 3 | - | 3 | 4 | 3 | 5 | 4 | 5 | 5 | 5 | - | 5 |
| | 1.25 | Post | 2 | 2 | 0 | 2 | 3 | 3 | 1 | 2 | 3 | 3 | 1 | 3 | 2 | - | 1 | 3 | 1 | 3 | 3 | 2 | 4 | 4 | 3 | 2 | 1 |
| 47.8 | 1.25 | Pre | 3 | 3 | 2 | 3 | 3 | 3 | 2 | 3 | 0 | 3 | 3 | 3 | 4 | 3 | - | 3 | 1 | 3 | 5 | 3 | 5 | 5 | 4 | - | 4 |
| | 1.25 | Post | 1 | 2 | 2 | 2 | 2 | 1 | 1 | 3 | 1 | 5 | 1 | 2 | 1 | - | 1 | 2 | 0 | 1 | 0 | 0 | 3 | 3 | 1 | 1 | 1 |
| 47.9 | 1.25 | Pre | 3 | 2 | 3 | 3 | 3 | 4 | 5 | 3 | 0 | 3 | 3 | 3 | 4 | 3 | - | 3 | 3 | 3 | 5 | 2 | 4 | 4 | 4 | - | 5 |
| | 1.25 | Post | 0 | 0 | 1 | 2 | 2 | 2 | 1 | 3 | 2 | 3 | 2 | 2 | 2 | - | 1 | 2 | 0 | 2 | 3 | 1 | 3 | 3 | 0 | 2 | 1 |
| 47.10 | 1.25 | Pre | 2 | 1 | 1 | 0 | 0 | 1 | 2 | 2 | 0 | 2 | 3 | 3 | 2 | 1 | - | 2 | 2 | 0 | 3 | 1 | 2 | 3 | 1 | - | 3 |
| | 1.25 | Post | 0 | 0 | 1 | 2 | 1 | 0 | 0 | 2 | 1 | 3 | 1 | 2 | 1 | - | 0 | 2 | 0 | 0 | 0 | 0 | 2 | 2 | 1 | 0 | 0 |
| 47.11 | 1.25 | Pre | 4 | 4 | 0 | 4 | 4 | 5 | 5 | 4 | 0 | 3 | 4 | 2 | 5 | 1 | - | 4 | 0 | 4 | 5 | 4 | 5 | 5 | 5 | - | - |
| | 1.25 | Post | 0 | 0 | 0 | 3 | 4 | 3 | 0 | 3 | 1 | 0 | 0 | 2 | 0 | - | 1 | 2 | 1 | 2 | 4 | 2 | 3 | 5 | 2 | 0 | 1 |
| 48 | 0.05 | Pre | 2 | 1 | 4 | 2 | 5 | 5 | 5 | - | - | - | - | - | - | - | - | - | - | 5 | 5 | 5 | 5 | 5 | 4 | 5 | 5 |
| | 0.05 | Post | | | | | | | | | | | | | | | | | | | | | | | | | |

**TABLE 1**

| COMPOUND (EXAMPLE NO. | RATE OF APPLICATION kg/ha | PRE- OR POST-EMERGENCE APPLICATION | TEST PLANTS | | | | | | | | | | | | | | | | | | | | | | | | |
|---|---|---|---|---|---|---|---|---|---|---|---|---|---|---|---|---|---|---|---|---|---|---|---|---|---|---|---|
| | | | Sb | Rp | Ct | Sy | Mz | Ww | Rc | Bd | Ip | Am | Pl | Ca | Ga | Xa | Xs | Ab | Co | Av | Dg | Al | St | Ec | Sh | Ag | Cn |
| 50 | 1.25 | Pre | 4 | 4 | 4 | 4 | 5 | 5 | 5 | 4 | 4 | 4 | 4 | 4 | 5 | 4 | - | 4 | 4 | 5 | 5 | 5 | 5 | 5 | 5 | - | 5 |
| | 1.25 | Post | 3 | 2 | 2 | 3 | 3 | 3 | 2 | 4 | 3 | 4 | 5 | 4 | 2 | - | 3 | 3 | 2 | 4 | 4 | 4 | 4 | 4 | 4 | 3 | 3 |
| 51 | 0.25 | Pre | 3 | 2 | 1 | 3 | 4 | 5 | 5 | 3 | 2 | 3 | 3 | 3 | 4 | 3 | - | 3 | 3 | 4 | 5 | 5 | 5 | 5 | 4 | - | 5 |
| | 0.25 | Post | | | | | | | | | | | | | | | | | | | | | | | | | |
| 52 | 0.25 | Pre | 2 | 2 | 3 | 2 | 2 | 2 | 1 | 3 | 0 | 3 | 3 | 3 | 3 | 0 | - | 3 | 0 | 0 | 3 | 2 | 3 | 4 | 0 | - | 0 |
| | 0.25 | Post | 0 | 0 | 0 | 1 | 1 | 0 | 0 | 2 | 2 | 2 | 0 | 1 | 0 | - | 2 | 2 | 1 | 0 | 0 | 0 | 0 | 2 | 0 | 0 | 0 |
| 53 | 0.25 | Pre | 3 | 0 | 3 | 3 | 5 | 2 | 5 | 2 | 2 | 2 | 4 | 2 | 3 | 0 | - | 3 | 0 | 2 | 5 | 4 | 5 | 5 | 3 | - | 3 |
| (isomer A) | 0.25 | Post | 0 | 0 | 0 | 2 | 2 | 1 | 0 | 2 | 2 | 2 | 1 | 1 | 3 | - | 3 | 3 | 1 | 0 | 2 | 0 | 0 | 2 | 2 | 2 | 0 |
| 56 | 1.25 | Pre | 4 | 2 | 4 | 1 | 2 | 0 | 1 | 4 | 1 | 4 | 4 | 4 | 4 | 3 | | 4 | 3 | 1 | 3 | 2 | 4 | 4 | 1 | 1 | 4 |
| | 1.25 | Post | 2 | 0 | 2 | 3 | 1 | 1 | 0 | 3 | 1 | 3 | 2 | 3 | 1 | | 0 | 2 | 1 | 0 | 0 | 1 | 1 | 1 | 1 | 0 | 0 |

The biotest results demonstrate that compounds and compositions of the present invention possess valuable and interesting herbicidal and/or plant growth regulatory properties, particularly against monocotyledon weed species.

The following non-limiting Examples are included as further illustration of the present invention. In each example optical rotations were recorded in chloroform solution at a concentration of 0.5 - 3 g/100 ml at ambient temperatures (approx 20°) on a Perkin Elmer 241 Polarimeter. $^{13}C$-nmr data were recorded in $CDCl_3$ at 20 MHz on a Varian FT-80 spectrometer. $^{1}H$-nmr data were recorded at 270 MHz, unless otherwise specified, in $CDCl_3$. Chemical shifts are given in ppm downfield from tetramethylsilane, and coupling constants (J) are in Hertz. h = hours; and psi = pounds per square inch (1 psi = 6.89 kpa). Flash chromatography was conducted according to W.C. Still et al., J.Org.Chem., (1978) 43, 2923-2925 on silica gel using mixtures of ethyl acetate and light petroleum as eluant. Thin layer chromatography (tlc) was performed on Merck Silica gel $GF_{254}$ plates.

## Specification of Molecular Chirality

The R and S nomenclature used to define the absolute stereochemistry on the examples and Preparative Examples was determined using the rules set out in papers by R.S. Cahn, C. Ingold and V. Prelog, Angew. Chem. Int. Edn. Engl., 5 (1966) 385-415 and V. Prelog and G. Helmchen, Angew. Chem. Int. Edn. Engl., 21 (1982) 567-583.

## General Preparative Example 1

### 0-Alkylations

To a stirred solution of the alcohol in dry tetrahydrofuran (10 ml/g of carbohydrate alcohol) at 0°C under an inert atmosphere, sodium hydride (60% or 80% dispersion in oil, 1.1 eq or more) was added slowly. When effervescence had ceased the appropriate alkyl halide (1.1 eq or more) was added portionwise and the resulting mixture was stirred at 0°C for 2 h and then at room temperature or 50°C if necessary until the reaction was complete (tlc evidence). Catalytic amounts of tetrabutylammonium iodide were added in some cases where reaction was slow. Methylation was thus effected using methyl iodide, ethylation using ethyl bromide in the presence of catalytic tetrabutylammonium iodide. Ether or preferably chloroform (20 ml/g) was added and the mixture was washed with water (30 ml/g). The aqueous phase was back extracted with ether or preferably chloroform (5 ml/g) and the combined organic extract was dried ($MgSO_4$ or $Na_2SO_4$) before being concentrated to a thick syrup. Purification was effected by flash chromatography on silica gel.

## General Preparative Example 2

### Swern Oxidations

(See A J Mancuso and D Swern, Synthesis, 1981, 179.) A stirred solution of oxalyl chloride (1.3 eq. per mole of alcohol) in dichloromethane was cooled under argon to -70°C and a solution of dimethylsulphoxide (2.6 eq. per mole of alcohol) in dichloromethane was added dropwise. Five minutes after the addition was complete a solution of the alcohol to be oxidized in dichloromethane was added slowly and the resulting mixture was stirred at -70°C for 30 min. Triethylamine (5 eq. per mole of alcohol) was then added and the mixture was stirred and allowed to warm to about 0-10°C, and then washed with water, 2 M aqueous hydrochloric acid, saturated aqueous sodium hydrogen carbonate and then dried ($MgSO_4$ or $Na_2SO_4$) and the solvent was removed under reduced pressure. The reaction may alternatively be conducted in tetrahydrofuran as solvent, in which case ether or chloroform may be added at the completion of the reaction to facilitate the separation of an organic phase.

Preparative Example 1

Substituted methanols

The following compounds were prepared for use in Example 1:

1 Tetrahydrothiopyran-4-methanol: tetrahydrothiopyran-4-one was converted to tetrahydrothiopyran-4-carboxaldehyde by Wittig methylenation - acid hydrolysis according to P.A. Aristoff (EP 0159784). The aldehyde (2.55 g) was reduced with sodium borohydride (0.4 g, 0.5 eq.) in ethanol, and after 20 min the reaction was quenched with excess acetone and the pH adjusted to 6 with acid resin. The mixture was filtered, concentrated, and the resulting syrup purified by flash chromatography to yield the title alcohol as a liquid (2.4 g), a single component by g.c.-m.s.

2 1-Cyclohexene-1-methanol: methyl 1-cyclohexene-1-carboxylate (10 g) in diethyl ether was added dropwise to a stirred solution of lithium aluminium hydride (3.2 g) in diethyl ether. After 30 min, saturated aqueous ammonium chloride then dilute hydrochloric acid were added, and the mixture was extracted with chloroform (x 3). The combined organic phase was dried ($Na_2SO_4$) and evaporated to yield the title alcohol as a syrup (9 g), single component by g.c.-m.s.

3 1-methylcyclohexane-1-methanol: 1-methylcyclohexane-1-carboxylic acid (10 g) in tetrahydrofuran was added dropwise to a stirred solution of lithium aluminium hydride (2.5 g) in tetrahydrofuran. After 1 h, saturated aqueous ammonium chloride then dilute hydrochloric acid were added, and the mixture was extracted with chloroform (x 3). The combined organic phase was dried ($Na_2SO_4$) and evaporated to yield the title alcohol as a colourless syrup (8 g), single component by g.c.-m.s.

4 Tetrahydrothiophene-2-methanol: the title alcohol was prepared from tetrahydrothiophene-2-carboxylic acid (J.T. Wrobel and E. Hejchman, Synthesis 1987, 452) by reduction with lithium aluminium hydride in ether (S. Ikegami, Tetrahedron 1974, 30, 2087).

5 (RS)-2-Cyclohexene-1-methanol: cyclohexenone (10 g) was added dropwise to a solution of lithium aluminium hydride (2 g) in diethyl ether (50 ml) and the mixture was stirred at ambient temperature for 2 h. Saturated ammonium chloride solution (150 ml) was added, followed by hydrochloric acid (2 M) to dissolve the grey precipitate, and the mixture was extracted with chloroform (x 3), dried ($MgSO_4$) and evaporated to a syrup. Fractional distillation under reduced pressure gave 2-cyclohexen-1-ol as a liquid, a single component by g.c.-m.s. The resulting 2-cyclohexen-1-ol (4 g) was converted to the title alcohol by the method of W. C. Still et al., (J. Am. Chem. Soc., 100 (1978) 1927-8, and after purification by flash crhomatography (silica; eluant ethyl acetate/light petroleum ether, 1:4) was obtained as a colourless liquid (2.5 g).

Preparative Example 2

De-O-benzylation

A solution 1R,2S,4R-2-benzyloxy-5-butyl-4-methoxy-6,8-dioxabicyclo[3.2.1]octane or its 5-methyl analogue (see Examples 15.4 and 15.1, respectively in EP 0229034A) in ethanol (20 ml/g) containing palladium hydroxide on carbon (20%; 0.05 g/g of benzyl ether) was hydrogenolysed at atmospheric pressure. When uptake of hydrogen was complete, the solution was filtered and evaporated to yield the corresponding 2-hydroxy-derivative in pure form. In this way the following compounds were prepared:

1 (1R,2S,4R)-5-butyl-2-hydroxy-4-methoxy-6,8-dioxabicyclo[3.2.l]octane, low melting crystals; $^1H$-nmr data (200 MHz) : 4.53 (m,H-1), 3.84 (dd,H-7exo, $J_{1,7exo}$ 5.1, $J_{7exo,7endo}$ 12.7), 3.74 (d,H-7endo), 3.72 (m,H-2), 3.41 (s,OMe), 3.13 (m,H-4), 2.05 (dm,H-3e, $J_{3a,3e}$ 15.3), 1.6-2.0 (m,H-3a and 5-$CH_2$Pr), 1.34 ($m,CH_2CH_2$) and 0.92 (t,C-$CH_3$).

2 (1R,2S,4R)-2-hydroxy-4-methoxy-5-methyl-6,8-dioxabicyclo[3.2.1]octane, white crystals; $^{13}C$-nmr data: 106.6 (C-5), 79.1 and 78.7 (C-1 and C-4), 66.3 (C-2), 66.0 (C-7), 57.6 (OMe), 26.8 (C-3) and 20.6 (C-$CH_3$).

Preparative Example 3

4-0-Benzyl-6-0-tert-butyldimethylsilyl-3-deoxy-2-0-methyl-D-ribohexono-l,5-lactone

A solution of 4-0-benzyl-3-deoxy-2-0-methyl-D-ribo-hexopyranose (prepared as detailed in Example 15 of EP 0229034A) (5.2 g) in dichloromethane (50 ml) containing triethylamine (5.4 ml), p-dimethylaminopyridine (0.12 g) and tert-butyldimethylsilyl chloride (3.0 g), was stirred at ambient temperature overnight. If necessary, more of the silylating agent can be added at this stage to ensure complete reaction of the starting material. When the reaction is essentially complete (tlc evidence) the solution is washed with water, 2 M aqueous hydrochloric acid and then saturated aqueous sodium hydrogen carbonate. Conventional processing gave a syrup which was subjected to Swern oxidation in dichloromethane (See General Preparative Example 2). Normal processing followed by chromatography on silica gel gave syrupy title lactone (4.8 g, 65%), $[\alpha]_D$ + 74.4°; $^{13}$C-nmr data: 170.2 (C-1), 137.4, 128.4, 127.9, 127.7 (aromatics), 82.2, 74.2 and 69.7 (C-2,-4, and -5), 71.6 (ArCH$_2$O), 62.1 (C-6), 58.5 (OCH$_3$), 31.9 (C-3), 25.8 [(CH$_3$ C], 18.2 [(CH$_3$)$_3$ C], -5.4 and -5.6 [Si(CH$_3$)2].

Preparative Example 4

(1R,2S,4R)-2-Benzyloxy-4-methoxy-5-(2-propen-1-yl)-6,8-dioxabicyclo[3.2.1]octane

Allylmagnesium chloride (5.5 ml, 2M solution in tetrahydrofuran, 11 mmoles) was added dropwise to a stirred solution of 4-0-benzyl-6-0-tert-butyldimethylsilyl-3-deoxy-2-0-methyl-D-ribohexono-1,5-lactone (see Preparative Example 3) (4.0 g) in dry tetrahydrofuran (30 ml) maintained at -70° C in an argon atmosphere. The resulting solution was stirred at -70° C for 1 h, and then acetic acid (1 ml) was added. The solution was diluted with water, extracted twice with chloroform and the combined extracts were washed with a saturated brine solution. After removal of the dried solvent, the resultant syrup was dissolved in an acetic acid - water mixture (50 ml, 9:1 v/v) and the solution was heated at 80° C for 3 h. The solution was concentrated and the residue was chromatographed on silica gel to give the syrupy title compound (2.66 g, 87%), $[\alpha]_D$ -24.1°; $^{13}$C-nmr data: 138.3, 128.3, 127.6, 127.5 (aromatics), 132.2 (CH$_2$CH = CH$_2$), 118.3 (CH$_2$CH = CH$_2$), 107.8 (C-5), 76.0, 75.7 and 71.4 (C-1,-2, and -4), 70.3 (ArCH$_2$O), 66.3 (C-7), 57.3 (OCH$_3$), 38.0 (CH$_2$CH = CH$_2$) and 23.8 (C-3).

Preparative Example 5

(1R,2SS,4R)-2-Benzyloxy-5-(3-fluoropropyl)-4-methoxy-6,8-dioxabicyclo[3.2.1]octane

Borane-tetrahydrofuran complex (10 ml, 1.0 M in tetrahydrofuran) was added dropwise to a cold (0 °C) stirred solution of (1R,2S,4R)-2-benzyloxy-4-methoxy-5-(2-propen-1-yl)-6,8-dioxabicyclo[3.2.1]octane (see Preparative Example 4) (1.35 g) in tetrahydrofuran (50 ml). The solution was stored at 4° C overnight and then allowed to warm to room temperature. Further borane-tetrahydrofuran complex (2 ml) was added and the solution was stirred another 8 h. Sodium hydroxide (20 ml, 1 M aqueous) was added followed by 30% hydrogen peroxide solution (2 ml). The mixture was stirred for 0.5 h and then partitioned between chloroform and water. Normal processing followed by chromatography on silica gel afforded syrupy (1R,2S,4R)-2-benzyloxy-5-(3-hydroxypropyl)-4-methoxy-6,8-dioxabicyclo[3.2.1]octane (1.18 g, 82%). This material was dissolved in dry dichloromethane (30 ml) containing p-toluenesulphonyl chloride (1 g), triethylamine (1.5 ml) and p-dimethylaminopyridine (0.05 g), and the solution was heated under reflux for 1 h. Conventional processing followed by chromatography on silica gel gave 1.46 g of tosylate. Half of this material was added to a solution of tetrabutylammonium fluoride in dry acetonitrile (50 ml). [The tetrabutylammonium fluoride was dried by taking 10 ml of a 1.0 M solution in tetrahydrofuran, and co-evaporating four times with toluene and once with toluene/acetonitrile (1:1 v/v).] The solution was heated under reflux for 2 h. Removal of the solvent and chromatography of the residue on silica gel afforded syrupy title compound (0.443 g), $[\alpha]_D$ -41.6°; $^{13}$C-nmr data: 138.3, 128.3, 127.6, 127.5 (aromatics), 108.0 (C-5), 84.2 (d, $J_{C,F}$ 164 Hz, CH$_2$CH$_2$CH$_2$F), 76.8, 76.1 and 71.4 (C-1,-2 and -4), 70.4 (ArCH$_2$O), 66.3 (C-7), 57.4 (OCH$_3$), 28.5 (d, $J_{C,F}$ 6Hz, CH$_2$CH$_2$CH$_2$F), 23.9 (C-3) and 23.3 (d, $J_{C,F}$ 20Hz, CH$_2$CH$_2$CH$_2$F).

Preparative Example 6

(1R,2S,4R)-2-Benzyloxy-5-(2-fluoroethyl)-4-methoxy-6,8-dioxabicyclo[3.2.1]octane

Sodium periodate (11.4 g) and then osmium tetroxide (0.1 g in water, 10 ml) were added to a solution of (1R,2S,4R)-2-benzyloxy-4-methoxy-5-(2-propen-1-yl)-6,8-dioxabicyclo[3.2.1]octane (7.75 g) (see Preparative Example 4) in ethanol/water (160 ml, 3:1 v/v). The mixture was stirred at room temperature for 3 h. Chloroform was then added and the mixture was filtered through a pad of celite. The filtrate was washed with water and the aqueous phase extracted again with chloroform. The combined chloroform solutions were concentrated to a syrup which was dissolved in ethanol. Sodium borohydride (1 g) was added with stirring to this solution and after 0.5 h the solution was neutralised by dropwise addition of acetic acid, and then the solvents were removed. Ethyl acetate was added to the residue and the resulting mixture was filtered through a pad of silica gel. The solids were washed with ethyl acetate and the combined filtrates were concentrated to dryness. The residue was dissolved in dry dichloromethane (50 ml) containing p-toluenesulphonyl chloride (4.5 g), triethylamine (7.5 ml) and p-dimethylaminopyridine (0.25 g). This solution was heated under reflux for 2 h. Conventional processing followed by chromatography on silica gel afforded a syrup (8.4 g) of (1R,2S,4R)-2-benzyloxy-4-methoxy-5-[2-(p-toluenesulphonyloxy)ethyl]-6,8-dioxabicyclo-[3.2.1]octane; $^{13}C$-nmr data: 128.7, 128.3, 127.8, 127.6 and 127.5 (aromatics), 106.7 (C-5), 76.6, 76.2 and 71.1 (C-1,-2 and -4), 70.4 ($Ar\underline{C}H_2O$), 66.2 and 66.0 (C-7 and $CH_2\underline{C}H_2OTs$), 57.3 ($OCH_3$), 32.2 ($\underline{C}H_2CH_2OTs$), 23.4 (C-3) and 21.5 ($Ar\underline{C}H_3$). A solution of this material (5.0 g) in dry acetonitrile (50 ml) was added to a solution of dry tetrabutylammonium fluoride in acetonitrile (250 ml). [The tetrabutylammonium fluoride (35 ml of a 1.0 M solution in tetrahydrofuran) was added to 300 ml of toluene/acetonitrile (1:1 v/v) and the solution was evaporated to dryness. Dry toluene (300 ml) was added and then evaported to dryness twice and then dry toluene/acetonitrile (300 ml, 1:1 v/v) was added and this solution was also evaporated to dryness. The residue of dry tetrabutylammonium fluoride was used as stated. ] This solution was heated under reflux for 0.1 h and then the cooled solution was made 20% aqueous. At this stage the reaction contains the chromatographically similar title compound and the 5-vinyl-analogue. N-Bromosuccinimide (5 g) was added and the solution was stirred at ambient temperature for 2 h. Most of the solvent was removed, and the residue was partitioned between ether and water. The organic layer was washed further with aqueous sodium thiosulphate, water, then dried and concentrated. Fractionation ofthe residue on a column of silica gel afforded the syrupy title compound (1.30 g), $[\alpha]_D$ -46.4°; $^{13}C$-nmr data: 138.3, 128,3, 127.7 and 127.6 (aromatics), 107.1 (d, J = 6 Hz, C-5), 79.8 (d, J = 163 Hz, $CH_2\underline{C}H_2F$), 76.5 (C-4), 76.1 (C-1), 71.2 (C-2), 70.4 ($Ar\underline{C}H_2O$), 66.3 (C-7), 57.3 ($OCH_3$), 33.9 (d, J = 20 Hz, $\underline{C}H_2CH_2F$) and 23.5 (C-3).

Preparative Example 7

2-0-Alkyl-6-0-tert-butyldimethylsilyl-4-0-cyclohexanemethyl-3-deoxy-D-ribo-hexono-1,5-lactones

(1R,2S,4R)-2-Cyclohexanemethyloxy-4-methoxy-6,8-dioxabicyclo[3.2.1]octane (Example 3.1) and its 4-ethoxy-analogue (Example 1.15) were separately converted into the corresponding 2-0-alkyl-4-0-cyclohexanemethyl-3-deoxy-D-ribohexopyranoses by sequential acetolysis ($Ac_2O$-$BF_3$.$OEt_2$) then de-O-acetylation (MeONa-MeOH) following the procedures detailed in Example 5. The diols so obtained were separately converted to the title lactones by the procedure detailed in Preparative Example 3, but using a 2-0-alkyl-4-0-cyclohexanemethyl-3-deoxy- D-ribo-hexopyranose in place of 4-0-benzyl-3-deoxy-2-0-methyl-D-ribo-hexopyranose. In this way the following compounds were prepared

1 6-0-tert-butyldimethylsilyl-4-0-cyclohexanemethyl-3-deoxy-2-0-methyl-D-ribo-hexono-1,5-lactone, as a syrup in 76% yield.

2 6-0-tert-butyldimethylsilyl-4-0-cyclohexanemethyl-3-deoxy-2-0-ethyl-D-ribo-hexono-1,5-lactone, as a syrup, in 70% yield.

Preparative Example 8

Methyl 4-0-benzyl-3-deoxy-2-0-methyl-α-D-ribo-hexopyranoside

To a stirred solution of methyl 4,6-0-benzylidene-3-deoxy-2-0-methyl-α-D-ribo-hexopyranoside (Preparative Example 5 in EP 0229034A) (19.3 g) in dry ether/dichloromethane (400 ml, 1:1 v/v), lithium aluminium hydride (4.2 g) was added in portions. The mixture was heated to reflux temperature, a solution of aluminium trichloride (14.5 g) in ether (150 ml) was added slowly and heating, under reflux, was continued for 15 min. After cooling to 0° C, aqueous ammonium chloride was added dropwise until all of the lithium aluminium hydride reacted. The product was partitioned between dichloromethane and aqueous hydrochloric acid (1% v/v). The organic phase was washed with water and aqueous sodium hydrogen carbonate, dried ($Na_2SO_4$) and evaporated to a yellow syrup. Purification by flash chromatography gave a yellow oil (16.7 g, 86%) consisting (by $^1$H-nmr) of the title compound and its 6-0-benzyl isomer in a ca 4:1 ratio; $^{13}$C-nmr data for the title compound: 137.9, 128.4, 127.7 and 127.6 (aromatic), 96.6 (C-1), 76.0, 72.1 and 71.0 (C-2, C-4 and C-5), 70.6 ($PhCH_2O$), 62.1 (C-6), 56.6 and 54.8 ($CH_3O$) and 29.1 (C-3).

Preparative Example 9

Methyl 4-0-benzyl-3-deoxy-2-0-methyl-6-(S) and 6-(R)-C-vinyl-α-D-ribo-hexopyranoside

Methyl 4-0-benzyl-3-deoxy-2-0-methyl-α-D-ribo-hexopyranoside (see Preparative Example 8) (3.6 g) in dry tetrahydrofuran (350 ml) was oxidised by the Swern Oxidation procedure (see General Preparative Example 2). After the triethylamine was added, the solution was allowed to warm to 0° C briefly, and was then recooled to -60° C and maintained at this temperature while vinylmagnesium bromide (50 ml, 1.0 M in tetrahydrofuran) was added. After 0.5 h, 2 M aqueous hydrochloric acid was added and the mixture was extracted (x 2) with chloroform. Processing of the organic extracts afforded a syrup (4.3 g) which was fractionated by chromatography on silica gel to give separately in a total yield of 3.78 g (96%), the 6-(S)- and then the 6-(R)-vinyl compounds in a ratio of 3:1. Methyl 4-0-benzyl-3-deoxy-2-0-methyl-6-(S)-C-vinyl-α-D-ribo-hexopyranoside; $^{13}$C-nmr data: 138.4 ($CH=CH_2$), 137.9, 128.4, 127.8 and 127.7 (aromatics), 115.3 ($CH=CH_2$), 96.7 (C-1), 75.9, 72.6, 71.9 and 70.4 (C-2,-4,-5, and -6) 70.9 ($OCH_2Ar$), 56.6 and 54.8 ($OCH_3$) and 29.3 (C-3). Methyl 4-0-benzyl-3-deoxy-2-0-methyl-6-(R)-C-vinyl-α-D-ribo-hexopyranoside; $^{13}$C-nmr data: 137.4, 128.4, 127.9 and 127.8 (aromatics), 136.4 ($CH=CH_2$), 116.7 ($CH=CH_2$), 96.5 (C-1), 75.9, 74.2, 73.3 and 72.4 (C-2,-4, -5 and -6), 70.1 ($OCH_2Ar$), 56.6 and 54.8 ($OCH_3$) and 29.0 (C-3).

Preparative Example 10

Methyl 2-0-benzyl-3-deoxy-4-0-methyl-DL-ribo-hexodialdopyranoside

A) Methyl 2-0-benzyl-3-deoxy-4-0-methyl-DL-ribo-hexopyranoside

A solution of (1RS,2SR,4RS)-4-benzyloxy-2-methoxy-6,8-dioxabicyclo[3.2.1.]octane (4.30 g), prepared as in Example 40, in methanol (50 ml), was warmed to 50° C and dry hydrogen chloride was bubbled into the solution for 30 min. Brine (100 ml) was then added, and the mixture was extracted with dichloromethane (2 x 50 ml). The combined dichloromethane extracts were dried ($MgSO_4$) and evaporated to leave a gum. This was chromatographed on silica gel (eluted with ethyl acetate/hexane mixtures) to give the title compound as a colourless oil (1.30 g), which was obtained as a 7:3 mixture of α - and β - anomers.

B) Methyl 2-0-benzyl-3-deoxy-4-0-methyl-DL-ribo-hexodialdopyranoside

A solution of dimethylsuphoxide (0.9 ml) in tetraydrofuran (5 ml) was cooled to -65° C under an argon atmosphere. Oxalyl chloride (0.89 ml) was added at such a rate that the temperature of the reaction mixture did not rise above -55° C. When effervescence had ceased, a solution of methyl 2-0-benzyl-3-deoxy-4-0-methyl-DL-ribo-hexopyranoside (1.27 g), prepared as in part A, in tetrahydrofuran (10 ml) was added at such a rate that the temperature of the reaction mixture did not rise above -55° C. The resultant yellow mixture was stirred for 5 min, then triethylamine (3.0 ml) was added dropwise, after which the mixture was allowed to warm to room temperature. After 1 h, saturated aqueous ammonium chloride (20 ml) was added and the mixture was extracted with diethyl ether (3 x 20 ml). The combined ether layers were washed with brine, dried ($MgSO_4$), and evaporated to leave an oil. This was chromatographed on silica gel (eluted with ethyl acetate/hexane mixtures) to give the crude title compound as syrup (0.72 g).

Preparative Example 11

Methyl 2-0-benzyl-3,7-dideoxy-4-0-methyl-DL-talo-heptopyranoside

A solution of methyl 2-0-benzyl-3-deoxy-4-0-methyl-DL-ribo-hexodialdopyranoside (0.72 g), prepared as in Preparative Example 10, in tetrahydrofuran (2 ml) was treated dropwise with a solution of methylmagnesium bromide in tetradyrofuran/toluene (1.5 M, 5 ml). The solution was stirred for 2 h then quenched with saturated aqueous ammonium chloride and the mixture was extracted thoroughly with diethyl ether. The ether extracts were washed with brine, dried ($MgSO_4$) and evaporated. The resulting colourless oil was subjected to preparative layer chromatography on silica gel plates, eluted with ethyl acetate/hexane (1:1) to give a mixture of isomers, predominantly the title compound (ca. 60% by gas chromatography) together with its DL-allo-stereoisomer (0.45 g).

Preparative Example 12

Methyl 3,7-dideoxy-4-0-methyl-2-0-(2-methylbenzyl)-DL-talo-heptopyranoside

A) Methyl 3-deoxy-4-0-methyl-2-0-(2-methylbenzyl)-DL-*ribo*-hexopyranoside

Hydrogen chloride gas was passed ito a solution of (1RS,2SR,4RS)-2-methoxy-4-(2-methylbenzyloxy)-6,8-dioxabicyclo[3.2.1]octane (4.00 g), prepared as in Example 41, in methanol (40 ml) for 1 min. The solution was then left to stand for 16 h, then heated under reflux for 30 min, cooled, and poured into water (100 ml). The mixture was extracted with dichloromethane (3 x 50 ml), and the combined organic layers were washed with brine, dried ($MgSO_4$) and evaporated to a pale yellow oil. Chromatography on silica gel eluted with ethyl acetate/hexane mixtures afforded the title compound as a colourless syrup (2.65 g) as a mixture of anomers.

B) Methyl 3,7-dideoxy-4-0-methyl-2-0-(2-methylbenzyl)-DL-talo-heptopyranoside

Dimethyl sulphoxide (0.63 ml) was added dropwise to a solution of oxalyl chloride (0.63 ml) in tetrahydrofuran at -65° C, then the temperature was allowed to rise to -50° C. When the effervescence had ceased, a solution of methyl 3-deoxy-4-0-methyl-2-0-(2-methylbenzyl)-DL-ribo-hexopyranoside (1.07 g), prepared as in part A, in tetrahydrofuran (5 ml) was added dropwise, and the temperature was allowed to rise to -10° C. The colourless solution was stirred at this temperature for 15 min, and was then cooled to -40° C and triethylamine (2.13 ml) was added dropwise. The resultant white suspension was stirred at -10° C for10 min, then cooled to -40° C and a solution of methylmagnesium bromide in tetrahydrofuran (1.5

M, 9.7 ml) was added at such a rate that the temperature did not rise above -30° C. The pale yellow mixture was then stirred at room temperature for 1 h, quenched with 2 M hydrochloric acid (20 ml). The organic phase was separated, and the aqueous phase was washed with diethyl ether (3 x 10 ml). The combined organic phases were dried ($MgSO_4$) and evaporated to an orange oil. Silica gel chromatography (eluted with ethyl acetate/hexane mixtures) afforded a mixture of isomers, predominantly the title compound together with its allo-steroisomer as a colourless oil (0.25 g). Further elution afforded recovered starting material (0.62 g).

Preparative Example 13

Methyl 2-0-benzyl-3,7-dideoxy-4-0-methyl-DL-ribo-heptopyranosid-6-ulose

Methyl 2-0-benzyl-3,7-dideoxy-4-0-methyl-DL-talo-heptopyranoside and its DL-allo-stereoisomer (0.35 g), prepared as in Preparative Example 11, was oxidized according to Swern (see General Preparative Example 2). The resulting crude reaction mixture was allowed to warm to room temperature and stirred for 2 h. Brine (10 ml) was added, and the mixture was extracted with ethyl acetate (2 x 5 ml). This was dried and evaporated to give the title compound as an oil (0.35 g).

Preparative Example 14

Methyl 2-0-benzyl-3,7-dideoxy-4-0-methyl-DL-allo-heptopyranoside and methyl 2-0-benzyl-3,7-dideoxy-4-0-methyl-DL-talo-heptopyranoside

A solution of methyl 2-0-benzyl-3,7-dideoxy-4-0-methyl-DL-ribo-heptopyranosid-6-ulose (200 mg), prepared as in Preparative Example 13, in methanol (2 ml) was treated portionwise with sodium borohydride (50 mg). The mixture was stirred for 30 min, then brine (5 ml) was added and the mixture was extracted with ethyl acetate (3 x 3 ml). The combined ethyl acetate layers were dried by filtration through phase separating paper, and evaporated to leave a colourless oil. Preparative layer chromatography on silica eluted with ethyl acetate/hexane (1:1) gave a mixture of the title compounds as a colourless oil (83 mg).

Preparative Example 15

Methyl 2-0-benzyl-3,7-dideoxy-4-0-methyl-6-C-methyl-DL-ribo-heptopyranoside

A solution of methyl 2-0-benzyl-3,7-dideoxy-4-0-methyl-DL-ribo-heptopryanosid-6-ulose (156 mg), prepared as in Preparative Example 13, in tetrahydrofuran (2 ml) was mixed with a solution of methylmagnesium bromide in tetrahydrofuran (1.5M, 1.2 ml). After 30 min the reaction was stopped by the addition of brine (4 ml) and neutralised with 2 M hydrochloric acid. The mixture was extracted with ethyl acetate (3 x 3 ml), and the combined ethyl acetate extracts were filtered through phase separating paper. Evaporation of the solvent left an oil which was subjected to preparative layer chromatography on silica gel, eluted with ethyl acetate/hexane (1:1) to give the syrupy title compound (54 mg) as a 4:1 mixture of its $\alpha$ - and $\beta$ - anomers.

Preparative Example 16

(1RS,2SR,4RS)-4-Hydroxy-2-methoxy-6,8-dioxabicyclo[3.2.1]octane

Trifluoroacetic acid (5 drops) was added to a solution of (1RS,2SR,4RS)-4-Benzyloxy-2-methoxy-6,8-dioxabicyclo[3.2.1]octane (7.00 g), prepared as in Example 40, in ethanol (50 ml). This mixture was hydrogenated over 5% palladium on carbon catalyst at a pressure of 150 p.s.i. at 40° C for 10 h. The mixture was filtered, and the filtrate evaporated to leave a pale green oil. Chromatography on silica gel, eluted with ethyl acetate/hexane (3:2) afforded the title compound as a colourless oil (3.90 g).

Preparative Example 17

(1R,2S,4R)-2-Benzyloxy-5-hydroxymethyl-4-methoxy-6,8-dioxabicyclo[3.2.1]octane

A) (1R,2S,4R)-2-Benzyloxy-5-(1′,3′-dithian-2′-yl)-4-methoxy-6,8-dioxabicyclo[3.2.1]octane

A mixture of 2-(4′-0-benzyl-3′-deoxy-5′,6′-0-isopropylidene-2′-0-methyl-D-allo-hexitol-1′-yl)dithiane and its altro-isomer (see Example 30, part A) (7.7 g) was oxidised according to General Preparative Example 2, but using trifluoroacetic anhydride (4.2 ml) instead of oxalyl chloride. The crude ketone thus obtained was stirred in aqueous acetic acid (70%, 180 ml) for 16 h at ambient temperature. The solvent was removed at 1 Torr/50° C and the oily residue was dissolved in toluene (300 ml) containing p-toluenesulphonic acid (0.5 g). After stirring at 50° C for 5 h, the cooled solution was neutralised with triethylamine and evaporated to a dark brown syrup. Flash chromatographic purification gave the title compound A (4.5 g, 68%) as white crystals which were recrystallised from light petroleum/ethyl acetate, m.p. 142-143° C, $[\alpha]_D$ -11.4°; $^{13}C$-nmr data: 138.1, 128.3, 127.6 and 127.5 (aromatic), 109.8 (C-5), 76.2, 73.8 and 71.2 (C-1, C-2 and C-4), 70.4 ($PhCH_2O$), 66.7 (C-7), 57.3 ($CH_3O$), 45.6 (C-2′), 27.9, 27.8 and 25.7 (C-3′, C-4′ and C-5′) and 24.2 (C-3).

B) (1R,2S,4R)-2-Benzyloxy-5-hydroxymethyl-4-methoxy-6,8-dioxabicyclo[3.2.1]octane

A solution of the dithiane from part A (4.5 g) in acetonitrile (80 ml) was added to a rapidly stirred mixture of N-bromosuccinimide (9 g), calcium carbonate (9 g), acetonitrile (80 ml) and water (20 ml) at 0° C. After 2 min, a mixture of powdered sodium thiosulphate (9 g) and sodium bicarbonate (9 g) was added. After a further 2 min the solids were removed by filtration. The filtrate was partitioned between ethyl acetate and water, the organic phase was dried ($MgSO_4$) and evaporated to a pale yellow oil. This was dissolved in aqueous acetonitrile (90%) and treated with sodium borohydride for 15 min at 0° C. After neturalising with acetic acid the solvent was removed and the residue was subjected to flash chromatography to give the title compound (2.3 g, 68%) as a colourless oil, [α]D -57.8°; $^{13}C$-nmr data: 138.3, 128.3 and 127.6 (aromatic), 106.8 (C-5), 76.3, 74.5 and 71.5 (C-1, C-2 and C-4), 70.5 ($PhCH_2O$), 66.3 (C-7), 62.6 ($CH_2OH$), 57.3 ($CH_3O$) and 23.8 (C-3).

Preparative Example 18

(1R,2S,4R)-2-Benzyloxy-5-halomethyl-4-methoxy-6,8-dioxabicyclo[3.2.1]octane

A solution of (1R,2S,4R)-2-Benzyloxy-5-hydroxymethyl-4-methoxy-6,8-dioxabicyclo[3.2.1]octane (4.0 g, 14.2 mmol) (prepared as described in Preparative Example 17) in dichloromethane (100 ml) was cooled to -40° C under argon. Pyridine (3.5 ml, 42.8 mmol) was added. Triflic anhydride (6.2 ml, 27.6 mmol) was then added at a rate such that the temperature of the solution did not exceed -30° C. The solution was stirred at -40° C for 10 min, then warmed to 0° C over 20 min. The resulting orange slurry was poured into a mixture of aqueous sodium hydrogen carbonate and ice and extracted twice with chloroform. The extracts were washed with a further portion of aqeuous sodium hydrogen carbonate, dried ($MgSO_4$) and evaporated. The

resulting brown crystalline mass was dissolved in hexamethylphosphoramide (12 ml). Benzyltriethylammonium chloride (9 g) or tetraethylammonium fluoride (5.7 g) was added and the resulting slurry stirred at 60° C for 3 h. Conventional extractive processing with diethyl ether and chromatography of the residue gave the title compounds. In this way, the following compounds were prepared:

1 (1R,2S,4R)-2-benzyloxy-5-chloromethyl-4-methoxy-6,8-dioxabicyclo[3.2.1)octane, $[\alpha]_D$ -50.1°; $^{13}$C-nmr data: 138.1, 128.3 and 127.6 (aromatic), 106.1 (C-5), 76.8, 73.8 and 71.2 (C-1, C-2 and C-4), 70.5 ($PhCH_2O$), 66.5 (C-7), 57.3 ($OCH_3$), 44.0 ($CH_2Cl$) and 23.8 (C-3).

2 (1R,2S,4R)-2-benzyloxy-5-fluoromethyl-4-methoxy-6,8-dioxabicyclo[3.2.1]octane, m.p. 48-51° C, $[\alpha]_D$ -67.0°; $^{13}$C-nmr data: 138.1, 128.3 and 127.6 (aromatic), 105.4 (d, $J_{C,F}$ 20.7 Hz, C-5), 81.4 (d, $J_{C,F}$ 171.9 Hz, $CH_2F$), 76.1 (C-1), 73.3 (d, J 2.3 Hz, C-4), 71.4 (C-2), 70.5 ($PhCH_2O$), 66.4 (C-7), 57.4 ($OCH_3$) and 23.6 (C-3).

Example 1

(1R,2S,4R)-2-(Substituted-methyloxy)-4-alkoxy-6,8-dioxabicyclo[3.2.1]octanes from Levoglucosenone

Levoglucosenone (1,6-anhydro-3,4-dideoxy-β-D-glycero-hex-3-enopyranos-2-ulose; Carboyhdr. Res. 1979, 71, 169-191) was mixed with a substituted-methanol (2-4 eq.), listed below, then trifluoroacetic acid (0.4-0.5 eq). The mixture, allowed to stand at room temperature for 6-30 days, was then concentrated on a rotary evaporator at ca. 0.1 torr, bath temperature 50-70° C. The resulting syrup was dissolved in dry tetrahydrofuran (10-20 ml/g) and cooled under argon to -78° C in a dry ice-acetone bath. A solution of lithium triethylborohydride or lithium tri-sec-butylborohydride in tetrahydrofuran (1 M, 16 ml/g of levoglucosenone) was added slowly. This mixture was stirred at -78° C for 30 min then methanol was added cautiously to destroy excess reagent. The solution was warmed to room temperature and partitioned between chloroform and water. The aqueous phase was extracted twice with additional portions of chloroform and the combined organic phase dried ($Na_2SO_4$), filtered and evaporated to a colourless syrup. The (1R,2S,4R)-2-(substituted-methyloxy)-4-hydroxy-6,8-dioxabicyclo[3.2.1]octane was isolated from this syrup as the predominant product by flash chromatography, and was O-alkylated according to General Preparative Example 1. Using the appropriate substituted methanol [ie: example 1.1, cyclobutanemethanol; 1.2, cyclopentanemethanol; 1.3, cycloheptanemethanol; 1.4, tetrahydropyran-2-yl-methanol; 1.5, tetrahydrofurfuryl alcohol; 1.6, (RS)-2-methylpentanol; 1.7, (RS)-2-methylbutanol; 1.8, 2,2-dimethylpropanol; 1.9, 2-methyl-2-propen-1-ol; 1.10, (RS)-3-cyclohexene-1-methanol; 1.11, (1S,2S,5S)-(-)-myrtanol; 1.12, (1R)-(-)-myrtenol; 1.13 and 1.14, tetrahydrothiopyran-4-methanol (see Preparative Example 1.1); 1.15, cyclohexanemethanol; 1.16 and 1.17, cyclooctanemethanol; 1.18 and 1.19, (1-methylcyclohexane)methanol (see Preparative Example 1.3); 1.20, tetrahydrothiophene-2-methanol (see Preparative Example 1.4); 1.21, 2-methylpropanol; 1.22, hexanol; 1.23, 2-ethylbutanol; 1.24, (RS)-2-cyclohexaneethanol; 1.25, cyclohexanol; 1.26, (RS)-2-cyclohexene-1-methanol (see Preparative Example 1.5); 1.27 and 1.28, 1-cyclohexene-1-methanol (see Preparative Example 1.2); 1.29, (RS)-2-ethylhexanol; 1.30 (RS)-2,2,4-trimethylpentanol; 1.31, (25)-2-methylbutamol] and the appropriate alkyl halide (ie. methyl iodide or ethyl bromide) the following compounds were prepared:

1 (1R,2S,4R)-2-cyclobutanemethyloxy-4-methoxy-6,8-dioxabicyclo[3.2.1]octane, $[\alpha]_D$ - 59.0°; $^{13}$C-nmr data: 100.1 (C-5), 74.7 and 74.5 (C-1 and C-4), 73.5 ($RCH_2O$), 73.3 (C-2), 65.3 (C-7), 56.9 ($OCH_3$), 34.8 (CH in cyclo-$C_4H_7$), 24.9, 24.8 and 18.3 ($CH_2$ in cyclo-$C_4H_7$) and 23.8 (C-3).

2 (1R,2S,4R)-2-cyclopentanemethyloxy-4-methoxy-6,8-dioxabicyclo[3.2.1]octane, $[\alpha]_D$ -55.6°; $^{13}$C-nmr data: 100.2 (C-5), 74.64 and 74.59 (C-1 and C-4), 73.55 ($RCH_2O$), 73.36 (C-2), 65.4 (C-7), 57.0 ($OCH_3$), 39.1 (CH in cyclo-$C_5H_9$), 29.4, 29.3 and 25.2 (x 2) ($CH_2$ in cyclo-$C_5H_9$) and 23.8 (C-3).

3 (1R,2S,4R)-2-cycloheptanemethyloxy-4-methoxy-6,8-dioxabicyclo[3.2.1]octane; $^{13}$C-nmr data: 100.2 (C-5), 74.9, 74.6 and 73.6 (C-1, C-2, C-4 and $RCH_2O$), 65.4 (C-7), 57.0 ($OCH_3$), 39.0 (CH in cyclo-$C_7H_{13}$, 31.0 28.5 and 26.3 (x 2, $CH_2$ in cyclo-$C_7H_{13}$) and 23.9 (C-3).

4 (1R,2S,4R)-4-methoxy-2-[(tetrahydropyran-2-yl)methyloxy]-6,8-dioxabicyclo[3.2.1]octane, as an ca. 1:1 mixture of diastereoisomers, $[\alpha]_D$ -55.2°; $^{13}$C-nmr data: 100.2 and 100.0 (C-5), 77.2, 77.1, 75.0, 74.6, 74.3, and 74.0 (C-1, C-2, C-4, and $OCHCH_2O$), 72.8 and 72.6 ($OCHCH_2O$), 68.2 ($OCH_2CH_2$), 65.4 (C-7), 57.1 ($OCH_3$), 28.4, 25.9, 24.3, 23.9 and 23.0 (C-3 and $CCH_2C$).

5 (1R,2S,4R)-4-methoxy-2-tetrahydrofurfuryloxy-6,8-dioxabicyclo[3.2.1]octane, as a mixture of diastereoisomers, $[\alpha]_D$-47.9°; $^1$H-nmr data (200 MHz): 5.49 (1H,s,H-5), 4.67 (1H,br s, H-1), 4.11 (1H, m, $CH_2CH(O)CH_2O$), 3.75 (4H, m, H-7endo, H-7exo, and $CH_2CH_2O$), 3.54 (2H, m, $CH(O)CH_2O$), 3.41 (4H, br s, $OCH_3$ and H-2), 3.16 (1H,d, H-4) and 1.89 (6H, m, H-3a, H-3e, and $CCH_2C$ x 2).

6 (1R,2S,4R)-4-methoxy-2-(2-methylpentyloxy)-6,8-dioxabicyclo[3.2.1]octane, as a ca. 1:1 mixture of diastereoisomers, $[\alpha]_D$ - 53.2°; $^{13}$C-nmr data: 100.1 (C-5), 74.8 ($C_5H_{11}CH_2O$), 74.5 (x 2) and 73.6 (C-4, C-2 and C-1), 65.3 (C-7), 56.8 (OMe), 23.7 (C-3), 32.8 (CH in $C_6H_{13}$), 35.7 and 19.8 ($CH_2$ in $C_6H_{13}$), 16.75 (0.5C), 16.82 (0.5C) and 14.1 ($CH_3$ in $C_6H_{13}$).

7 (1R,2S,4R)-4-methoxy-2-(2-methylbutyloxy)-6,8-dioxabicyclo[3.2.1]octane, as a ca. 1:1 mixture of diastereoisomers, $[\alpha]_D$ - 59.9°; $^{13}$C-nmr data: 100.1 (C-5), 74.3 ($C_4H_9CH_2O$), 74.3 (x 2) and 73.5 (C-4, C-2 and C-1), 65.2 (C-7), 56.7 (OMe), 25.9 (C-3), 34.6 (CH in $C_5H_{11}$), 23.6 ($CH_2$ in $C_5H_{11}$), 16.15 (0.5C), 16.29 (0.5C) and 10.9 ($CH_3$ in $C_5H_{11}$).

8 (1R,2S,4R)-4-methoxy-2-(2,2-dimethylpropyloxy)-6,8-dioxabicyclo[3.2.1]octane, $[\alpha]_D$ - 60.4°; $^{13}$C-nmr data: 100.3 (C-5), 79.1 ($C_4H_9CH_2O$) 74.4 (x 2) and 74.1 (C-1, C-2 and C-4), 65.2 (C-7), 56.4 (OMe), 31.9 (q-C in $C_5H_{11}$), 26.4 (3 x $CH_3$ in $C_5H_{11}$) and 23.2 (C-3).

9 (1R,2S,4R)-4-methoxy-2-(2-methyl-2-propen-1-yloxy)-6,8-dioxabicyclo[3.2.1]octane; $^{13}$C-nmr data: 142.1 (q-C in $C_4H_7$), 112.5 ($CH_2$ in $C_4H_7$), 100.1 (C-5), 74.6, 74.4 and 71.6 (C-1, C-2 and C-4), 72.5 ($CH_2O$ in $C_4H_7$), 65.4 (C-7), 57.2 (OMe), 24.4 (C-3) and 19.3 ($CH_3$ in $C_4H_7$).

10 (1R,2S,4R)-2-(3-cyclohexene-1-methyloxy)-4-methoxy-6,8-dioxabicyclo[3.2.1]octane, as a ca. 1:1 mixture of diastereoisomers, $[\alpha]_D$ -38.0°; $^{13}$C-nmr data: 126.6 and 125.7 (CH in cyclo-$C_6H_9$), 100.0 (C-5), 74.4 (x 2) and 73.4 (C-1, C-2 and C-4), 73.7 ($RCH_2O$), 65.1 (C-7), 56.7 (OMe), 33.4 (CH in cyclo-$C_6H_9$), 28.2 (0.5C), 28.1 (0.5C), 25.3 and 24.2 ($CH_2$ in cyclo-$C_6H_9$) and 23.5 (C-3).

11 (1R,2S,4R)-4-methoxy-2-[(1S,2S,5S)-myrtanyloxy]-6,8-dioxabicyclo[3.2.1]octane, $[\alpha]_D$ -53.2°; $^{13}$C-nmr data: 100.1 (C-5), 74.7, 74.6 and 73.7 (C-1, C-2 and C-4), 73.5 ($RCH_2O$), 65.4 (C-7), 57.0 (OMe), 42.4, 40.9 and 34.7 (CH in $C_9H_{15}$), 31.9 (q-C in $C_9H_{15}$), 25.6 and 20.0 ($CH_3$ in $C_9H_{15}$), 23.4 (C-3), 24.0, 23.8 and 18.5 ($CH_2$ in $C_9H_{15}$).

12 (1R,2S,4R)-4-methoxy-2-[(1R)-myrtenyloxyl]-6,8-dioxabicyclo[3.2.1]octane, $[\alpha]_D$ -48.9°; $^{13}$C-nmr data: 145.2 (vinylic q-C in $C_9H_{15}$), 120.1 (vinylic CH in $C_9H_{15}$), 100.1 (C-5), 74.6 (x 2) and 70.8 (C-1, C-2 and C-4), 71.0 ($RCH_2O$), 65.3 (C-7), 57.1 (OMe), 42.8 and 40.1 (CH in $C_9H_{13}$), 37.8 (q-C in $C_9H_{13}$), 31.3 and 31.1 ($CH_2$ in $C_9H_{13}$), 26.1 and 20.9 ($CH_3$ in $C_9H_{13}$) and 24.0 (C-3).

13 (1R,2S,4R)-4-methoxy-2-(tetraydydrothiopyran-4-methyloxy)6,8-dioxabicyclo[3.2.1]octane, $[\alpha]_D$ -43.1°; $^{13}$C-nmr data: 100.0 (C-5), 74.5 (x 3) and 73.8 (C-1, C-2, C-4 and $RCH_2O$), 65.4 (C-7), 57.2 (OMe), 37.5 (CH in cyclo-$C_5H_9S$), 31.0, 30.9 and 28.2 (x 2) ($CH_2$ in cyclo-$C_5H_9S$) and 23.9 (C-3).

14 (1R,2S,4R)-4-ethoxy-2-(tetraydrothiopyran-4-methyloxy)-6,8-dioxabicyclo[3.2.1]octane, $[\alpha]_D$ -47.8°; $^{13}$C-nmr data: 100.7 (C-5), 74.6, 74.3, 73.8 and 72.8 (C-1, C-2, C-4 and $RCH_2O$), 65.3 (C-7), 64.8 ($OCH_2CH_3$), 37.6 (CH in cyclo-$C_5H_9S$), 31.1, 30.9 and 28.2 (x 2) ($CH_2$ in cyclo-$C_5H_9S$), 24.2 (C-3) and 15.3 ($OCH_2CH_3$).

15 (1R,2S,4R)-2-cyclohexanemethyloxy-4-ethoxy-6,8-dioxabicyclo[3.2.1]octane, $[\alpha]_D$ -41.1°; $^{13}$C-nmr data: 100.8 (C-5), 74.9, 74.8, 73.6 and 72.9 (C-1, C-2, C-4 and $RCH_2O$), 65.4 (C-7), 64.7 ($OCH_2CH_3$), 37.8 (CH in cyclo-$C_6H_{11}$), 30.1, 29.9, 26.6, 26.5 and 25.8 ($CH_2$ in cyclo-$C_6H_{11}$), 24.2 (C-3) and 15.3 ($OCH_2CH_3$).

16 (1R,2S,4R)-2-cyclooctanemethyloxy-4-methoxy-6,8-dioxabicyclo[3.2.1]octane, $[\alpha]_D$ -54.0°; $^{13}$C-nmr data: 100.2 (C-5), 75.3 ($RCH_2O$), 74.6, 74.5 and 73.6 (C-1, C-2 and C-4), 65.3 (C-7), 56.9 (OMe), 37.2 (CH in cyclo-$C_8H_{15}$), 29.3 (x 2), 26.7 (x 2), 26.2, 25.3 (x 2) ($CH_2$ in cyclo-$C_8H_{15}$) and 23.8 (C-3).

17 (1R,2S,4R)-2-cyclooctanemethyloxy-4-ethoxy-6,8-dioxabicyclo[3.2.1]octane, $[\alpha]_D$ -49.9°; $^{13}$C-nmr data: 100.8 (C-5), 75.2 ($RCH_2O$), 74.6, 73.6 and 72.9 (C-1, C-2 and C-4), 65.4 (C-7), 64.7 ($OCH_2CH_3$), 37.4 (CH in cyclo-$C_8H_{15}$), 29.3 (x 4), 26.8, 26.3 and 25.4 ($CH_2$ in cyclo-$C_8H_{15}$), 24.2 (C-3) and 15.3 ($OCH_2CH_3$).

18 (1R,2S,4R)-4-methoxy-2-(1-methylcyclohexane-1-methyloxy)-6,8-dioxabicyclo[3.2.1]octane, $[\alpha]_D$ -55.5°; $^{13}$C-nmr data: 100.3 (C-5), 78.3 ($RCH_2O$), 79.4 (x 2) and 79.1 (C-1, C-2 and C-4), 65.3 (C-7), 56.5 (OMe), 34.3 (x 3), 26.2 (x 3) ($CH_2$ and q-C in cyclo-$C_7H_{13}$), 23.2 (C-3) and 22.4 (C-$CH_3$).

19 (1R,2S,4R)-4-ethoxy-2-(1-methylcyclohexane-1-methyloxy)-6,8-dioxabicyclo[3.2.1]octane, $[\alpha]_D$ -55.9°; $^{13}$C-nmr data: 100.0 (C-5), 78.3 ($RCH_2O$), 74.5, 74.1 and 72.9 (C-1, C-2 and C-4), 65.4 (C-7), 64.3 ($OCH_2CH_3$) 34.4 (x 3), 26.3 and 21.7 (x 2), ($CH_2$ and q-C in cyclo-$C_7H_{13}$), 23.9 (C-3) and 22.5 (C-$CH_3$).

20 (1R,2S,4R)-4-methoxy-2-(tetraydrothiophene-2-methyloxy)-6,8-dioxabicyclo[3.2.1]octane, as a mixture of diastereoisomers; $^{13}$C-nmr data: 100.1 and 100.0 (C-5), 74.8 and 74.3 (C-1), 74.5 (C-4), 73.9 and 73.6 (C-2), 73.3 and 73.1 ($RCH_2O$), 65.3 (C-7), 57.1 (OMe), 47.1 and 47.0 (CH in cyclo-$C_4H_7S$), 33.4 (x 2), 31.9 (x 2), 29.43, 29.36, 24.1 and 23.7 ($CH_2$ in cyclo-$C_4H_7S$ and C-3).

21 (1R,2S,4R)-2-(2-methylpropyloxy)-4-methoxy-6,8-dioxabicyclo[3.2.1]octane, $[\alpha]_D$ -60.1°; $^{13}$C-nmr data: 100.1 (C-5), 76.1 ($RCH_2O$), 74.6 and 73.6 (C-1, -2 and -4), 65.3 (C-7), 57.9 ($OCH_3$), 28.0 (CH in 2-methylpropyloxy), 23.8 (C-3), 19.3 and 19.2 ($CH_3$ in 2-methylpropyloxy.

22 (1R,2S,4R)-2-hexyloxy-4-methoxy-6,8-dioxabicyclo[3.2.1]octane, $[\alpha]_D$ -51.6°; $^{13}$C-nmr data: 100.1 (C-5), 74.7, 74.6 and 73.4 (C-1, -2 and -4), 69.3 ($RCH_2O$), 65.4 (C-7), 57.2 ($OCH_3$), 31.6, 29.6 and 25.7 ($CH_2$ in hexyloxy), 24.2 (C-3), 22.5 ($CH_2$ in hexyloxy) and 13.9 ($CH_3$ in hexyloxy).

23 (1R,2S,4R)-2-(2-ethylbutyloxy)-4-methoxy-6,8-dioxabicyclo[3.2.1]octane, $[\alpha]_D$ -54.6°; $^{13}$C-nmr data: 100.1 (C-5), 74.5 (x 2) and 73.5 (C-1, -2 and -4), 71.5 ($RCH_2O$), 65.2 (C-7), 56.7 ($OCH_3$), 40.6 (CH in 2-ethylbutyloxy), 23.5 (C-3), 22.9 (2 x $CH_2$ in 2-ethylbutyloxy), 10.8 and 10.7 ($CH_3$ in 2-ethylbutyloxy).

24 (1R,2S,4R)-2-(-2-cyclohexylethyloxy)-4-methoxy-6,8-dioxabicyclo[3.2.1]octane, $[\alpha]_D$ -37.5°; $^{13}$C-nmr data: 99.9 (C-5), 74.5, 74.4 and 73.1 (C-1, -2 and -4), 66.7 ($RCH_2O$), 65.1 (C-7), 56.7 ($OCH_3$), 34.2 (CH in cyclohexylethyloxy), 36.8, 33.1, 32.9, 26.3 and 25.9 (x 2) ($CH_2$ in cyclohexylethyloxy), and 23.7 (C-3).

25 (1R,2S,4R)-2-cyclohexyloxy-4-methoxy-6,8-dioxabicyclo-[3.2.1]octane, m.p. 82-85°; $[\alpha]_D$ -34.1°; $^{13}$C-nmr data: 100.1 (C-5), 76.0 and 74.7 (C-1, -2 and -4), 70.3 (CH in cyclohexyloxy), 65.5 (C-7), 57.0 ($OCH_3$), 32.8 and 32.3 ($CH_2$ in cyclohexyloxy), 25.6, 24.7 (x 2) and 24.3 ($CH_2$ in cyclohexyloxy and C-3).

26. (1R,2S,4R)-2-(2-cyclohexene-1-methyloxy)-4-methoxy-6,8-dioxabicyclo[3.2.1]octane, as a mixture of diastereoisomers; $^{13}$C-nmr data: 128.5 (0.5C), 128.3 (br, 1C) and 128.1 (0.5C) (CH=CH in cyclo-$C_6H_9$), 100.2 (C-5), 74.6 (x2) (C-1 and C-4), 73.7 (C-2), 73.5 (0.5C) and 73.4 (0.5C) ($RCH_2O$), 65.4 (C-7), 57.1 ($OCH_3$), 35.52 (0.5C) and 35.46 (0.5C) (CH in cyclo-$C_6H_9$), 25.8, 25.3, 20.7 (0.5C) and 20.6 (0.5C) ($CH_2$ in cyclo-$C_6H_9$) and 23.9 (C-3).

27. (1R,2S,4R)-2-(1-cyclohexene-1-methyloxy)-4-methoxy-6,8-dioxabicyclo[3.2.1]octane, $[\alpha]_D$ = 51.5°; $^{13}$C-nmr data: 134.7 (q-C in cyclo-$C_6H_9$), 100.0 (C-5), 74.6, 74.5 and 71.1 (C-1, -2 and -4), 73.1 ($RCH_2O$), 65.3 (C-7), 57.1 ($OCH_3$), 26.7, 24.8, 24.2 and 22.3 (x2) ($CH_2$ in cyclo-$C_6H_9$ and C-3).

28. (1R,2S,4R)-2-(1-cyclohexene-1-methyloxy)-4-ethoxy-6,8-dioxabicyclo[3.2.1]octane; $^{13}$C-nmr data: 134.9 (q-C in cyclo-$C_6H_9$), 124.8 (CH in cyclo-$C_6H_9$), 100.8 (C-5), 74.7, 73.2 and 71.5 (C-1, -2 and -4), 72.9 ($RCH_2O$), 65.4 (C-7), 64.8 ($OCH_2CH_3$), 25.8, 24.9, 24.5 and 22.4(x2) ($CH_2$ in cyclo-$C_6H_9$ and C-3) and 15.3 ($OCH_2CH_3$).

29. (1R,2S,4R)-2-(2-ethylhexyloxy)-4-methoxy-6,8-dioxabicyclo[3.2.1]octane, as a mixture of diastereoisomers, $[\alpha]_D$ -81.8°; $^{13}$C-nmr data: 100.3 (C-5), 74.6 (x2) and 73.7 (C-1, -2 and -4), 72.2 ($RCH_2O$), 65.4 (C-7), 56.9 ($OCH_3$), 39.2 (CH in $C_8H_{17}$), 30.2, 28.9, 23.7, 23.5 and 23.0 (C-3 and $CH_2$ in $C_8H_{17}$), 14.0 and 10.9 ($CH_3$ in $C_8H_{17}$).

30. (1R,2S,4R)-4-methoxy-2-(2,44-trimethylpentyloxy)-6,8-bicyclo[3.2.1]octane, as a mixture of diastereoisomers, $[\alpha]_D$ -51.8°; $^{13}$C-nmr data: 100.2 (C-5), 75.8 and 75.7 ($RCH_2O$), 74.5 and 74.4 (C-1 and C-4), 73.6 and 73.5 (C-2), 65.3 (C-7), 56.8 ($OCH_3$), 47.4 and 47.3 ($CH_2$ in trimethylpentyl), 30.8 ($Me_3C$-), 29.8 (x 2) and 29.6 [$(CH_3)_3C$], 23.8 and 23.5 (C-3) and 19.9 ($CH_3CH$).

31. (1R,2S,4R)-2-[(2S)-2-methylbutyloxy]-4-methoxy-6,8-dioxabicyclo[3.2.1]octane, $[\alpha]_D$ -60.7°; $^{13}$C-nmr data: 100.2 (C-5), 74.6 (x 2) and 73.6 (C-2, C-4 and C-1), 74.5 ($RCH_2O$), 65.3 (C-7), 56.9 ($OCH_3$), 34.6 [$CH_3CH_2(CH_3)CH$], 26.0 ($MeCH_2CH$), 23.7 (C-3), 16.4 and 11.1 (C-$CH_3$).

Example 2

(1R,2S,4R)-4-Methoxy-2-phenoxy-6,8-dioxabicyclo[3.2.1]octane

To a solution of (1R,2S,4R)-2-hydroxy-4-methoxy-6,8-dioxabicyclo[3.2.1]octane in tetrahydropuran at 0°C was added sodium hydride (60% dispersion in oil, 1.1 eq. or more) and diphenyliodinium chloride (1.2 eq.). The mixture was stirred at room temperature for several days, then methanol was added to destroy excess hydride. The solution was diluted with water and extracted with chloroform (x 3). The combined organic phase was dried ($MgSO_4$), filtered and evaporated to a residue which was subjected to flash chromatography to yield the title compound as a syrup,$[\alpha]_D$ -61.2°; $^{13}$C-nmr data: 157.1 (q,PhO), 129.5 (x 2), 121.3 and 116.3 (x 2) (CH in PhO), 100.2 (C-5) 74.7, 74.4 and 71.3 (C-1, C-2 and C-4), 65.4 (C-7), 57.2 (OMe), and 23.4 (C-3).

Example 3

O-Alkylation of 2-hydroxy-4-methoxy-6,8-dioxabicyclo[3.2.1]octane and its 5-alkyl analogues

1,6-Anhydro-3-deoxy-2-0-methyl-β-D-ribo-hexopyranose (Preparative Examples 1 or 6 in EP 0229034A) or the appropriate 5-alkyl-2-hydroxy-4-methoxy-6,8-dioxabicyclo[3.2.1.]octane (see Preparative Example 2) were separately O-alkylated according to General Preparative Example 1. In cases where the alkyl halide was consumed before complete alkylation of the carbohydrate alcohol had occurred, additional portions of sodium hydride and alkyl halide were added. Purification was effected by flash chromatography.

Using the appropriate dioxabicyclooctane (ie, examples 2.1 and 2.2, 1,6-anhydro-3-deoxy-2-0-methyl-β-D-ribo-hexopyranose, alternatively named (1R,2S,4R)-2-hydroxy-4-methoxy-6,8-dioxabicyclo[3.2.1.]octane; 2.3, (1R,2S,4R)-5-butyl-2-hydroxy-4-methoxy-6,8-dioxabicyclo[3.2.1.]octane; 2.4, (1R,2S,4R)-2-hydroxy-4-methoxy-5-methyl-6,8-dioxabicyclo[3.2.1.] octane] and the appropriate alkyl halide (examples 2.1, 2.3 and 2.4, cyclohexanemethyl bromide; 2.2, cyclopropanemethyl bromide) the following compounds were prepared:

1 (1R,2S,4R)-2-cyclohexanemethyloxy-4-methoxy-6,8-dioxabicyclo[3.2.1.]octane, $[\alpha]_D$ -51.4°; $^{13}C$-nmr data: 101.2 (C-5), 75.0 ($C_6H_{11}CH_2O$), 74.6 (x 2) and 73.6 (C-1, C-2 and C-4), 65.4 (C-7), 57.0 ($OCH_3$), 37.7 ($CHCH_2O$), 30.0, 26.5, 25.7 (x 3), and 23.8 ($CH_2$ in cyclo-$C_6H_{11}$).

2 (1R,2S,4R)-2-cyclopropanemethyloxy-4-methoxy-6,8-dioxabicyclo[3.2.1]octane, $[\alpha]_D$ -58.0°; $^{13}C$-nmr data: 100.1 (C-5), 74.9, 74.7 and 72.7 (C-1, C-2 and C-4), 73.4 ($C_3H_5CH_2O$), 65.4 (C-7), 57.2 ($OCH_3$), 24.3 (C-3), 10.6 ($CHCH_2O$) and 3.0 ($CH_2$ in cyclo-$C_3H_5$).

3 (1R,2S,4R)-5-butyl-2-cyclohexanemethyloxy-4-methoxy-6,8-dioxabicyclo[3.2.1]octane, light yellow syrup (69%), $[\alpha]_D$ -49.1°; $^{13}C$-nmr data: 108.4 (C-5), 76.3 and 76.1 (C-1 and C-4), 75.2 ($RCH_2O$), 73.6 (C-2), 66.2 (C-7), 57.0 (OMe), 37.6 (CH in cyclo-$C_6H_{11}$), 32.7, 30.1, 29.9, 26.6, 25.7 (x 2), 24.3, 23.7 and 22.8 (9 x $CH_2$) and 13.9 (C-$CH_3$).

4 (1R,2S,4R)-2-cyclohexanemethyloxy-4-methoxy-5-methyl-6,8-dioxabicyclo[3.2.1]octane, colourless syrup (86%), $[\alpha]_D$ -75.2°; $^1H$-nmr data (200 MHz): 4.63 (m,H-1), 3.88 (dd,H-7exo, $J_{1,7exo}$ 5.4 $J_{7,7}$ 7.4), 3.70 (dd, H-7endo, $J_{1,7endo}$ 0.6), 3.40 (OMe), 3.0-3.4 (m,$RCH_2O$), 3.19 (m,H-2), 3.05 (dm,H-4, $J_{3a,4}$ 3.9), 2.15 (H-3e, $J_{3a,3e}$ 15.7), 1.6-1.9 (m,H-3a and cyclo-$C_6H_{11}$), 1.55 (C-Me), 1.15-1.30 and 0.85-0.95 (m, cyclo-$C_6H_{11}$).

Example 4

Hydrogenation of aralykyl moieties

A 4-alkoxy-2-aralkoxy-6,8-dioxabicyclo[3.2.1]octane was dissolved in methanol or ethanol and 5% rhodium on alumina catalyst (0.2 g per g of substrate) was added. The mixture was then hydrogenated at room temperature and at atmospheric pressure or 80 p.s.i. The mixture was filtered and evaporated, and the resulting colourless syrup was purified by chromatography on silica gel eluted with hexane-ethyl acetate mixtures.

Using this procedure the following compounds were prepared:

1 (1R,2S,4R)-2-cyclohexanemethyloxy-4-methoxy-6,8-dioxabicyclo[3.2.1]octane, colourless gum from (1R,2S,4R)-2-benzyloxy-4-methoxy-6,8-dioxabicyclo[3.2.1]octane [EP 0229034A, Example 3.1] in 78% yield; $^1H$-nmr data: 0.95 (2H,m), 1.2 (3H,m), 1.7 (3H,m), 1.85 (4H,m), 2.02 (1H,d), 3.15 (1H,d), 3.20 (1H,d), 3.28 (2H,dd), 3.40 (3H,s), 3.72 (1H,d), 3.80 (1H,dd), 4.61 (1H,d) and 5.48 (1H,s).

2 (1R,2S,4R,1′RS)-2-(1′-cyclohexyl-ethyloxy)-4-methoxy-6,8-dioxabicyclo[3.2.1]octane, colourless gum from (1R,2S,4R)-2-(1′-phenylethyloxy)-4-methoxy-6,8-dioxabicyclo[3.2.1]octane [EP 0229034A, Example 4.3] in 29% yield; $^1H$-nmr data: 0.95 (2H,m), 1.15 (6H,m), 1.4 (1H,m), 1.8 (7H,m), 3.2 (3H,m), 3.38 and 3.40 (3H, 2 x s), 3.75 (2H,m), 4.50 and 4.55 (1H, 2 x d), 5.46 and 5.48 (1H, 2 x s).

3 (1R,2S,4R,4′RS)-4-methoxy-2-(4′-methylcyclohexane)methyloxy-6,8-dioxabicyclo[3.2.1]octane, colourless gum from (1R,2S,4R)-2-(4-methylbenzyloxy)-4-methoxy-6,8-dioxabicyclo[3.2.1]octane [EP 0229034A, Example 4.7] in 25% yield; $^1H$-nmr data: 0.9 (4H,m), 1.2 (2H,m) 1.5 (4H,m), 1.65 (2H,m), 1.8 (2H,dt), 2.0 (1H,d), 3.2 (2H,m), 3.40 (3H,s), 3.3 (2H,m), 3.72 (1H,d), 3.80 (1H,t), 4.60 (1H,s) and 5.48 (1H,s).

4 (1R,2S,4R,2′RS)-4-methoxy-2-(2′-methylcyclohexane)methyl-oxy-6,8-dioxabicyclo[3.2.1]octane, colourless gum from (1R,2S,4R)-2-(2-methylbenzyloxy)-4-methoxy-6,8-dioxabicyclo[3.2.1]octane [EP 0229034A, Example 4.5] in 42% yield; $^1$H-nmr data: 0.85 (3H,m), 1.25 (2H,m), 1.35 (5H,m), 1.6 (1H,m), 1.85 (2H,m), 1.95 (2H,m), 3.15 (1H,d), 3.20 (1H,d), 3.30 (2H,m), 3.40 (3H,s), 3.74 (1H,d), 3.80 (1H,t), 4.61 (1H,d) and 5.49 (1H,s).

5 (1R,2S,4R)-2-cyclohexanemethyloxy-4-methoxy-5-propyl-6,8-dioxabicyclo[3.2.1]octane, colourless gum from (1R,2S,4R)-2-benzyloxy-4-methoxy-5-propyl-6,8-dioxabicyclo[3.2.1]octane [EP 0229034A, Example 15.5] in 45% yield; $^1$H-nmr data: 0.85 (2H,m), 0.94 (3H,t), 1.2 (2H,m), 1.45 (2H,m), 1.7 (9H,m), 1.95 (1H,m), 2.12 (1H,d), 3.06 (1H,d), 3.17 (1H,d), 3.24 (1H,t), 3.32 (1H,dd), 3.36 (1H,s), 3.68 (1H,d), 3.79 (1H,dd) and 4.60 (1H,d).

6 (1R,2S,4R)-2-cyclohexanemethyloxy-5-ethyl-4-methoxy-6,8-dioxabicyclo[3.2.1]octane, colourless gum from (1R,2S,4R)-2-benzyloxy-5-ethyl-4-methoxy-6,8-dioxabicyclo[3.2.1]octane [EP 0229034A, Example 15.2] in 42% yield; $^1$H-nmr data: 0.85 (2H,m), 0.93 (3H,t), 1.20 (3H,m), 1.7 (8H,m), 1.96 (1H,m), 2.10 (1H,d), 3.07 (1H,d), 3.16 (1H,d), 3.23 (1H,d), 3.30 (1H,dd), 3.36 (3H,s), 3.66 (1H,d), 3.78 (1H,dd) and 4.58 (1H,d).

7 (1RS,2SR,4RS)-4-cyclohexanemethyloxy-2-methoxy-6,8-dioxabicyclo[3.2.1]octane, colourless gum from (1RS,2SR,4RS)-4-benzyloxy-2-methoxy-6,8-dioxabicyclo[3.2.1]octane (see Example 40) in 60% yield; $^1$H-nmr data: 0.85 (2H,m), 1.2 (3H,m), 1.6 (4H,m), 1.8 (3H,m), 2.01 (1H,d), 3.18 (2H,s), 3.28 (2H,m), 3.41 (3H,s), 3.73 (1H,t), 3.80 (1H,dd), 4.65 (1H,d) and 5.49 (1H,s).

8 (1R,2S,4R)-2-cyclohexanemethyloxy-5-(3-fluoropropyl)-4-methoxy-6,8-dioxabicyclo[3.2.1]octane, from (1R,2S,4R)-2-benzyloxy-5-(3-fluoropropyl)-4-methoxy-6,8-dioxabicyclo[3.2.1]octane (see Preparative Example 5) in 82% yield; $^{13}$C-nmr data: 107.9 (C-5), 84.2 ($CH_2F$, $J_{C,F}$ 164Hz) and 76.8 and 76.3 (C-1 and C-4), 75.3 ($R\underline{C}H_2O$), 73.5(C-2), 66.4 (C-7), 57.1 ($OCH_3$), 37.6 (CH in cyclo-$C_6H_{11}$), 30.1, 29.9, 25.7 and 23.6 (C-3 and $\underline{C}H_2$ in cyclo-$C_6H_{11}$), 28.5 ($\underline{C}H_2CH_2CH_2F$, $J_{C,F}$ 6.2 Hz) and 23.5 ($CH_2\underline{C}H_2CH_2F$, $J_{C,F}$ 20.0Hz).

9 (1R,2S,4R)-2-cyclohexanemethyloxy-5-(2-fluoroethyl)-4-methoxy-6,8-dioxabicyclo[3.2.1]octane, from (1R,2S,4R)-2-benzyloxy-5-(2-fluoroethyl)-4-methoxy-6,8-dioxabicyclo[3.2.1]octane (see Preparative Example 6); $^1$H-nmr data: 0.9 (2H,m), 1.2 (3H,m), 1.6-1.8 (7H,m), 2.15 (1H,d), 2.3(1H,q), 2.4 (1H,m), 3.1 (1H,d), 3.2 (1H,d), 3.3 (2H,dq), 3.37 (3H,s), 3.7 (1H,d), 3.8(1H,dd), 4.6 (1H,d), 4.55 and 4.75 (2H,m).

10 (1R,2S,4R)-2-cyclohexanemethyloxy-5-fluoromethyl-4-methoxy-6,8-dioxabicyclo[3.2.1]octane, from (1R,2S,4R)-2-benzyloxy-5-fluoromethyl-4-methoxy-6,8-dioxabicyclo[3.2.1]octane (Preparative Example 18.2); $[\alpha]_D$ -57.8°; $^{13}$C-nmr data: 105.3 (d,$J_{C,F}$ 20.1 Hz, C-5), 82.1 (d,J 171.8 Hz, $CH_2F$), 76.2 (C-1), 75.4 ($C_6H_{10}CH_2O$), 73.5 (C-2), 73.2 (d,J 4.7 Hz, C-4), 66.4 (C-7), 57.2 ($OCH_3$), 37.6 (cyclohexane CH), 30.1, 29.9, 26.6 and 25.7 (x 2) (cyclohexane $CH_2$) and 23.4 (C-3).

11 (1R,2S,4R)-5-chloromethyl-2-cyclohexanemethyloxy-4-methoxy-6,8-dioxabicyclo[3.2.1]octane, from (1R,2S,4R)-2-benzyloxy-5-chloromethyl-4-methoxy-6,8-dioxabicyclo[3.2.1]octane (Preparative Example 18.1); m.p. 65-68°C, $[\alpha]_D$ -66.6°; $^{13}$C-nmr data: 106.1 (C-5), 75.4 ($R\underline{C}H_2O$), 77.0, 73.7 and 73.3 (C-1, -2 and -4), 66.6 (C-7), 57.2 (OMe), 44.1 ($CH_2Cl$), 37.6 (CH in cyclo-$C_6H_{11}$), 30.1, 30.0, 26.6 and 25.7 (x 2) ($CH_2$ in cyclo-$C_6H_{11}$) and 23.6 (C-3).

## Example 5

### (1R,2S,4R)-5-Alkyl-2-cyclohexanemethyloxy-4-ethoxy-6,8-dioxabicyclo[3.2.1]octanes by C-5 alkylation

A solution of 1,6-anhydro-4-0-cyclohexanemethyloxy-3-deoxy-2-0-ethyl-$\beta$ -D-ribo-hexopyranose (see Example 2.1) (10 g) in acetic anhydride (40 ml) was stirred in an ice-bath while boron trifluoride diethyl etherate (10 drops) was added. The solution was stirred at ambient temperature for 2 h. Sodium hydrogen carbonate (65 g) and then water (500 ml) were carefully added, and the mixture was extracted with chloroform. The extract was dried ($MgSO_4$) and evaporated. The crude diacetate so obtained was dissolved in methanol (100 ml) and a solution of sodium methoxide in methanol was added until the pH was 10. The resulting solution was allowed to stand at ambient temperature overnight before acid resin was added until the pH was 7.

Filtration and evaporation gave a quantitative yield of syrupy diol. This diol (Xg) was then dissolved in dry tetrahydrofuran (25X ml) and 4-5 mole equivalents of the appropriate alkylmagnesium halide (2-3M solution in diethyl ether) was added. The resulting solution was heated under reflux in an inert atmosphere until the diol was all consumed (generally 4-6 h) as evidenced by thin layer chromatography. Excess Grignard reagent was destroyed by the careful addition of water, and then saturated ammonium chloride

solution was added and the mixture was extracted three times with chloroform. The combined chloroform extracts were dried ($MgSO_4$) and concentrated. The residual syrup was dissolved in acetone (10X ml) and stirred while anhydrous cupric sulphate (0.2X g) and (±)-10-camphorsulphonic acid (0.05X g) were added. The resulting mixture was stirred at ambient temperature until reaction was complete (1-2 h; tlc evidence) and then sodium hydrogen carbonate (1X g) was added and the mixture stirred at ambient temperature for 1 h, filtered with the assistance of filter aid and evaporated. The resulting crude alcohol was oxidised according to Swern (see General Preparative Example 2). The product was purified by flash chromatography and then cyclised by reaction in acetic acid - water (9:1 v/v; 50X ml) at 50 °C overnight. The solvent was then evaporated and the residue purified by flash chromatography to give (1R,2S,4R)-5-alkyl-2-cyclohexanemethyloxy-4-ethoxy-6,8-dioxabicyclo[3.2.1]octanes.

Using the appropriate alkylmagnesium halide, the following compounds were prepared by the above procedure:

1 (1R,2S,4R)-2-cyclohexanemethyloxy-4-ethoxy-5-ethyl-6,8-dioxabicyclo[3.2.1]octane, $[\alpha]_D$ -59.4°; $^{13}C$-nmr data: 108.0 (C-5), 76.0, 74.2 and 73.3 (C-1, C-2 and C-4), 74.6 ($R\underline{C}H_2O$), 65.8 (C-7), 64.4 ($O\underline{C}H_2CH_3$), 37.4 (CH in cyclo-$C_6H_{11}$), 29.8, 29.6, 26.2 and 25.4 (x 3) ($CH_2$ in cyclo-$C_6H_{11}$ and $\underline{C}H_2CH_3$), 24.1 (C-3), 15.0 ($OCH_2\underline{C}H_3$) and 5.9 ($CH_2\underline{C}H_3$).

2 (1R,2S,4R)-2-cyclohexanemethyloxy-4-ethoxy-5-propyl-6,8-dioxabicyclo[3.2.1]octane $[\alpha]_D$ -58.3°; $^{13}C$-nmr data: 108.2 (C-5), 76.2, 74.6 and 73.5 (C-1, C-2 and C-4), 75.0 ($R\underline{C}H_2O$), 66.0 (C-7), 64.7 ($O\underline{C}H_2CH_3$), 37.7 (CH in cyclo-$C_6H_{11}$), 35.0, 30.1, 29.9, 26.5, 25.6 (x 2) ($\underline{C}H_2CH_2CH_3$ and $CH_2$ in cyclo-$C_6H_{11}$), 24.3 (C-3), 15.3 (x 2) and 14.1 ($CH_2\underline{C}H_2CH_3$, $CH_2CH_2\underline{C}H_3$ and $OCH_2\underline{C}H_3$).

3 (1R,2S,4R)-2-cyclohexanemethyloxy-4-ethoxy-5-methyl-6,8-dioxabicyclo[3.2.1]octane, $[\alpha]_D$ -60.6°; $^{13}C$-nmr data: 106.7 (C-5), 76.2, 75.5 and 72.9 (C-1, C-2 and C-4), 74.7 ($R\underline{C}H_2O$), 65.6 (C-7), 64.6 ($O\underline{C}H_2CH_3$), 37.3 (CH in cyclo-$C_6H_{11}$), 29.8, 29.6, 26.3 and 25.4 (x 2) ($CH_2$ in cyclo-$C_6H_{11}$), 24.1 (C-3), 20.5 (C-$\underline{C}H_3$) and 15.0 ($OCH_2\underline{C}H_3$).

## Example 6

### (1R,2S,4R)-4-alkoxy-5-alkyl-2-(3-cyclohexene-1-methyloxy)-6,8-dioxabicyclo[3.2.1]octanes by C-5 alkylation

Using a method analogous to that of Example 5, but starting from 2-(3-cyclohexane-1-methyloxy)-4-methoxy-6,8-dioxabicyclo[3.2.1]octane (see Example 1.10) or its 4-ethoxy-homologue prepared from levoglucosenone by the method described in Example 1) as appropriate, the following compounds were prepared as mixtures of diastereoisomers:

1 (1R,2S,4R)-2-(3-cyclohexene-1-methyloxy)-4-methoxy-5-methyl-6,8-dioxabicyclo[3.2.1]octane, $[\alpha]_D$ -75.5°; $^{13}C$-nmr data: 126.6, 125.6 (vinylic CH in cyclo-$C_6H_9$), 106.7 (C-5), 77.3, 76.1 and 73.1 (C-1, C-2 and C-4), 73.9 ($RCH_2O$), 65.8 (C-7), 56.9 ($OCH_3$), 33.3 (CH in cyclo-$C_6H_9$), 28.3, 28.1, 25.3, 24.1 and 23.5 ($CH_2$ in cyclo-$C_6H_9$), 23.4 (C-3) and 20.6 ($CH_3$).

2 (1R,2S,4R)-2-(3-cyclohexene-1-methyloxy)-4-methoxy-5-ethyl-6,8-dioxabicyclo[3.2.1]octane, $[\alpha]_D$ -101.9°; $^{13}C$-nmr data: 126.8 and 125.8 (vinylic CH in cyclo-$C_6H_9$), 108.3 (C-5), 76.1, 75.9 and 73.6 (C-1, C-2 and C-4), 74.0 ($RCH_2O$), 66.1 (C-7), 56.9 ($OCH_3$), 33.4 (CH in cyclo-$C_6H_9$), 28.4, 28.2, 25.8, 25.4 and 24.3 ($CH_2$ in cyclo-$C_6H_9$), 23.5 (x 2) (C-3 and $\underline{C}H_2CH_3$) and 6.3 ($CH_2\underline{C}H_3$).

3 (1R,2S,4R)-2-(3-cyclohexene-1-methyloxy)-4-ethoxy-5-ethyl-6,8-dioxabicyclo[3.2.1]octane, $[\alpha]_D$ -61.1°; $^{13}C$-nmr data: 126.6 and 125.6 (vinylic CH in cyclo-$C_6H_9$), 108.7 (C-5), 76.2, 74.2 and 73.5 (C-1, C-2 and C-4), 73.8 ($RCH_2O$), 65.9 (C-7), 64.6 ($O\underline{C}H_2CH_3$), 33.3 (CH in cyclo-$C_6H_9$), 28.1, 28.3, 25.6, 25.3, 24.2 ($CH_2$ in cyclo-$C_6H_9$), 24.1 (C-3), 15.2 ($OCH_2\underline{C}H_3$) and 6.1 ($CH_2\underline{C}H_3$).

4 (1R,2S,4R)-2-(3-cyclohexene-1-methyloxy)-4-ethoxy-5-methyl-6,8-dioxabicyclo[3.2.1]octane, $[\alpha]_D$ -76.2°; $^{13}C$-nmr data: 126.9 and 125.9 (vinylic CH in cyclo-$C_6H_9$), 107.1 (C-5), 76.4, 75.7 and 73.3 (C-1, C-2 and C-4), 74.1 ($R\underline{C}H_2O$), 65.9 (C-7), 65.1 ($O\underline{C}H_2CH_3$), 33.5 (CH in cyclo-$C_6H_9$), 28.5, 28.3, 25.5, 24.7 and 24.6 (CH2 in cyclo-$C_6H_9$), 24.4 (C-3), 20.8 (C-$\underline{C}H_3$) and 15.3 ($OCH_2\underline{C}H_3$).

5 (1R,2S,4R)-2-(3-cyclohexene-1-methyloxy)-4-ethoxy-5-propyl-6,8-dioxabicyclo[3.2.1]octane, $[\alpha]_D$ -42.7°; $^{13}C$-nmr data: 126.7 and 125.7 (vinylic CH in cyclo-$C_6H_9$), 108.1 (C-5), 76.7, 74.6 and 73.6 (C-1, C-2 and C-4), 73.9 ($R\underline{C}H_2O$), 66.0 (C-7), 64.7 ($O\underline{C}H_2CH_3$), 34.9, 33.4, 28.4, 28.2, 24.2 (x 2), 15.2 (x 2) and 14.1 ($CH_2$ and CH in cyclo-$C_6H_9$, $OCH_2\underline{C}H_3$ and $\underline{C}H_2\underline{C}H_2CH_3$)

Example 7

(1R,2S,4R)-4-Alkoxy-2-cyclohexanemethyloxy-5-(2-propen-1-yl)-6,8-dioxabicyclo[3.2.1]octanes

6-O-tert-Butyldimethylsilyl-4-O-cyclohexanemethyl-3-deoxy-2-O-methyl-D-ribo-hexono-1,5-lactone (Preparative Example 7.1) and its 2-O-ethyl analogue (Preparative Example 7.2) were separately converted into the title compounds by the procedure detailed in Preparative Example 4, but using one of the above lactones in place of 4-O-benzyl-6-O-tert-butyldimethylysilyl-3-deoxy-2-O-methyl-D-ribo-hexono-1,5-1actone. In this way the following compounds were prepared:

1. (1R,2S,4R)-2-cyclohexanemethyloxy-4-methoxy-5-(2-propen-1-yl)-6,8-dioxabicyclo[3.2.1]octane, $[\alpha]_D$ -33.7°; $^{13}C$-nmr data: 132.2 ($CH=CH_2$), 118.0 ($CH=CH_2$), 107.6 (C-5), 76.1, 75.6 and 73.4 (C-1, C-2 and C-4) 75.1 ($RCH_2O$), 66.2 (C-7), 56.8 ($OCH_3$), 37.8 ($CH_2CH=CH_2$), 37.8 (CH in cyclo-$C_6H_{11}$), 30.0, 29.9, 26.5 and 25.7 (x 2) ($CH_2$ in cyclo-$C_6H_{11}$) and 23.4 (C-3).

2. (1R,2S,4R)-2-cyclohexanemethyloxy-4-ethoxy-5-(2-propen-1-yl)-6,8-dioxabicyclo[3.2.1]octane, $[\alpha]_D$ -38.6°; $^{13}C$-nmr data: 132.4 ($CH=CH_2$), 118.0 ($CH=CH_2$), 107.7 (C-5), 75.2, 74.0 and 73.5 (C-1, C-2 and C-4), 74.2 ($RCH_2O$), 66.2 ($OCH_2CH_3$), 64.8 (C-7), 37.9 (CH in cyclo-$C_6H_{11}$), 37.6 ($CH_2CH=CH_2$), 30.1, 29.9, 26.6 and 25.7 (x2) ($CH_2$ in cyclo-$C_6H_{11}$), 24.3 (C-3) and 15.4 ($OCH_2CH_3$).

Example 8

(1R,2S,4R)-4-Alkoxy-2-cyclohexanemethyloxy-5-[2-(p-toluenesulphonyloxy)ethyl]-6,8-dioxabicyclo[3.2.1]-octanes

By the procedure in Preparative Example 6 for converting (1R,2S,4R)-2-benzyloxy-4-methoxy-5-(2-propen-1-yl)-6,8-dioxabicyclo[3.2.1]octane into its 5-[2-(p-toluenesulphonyloxy)ethyl]-analogue, (1R,2S,4R)-2-cyclohexanemethyloxy-4-methoxy-5-(2-prop-1-enyl)-6,8-dioxabicyclo[3.2.1)octane (Example 7.1) and its 4-ethoxy-analogue (Example 7.2) were separately converted into the title compounds. In this way the following compounds were obtained:

1. (1R,2S,4R)-2-cyclohexanemethyloxy-4-methoxy-5-[2-(p-toluenesulphonyloxy)ethyl]-6,8-dioxabicyclo[3.2.1]octane as a yellow oil.

2. (1R,2S,4R)-2-cyclohexanemethyloxy-4-ethoxy-5-[2-(p-toluenesulphonyloxy)ethyl]-6,8-dioxabicyclo[3.2.1]octane, as a syrup.

Example 9

(1R,2S,4R)-2-Cyclohexanemethyloxy-4-ethoxy-5-(2-fluoroethyl)-6,8-dioxabicyclo[3.2.1]octane

and

(1R,2S,4R)-2-Cyclohexanemethyloxy-4-ethoxy-5-vinyl-6,8-dioxabicyclo[3.2.1]octane

(1R,2S,4R)-2-Cyclohexanemethyloxy-4-ethoxy-5-[2-(p-toluenesulphonyloxy)ethyl]-6,8-dioxabicyclo-[3.2.1]octane (Example 8.2) (1.43 g) and tetrabutylammonium fluoride [9.4 ml (1M in THF) dried as in Preparative Example 6] were heated in acetonitrile (100 ml) under reflux for 1 h. The mixture was evaporated onto silica gel and chromatographed in the usual way. The eluant contained both title compounds and crystallised to a white solid. This mixture was applied in methanol (3 ml) to a column of Dowex 50 WX 8, 200-400 mesh resin in the Ag(I)-form (35 ml). Elution with distilled water yielded the first title compound as a syrup; $^{13}C$-nmr data: 107.1 (d, C-5, $J_{C,F}$ 6 Hz), 80.0 (d, $CH_2F$, $J_{C,F}$ 162.6 Hz), 76.4, 74.8 and 73.3 (C-1, -2 and -4), 75.3 ($RCH_2O$), 66.3 (C-7), 64.9 ($OCH_2CH_3$), 37.7 (CH in cyclo-$C_6H_{11}$), 33.8 (d,

$CH_2CH_2F$, JC,F 20.2Hz), 30.2, 29.9, 26.6 and 25.7 (x2) ($CH_2$ in cyclo-$C_6H_{11}$), 24.1 (C-3) and 15.3 ($OCH_2CH_3$). Further elution with 5-10% acetonitrile in water yielded the second title compound as a syrup, $^{13}C$-nmr data: 134.4 ($CH=CH_2$), 116.9 ($CH=CH_2$), 105.7 (C-5), 76.4, 75.6 and 73.1 (C-1, C-2 and C-4), 75.1 ($RCH_2O$), 66.0 ($OCH_2CH_3$), 65.1 (C-7), 37.6 (CH in cyclo-$C_6H_{11}$), 30.1, 29.9, 26.6 and 25.7 (x2) ($CH_2$ in cyclo-$C_6H_{11}$), 24.9 (C-3) and 15.3($OCH_2CH_3$).

Example 10

(1R,2S,4R)-5-(2-Chloroethyl)-2-cyclohexanemethyloxy-4-methoxy-6,8-dioxabicyclo[3.2.1]octane

A solution of (1R,2S,4R)-2-cyclohexanemethyloxy-4-methoxy-5-[2-(p-toluenesulphonyloxy)ethyl]-6,8-dioxabicyclo[3.2.1]octane (Example 8.1) (1.5 g) and tetrabutylammonium chloride (3.0 g) in acetonitrile (50 ml) was heated under reflux for 4 h. The solvent was then removed by evaporation and the residue was partitioned between chloroform and water. The aqueous phase was extracted with chloroform and the combined organic phase was dried ($Na_2SO_4$) and evaporated. The residue was subjected to flash chromatography to yield the title compound as white crystals; $^{13}C$-nmr data; 107.1 (C-5), 77.0, 76.6 and 73.3 (C-1, -2 and -4), 75.3 ($RCH_2O$), 66.4 (C-7), 57.1 (OMe), 38.8, 37.6, 36.2, 30.1, 29.9, 26.6, 25.7 (x2) and 23.3 (cyclo-$C_6H_{11}$, $CH_2CH_2Cl$ and C-3).

Example 11

(1R,2S,4R)-2-Cyclohexanemethyloxy-4-methoxy-5-vinyl-6,8-dioxabicyclo[3.2.1]octane

A solution of (1R,2S,4R)-2-cyclohexanemethyloxy-4-methoxy-5-[2-(p-toluenesulphonyloxy)ethyl]-6,8-dioxabicyclo[3.2.1]octane (Example 8.1) (1.5 g) and sodium iodide (1.5 g) in acetone (100 ml) was heated under reflex for 16 h. Saturated sodium chloride solution was added and the mixture was extracted with chloroform (x3). The combined extracts were dried ($MgSO_4$) and evaporated to a syrup. This syrup was dissolved in a toluene solution of 1,8-diazabicyclo[5.4.0]undec-7-ene (2 M, 100 ml) and brought to reflux for 0.5 h, then chloroform was added and the mixture washed with sodium hydroxide (1 M), hydrochloric acid (2 M) and water. The organic phase was dried ($MgSO_4$) and evaporated onto silica gel. Flash chromatography gave the title compound as a white solid (0.6 g); $^{13}C$-nmr data: 13.3 ($CH=CH_2$), 117.2 ($CH=CH_2$), 77.3, 76.3 and 73.1 (C-1, C-2 and C-4), 75.3 ($RCH_2O$), 66.0 (C-7), 57.4 ($OCH_3$), 37.6 (CH in cyclo-$C_6H_{11}$), 30.1, 30.1, 26.6 and 25.7 (x 2) ($CH_2$ in cyclo-$C_6H_{11}$) and 24.1 (C-3).

Example 12

(1R,2S,4R)-2-Benzyloxy-4-methoxy-1-methyl-6,8-dioxabicyclo[3.2.1]octane

A) 4-0-Benzyl-3-deoxy-2-0-methyl-D-ribo-hexose ethylene dithioacetal

To add solution of 1,6-anhydro-4-0-benzyl-3-deoxy-2-0-methyl-$\beta$-D-ribo-hexopyranose (EP 0229034A, Example 3.1) (1.0 g) in dry dichloromethane (30 ml) containing 1,2-ethanedithiol (1.0 ml) at -10°C, boron trifluoride diethyl etherate (1.1 ml) was added dropwise. The resulting solution was stirred, allowed to warm to 10°C over 2 h, then washed with 0.5M aqueous sodium hydroxide (x 2), water, dried ($MgSO_4$) and concentrated. The residue was flash chromatographed to give the syrupy title compound A (1.19 g, 86%) $[\alpha]_D$ + 47°; $^{13}C$ nmr data: 137.9, 128.4, 128.0, 127.8 (aromatic), 81.0 and 77.0 (C-2 and C-4), 72.3 and 71.4 (C-5 and $ArCH_2O$), 63.7 (C-6), 58.0 ($OCH_3$), 55.5 [$CH(SR)_2$], 38.6 ($CH_2S$) and 30.8 (C-3).

B) 4-0-Benzyl-6-0-tert-butyldiphenylsilyl-3-deoxy-2-0-methyl-D-ribo-hexose ethylene dithioacetal

To a stirred solution of 4-0-benzyl-3-deoxy-2-0-methyl-D-ribo-hexose ethylene dithioacetal (3.2 g), prepared as in part A, in N,N-dimethyformamide (25 ml), tert-butylchlorodiphenylsilane (2.7 g, 1.05 eq.) and imidazole (1.9 g, 3 eq.) were added. After 2 h water (200 ml) was added and the mixture was extracted twice with 100 ml of chloroform. The combined organic extracts were washed with 1M aqueous hydorchloric acid, saturated aqueous sodium hydrogen carbonate, water (x 2) then dried ($MgSO_4$) and concentrated to a syrup. Flash chromatography gave pure title compound B as a syrup (4.64 g, 86%), $[\alpha]_D$ + 9.5°; $^{13}$C-nmr data: 138.2, 135.6, 133.3, 129.7, 128.3, 127.9, 127.7 and 127.6 (aromatic), 82.0 and 76.2 (C-2 and C-4), 73.2 (C-5), 71.5 ($PhCH_2O$), 64.9 (C-6), 58.2 and 56.2 [$OCH_3$ and CH(S-Alkyl)$_2$], 38.6 ($SCH_2$), 32.2 (C-3), 26.9 [C-($CH_3$)$_3$] and 19.2 [C($CH_3$)$_3$].

C) 4-0-Benzyl-3-deoxy-5-C-methyl-2-0-methyl-6-0-tert-butyldiphenylsilyl-D-ribo-hexose ethylene dithioacetal

A solution of oxalyl chloride (0.5 ml) in dry tetrahydrofuran was stirred and cooled under argon to -70°C and dimethylsulphoxide (0.5 ml) in dry tetrahydrofuran was stirred and cooled under argon to -70°C and dimethylsulphoxide (0.5 ml) was added dropwise. After 10 min a solution of 4-0-benzyl-3-deoxy-2-0-methyl-6-0-tert-butyldiphenylsilyl-D-ribo-hexose ethylene dithioacetal (1.5 g), prepared as in Part B, in tetrahydrofuran was added dropwise and the solution was stirred for 30 min at -70°C. Triethylamine (2.5 ml) was added and the resulting mixture was allowed to warm to ambient temperature, stirred at this temperature for 10 min then cooled to -70°C. A solution of methylmagnesium iodide (3M in ether, 2.5 ml, 3 eq.) was added dropwise, the mixture was stirred at -70°C for 1 h and poured onto 1 M aqueous hydrochloric acid. The resulting mixture was immediately extracted three times with chloroform and the combined extracts were washed with saturated aqueous sodium hydrogen carbonate, dried ($MgSO_4$) and concentrated. The residue was flash chromatographed to give the syrupy title compound C (1.31 g, 85%) as a single isomer; $^{13}$C-nmr data: 138.6, 135.6, 132.9, 129.8, 128.1, 127.7, 127.5 and 127.3 (aromatic), 83.3 and 78.2 (C-2 and C-4), 75.2 (C-5), 72.2 ($PhCH_2O$), 68.8 (C-6), 58.4 and 56.6 [$OCH_3$ and CH(S-Alkyl)$_2$], 38.5 ($SCH_2$), 26.9 [C($CH_3$)$_3$], 19.3 [C($CH_3$)$_3$] and 18.8 ($CH_3$).

D) (1R,2S,4R)-2-Benzyloxy-4-methoxy-1-methyl-6,8-dioxabicyclo[3.2.1]octane

A solution of 4-0-benzyl-3-deoxy-5-C-methyl-2-0-methyl-6-0-tert-butyldiphenylsilyl-D-ribo-hexose ethylene dithioacetal (2.3 g), prepared as in part C, in tetrahydrofuran containing tetrabutylammonium fluoride (1.5 eq.) was stirred at ambient temperature for 1 h then concentrated to a syrup which was flash graphed to give syrupy 4-0-benzyl-3-deoxy-5-C-methyl-2-0-methyl-D-ribo-hexose ethylene dithioacetal (1.2 g, 90%). This material was added dropwise, as a solution in methanol, to a stirred solution of N-bromosuccinimide (1.8 g) in methanol. The entire solution was stirred at ambient temperature for 1 h then added to saturated aqueous sodium hydrogen carbonate. The resulting mixture was extracted twice with chloroform and the combined extracts were dried ($MgSO_4$) and evaporated. The residue was dissolved in toluene (30 ml) containing camphorsulphonic acid (0.01 g) and the solution heated under reflux for 1 h. After cooling, the solution was washed with saturated aqueous sodium hydrogen carbonate, dried ($MgSO_4$) and evaporated. Flash chromatography of the residue gave the title compound as a syrup (0.11 g, 10% overall), $[\alpha]_D$ + 39°; $^{13}$C-nmr data: 138.1, 128.2, and 127.6 (aromatic), 100.9 (C-5), 80.8 (C-2), 74.0 and 73.9 (C-2 and C-4), 71.5 and 71.1 ($PhCH_2O$ and C-7), 57.2 ($OCH_3$), 23.6 (C-3) and 18.6 ($CH_3$).

Example 13

(1RS,2S,4R,7S)-2-Benzyloxy-4-methoxy-7-methyl-6,8-dioxabicyclo[3.2.1]octane

A) Methyl 4-0-benzyl-3,7-dideoxy-2-0-methyl-β -L-talo-heptopyranoside

A stirred solution of oxalyl chloride (1.5 ml) in dry tetrahydrofuran (30 ml) under argon was cooled to -70 °C and treated dropwise slowly with dimethylsulphoxide (1.5 ml). Ten min later a solution of methyl 4-0-benzyl-3-deoxy-2-0-methyl-α-D-ribo-hexopyranoside and its 6-0-benzyl isomer (Preparative Example 8) (2.8 g) in tetrahydrofuran (20 ml) was added dropwise and after 10 min triethylamine (5.0 ml) was added. The resulting mixture was stirred and allowed to warm to -10 °C by which time tlc showed that there was no starting hexopyranoside present. The reaction mixture was then cooled to -40 °C and a solution of methylmagnesium iodide (15 ml, 2M in ether) was added dropwise. After 15 min the mixture was poured slowly onto 200 ml of 2M aqueous hydrochloric acid then extracted twice with chloroform. The combined extracts were washed with saturated aqueous sodium hydrogen carbonate, dried ($MgSO_4$) and evaporated. Flash chromatography of the residue gave the syrupy title compound A (1.55 g).

B) (1RS,2S,4R,7S)-2-Benzyloxy-4-methoxy-7-methyl-6,8-dioxabicyclo[3.2.1]octane

Methyl 3-0-benzyl-3,7-dideoxy-2-0-methyl-β -L-talo-heptopyranoside (1.55 g), as prepared in Part A, was dissolved in toluene (30 ml), containing p-toluenesulphonic acid (0.10 g), and heated under reflux for 8 h. The resulting black solution was washed with aqueous sodium hydrogen carbonate, dried ($MgSO_4$) and evaporated. Flash chromatography of the residue gave the syrupy title compound B (a 7-exo-isomer) (0.76 g) $[\alpha]_D$ -39.0°; $^{13}$C-nmr data: 138.3, 128.3, and 127.5 (aromatic), 100.6 (C-5), 79.7, 74.5, 72.7 and 71.3 (C-1, C-2, C-4, and C-7), 70.2 ($PhCH_2O$), 57.2 ($OCH_3$), 24.5 (C-3) and 21.0 ($CH_3$).

## Example 14

(1RS,2S,4R,7S)-2-Benzyloxy-7-ethyl-4-methoxy-6,8-dioxabicyclo[3.2.1]octane

By the same procedure as in Example 13, except using ethylmagnesium bromide in place of methylmagnesium iodide, the title compound (a 7-exo-isomer was obtained as a syrup with $[\alpha]_D$ -34°; $^{13}$C-nmr data: 138.3, 128.3, 127.6 and 127.5 (aromatic), 100.5 (C-5), 78.1, 78.1, 74.5 and 71.6 (C-1, C-2, C-4 and C-7), 70.3 ($PhCH_2$), 57.2 ($OCH_3$), 27.8 ($CH_2CH_3$), 24.7 (C-3) and 9.6 ($CH_2CH_3$).

## Example 15

(1S,2S,4R,7R)-2-Benzyloxy-4-methoxy-7-methyl-6,8 dioxabicyclo[3.2.1]octane and its (7S)-isomer

A) Methyl 4-0-benzyl-3,7-dideoxy-2-0-methyl-α-D-ribo-heptopyranosid-6-ulose

A stirred solution of oxalyl chloride (1.0 ml) in dry tetrahydrofuran (10 ml) under argon was cooled to -70 °C and a solution of dimethylsulphoxide (1.2 ml) in dry tetrahydrofuran (10 ml) was added slowly. After 10 min a solution of methyl 4-0-benzyl-3,7-dideoxy-2-0-methyl-β-L-talo-heptopyranoside (Example 13, Part A) (1.56 g) in dry tetrahydrofuran (10 ml) was added slowly. After 15 min triethylamine (3 ml) was added and the thick suspension was warmed slowly to room temperature and partitioned between ether and dilute hydrochloric acid. The ether layer was washed with water (x 2), dried ($Na_2SO_4$) and evaporated to give title compound A as a pale yellow oil (0.75 g), single component by tlc.

B) (1RS,2S,4R,7R)-2-Benzyloxy-4-methoxy-7-methyl-6,8-dioxabicyclo[3.2.1]octane and its (7S)-isomer

The ketone from Part A (0.35 g) was dissolved in absolute ethanol and sodium borohydride (0.135 g, 3 eq.) was added. The solution was stirred at room temperature for 1 day, diluted with aqueous ammonium chloride, and extracted with ether. The ether extract was dried ($Na_2SO_4$) and concentrated to an oil (0.32 g), shown by tlc to be predominantly the single product methyl 4-0-benzyl-3,7-dideoxy-2-0-methyl-α-D-allo-heptopyranoside of slightly greater mobility than the L-talo-isomer (see Example 13, Part A) also present as a minor component. This oil was dissolved in dry toluene, and 4A molecular sieve (7 g) and Amberlyst 15 acidic ion-exchange resin (1.0 g) were added. The mixture was stirred at 60 °C for 2 days and at room temperature for 2 days then filtered. The solids were washed with ethyl acetate and the combined filtrate evaporated to produce a brown oil which was subjected to preparative tlc (silica gel) to yield the first title compound (the 7-endo-isomer) in a syrupy mixture (0.023 g) with its (7S)-exo-isomer (cf. Example 13), in a ratio ca. 65:35; $^1$H-nmr data from the mixture for the (7R)-isomer: 5.46 (H-1), 4.64 ($PhCH_2O$) and 1.25 (d,C-$CH_3$); and for the (7S)-isomer: 5.54 (H-1), 4.66 ($PhCH_2O$) and 1.27 (d, C-$CH_3$).

Example 16

1S,2S,4R)-2-Benzyloxy-4-methoxy-7,7-dimethyl-6,8-dioxabicyclo[3.2.1]octane

A solution of methyl 4-0-benzyl-3,7-dideoxy-2-0-methyl-α-D-ribo-heptopyranosid-6-ulose (Example 15, Part A) (0.43 g) in dry tetrahydrofuran (10 ml) was added to a solution of methylmagnesium iodide in ether, cooled to 0 °C under argon [5 ml; prepared from magnesium turnings (0.053 g) and methyl iodide (0.092 g)-]. After 30 min the mixture was partitioned between ether and aqueous ammonium chloride. The ether extract was dried ($Na_2SO_4$) and evaporated to an oil (0.36 g). A portion of this oil (0.31 g) was stirred in toluene solution (10 ml) with 4A molecular sieve (2.0 g) and Amberlyst 15 acidic ion-exchange resin (0.5 g) for 2 days at room temperature. Filtration and evaporation yielded the crude title compound as a clear colourless oil (0.14 g), which was purified by preparative tlc (silica gel, eluant 3:7 ethylacetate/hexane) to yield the pure title compound (0.032 g); $^1$H-nmr data: 7.37 (5H,m,Ph), 5.48 (1H,narrow m, H-5), 4.64 (2H,narrow m, $PhCH_2O$), 4.01 (1H,narrow m, H-1), 3.42 (4H,m,$OCH_3$ and H-2), 3.23 (1H,m,H-4), 2.01 (2H,m,H-3,3′), 1.31 and 1.30 (2 x 3H,s,C-$CH_3$).

Example 17

(1S,2S,4R,7R)-2-Benzyloxy-7-ethyl-4-methoxy-6,8-dioxabicyclo[3.2.1]octane

The title compound (the 7-endo-isomer was prepared from methyl 4-0-benzyl-3-deoxy-2-0-methyloxy-α-D-ribo-hexopyranoside and its 6-O-benzyl isomer (Preparative Example 8) in the same way as described for the 7-methyl analogue (see Example 13, part A and Example 15) but using ethylmagnesium bromide instead of methylmagnesium chloride. It was obtained free of the 7S-isomer. $^1$H-nmr data: 0.92 (3H,d, J 6Hz), 1.36-1.51 (1H,m), 1.60-1.76 (1H,m), 2.00 (2H,m), 3.20 (1H,m), 3.40 (1H,m), 3.43 (3H,s), 3.93 (1H,m), 4.36 (1H,m) 4.66 (2H,m), 5.47 (1H,s) and 7.24-7.44 (5H,m).

Example 18

(1S,2S,4R,7S)-2-Benzyloxy-4-methoxy-7-butyl-6,8-dioxabicyclo[3.2.1)octane

By the same procedure as in Example 13, except using butylmagnesium bromide in place of methylmagnesium iodide, the title compound was obtained as the pure 7-exo-isomer; $^1$H-nmr data: 0.89 (3H,br t), 1.10-1.74 (6H,m), 1.84 (1H,dt,$J_1$ = 15Hz,$J_2$ = 5Hz), 2.03 (1H,d,J 15Hz), 3.12 (1H,m), 3.27 (1H,m), 3.42 (3H,s), 3.74 (1H,t,J 6Hz), 4.20 (1H,s), 4.66 (2H,s), 5.50 (1H,s) and 7.20-7.40 (5H,m).

Example 19

(1RS,2SR,4RS,7RS)-2-Benzyloxy-4-methoxy-5,7-dimethyl-6,8-dioxabicyclo[3.2.1]octane

A) (2RS,3RS)-1-Benzyloxy-2,3-epoxybutane

To a stirred suspension of powdered potassium hydroxide (22.4 g, 400 mmol) in DMSO (50 ml) was added crotyl alcohol (7.2 g, 100 mmol) followed, dropwise, by benzyl bromide (13 ml, 110 mmol) in DMSO (40 ml). Ethyl acetate (750 ml) was added, the mixture was filtered, and the filtrate washed four times with water, dried ($MgSO_4$), filtered, and concentrated to yield E-1-benzyloxybut-2-ene as an oil (20 g) which was used in the preparation of the title compound without further purification. To a stirred solution of E-1-benzyloxybut-2-ene (20 g) in dichloromethane (150 ml) was added 3-chloroperoxybenzoic acid (40 g). After 15 h, the mixture was diluted with ether, washed twice each with saturated aqueous sodium metabisulphite, saturated aqueous sodium carbonate, and water, dried ($MgSO_4$), filtered, and concentrated to yield an oil (17.4 g) which was chromatographed on silica eluted with hexane - ethyl acetate (5:1) to yield the title compound A as a colourless liquid (9.4 g).

B) (4RS,5SR)-4-Benzyloxymethyl-2,2,5-trimethyl-1,3-dioxolane

To a stirred solution of the title compound from part A (8.3 g, 47 mmol) and acetone (3.8 ml, 1.1 eq) in dichloromethane (50 ml) at -35 °C was added dropwise tin(IV) chloride (1.1 ml, 9.4 ml). After 5 min, saturated aqueous sodium bicarbonate was added to quench the reaction, and the product was extracted three times with dichloromethane. The organic phase was dried ($MgSO_4$), filtered, and concentrated to yield an oil (9.75 g) which was chromatographed on silica eluted with hexane - ethyl acetate (8:1) to afford pure title compound B (7.82 g, 71%).

C) (4′RS,5′SR,5RS)-5-Hydroxy-2,2-dimethoxy-5-(2,2,5-trimethyl-1,3-dioxolan-4-yl)pentan-3-one

Title compound from part B (13 g) in ethyl acetate containing a drop of triethylamine was hydrogenated at 350 atmospheres and 100 °C for 20 h using 10% Pd on C as catalyst. The catalyst was removed by filtration and the filtrate concentrated under reduced pressure. The crude product so obtained was distilled in a Kugelrohr apparatus at 200 °C (bath temp.) and 12 mm Hg giving an oil shown by gc to contain approximately 50% of (4RS,5SR)-4-hydroxymethyl-2,2,5-trimethyl-1,3-dioxolane and used without further purification in the preparation of title compound C. Dimethylsulphoxide (5.4 ml, 77 mmol) was added dropwise to a stirred solution of oxalyl chloride (3.2 ml, 38 mmol) in dry THF at -78 °C under $N_2$. After 5 min, crude (4RS,5SR)-4-hydroxymethyl-2,2,5-trimethyl-1,3-dioxolane (50%; 8 g, ca. 34 mmol) in dry THF (10 ml) was added followed after 15 min, by triethylamine (23 ml, 170 mmol). The mixture was allowed to warm to -10 °C and filtered under $N_2$. The solution of aldehyde so prepared was cooled to -78 °C and added rapidly to the lithium enolate of 3,3-dimethoxybutan-2-one [prepared from 3,3-dimethoxybutan-2-one (4.5 g, 34 mmol) and lithium hexamethyldisilazide (1M in THF; 43 ml, 42.5 mmol) at -78 °C under $N_2$]. After 30 min, the reaction was quenched by addition of acetic acid (8 ml) in THF (20 ml). Saturated aqueous ammonium chloride was added, and the mixture allowed to warm to room temperature. The organic layer

was separated, and the aqueous phase extracted with ethyl acetate (3 x 50 ml). The combined organic phase was washed with saturated aqueous sodium bicarbonate, dried ($MgSO_4$), filtered, and concentrated to yield an oil (12 g) which was chromatographed on silica eluted with hexane-ethyl acetate (2:1) to afford pure title compound C as a liquid which crystallised on standing (5.46 g, 58%), m.p. 75-78° C.

D) (4′RS,5′RS,5SR)-5-Benzyloxy-2,2-dimethoxy-5-(2,2,5-trimethyl-1,3-dioxolan-4-yl)pentan-3-ol

To a stirred solution of title compound C (1.04 g, 4 mmol) in dichloromethane (15 ml) at -78° C under nitrogen was added boron trifluoride etherate (50 μl) followed by phenyl diazomethane (ca. 1 M; 40 ml). Reaction was monitored by tlc and further boron trifluoride etherate (50 μl) was added until tlc indicated that reaction was complete. The reaction was quenched by addition of excess acetic acid and the mixture was allowed to warm to room temperature. Water (80 ml) was added, the organic layer was separated, and the aqueous phase extracted with dichloromethane (3 x 60 ml). The organic phases were combined, dried ($MgSO_4$), and concentrated to yield an oil which was dissolved in ethanol (50 ml) and mixed with sodium borohydride (1.2 g). When tlc indicated reaction was complete, water was added and the solvents removed under reduced pressure. The residue was partitioned between water and dichloromethane, the organic phase separated, and the aqueous phase further extracted twice with ethyl acetate. The combined organic phase was dried ($MgSO_4$) and concentrated to yield a yellow oil (3.68 g) which was chromatcgraphed on silica eluted with hexane - ethyl acetate (2:1) to afford pure title compound D (1.01 g, 69%) as a 60:40 mixture of epimers at the 3-position ($^1$H-nmr evidence).

E) (1RS,2SR,4RS,7RS)-2-Benzyloxy-4-hydroxy-5,7-dimethyl-6,8-dioxabicyclo[3.2.1]octane

A solution of title compound D (900 mg) in acetic acid-water (9:1 v/v; 15 ml) was heated at 70° C. After 40 min, the solvent was removed under reduced pressure, the residue azeotroped twice with toluene and chromatographed on silica eluted with hexane - ethyl acetate (1.5:1) to yield title compound E (305 mg) followed by its (4SR)-epimer (230 mg) (white solid, m.p. 220-221° C) (combined yield 83%).

F) (1RS,2SR,4RS,7RS)-2-Benzyloxy-4-methoxy-5,7-dimethyl-6,8-dioxabicyclo[3.2.1]octane

To a stirred suspension of powdered potassium hydroxide (180 mg, 3.2 mmol) in DMSO (4 ml) was added title compound E (210 mg, 0.8 mmol) followed by iodomethane (496 μl, 8 mmol). After 30 min, the mixture was diluted with ethyl acetate, filtered, and washed four times with water. The organic phase was dried ($MgSO_4$) and concentrated to yield an oil (173 mg) which was purified by chromatography on silica eluted with hexane - ethyl acetate (2:1). The title compound (with a 7-endo-methyl group) was obtained as an oil (173 mg, 79%); $^1$H-nmr data: 1.21 (3H,d), 1.54 (3H,s), 1.94 (1H,dt), 2.14 (1H,d,J 16Hz), 3.08 (1H,d), 3.38 (1H,d), 3.42 (3H,s), 4.24 (1H,m), 4.35 (1H,m), 4.63 (2H, AB-system,J = 13Hz) and 7.23-7.41 (5H,m).

Example 20

(1RS,2SR,4RS)-2-Benzyloxy-4-methoxy-1,5-dimethyl-6,8-dioxabicyclo[3.2.1]octane

A) (4′RS,5SR)-5-Hydroxy-2,2-dimethoxy-5-(2,2,4-trimethyl-1,3dioxolan-4-yl)pentan-3-one

Dimethylsulphoxide (6.3 ml, 88 mmol) in dry THF (6 ml) was added dropwise to a stirred solution of oxalyl chloride (3.9 ml, 44 mmol) in dry THF (20 ml) at -78° C under argon. After 2 min, (4RS)-4-hydroxymethyl-2,2,4-trimethyl-1,3-dioxolane (GB 1293546) (5.9 g, 40 mmol) in dry THF (10 ml) was added followed, after 25 min, by triethylamine (28 ml, 200 mmol). The mixture was allowed to warm to room temperature and filtered under argon. The solution of the aldehyde so prepared was cooled to -78° C and added rapidly to the lithium enolate of 3,3-dimethoxybutan-2-one [prepared from 3,3-dimethoxybutan-2-one

(4.85 g, 37 mmol) and lithium hexamethyldisilazide (1M in hexane; 37 ml, 37 mmol) at -78°C under argon]. After 30 min, the reaction was quenched by addition of acetic acid (2.1 ml) in THF (10 ml). Saturated aqueous sodium bicarbonate was added, and the mixture allowed to warm to room temperature. The organic layer was separated, and the aqueous phase extracted with ethyl acetate (3 x 100 ml). The combined organic phase was washed with saturated aqueous sodium bicarbonate, dried ($Na_2SO_4$), filtered, and concentrated to give an oil (9.1 g) which was chromatographed on silica eluted with hexane-ethyl acetate (4:1 increasing) to afford title compound A (6.07 g, 55%).

B) (4′RS,5SR)-5-Benzyloxymethoxy-2,2,3-trimethoxy-5-(2,2,4-trimethyl-1,3-dioxolan-4-yl)pentane

Benzyloxymethyl chloride (10.24 g, ca. 33 mmol) was added to a solution of the title compound from part A (3 g, 10.9 mmol) and N,N-dimethylaniline (2.64 g, 21.8 mmol) in dichloromethane (15 ml) and the mixture was stirred for 15 h. Saturated aqueous sodium bicarbonate was added and the mixture extracted with ethyl acetate, dried ($MgSO_4$), filtered, and concentrated to yield an oil. The residue was dissolved in ethanol (50 ml) and mixed with sodium borohydride (1.24 g). After 2 days, water was added, the solvent removed under reduced pressure, and the residue partitioned between ethyl acetate and water. The organic phase was separated, washed with water, dried ($MgSO_4$), and concentrated to yield a yellow oil (4.34 g) which was purified by chromatography on silica eluted with hexane - ether. (4′RS,5SR)-5-Benzyloxymethoxy-2,2-dimethoxy-5-(2,2,4-trimethyl-1,3-dioxolan-4-yl)pentan-3-ol was obtained as a pale yellow oil (4.34 g, 100%), indicated by $^1$H-nmr to be a 6:4 mixture of epimers at the 3-position. Sodium hydride (50% in oil; 0.43 g,9 mmol) was added in portions to a stirred solution of these pentan-3-ol derivatives (3 g, 7.5 mmol) in dry THF under argon at 0°C. After 5 min, iodomethane (0.93 ml, 15 mmol) was added. Tlc indicated reaction was only 50% complete in 1 h so further iodomethane (0.46 ml) and sodium hydride (50% m oil, 0.22 g) were added after which tlc showed complete reaction (1.5 h). The reaction was quenched with water, extracted with ethyl acetate, dried ($MgSO_4$), filtered, and concentrated to give a brown oil (3.25 g). Chromatography on silica eluted with hexane - ethyl acetate (9:1) afforded title compound B as a colourless oil (1.76 g, 57%) shown by $^1$H-nmr to be a 6:4 mixture of epimers at the 3-position.

C) (1RS,2SR,4RS)-2-Hydroxy-4-methoxy-1,5-dimethyl-6,8-dioxabicyclo[3.2.1]octane

The title compound from part B (1.5 g) was dissolved in trifluoro-acetic acid (4.5 ml) and kept for 20 h at room temperature. The solvent was removed under reduced pressure and the residue purified by chromatography on silica eluted with hexane - ethyl acetate (9:1 increasing) to yield title compound C (274 mg, 34%) followed by its (4SR)-epimer (172 mg, 22%).

D) (1RS,2SR,4RS)-2-Benzyloxy-4-methoxy-1,5-dimethyl-6,8-dioxabicyclo[3.2.1]octane

The title compound from part C (274 mg) was dissolved in DMSO (15 ml) and powdered potassium hydroxide (327 mg) added followed by benzyl bromide (498 mg). After 15 h, water (15 ml) was added, and the product extracted with ethyl acetate. The extracts were dried ($MgSO_4$), filtered, and concentrated to yield a yellow oil (1.2 g). Chromatography on silica eluted with hexane - ethyl acetate (9:1 increasing) gave the title compound as a pale yellow oil (335 mg, 83%); $^1$H-nmr data: 1.38 (3H,s), 1.53 (3H,s), 1.73 (1H,dt), 2.23 (1H,d,J 15Hz), 3.04 (1H,d), 3.13 (1H,d), 3.40 (3H,s), 3.46 (1H,d,J 10Hz), 3.67 (1H,d,J 10Hz), 4.5 (1H,d,J 13Hz), 4.74 (1H,d,J 13Hz) and 7.24-7.40 (5H,m).

Example 21

(1S,2S,4R,7S)-2-Benzyloxy-4-methoxy-7-vinyl-6,8-dioxabicyclo[3.2.1]octane

A solution of camphorsulphonic acid (1.5 g) and methyl 4-0-benzyl-3-deoxy-2-0-methyl-6-(S)-C-vinyl-α-D-ribo-hexopyranoside (see Preparative Example 9) (1.9 g) in toluene (60 ml) was heated under reflux for 1.5 h. Conventional processing followed by chromatography on silica gel afforded 1.39 g (81%) of title compound (the 7-exo-isomer), $[\alpha]_D$ -52.2°; $^{13}$C-nmr data: 138.2, 128.3 and 127.5 (aromatics), 136.8 ($CH=CH_2$), 116.8 ($CH=CH_2$), 101.0 (C-5), 79.2 and 77.4 (C-1 and -4), 74.4 (C-2), 71.3 (C-7), 70.3 ($OCH_2Ar$), 57.3 ($OCH_3$) and 24.6 (C-3).

Example 22

(1S,2S,4R,7R)-2-Benzyloxy-4-methoxy-7-vinyl-6,8-dioxabicyclo[3.2.1]octane

A solution of camphorsulphonic acid (0.8 g) and methyl 4-0-benzyl-3-deoxy-2-0-methyl-6(R)-C-vinyl-α-D-ribo-hexopyranoside (see Preparative Example 9) (1.1 g) in toluene (40 ml) was heated under reflux for 1 h. Conventional processing followed by chromatography on silica gel afforded 0.876 g (88%) of the title compound (a 7-endo-isomer) $[\alpha]_D$ -20.7°; $^{13}$C-nmr data: 138.3, 128.3, 127.5, 127.4 (aromatics), 131.6 ($CH=CH_2$), 118.7 ($CH=CH_2$), 100.6 (C-5), 78.0, 77.4 (C-1 and -4), 74.6 (C-2), 70.5 ($OCH_2Ar$), 69.4 (C-7), 57.3 ($OCH_3$) and 25.1 (C-3).

Example 23

(1S,2S,4R,7S)-2-(2-Chlorobenzyloxy)-4-methoxy-7-p-toluenesulphonyloxymethyl-6,8-dioxabicyclo[3.2.1]-octane

A) (1S,2S,4R,7S)-2-Benzyloxy-4-methoxy-7-p-toluenesulphonyloxy methyl-6,8-dioxabicyclo[3.2.1)octane

Sodium periodate (3.2 g) and a solution of osmium tetroxide (0.025 g) in water (5 ml) were added to a solution of (1S,2S,4R,7S)-2-benzyloxy-4-methoxy-7-vinyl-6,8-dioxabicyclo[3.2.1]octane (see Example 21) (2.35 g) in ethanol and water (40 ml, 3:1 v/v). The mixture was stirred at room temperature for 2 h and then partitioned between chloroform and water. The aqueous phase was extracted twice more with chloroform and the combined extracts were processed to give a syrup which was dissolved in ethanol (40 ml) and stirred with sodium borohydride (0.35 g) for 0.5 h. The mixture was made slightly acidic with acetic acid and then the solvent was removed. Ethyl acetate was added to the residue and the mixture was filtered through a pad of silica gel and the solids were washed further with ethyl acetate. The combined filtrate was concentrated to a syrup which was dissolved in dichloromethane (50 ml) containing triethylamine (2.4 ml), p-dimethylaminopyridine (0.15 g) and p-toluenesulphonyl chloride (2.3 g). This solution was heated under reflux for 1 h. Normal processing of the reaction followed by chromatography gave 2.38 g (64%) of the title compound A; $^{13}$C-nmr data: 145.1, 138.0, 132.5, 129.9, 128.3, 127.8 and 127.6 (aromatics), 101.1 (C-5), 76.3, 74.0, 73.2 and 71.0 (C-1,-2,-4 and -7), 70.4 ($PhCH_2O$), 69.1 ($CH_2OTs$), 57.2 ($OCH_3$), 24.4 (C-3) and 21.5 ($ArCH_3$).

B) (1S,2S,4R,7S)-2-(2-Chlorobenzyloxy)-4-methoxy-7-p-toluenesulphonyloxymethyl-6,8-dioxabicyclo[3.2.1]-octane

A solution of the product of part A above (2.25 g) in ethanol (200 ml) was stirred with 20% $Pd(OH)_2$ on carbon (0.2 g) in an atmosphere of hydrogen until the reaction was complete. (Hydrogen uptake 120 ml). The solution was filtered through a pad of celite and concentrated. To a solution of the residue in tetrahydrofuran (50 ml) was added 2-chlorobenzyl bromide (2 g) and then sodium hydride (80% dispersion,

0.2 g). The mixture was stirred at room temperature for 2 h, and then was partitioned between chloroform and water. Normal processing followed by chromatography on silica gel afforded the title compound (a 7-exo-isomer) (2.15 g, 88%), $[\alpha]_D$ -45.2°; $^{13}C$-nmr data: 145.2, 135.7, 132.5, 129.9, 129.2, 129.0, 128.6, 127.9 and 126.8 (aromatics) 101.1 (C-5), 76.2, 73.9, 73.2 and 72.1 (C-1, -2, -4 and -7), 68.9 and 67.6 ($ArCH_2O$ and $CH_2OTs$), 57.3 ($OCH_3$), 24.5 (C-3) and 21.6 ($ArCH_3$).

## Example 24

### (1S,2S,4R,7R)-2-(2-Chlorobenzyloxy)-7-chloromethyl-4-methoxy-6,8-dioxabicyclo[3.2.1]octane

A solution of (1S,2S,4R,7S)-2-(2-chlorobenzyloxy-4-methoxy-7-p-toluenesulphonyloxymethyl-6,8-dioxabicyclo[3.2.1]octane (see Example 23) (0.80 g) and tetrabutylammonium chloride (1.0 g) in dry acetonitrile (30 ml) was heated under reflux in an argon atmosphere for 24 h. The solvent was removed and the residue chromatographed on silica gel to give the title compound (a 7-exo-isomer) (0.44 g, 74%), $[\alpha]_D$ -9.8°; $^{13}C$-nmr data: 135.8, 132.6, 129.2, 129.0, 128.6 and 126.8 (aromatics), 101.4 (C-5), 76.9, 76.2, 74.0 and 72.3 (C-1, -2, -4 and -7), 67.6 ($OCH_2Ar$), 57.3 ($OCH_3$), 43.8 ($CH_2Cl$), and 24.6 (C-3).

## Example 25

### (1S,2S,4R,7R)-2-(2-Chlorobenzyloxy)-7-fluoromethyl-4-methoxy-6,8-dioxabicyclo[3.2.1]octane

Tetrabutylammonium fluoride (5 ml, 1.0M in tetrahydrofuran) was dried by evaporation of acetonitrile/toluene (60 ml 1:1 v/v) from it three times under reduced pressure. The residue was dissolved in dry acetonitrile (40 ml) containing (1S,2S,4R,7S)-2-(2-chlorobenzyloxy-)-4-methoxy-7-p-toluenesulphonyloxymethyl-6,8-dioxabicyclo[3.2.1]octane (see Example 23) (0.80 g) and the solution was heated under reflux for 4 h. Removal of the solvent and fractionation of the residue by silica gel column chromatography afforded the title compound (a 7-exo-isomer) (0.192 g, 34%), $[\alpha]_D$ -34.1°; $^{13}C$-nmr data: 135.8, 132.5, 129.1, 129.0, 128.6 and 126.8 (aromatics), 101.0 (C-5), 82.3 (d, $J_{C,F}$ 172Hz, $CH_2F$), 75.8 (d, J 3Hz, C-1), 74.1 (C-4), 73.8 (d, J 25Hz, C-7), 72.3 (C-2), 67.6 ($OCH_2Ar$), 57.3 ($OCH_3$) and 24.7 (C-3).

## Example 26

### (1R,2S,4R)-2-Benzyloxy-1-ethyl-4-methoxy-6,8-dioxabicyclo[3.2.1]octane

A solution of 1,6-anhydro-4-0-benzyl-3-deoxy-2-0-methyl-β--D-ribo-hexopyranose [EP 0229034A, Example 3.1] (5 g) in acetic anhydride (30 ml) was stirred in an ice-bath while boron trifluoride diethyl etherate (5 drops) was added. The solution was stirred at ambient temperature for 2 h, then sodium hydrogen carbonate (48 g) and water (400 ml) were added. The mixture was extracted with chloroform, dried ($MgSO_4$) and the solvent removed by evaporation. The crude diacetate was dissolved in methanol (70 ml) and a solution of sodium methoxide in methanol was added until the pH was 10. The resulting solution was allowed to stand at ambient temperature overnight before acid resin was added until the pH was 7. Filtration and evaporation gave a syrupy diol (4 g). The crude diol, dissolved in dichloromethane (200 ml) was cooled to -20° and boron trifluoride diethyl etherate (4 ml) and 1,3-propanedithiol (4.8 ml) were added dropwise. After 1 h at -20°, sodium hydroxide solution (1M, 200 ml) was added, the mixture was extracted with chloroform, back-extracted with sodium hydroxide solution, washed with water then dried ($MgSO_4$) and evaporated. The residue was purified by flash chromatography to yield a syrupy lactol (3.57 g).

This lactol was dissolved in dimethylformamide (30 ml) and tertbutyldiphenylsilyl chloride (2.8 ml) and imidazole (2 g) were added. After 3 h at room temperature, water was added and the mixture was extracted with chloroform, washed with dilute hydrochloric acid, saturated aqueous sodium hydrogen carbonate, water

then dried ($MgSO_4$) and evaporated. Purification of the resulting residue by flash chromatography gave 4-0-benzyl-6-0-tert-butyldiphenylsilyl-3-deoxy-D-ribo-hexose propylene dithioacetal as a syrup (4.85 g).

This syrup was oxidised by an alternative Swern oxidation (see S.L. Huang, K Omura and D Swern, J Org Chem 1976, 41, 3329). To a stirred solution of dry dimethylsulphoxide (2 ml) in dichloromethane (200 ml) at -78° C was added trifluoroacetic anhydride (3 ml) dropwise, the temperature being kept less than -65° C. After stirring for 10 min, the above syrupy alcohol dissolved in dichloromethane (50 ml) was added dropwise, the temperature being kept below -65° C during the addition and for a further 30 min, when triethylamine (5.7 ml) was added. The reaction mixture was allowed to come to ambient temperature and was then washed with water. The aqueous layer was extracted with chloroform, and the combined organic phase was dried ($MgSO_4$) and evaporated.

The crude ketone so obtained was dissolved in tetrahydrofuran (100 ml), cooled to -78° C under argon, and ethylmagnesium bromide (14 ml, 3M in diethyl ether) was added, slowly, the temperature being kept at less than -65° C. The resulting mixture was poured carefully into saturated aqueous ammonium chloride solution (300 ml), extracted with chloroform (x 3), dried ($MgSO_4$), evaporated and the residue was purified by flash chromatography to yield a C-methyl derivative (3.07 g).

This derivative was then taken up in tetrahydrofuran (5 ml) and added dropwise to a suspension of lead dioxide (1.8 g) in 20% aqueous tetrahydrofuran (60 ml) containing boron trifluoride diethyl etherate (2.5 ml). (See Ghiringhelli, Synthesis 1982, 580-582.) Diethyl ether (200 ml) was added, the solution filtered and the filtrate washed with saturated aqueous sodium hydrogen carbonate, saturated sodium chloride solution, dried ($MgSO_4$) and evaporated. The resulting syrup was dissolved in trifluoroacetic acid at 0° C and stirred overnight at ambient temperature, the solvent was removed and the residue dissolved in tetrahydrofuran, cooled to 0° C and stirred with sodium hydride (80% dispersion in oil, 0.2 g) and benzyl bromide (0.6 ml) for 1.5 h. Methanol (5 ml) and water (100 ml) were added and the mixture was extracted with chloroform. The extract was dried ($MgSO_4$) and evaporated. Flash chromatography of the residue gave the title compound as a solid (0.15 g), $[\alpha]_D$ -37.1°; $^{13}$C-nmr data: 128.4, 128.2 and 126.7 (phenyl), 100.9 (C-5), 83.6 (C-1), 74.5 (C-4), 71.1 (C-2), 70.8 (Ph$\underline{C}H_2$O), 69.4 (C-7), 57.2 ($OCH_3$), 24.5 ($\underline{C}H_2CH_3$), 23.2 (C-3) and 7.6 ($CH_2\underline{C}H_3$).

## Example 27

### (1R,6R,8S)-8-benzoyloxy-4-methoxy-5,11,12-trioxatricyclo-[7.2.1.$0^{1,6}$]dodecane

#### A) Methyl 2-0-allyl-4-0-benzyl-3-deoxy-α-D-ribo-hexopyranoside

Methyl 2-0-allyl-4,6-0-benzylidene-3-deoxy-α-D-ribo-hexopyranoside was prepared from methyl 4,6-O-benzylidene-3-deoxy-α-D-ribo-hexopyranoside (Vis and Karrer, Helv. Chim. Acta, 1954, 46, 378-381; see also EP 0229034A, Preparative Example 5) by 2-0-allylation according to the method described in General Method 1 using allyl bromide. A solution of this compound (24 g, 78 mmol) in dry methanol (500 ml) was stirred overnight with Amberlyst 15 ($H^+$- form; 43 g). The solution was filtered and Amberlyst A-21 (free base form) was added until the solution was neutral. The solution was filtered, and after a trace of sodium methoxide had been added, evaporated to dryness under reduced pressure at temperatures up to 80° C. The residue was purified by flash chromatography to give syrupy methyl 2-0-allyl-3-deoxy-α-D-ribo-hexopyranoside (11 g, 54 mmol). This compound was dissolved in pyridine (200 ml) and trityl chloride (16.9 g, 1.1 eq.) was added. The solution was stirred at room temperature for nine days, by which time tlc indicated that no starting material remained. The mixture was partitioned between chloroform and saturated aqueous sodium hydrogen carbonate. The organic phase was dried ($MgSO_4$) and evaporated to dryness. The residue was dissolved in DMF (200 ml). Sodium hydride (80% in oil, 1.8 g, 1.2 eq.) and benzyl bromide (7.1 ml, 1.1 eq.) were added portionwise. The mixture was stirred at room temperature overnight and then heated to 80° C. The benzylation reaction was incomplete and not progressing (tlc evidence). The mixture was quenched with methanol and partitioned between chloroform and water. The organic phase was dried ($MgSO_4$) and concentrated. Flash chromatography on the residue gave methyl 2-0-allyl-4-0-benzyl-3-deoxy-6-O-trityl-α-D-ribo-hexopyranoside (17 g, 31 mmol) and methyl 2-0-allyl-3-deoxy-6-0-trityl-α-D-ribo-hexopyranoside (7 g), which was recycled to give a further 7.2 g (13 mmol) of the desired 4-0-benzyl ether. A

solution of methyl 2-0-allyl-4-0-benzyl-3-deoxy-6-0-trityl-α-D-ribo-hexopyranoside (17 g) in methanol (300 ml) was stirred overnight with a portion of Amterlyst 15 ($H^+$ form. The mixture was filtered and Amberlyst A-21 (free base form) added to bring the solution to neutrality. The solution was refiltered and evaporated to dryness. Flash chromatography gave the title compound A (9.4 g, 30.5 mmol, 56% from methyl 2-0-allyl-4,6-0-benzylidene-3-deoxy-α-D-ribo-hexopyranoside), $[\alpha]_D$ + 123°; $^{13}$C-nmr data: 138.0 (aromatic, q), 134.6 ($CH_2\underline{C}H = CH_2$), 128.3, 127.6 (aromatic), 117.4 ($CH=\underline{C}H_2$), 97.0 (C-1), 73.8 (C-5), 72.1 (C-4), 71.1 (C-2), 70.6 and 70.1 ($Ph\underline{C}H_2O$ and $CH_2=CH\underline{C}H_2O$), 62.0 (C-6), 54.7 ($OCH_3$) and 29.7 (C-3).

B) 2-0-Allyl-4-0-benzyl-6-0-tert-butyldimethylsilyl-3-deoxy-D-ribo-hexonolactone

A solution of methyl 2-0-allyl-4-0-benzyl-3-deoxy-α-D-ribo-hexopyranoside (12 g, 39 mmol), prepared as in Part A, in acetic anhydride (50 ml) was cooled in an ice-water bath. Boron trifluoride diethyl etherate (2 ml) was added. The solution was stirred for 30 min then poured into a mixture of sodium hydrogen carbonate (84 g) and water (100 ml) and stirred until effervescence ceased. The mixture was extracted twice with chloroform and the combined organic extracts were dried ($MgSO_4$) and evaporated to dryness. Flash chromatography gave syrupy 1,6-di-0-acetyl-2-0-allyl-4-0-benzyl-3-deoxy-α-D-ribo-hexopyranose (12.2 g, 32.2 mmol), which was dissolved in methanol (50 ml). Sodium methoxide solution (1% in methanol, 4 ml) was added. After 1 h the solution was neutralised with acetic acid and evaporated to dryness. Flash chromatography gave 2-0-allyl-4-0-benzyl-3-deoxy-αβ--D-ribo-hexopyranose (9.6 g, 33 mmol) as a 1:1 mixture of α- and β- anomers. This product was dissolved in dichloromethane and treated with tert-butyldimethylsilyl chloride (5.3 g, 1.1 eq.), triethylamine (4.9 ml, 1.1 eq.) and dimethylaminopyridine (200 mg). The mixture was stirred overnight then partitioned between chloroform and water. The organic phase was dried ($MgSO_4$) and evaporated to dryness. Flash chromatography gave 2-0-allyl-4-0-benzyl-3-deoxy-6-0-tert-butyldimethylsilyl-αβ-D-ribo-hexopyranose (9.8 g, 24 mmol). This compound (4.6 g, 11.2 mmol) was subjected to Swern oxidation (General Preparative Example 2) to give the syrupy title compound B (3.6 g, 8.9 mmol, 48% yield from methyl 2-0-allyl-4-0-benzyl-3-deoxy-α-D-ribo-hexopyranoside), $[\alpha]_D$ +65°; $^{13}$C-nmr data: 170.4 (C-1), 137.5 (aromatic, q), 133.9 ($CH_2\underline{C}H=CH_2$), 128.4 and 127.7 (aromatic), 117.8 ($CH=\underline{C}H_2$), 82.3 (C-5), 71.6 (x 3) (C-4, $Ph\underline{C}H_2O$- and $CH_2=CH\underline{C}H_2O$), 69.8 (C-2), 62.1 (C-6), 32.3 (C-3), 25.8 (x 3) [$C(\underline{C}H_3)_3$], 18.2 ($Si\underline{C}Me_3$), and -5.36 (x 2) ($Si\ CH_3$).

C) (1R,2S,4R)-4-Allyloxy-2-benzyloxy-5-(3,3-dimethoxypropyl)-6,8-dioxabicyclo[3.2.1]octane

A solution of 2-(2-bromoethyl)-1,3-dioxalane (1.3 ml, 10.5 mmol) and 1,2-dibromoethane (70 μl) in dry tetrahydrofuran (14 ml) was added to magnesium turnings (0.77 g) (previously activated by grinding) at a rate such that the temperature of the solution did not exceed 35°C. When the mixture had cooled to room temperature it was further cooled to -70° and a solution of 2-0-allyl-4-0-benzyl-6-0-tert-butyldimethylsilyl-3-deoxy-D-ribo-hexonolactone (2 g, 4.9 mmol) (prepared as described in Part B) in tetrahydrofuran (10 ml) was added over slowly. The mixture was then warmed to 0°, quenched with saturated aqueous ammonium chloride (5 ml), and filtered. The filtrate was partitioned between dilute hydrochloric acid and chloroform. The organic phase was washed with saturated aqueous sodium hydrogen carbonate, dried ($MgSO_4$) and evaporated to dryness. The residue was taken up in dry methanolic hydrogen chloride (1%, 50 ml) and stirred for 3 h. The solution was partitioned between chloroform and saturated aqueous sodium chloride. The organic phase was dried ($MgSO_4$) and evaporated to dryness. Flash chromatography gave the syrupy title compound C (1.5 g, 4.0 mmol, 81%), $[\alpha]_D$ -32°; $^{13}$C-nmr data: 138.4 (aromatic, q), 135.2 (-$CH_2\underline{C}H=CH_2$), 128.2 and 127.6 (aromatic), 117.0 ($CH_2CH=\underline{C}H_2$), 108.1 (C-5), 104.3 [$\underline{C}H(OMe)_2$], 74.1 (C-2), 71.5 (C-4), 70.7 and 70.3 ($Ph\underline{C}H_2O$ and $CH_2=CH\underline{C}H_2O$), 66.3 (C-7), 52.4 and 52.3 ($OCH_3$), 27.8 [$CH_2\underline{C}H_2CH(OMe)_2$], 25.1 and 24.7 [$\underline{C}H_2CH_2CH(OMe)_2$ and C-3].

D) (1R,6R,8S)-8-Benzyloxy-4-methoxy-5,11,12-trioxatricyclo[7.2.1.0$^{1,6}$]dodecane

A solution of (1R,2S,4R)-4-allyloxy-2-benzyloxy-5-(3,3-dimethoxypropyl)-6,8-dioxabicyclo[3.2.1]octane (0.8 g) (prepared as outlined in Part C) in methanol (25 ml) was added to palladium on carbon (10%, 0.20 g) and Amberlyst 15 ($H^+$ form, 0.15 g). The mixture was stirred at 70° for 24 h. It was then filtered and most of the solvent removed under reduced pressure. The residue was taken up in toluene and stirred with

Amberlyst 15 ($H^+$ form; 0.15 g) at 70° for 1 h. The mixture was filtered and the filtrate concentrated. Flash chromatography gave a single isomer of the title compound (0.36 g, 56%) as a syrup, $[\alpha]_D$ 44°; $^{13}C$-nmr data: 138.4 (aromatic, q), 128.3 and 127.7 (aromatic), 103.4 (C-1), 97.4 (C-4), 75.6 (C-9), 70.3 (C-8), 70.1 ($PhCH_2O$), 66.1 (C-6), 64.4 (C-2), 54.5 ($OCH_3$), 28.2 (C-3), and 26.9 (x 2) (C-2 and C-7).

## Example 28

### (1R,6R,8S)-8-Benzyloxy-5,11,12-trioxatricyclo[7.2.1.0$^{1,6}$]dodecane

A solution of (1R,6R,8S)-8-benzyloxy-4-methoxy-5,11,12-trioxatricyclo[7.2.1.0$^{1,6}$]dodecane (0.36 g, 1.4 mmol) (see Example 27) in acetonitrile (10 ml) was added to a solution of triethylsilane (380 µl, 2 eq.) and boron trifluoride diethyl etherate (300 µl, 2 eq.) in acetonitrile (10 ml). After 10 min the solution was partitioned between chloroform and saturated aqueous sodium hydrogen carbonate. The organic phase was dried ($MgSO_4$) and evaporated to dryness. Flash chromatography of the residue gave the title compound - (0.3 g, 1.1 ml, 81%) as a syrup that solidified on standing, m.p. 97-99°, $[\alpha]_D$ -6.2°; $^{13}C$-nmr data: 138.4 (aromatic, q), 128.3, 127.6 and 127.5 (aromatic), 103.6 (C-1), 76.0 (C-9), 74.2 (C-6), 70.5 (C-8), 70.0 ($PhCH_2O$), 67.6 (C-4), 66.0 (C-10), 31.9 (C-3), 27.5 (C-2) and 24.4 (C-7).

## Example 29

### (1R,2R,3S,4S)-2-Benzyloxy-3-fluoro-4-methoxy-6,8-dioxabicyclo[3.2.1]octane

#### A) (1R,2S)-2-Benzyloxy-6,8-dioxabicyclo[3.2.1]oct-3-ene

To a solution of (1R,2S,4R)-2-benzyloxy-4-hydroxy-6,8-dioxabicyclo[3.2.1]octane (EP 0229034A, Examples 2 and 14A) (7.7 g, 33 mmol) in dichloromethane (100 ml) was added trifluoromethanesulphonic anhydride (6.0 ml, 1.1 eq.) and pyridine (5.8 ml, 2.2 eq.). After 10 min, the reaction mixture was partitioned between chloroform and water. The dried organic layer was concentrated to a residue, and 1,8-dioxabicyclo-[5.4.0]undec-7-ene (20 ml) was added. After 1 h, the reaction mixture was partitioned between chloroform and dilute aqueous hydrochloric acid. The organic phase was then extracted with saturated aqueous sodium bicarbonate, dried ($Na_2SO_4$) and concentrated. The residue was chromatographed on silica gel ($CH_2Cl_2$-$CH_3OH$, 99:1), to yield title compound A (alternatively named 1,6-anhydro-4-0-benzyl-2,3-dideoxy-β-D-erythro-hex-2-enopyranose) as a syrup (3.8 g, 54%).

#### B) (1R,2RS,3R,4R)-2-Benzyloxy-3,4-epoxy-6,8-dioxabicyclo[3.2.1]octane

To a solution of the product from part A (2.6 g, 12 mmole) in dichloromethane (75 ml) was added 3-chloroperoxybenzoic acid (5.5 g, 2.3 eq.). After 1 month, the reaction mixture was filtered and the filtrate partitioned between chloroform and saturated aqueous sodium bicarbonate. The organic layer was concentrated and chromatographed on silica gel (eluted with petroleum ether/ethyl acetate, 1:1) to give the syrupy title compound B (alternatively named 1,6:2,3-dianhydro-4-0-benzyl-β-D-allopyranose) (1.9 g, 69%).

#### C) (1R,2R,3R,4S)-2-Benzyloxy-3-fluoro-3-hydroxy-6,8-dioxabicyclo[3.2.1]octane

A portion of the product (from part B) (0.72 g, 3.1 mmol) was dissolved in ethylene glycol (50 ml, dry), potassium bifluoride (4 8 g, 20 eq.) was added, and the solution was heated under reflux overnight. The reaction mixture was then partitioned between chloroform and water and the dried organic phase was

concentrated and chromatographed on silica gel (eluted with petroleum ether/ethyl acetate), to yield the title compound C (alternatively named 1,6-anhydro-4-0-benzyl-3-deoxy-3-fluoro-β-D-glucopyranose) as a syrup (0.32 g, 28% from the alkene of part A) along with recovered starting material (0.09 g) and an adduct of the D-allo-epoxide with ethylene glycol (0.11 g).

D) (1R,2R,3R,4S)-2-Benzyloxy-3-fluoro-4-methoxy-6,8-dioxabicyclo[3.2.1]octane

Using the method of General Preparative Example 1 the compound frcm part C was methylated using methyl iodide (10 eq.), followed by sodium hydride (2 eq.). The title compound (alternatively named 1,6-anhydro-4-0-benzyl-3-deoxy-3-fluoro-2-0-methyl-β-D-glucopyranose) was obtained as a colourless syrup $[\alpha]_D$ -32.2°; $^{13}$C-nmr data: 137.5, 128.4, 127.8, 127.7 (aromatic), 99.8 (d,C-5, $J_{5,F}$ 3.4Hz), 89.8 (d,C-3, $J_{3,F}$ 179.9), 78.4 (d,C-2, $J_{2,F}$ 23.3), 76.2 (d,C-4, $J_{4,F}$ 25.4), 74.2 (d,C-1, J1,F 3.2), 71.4 ($ArCH_2O$), 65.6 (d,C-7, $J_{7,F}$ 2.1) and 57.9 ($OCH_3$)

Example 30

(1S,2R,4S)-4-Benzyloxy-2-methoxy-6,8-dioxabicyclo[3.2.1]octane

A) 2-(4′-O-Benzyl-3′-deoxy-5′,6′-O-isopropylidene-2′-O-methyl-D-allo-hexitol-1′-yl)dithiane

To a stirred solution of dithiane (0.65 g) in dry tetrahydrofuran (10 ml), n-butyllithium in hexane (2.0 ml, 2.7M) was added dropwise under argon at -30°C. Stirring was continued at -30°C for 5 h, then, after cooling to -60°C, a solution of 4-0-benzyl-3-deoxy-5,6-0-isopropylidene-2-0-methyl-aldehydo-D-ribo-hexose (EP 0229034A, Preparative Example 8) (0.9 g) in tetrahydrofuran (2.0 ml) was added. After 10 min at -60°C, the reaction mixture was slowly warmed to 0°C, and partitioned between chloroform and water. The organic phase was dried ($Na_2SO_4$) and evaporated to a brown syrup. Flash chromatography gave the title allo-compound A (0.20 g) as a colourless oil, $[\alpha]_D$ -13.9°, its altro-isomer (0.24 g) as a pale yellow solid m.p. 185-188°C, [ $]_D$ -10.8°, and a ca. 1:1 mixture of the two isomers (0.47 g, total 1.1 g, 86%); $^{13}$C-nmr data, allo-isomer: 137.7, 128.4, 128.1 and 127.8 (aromatic), 109.1 ($Me_2C$), 78.1, 78.1, 75.7 and 74.9 (C-1′, C-2′, C-4, and C-5′), 72.8 ($PhCH_2O$), 65.8 (C-6′), 56.9 ($CH_3O$), 49.0 (C-2), 30.4, 29.4, 28.9 and 25.8 (C-3′, C-3, C-4 and C-5), 26.4 and 25.1 ($CH_3C$); $^{13}$C-nmr data, altro-isomer: 138.2, 128.3, 127.8 and 127.6 (aromatic), 109.1 ($Me_2C$), 77.9, 76.3, 76.2 and 72.6 (C-1′, C-2′, C-4′ and C-5′), 72.7 ($PhCH_2O$), 66.1 (C-6′), 57.6 ($CH_3O$), 48.5 (C-2), 31.2, 28.0, 27.4 and 25.6 (C-3′, C-3, C-4 and C-5), 26.5 and 25.2 ($CH_3O$).

B) 1,2-Di-O-acetyl-5-0-benzyl-4-deoxy-6,7-0-isopropylidene-3-0-methyl-D-allo-heptitol

The allo-dithiane from part A (1.8 g) was stirred in refluxing aqueous acetonitrile (30 ml, 80%) in the presence of mercury(II) chloride (3.6 g) and mercury(II) oxide (1.8 g) for 20 min. The cooled mixture was filtered, the filtrate was partitioned between dichloromethane and water, and the solids were washed with several portions of dichloromethane. The combined organic extracts were washed with aqueous ammonium chloride and water, dried ($Na_2SO_4$) and evaporated to a colourless oil. Sodium borohydride (0.4 g) was added to a stirred solution of this oil in aqueous acetonitrile (30 ml, 95%) at ambient temperature. After 30 min glacial acetic acid was added until neutral, the solvent was removed by evaporation and re-evaporation with toluene and the residue was treated with pyridine/acetic anhydride (20 ml, 1:1 v/v) for 70 h at ambient temperature. The reaction mixture was then poured into ice water. This mixture was extracted with chloroform, the extract was washed with water, dried ($Na_2SO_4$) and evaporated to yield an orange syrup which was flash chromatographed to give the title compound B (1.1 g, 60%) as a colourless oil, $[\alpha]_D$ +6.2°; $^{13}$C-nmr data: 170.6 and 170.1 (C=O), 138.1, 128.3, 127.8 and 127.6 (aromatic), 109.1 ($Me_2C$, 78.1, 77.4, 76.7 and 72.6 (C-2, C-3, C-5 and C-6), 72.2 ($PhCH_2O$), 66.4 (C-7), 62.5 (C-1), 57.9 ($CH_3O$), 33.0 (C-4), 26.4 and 25.1 ($CH_3C$), 20.9 and 20.6 (Ac).

C) (1S,2R,4S)-4-Benzyloxy-2-methoxy-6,8-dioxabicyclo[3.2.1]octane

The diacetate from part B (0.90 g) was treated with aqueous acetic acid (8 ml, 70%) for 16 h at ambient temperature. Water (4 ml) was added followed by vigorous stirring and sodium periodate (0.64 g). After stirring for 15 min the reaction mixture was partitioned between chloroform and water and the organic phase was dried ($Na_2SO_4$) and evaporated. The residue was stirred in dry methanol in the presence of anhydrous potassium carbonate (0.2 g) for 75 min. The solids were removed by filtration, the filtrate evaporated and the residue taken up in toluene. The solution was acidified by addition of p-toluenesulphonic acid and left at ambient temperature for 16 h. It was then neutralised with triethylamine and evaporated to a brown syrup. Purification by flash chromatography gave the title compound (0.36 g, 66%) as a colourless oil, $[\alpha]_D$ +78°; $^{13}$C-nmr data: 138.2, 128.3, 127.7 and 127.5 (aromatic), 100.8 (C-1), 75.0, 73.7 and 71.6 (C-2, C-4 and C-5), 71.1 (Ph$CH_2$O), 65.2 (C-6), 56.6 ($CH_3$O) and 24.4 (C-3).

## Example 31

(1S,2R,4S)-4-Benzyloxy-5-chloromethyl-2-methoxy-6,8-dioxabicyclo[3.2.1]octane

A) (1S,2R,4S)-4-Benzyloxy-5-hydroxymethyl-2-methoxy-6,8-dioxabicyclo[3.2.1]octane

1,2-Di-O-acetyl-5-O-benzyl-4-deoxy-6,7-O-isopropylidene-3-O-methyl-D-allo-heptitol (prepared as described in Example 30, Part B, 2.3 g) was treated with aqueous acetic acid (20 ml, 70%) for 48 h at ambient temperature. The solvent was removed by evaporation and the crude diol was monosilylated by treatment with tertbutyldiphenylsilyl chloride (1.7 ml) in N,N-dimethylformamide (5.5 ml) in the presence of imidazole (1.2 g) for 16 h at ambient temperature. The reaction mixture was partitioned between chloroform and water, the organic phase was washed with dilute hydrochloric acid followed by aqueous sodium bicarbonate, dried ($MgSO_4$) and evaporated. The primary silyl ether was oxidised according to Swern (see General Preparative Example 2). The product was purified by flash chromatography before deacetylation with methanolic sodium methoxide (20 ml, 0.1%) for 4 h at ambient temperature. After stirring with Amberlyst 15 ($H^+$-form) ion exchange resin for 30 min, removal of the resin by filtration and evaporation of the filtrate, the residue was stirred with dry toluene (20 ml) containing catalytic amounts of p-toluenesulphonic acid for 5 h at 40-50°C under argon. The solution was neutralised with triethylamine and evaporated to a syrup which was purified by flash chromatography. Removal of the silyl protecting group was effected by stirring in tetrahydrofuran (20 ml) containing tetrabutylammonium fluoride (5 mmol) for 48 h at ambient temperature. The solution was neutralised with acetic acid and evaporated to a thick syrup. Flash chromatography gave the title compound A (0.7 g, 46%) as a colourless oil, $[\alpha]_D$ +89°; $^{13}$C-nmr data: 137.8, 128.3, 128.2 and 127.7 (aromatic), 107.0 (C-5), 75.3, 74.8 and 71.3 (C-1, C-2 and C-4), 71.3 (Ph$CH_2$O), 66.2 (C-7), 62.4 ($CH_2$OH), 56.6 ($CH_3$O) and 24.2 (C-3).

B) (1R,2R,4S)-4-Benzyloxy-2-methoxy-5-trifluoromethanesulphonyloxymethyl-6,8-dioxabicyclo[3.2.1]octane

To a stirred solution of the alcohol from Part A (0.60 g) in dichloromethane (20 ml) at -30°C under argon, pyridine (0.5 ml) and trifluoromethanesulphonic anhydride (1.0 ml) were added. After 30 min at -30°C and 30 min at 0°C the reaction mixture was poured into ice-cold aqueous bicarbonate. Three extractions with dichloromethane gave, after washing with water, drying ($MgSO_4$) and removal of the solvent, a dark red oil. Purified by flash chromatography the title compound B (0.60 g, 68%) was obtained as white crystals, m.p. 61-62°C, $[\alpha]_D$ +69°; $^1$H-nmr data (200 MHz): 7.3 (4H,m, aromatic), 4.75 (1H,m,H-1), 4.71 and 4.43 (2H,2d,Ph$CH_2$O), 4.64 and 4.49 (2H,2d,$CH_2$OTf), 3.95 (1H,dd,H-7exo), 3.79 (1H,dd,H-7endo), 3.44 (3H,s,$CH_3$O), 3.4 (1H,m,H-4), 3.2 (1H,m,H-2), 2.17 (1H,dm,H-3e) and 1.83 (1H,dt,H-3a).

C) (1S,2R,4S)-4-Benzyloxy-5-chloromethyl-2-methoxy-6,8-dioxabicyclo[3.2.1]octane

A solution of the triflate from Part B (0.60 g) and tetrabutylammonium chloride (1.5 g) in hexamethylphosphoramide (2 ml) was stirred under argon for 2 h at ambient temperature. The reaction mixture was partitioned between ether and water, the ether extract was dried ($MgSO_4$) and evaporated to an oil which was purified by flash chromatography to give the title compound (0.35 g, 81%) as a colourless syrup, $[\alpha]_D$ +87°; $^{13}C$-nmr data: 137.6, 128.2 and 127.6 (aromatic), 106.1 (C-5), 75.8, 74.3 and 71.1 (C-1,C-2 and C-4), 71.5 ($PhCH_2O$), 66.3 (C-7), 56.5 ($CH_3O$), 43.9 ($CH_2Cl$) and 24.0 (C-3).

Example 32

(1S,2R,4S)-4-Benzyloxy-2-methoxy-5-methyl-6,8-dioxabicyclo[3.2.1]octane

(1S,2R,4S)-4-Benzyloxy-5-chloromethyl-2-methoxy-6,8-dioxabicyclo[3.2.1]octane (Example 31, Part C) (0.35 g) in toluene (10 ml) containing azobisisobutyronitrile (catalytic amounts) was treated with tributyltin hydride (1.5 ml in several portions) for 16 h at 80-85°C. Evaporation of the solvent, partitioning between acetonitrile and light petroleum ether and flash chromatography gave the title compound (0.24 g, 77%) as a colourless oil, $[\alpha]_D$ +116°; $^1H$-nmr data (200 MHz): 7.3 (4H,m, aromatic), 4.7 (1H,m,H-1), 4.73 and 4.48 (2H,2d,$PhCH_2O$), 3.88 (1H,dd,H-7exo), 3.67 (1H,dd,H-7endo), 3.43 (3H,s,$CH_3O$), 3.2 (2H,m,H-2 and H-4), 2.12 (1H,dm,H-3e), 1.81 (1H,dt,H-3a) and 1.47 (3H,C-$CH_3$).

Example 33

(1S,2R,4S)-4-Benzyloxy-2-methoxy-1-propyl-6,8-dioxabicyclo[3.2.1]octane

To a stirred suspension of potassium tert-butoxide (0.96 g) in dry tert-butyl methyl ether (40 ml) at -75°C in an argon atmosphere was added dropwise sec-butyllithium (6.0 ml, 1.4 M in cyclohexane). The resulting yellow suspension was stirred at -75°C for 2 h, and then lithium bromide (9.0 ml, 2 M solution in tetrahydrofuran) was added. The mixture was warmed to -20°C for 0.5 h, and then recooled to -75°C. The resultant thick pale yellow mixture was stirred while a solution of 4-0-benzyl-6-0-tert-butyldimethylsilyl-3-deoxy-2-0-methyl-D-ribo-hexono-1,5-lactone (Preparative Example 3) (1.0 g) in tetrahydrofuran (10 ml) was added dropwise. After 0.25 h, the reaction was quenched with acetic acid (2 ml) and partitioned between water and toluene. Conventional processing afforded 1.2 g of colourless syrup representing an essentially quantitative yield of 5-0-benzyl-1-0-tert-butyl-7-0-tertbutyldimethylsilyl-4-deoxy-3-0-methyl-D-ribo-hept-2-ulose. Most of this material (1.1 g) was dissolved in dry ether (10 ml) and propylmagnesium chloride (10 ml, 2 M in ether) was added. The solution was stirred at ambient temperature for 24 h. Careful quenching with saturated aqueous ammonium chloride was followed by two extractions with ether. Conventional processing of the organic phase gave a syrup which by tlc examination contained two components. To a solution of this material in tetrahydrofuran (10 ml) was added tetrabutylammonium fluoride (3.5 ml of 1.0 M solution in tetrahydrofuran). After 1 h the solvent was removed and the residue was dissolved in ethanol (45 ml) and water (15 ml). Sodium periodate (0.68 g) was added to the stirred solution, and after 2 h a further 0.3 g was added. After another 2 h, the mixture was partitioned between chloroform and water. The aqueous phase was extracted twice more with chloroform and the combined chloroform extracts were concentrated to a syrup. Chromatography on silica gel of this material afforded first 0.15 g of the minor component and then 0.504 g of 2-0-benzyl-6-0-tert-butyl-3-deoxy-4-0-methyl-5-C-propyl-L-ribo-hexopyranose. This material was dissolved in cold (0°C) trifluoroacetic acid and then the solution was allowed to warm to ambient temperature and was stirred overnight. The resulting black solution was evaporated to dryness and then dissolved in methanol containing triethylamine to solvolyse a small quantity of trifluoroacetate ester that had formed. After evaporation the residue was chromatographed on silica gel to give 0.25 g of syrup. This

material was benzylated in the usual manner (see General Preparative Example 1) to give the title compound as a syrup (0.322 g), $[\alpha]_D$ +25.0°; $^{13}C$-nmr data: 138.2, 128.2, 127.8 and 127.5 (aromatics), 101.3 (C-5), 82.9 (C-1), 75.0 and 71.4 (C-2 and -4), 71.2 ($ArCH_2O$), 69.7 (C-7), 57.0 ($OCH_3$), 33.9 ($CH_2CH_2CH_3$), 23.2 (C-3), 17.1 ($CH_2CH_2CH_3$) and 14.6 ($CH_2CH_2CH_3$).

Example 34

(1RS,2SR,4RS,7RS)-4-Benzyloxy-2-methoxy-5,7-dimethyl-6,8-dioxabicyclo[3.2.1]octane

A) (4′RS,5′RS,5SR)-2,2,5-trimethoxy-5-(2,2,5-trimethyl-1,3-dixolan-4-yl)pentan-3-ol

To a solution of (4′RS,5′SR,5RS)-5-hydroxy-2,2-dimethoxy-5-(2,2,5-trimethyl-1,3-dixolan-4-yl)pentan-3-one (prepared as described in example 19, part C) (1.1 g, 4 mmol) and 2,6-di-tert-butyl-4-methylpyridine (2.04 g, 10 mmol) in dry dichloromethane (20 ml) was added methyl trifluoromethanesulphonate (1.12 ml, 10 mmol). The reaction was heated under reflux for 20 h, excess dichloromethane was added, and the mixture washed twice with water. The organic layer was dried ($MgSO_4$) and concentrated to give an oil which was dissolved in ethanol (20 ml) and mixed with sodium borohydride (1.5 g). The reaction was quenched by addition of water, the solvents were removed under reduced pressure, and the residue dissolved in ethyl acetate (50 ml). The solution was washed with water, dried ($MgSO_4$) and concentrated to yield a crude product which was chromatographed on silica eluted with hexane - ethyl acetate (2:1). Title compound A was obtained as an oil (910 mg, 78%). $^1H$-nmr indicated it to be a (6:4) mixture of epimers at 3-position.

B) (1RS,2SR,4RS,7RS)-4-Hydroxy-2-methoxy-5,7-dimethyl-6,8-dioxabicyclo[3.2.1]octane

Title compound A (approx. 900 mg) in acetic acid-water (9:1, v/v; 20 ml) was heated at 70°C for 35 min. The solvents were removed under reduced pressure and the residue chromatographed on silica eluted with hexane - ethyl acetate (3:2). Title compound B (150 mg) followed by its (4SR)-epimer (90 mg) were obtained.

C) (1RS,2SR,4RS,7RS)-4-Benzyloxy-2-methoxy-5,7-dimethyl-6,8-dioxabicyclo[3.2.1]octane

To a stirred suspension of powdered potassium hydroxide (224 mg, 4 mmol) in DMSO (5 ml) was added title compound from part B (150 mg) followed by benzyl bromide (238 µl, 2 mmol). After 20 min, the mixture was diluted with excess ethyl acetate, filtered, washed three times with water, dried ($MgSO_4$), filtered, and concentrated to give a yellow oil (300 mg). Chromatography on silica eluted with hexane - ethyl acetate (3:2) gave the title compound (with a 7-endo-methyl group) as an oil which crystallised on standing, m.p. 51-53°C; $^1H$-nmr data: 1.25 (3H,d), 1.45 (3H,s), 1.94 (1H,dt), 2.1 (1H,d,J 16Hz), 3.20-3.28 (2H,m), 3.41 (3H,s), 4.20-4.30 (1H,m), 4.36-4.43 (1H,m), 4.49 (1H,d,J 13Hz), 4.71 (1H,d,J 13Hz) and 7.23-7.38 (5H,m).

Example 35

(1RS,2SR,4RS)-4-Benzyloxy-2-methoxy-1,5-dimethyl-6,8-dioxabicyclo[3.2.1]-otane

A) (4′RS,5SR)-2,2,5-trimethoxy-5-(2,2,4-trimethyl-1,3-dioxolan-4-yl)pentan-3-ol

Methyl trifluoromethanesulphonate (1.42 ml, 12.5 mmol) was added in one portion to a stirred solution of (4′RS,5SR)5-hydroxy-2,2-dimethoxy-5-(2,2,4-trimethyl-1,3-dioxolan-4-yl)pentan-3-one (2.77 g, 10 mmol) (prepared in example 20, part A) and 2,6-di-tert-butyl-4-methylpyridine (2.58 g, 12.5 mmol) in carbon tetrachloride (50 ml) at room temperature under argon. The mixture was heated under reflux for 24 h after which time tlc showed incomplete reaction. Further methyl trifluoromethanesulphonate (1.4 ml) and 2,6-di-tert-butyl-4-methylpyridine (2.58 g) were added and the heating was continued for a further 48 h. The reaction mixture was cooled in ice and filtered. The filtrate was washed twice with water, dried ($MgSO_4$), and concentrated to give a brown oil (4.5 g) which was dissolved in ethanol (50 ml) and treated with sodium borohydride (1.13 g, 30 mmol). After 15 min, the solvent was evaporated and the residue partitioned between ethyl acetate and water. The organic layer was separated and the aqueous phase extracted twice with ethyl acetate. The combined organic phase was dried ($MgSO_4$), filtered, and concentrated to yield an oil (4.5 g) containing title compound A as a mixture of epimers at the 3-position, which was cyclised without further purification.

B) (1RS,2SR,4RS)-4-Hydroxy-2-methoxy-1,5-dimethyl-6,8-dioxabicyclo[3.2.1]octane

Crude product (4.5 g) from Part A containing title compound A was dissolved in trifluoroacetic acid (5 ml) and allowed to stand at room temperature for 15 h. The solvent was removed under reduced pressure and the residue chromatographed twice on silica eluted with hexane - ethyl acetate (7:3 increasing) to yield title compound B (370 mg) followed by its (4SR)-epimer (300 mg).

C) (1RS,2SR,4RS)-4-Benzyloxy-2-methoxy-1,5-dimethyl-6,8-dioxabicyclo[3.2.1]octane

The title compound from part B (370 mg, 1.97 mmol) was dissolved in DMSO (30 ml) and powdered potassium hydroxide (441 mg, 7.86 mmol) added followed by benzyl bromide (672 mg, 3.93 mmol). After 15 h, water (15 ml) was added and the product extracted with ethyl acetate. The extracts were dried ($MgSO_4$), filtered, and concentrated to yield a yellow oil (1.13 g). Chromatography on silica eluted with hexane - ethyl acetate (7:3) gave the title compound as a colourless oil (160 mg, 30%); $^1$H-nmr data: 1.44 (3H,s), 1.46 (3H,s), 1.72 (1H,dt), 2.18 (1H,d,J 14Hz), 2.98 (1H,d), 3.17 (1H,d), 3.41 (3H,s), 3.46 (1H,d,J 10Hz), 3.69 (1H,d,J 10Hz), 4.49 (1H,d,J 13Hz), 4.66 (1H,d,J 13Hz) and 7.24-7.40 (5H,m).

## Example 36

### (1RS,2RS,4SR,7RS)-4-Benzyloxy-2-methoxy-7-phenyl-6,8-dioxabicyclo[3.2.1]octane

A) 3,4-Dihydro-2H-pyran-2-(phenylmethanol)

To a stirred solution of 3,4-dihydro-2H-pyran-2-carboxaldehyde (22.4 g) in tetrahydrofuran (100 ml) cooled in an ice-bath and under argon, a solution of phenylmagnesium bromide in tetrahydrofuran (2.0 M, 100 ml) was added dropwise at such a rate that the temperature of the reaction mixture did not rise above 10° C. The solution was then allowed to warm to room temperature and was left to stand for 16 h. The mixture was quenched with saturated aqueous ammonium chloride, and made slightly acid with 2M hydrochloric acid. The organic phase was separated, and the aqueous layer extracted with diethyl ether (2 x 50 ml). The combined organic layers were washed with brine, dried ($MgSO_4$), and evaporated to leave a pale yellow oil (45.8 g). Filtration through silica gel, eluted with ethyl acetate/hexane (1:5) gave the title compound A as a colourless oil (32.1 g).

B) (1RS,3SR,4SR,7RS)-3,4-Epoxy-7-phenyl-6,8-dioxabicyclo[3.2.1]octane

To a solution of 3,4-dihydro-2H-pyran-2-(phenylmethanol) (14.0 g), prepared as in part A), in dichloromethane (50 ml) was added dropwise a solution of bromine (3.8 ml) in dichloromethane at such a rate that a gentle reflux was maintained. After the addition, solid potassium carbonate was added portionwise until no further effervescence was observed, then water (100 ml) was added. The dichloromethane layer was separated, washed with saturated aqueous sodium bicarbonate solution, dried ($MgSO_4$) and evaporated to yield 4-bromo-7-phenyl-6,8-dioxabicyclo[3.2.1]octane (mixture of stereoisomers) as an orange oil (20.0 g).

This product was dissolved in ethanol (50 ml) and a solution of potassium hydroxide (5.0 g) in water (2 ml) was added. The mixture was heated under reflux. Further quantities of potassium hydroxide were added after 2 h (2.5 g) and 5 h (1.0 g). After 7 h, the mixture was allowed to cool and poured into brine (100 ml). This mixture was extracted with dichloromethane (3 x 25 ml), and the combined organic phases were dried $MgSO_4$ and filtered. 3-Chloroperbenzoic acid (18.0 g) was then added to the filtrate and the resulting solution was allowed to stand for 2 days, after which time a solid had precipitated. Aqueous sodium metabisulphite solution (10%) was cautiously added until a sample of the mixture gave a negative reaction to starch iodide paper, then the resulting suspension was filtered and the organic phase was separated, washed thoroughly with 1M sodium hydroxide solution, then water, dried $MgSO_4$ and evaporated to yield a pale yellow gum (13.2 g). Silica gel chromatography, with hexane/ethyl acetate/ether mixtures as eluant afforded firstly a mixture consisting predominantly of isomers of 4-bromo-7-phenyl-6,8-dioxabicyclo[3.2.1]-octane (10.8 g), followed by the title compound B (the 7-exo-isomer) (2.4 g), which crystallised on standing, m.p. 57-60° C.

C) (1RS,4SR,7RS)-4-Hydroxy-7-phenyl-6,8-dioxabicyclo[3.2.1]oct-2-ene

A solution of (1RS,3SR,4SR,7RS)-3,4-epoxy-7-phenyl-6,8-dioxabicyclo[3.2.1]octane (1.6 g), prepared as in part B, in tetrahydrofuran (10 ml) was treated with a solution of lithium bis(trimethylsilylamide) in tetrahydrofuran (1.0 M, 10 ml). The mixture was heated under reflux for 30 min, then allowed to cool and quenched with brine (50 ml). This mixture was extracted with chloroform (3 x 20 ml). The combined chloroform extracts were washed with brine, and dried ($MgSO_4$) and evaporated to yield a semi-crystalline orange residue, which crystallised from ethyl acetate/hexane to give the title compound C as colourless crystals (0.9 g), m.p. 109-110° C.

D) (1RS,4SR,7RS)-4-Benzyloxy-7-phenyl-6,8-dioxabicyclo[3.2.1]oct-2-ene

4-Hydroxy-7-phenyl-6,8-dioxabicyclo[3.2.1]oct-2-ene (1.09 g), prepared as in part C, was benylated according to General Preparative Example 1 using benyl bromide to give without chromatographic purification a yellow oil (1.90 g) which crystallised on standing, containing title compound D.

E) (1RS,2SR,3RS,4RS,7SR)-4-Benzyloxy-2,3-epoxy-7-phenyl-6,8-dioxabicyclo[3.2.1]octane

A solution of the crude product of part C (1.90 g) containing 4-benzyloxy-7-phenyl-6,8-dioxabicyclo-[3.2.1]oct-2-ene, in dichloromethane (20 ml) was mixed with 3-chloroperbenzoic acid (2.30 g) The mixture was left to stand for 13 days, then 10% aqueous sodium metabisulphite was added slowly until all exothermic reaction ceased. The resultant suspension was filtered, and the filtrate was thoroughly washed with 1 M sodium hydroxide solution, then saturated sodium bicarbonate solution, dried ($MgSO_4$), and evaporated to yield a white solid. This was chromatographed on silica gel eluted with ethyl acetate/hexane mixtures, to give the title compound E as a white solid (1.18 g), m.p. 121.5-123.5° C.

F) (1RS,2RS,4SR,7RS)-4-Benzyloxy-2-hydroxy-7-phenyl-6,8-dioxabicyclo[3.2.1]octane

To a solution of 4-benzyloxy-2,3-epoxy-7-phenyl-6,8-dioxabicyclo[3.2.1]octane (1.18 g), prepared as in part E, in tetrahydrofuran (20 ml) was added lithium aluminium hydride (0.15 g). This mixture was allowed to stand at room temperature for 16 h, and was then heated under reflux for 2 h. The mixture was cooled to

room temperature and excess lithium aluminium hydride was destroyed by the dropwise addition of water. The mixture was made slightly acid with 2 M hydrochloric acid and extracted with ethyl acetate (3 x 20 ml). The combined ethyl acetate extracts were washed with brine, dried ($MgSO_4$), and evaporated to yield the title compound F (0.97 g).

G) (1RS,2RS,4SR,7RS)-4-Benzyloxy-2-methoxy-7-phenyl-6,8-dioxabicyclo[3.2.1]octane

To a solution of 4-benzyloxy-2-hydroxy-7-phenyl-6,8-dioxabicyclo[3.2.1]octane (0.97 g), prepared as in part F, in dimethylformamide (10 ml) was added a dispersion of sodium hydride in oil (50%, 0.15 g). After effervescence had ceased, methyl iodide (0.8 ml) was added. The resultant colourless solution was stirred for 30 min, then quenched with brine (20 ml). The mixture was extracted with ethyl acetate (2 x 25 ml). The combined ethyl acetate layers were washed thoroughly with water, dried ($MgSO_4$) and evaporated to yield a white solid, which was chromatographed on silica gel (eluant, ethyl acetate/hexane mixtures) The resulting product was recrystallised from ethyl acetate/hexane to give the title compound G (the 7-exo-isomer) as white crystals (0.42 g), m.p. 77.5-79.5°C; $^1$H-nmr data: 1.95-2.15 (2H,m), 3.33-3.42 (2H,m), 3.43 (3H,s), 4.47 (1H,br s), 4.61 (1H,d), 4.71 (1H,d), 4.79 (1H,s), 5.68 (1H,s) and 7.24-7.42 (10H,m).

Example 37

(1RS,2RS,4SR,7RS)-4-Benzyloxy-7-ethyl-2-methoxy-6,8-dioxabicyclo[3.2.1]octane

By the same procedure as in Example 36, but using ethylmagnesium bromide in place of phenylmagnesium bromide in stage A, the title compound (a 7-exo-isomer) was obtained as a syrup; $^1$H-nmr data: 0.91 (3H,t), 1.53 (2H,m), 1.85 (1H,dt), 1.99 (1H,d), 3.16 (1H,d), 3.25 (1H,d), 3.44 (3H,s), 3.72 (1H,t), 4.26 (1H,s), 4.55 (1H,d), 4.65 (1H,d), 5.40 (1H,s) and 7.20-7.36 (5H,m).

Example 38

(1RS,2RS,4SR,7RS)-4-Benzyloxy-2-methoxy-7-propyl-6,8-dioxabicyclo[3.2.1]octane

A) (1RS,3SR,4SR,7RS)-3,4-Epoxy-7-propyl-6,8-dioxabicyclo[3.2.1]octane

A 1:1:1:1 mixture of the possible stereoisomers of 4-bromo-7-propyl-6,8-dioxabicyclo[3.2.1]octane was prepared from 3,4-dihydro-2H-pyran-2-carboxaldehyde using the procedure described for 4-bromo-7-phenyl-6,8-dioxabicyclo[3.2.1]octane (see Example 36, parts A and B) but using propylmagnesium chloride (in diethyl ether) in place of phenylmagnesium bromide. This mixture of 4-bromo-7-propyl-6,8-dioxabicyclo-[3.2.1]octane stereoisomers (8.77 g) in ethanol (50 ml) was mixed with a solution of potassium hydroxide (4.2 g) in water (2 ml). The mixture was heated under reflux for 1 h, then cooled and left to stand for 72 h. The mixture was then heated under reflux for a further 4 h (potassium hydroxide pellets, 4.2 g, were added after 2 h). The resultant mixture was allowed to cool, and water (500 ml) was added. This mixture was extracted with dichloromethane (2 x 200 ml, then 100 ml). The combined organic layer was dried ($MgSO_4$), filtered, and to the resulting filtrate was added 3-chloroperbenzoic acid (6.55 g). The solution was left to stand for 5 days, then 3-chloroperbenzoic acid (1.0 g) was added. After 16 h, excess 3-chloroperbenzoic acid was destroyed by the addition of a 10% aqueous solution of sodium metabisulphite, and the mixture was thoroughly extracted with saturated aqueous sodium bicarbonate solution. The dichloromethane layer was dried ($MgSO_4$) and evaporated to leave a clear oil. Chromatography on silica gel eluted with ethyl

acetate/hexane mixtures afforded firstly a mixture of the (1RS,4SR,7SR)-, (1RS,4RS,7RS)-, and (1RS,4RS,7SR)-stereoisomers of 4-bromo-7-propyl-6,8-dioxabicyclo[3.2.1]octane (4.11 g), then a yellow oil (1.61 g) comprised of the title compound (a 7-exo-isomer) and its (1RS,3SR,4SR,7SR)-(7-endo)stereoisomer as a 4:1 mixture.

B) (1RS,2RS,4SR,7RS)-4-Benzyloxy-2-methoxy-7-propyl-6,8-dioxabicyclo[3.2.1]octane

The 4:1 mixture of (1RS,3SR,4SR,7RS)-3,4-Epoxy-7-propyl-6,8-dioxabicyclo[3.2.1]octane and its (1RS,3SR,4SR,7SR)-stereoisomer from part A was converted using the procedure detailed in Example 36, stages C to G into the title compound (7-exo-isomer), obtained as a syrup which contained 20% of its (1RS,2RS,4SR,7SR)-(7-endo)-stereoisomer; $^1$H-nmr data: (major isomer only) 0.93 (3H,t), 1.22-1.67 (4H,m), 1.87 (1H,dt), 2.01 (1H,d,br), 3.16 (1H,m), 3.24 (1H,m), 3.41 (3H,s), 3.80 (1H,m), 4.26 (1H,br s), 4.56 (1H,d), 4.66 (1H,d), 5.41 (1H,br s) and 7.24-7.39 (5H,m).

## Example 39

### (1RS,2RS,4SR,7SR)-4-Benzyloxy-2-methoxy-7-propyl-6,8-dioxabicyclo[3.2.1]octane

A) (1RS,3SR,4SR,7SR)-3,4-Epoxy-7-propyl-6,8-dioxabicyclo[3.2.1]octane

The mixture of (1RS,4SR,7SR)-, (1RS,4RS,7RS)- and (1RS,4RS,7SR)-4-bromo-7-propyl-6,8-dioxabicyclo[3.2.1]octanes (4.11 g), prepared in Example 38 part A, was dissolved in ethanol (30 ml) and a solution of potassium hydroxide (5.0 g) in water (5 ml) was added. This mixture was heated under reflux for 3 h, then allowed to cool and left to stand for 6 days. The mixture was then heated under reflux for 5 h, with the addition of potassium hydroxide pellets at the following times (0 h, 5.0 g; 1 h, 5.0 g; 3 h, 2.5 g). The mixture was then cooled and water (100 ml) was added. The mixture was extracted with dichloromethane (3 x 30 ml). The combined dichloromethane extracts were dried ($MgSO_4$), filtered, and the filtrate was mixed with 3-chloroperbenzoic acid (1.90 g). This solution was allowed to stand for 3 days, then a further quantity of 3-chloroperbenzoic acid (2.0 g) was added. After a further 3 days, excess 3-chloroperbenzoic acid was destroyed by the addition of a 10% aqueous solution of sodium metabisulphite and the mixture was thoroughly extracted with saturated aqueous sodium bicarbonate solution. The dichloromethane layer was dried ($MgSO_4$), and evaporated to leave a pale yellow oil. Chromatography on silica gel eluted with ethyl acetate/hexane mixtures afforded firstly a mixture of (1RS,4RS,7RS)- and (1RS,4RS,7SR)-4-bromo-7-propyl-6,8-dioxabicyclo[3.2.1]octane (2.20 g), then a clear oil (1.12 g) mixture of the title compound (a 7-endo-isomer) and its (1RS,3SR,4SR,7RS)-(7-exo)-stereoisomer.

B) (1RS,2RS,4SR,7SR)-4-Benzyloxy-2-methoxy-7-propyl-6,8-dioxabicyclo[3.2.1]octane

By the same procedure as described in Example 36, stages C to G, the mixture containing predominatly (1RS,3SR,4SR,7SR)-3,4-epoxy-7-propyl-6,8-dioxabicyclo[3.2.1]octane prepared in part B, was converted to the title compound. This compound, more than 98% pure 7-endo-isomer, was obtained as a colourless gum; $^1$H-nmr data: 0.96 (3H,t), 1.25-1.70 (4H,m), 1.98 (2H,m), 3.24 (1H,m), 3.31 (1H,m), 3.42 (3H,s), 4.01 (1H,m), 4.39 (1H,d), 4.57 (1H,d), 4.66 (1H,d), 5.36 (1H,d) and 7.21-7.41 (5H,m).

## Example 40

(1RS,2SR,4RS)-4-Benzyloxy-2-methoxy-6,8-dioxabicyclo[3.2.1]octane

By the same procedure as in Example 36, stages C to G, (1RS,3SR,4SR)-3,4-epoxy-6,8-dioxabicyclo-[3.2.1]octane (F Sweet and R K Brown, Can J Chem., 1968, 46, 2889) was converted to the title compound which was obtained as a syrup; $^1$H-nmr data: (400MHz) 1.82 (1H,dt), 2.01 (1H,dd), 3.18 (1H,d), 3.28 (1H,d), 3.45 (3H,s), 3.71 (1H,m), 3.79 (1H,m), 4.58 (1H,d), 4.64 (1H,m), 4.65 (1H,d), 5.41 (1H,s) and 7.23-7.38 (5H,m).

Example 41

(1RS,2SR,4RS)-2-Methoxy-4-(2-methylbenzyloxy)-6,8-dioxabicyclo[3.2.1]octane

By the same procedure as in Example 36, stages C to G, but using 2-methylbenzyl bromide in place of benzyl bromide in stage D, (1RS,3SR,4SR)-3,4-epoxy-6,8-dioxabicyclo[3.2.1]octane was converted to the title compound which was obtained as a colourless oil; $^1$H-nmr data: 1.83 (1H,dt), 2.01 (1H,br d), 2.36 (3H,s), 3.17 (1H,d), 3.29 (1H,d), 3.44 (3H,s), 3.71 (1H,d), 3.79 (1H,m), 4.58 1H,d), 4.62 (1H,d), 4.66 (1H,m), 5.44 (1H,s) and 7.10-7.35 (4H,m).

Example 42

(1RS,2SR,4RS,7SR)-4-Benzyloxy-2-methoxy-7-methyl-6,8-dioxabicyclo[3.2.1]octane

A solution of methyl 2-0-benzyl-3,7-dideoxy-4-0-methyl-DL-talo-heptopyranoside (containing some of its DL-allo-stereoisomer) (110 mg), prepared as in Preparative Example 1, in toluene (1 ml) was stirred with a 3A molecular sieve (50 mg) and AmBerlyst 15 ion exchange resin ($H^+$ form; 100 mg) for nine days. The mixture was filtered through Hyflo$^R$ filter aid and the filter aid was washed thoroughly with ethyl acetate and the combined filtrates were evaporated to leave a green oily residue. Preparative layer chromatography on silica gel, eluted with ethyl acetate/hexane (2:1) gave an oil (61 mg), which was further purified by preparative layer chromatography on silica gel eluted with chloroform/methanol (19:1) to give a colourless oil (31 mg) containing the title compound (the 7-exo-isomer) in a 7:1 mixture with its (1RS,2SR,4RS,7RS)-(7-endo)-stereoisomer; $^1$H-nmr data: (major isomer only) 1.25 (3H,d), 1.89 (1H,dt), 2.00 (1H,br d), 3.19 (1H,d), 3.22 (1H,d), 3.41 (3H,s), 4.01 (1H, q), 4.21 (1H, br s), 4.56 (1H,d), 4.65 (1H,d), 5.42 (1H,s) and 7.22-7.39 (5H,m).

Example 43

(1RS,2SR,4RS,7SR)-2-Methoxy-7-methyl-4-(2-methylbenzyloxy)-6,8-dioxabicyclo[3.2.1]octane and (1RS,2SR,4RS,7SR)-4-Hydroxy-2-methoxy-7-methyl-6,8-dioxabicyclo[3.2.1]octane

By the same procedure as in Example 42, methyl 3,7-dideoxy-4-0-methyl-2-0-(2-methylbenzyl)-DL-talo-heptopyranoside (containing some of its DL-allo-stereoisomer), prepared as in Preparative Example 12, was converted to a 4:1 mixture of the title compounds. These were separated by preparative layer chromatography on silica eluted with ethyl acetate/hexane (7:3) to give a colourless oil (85 mg) containing the first title compound (a 7-exo-isomer) as a 5:1 mixture with its (1RS,2SR,4RS,7RS)-stereoisomer; $^1$H-nmr data: (400MHz) (major isomer only) 1.23 (3H,d), 1.84 (1H,dt), 1.99 (1H,broad d), 2.35 (3H,s), 3.28 (1H,d), 3.23 (1H,d), 3.41 (3H,s), 4.01 (1H,q), 4.20 (1H,br s), 4.58 (1H,d), 4.62 (1H,d), 5.46 (1H,s), 7.10-7.21 (3H,m) and 7.31 (1H,m); and then a colourless gum (18 mg) containing the second title compound (the 7-exo-isomer) as a 9:1 mixture with its (1RS,2SR,4RS,7RS)-(7-endo-)-stereoisomer.

Example 44

(1RS,2SR,4RS,7RS)-4-Benzyloxy-2-methoxy-7-methyl-6,8-dioxabicyclo[3.2.1]octane and its (1RS,2SR,4RS,7SR)-stereoisomer

By the same procedure as in Example 42, a mixture of methyl 2-0benzyl-3,7-dideoxy-4-0-methyl-DL-allo-heptopyranoside and methyl 2-0-benzyl-3,7-dideoxy-4-0-methyl-DL-talo-heptopyranoside, prepared as in Preparative Example 14, was converted to an inseparable 1:1 mixture of the title compounds, which was obtained as a colourless oil; $^1$H-nmr data: 1.20-1.29 (3H,d x 2), 1.80-1.90 (1H,m), 1.99 (1H,m), 3.16-3.40 (2H,m), 3.42 (1.5H,s), 3.43 (1.5H,s), 4.00 (0.5H,q), 4.15 (0.5H,m), 4.20 (0.5H,m), 4.35 (0.5H,m), 4.55 (0.5H,d), 4.57 (0.5H,d), 4.64 (1H,d), 5.34 (0.5H,s), 5.42 (0.5H,s) and 7.20-7.40 (5H,m).

Example 45

(1RS,2RS,4SR)-4-Benzyloxy-2-methoxy-7,7-dimethyl-6,8-dioxabicyclo[3.2.1]octane

By the same procedure as in Example 42, methyl 2-0-benzyl-3,7-dideoxy-4-0-methyl-6-C-methyl-DL-ribo-heptopyranoside, prepared as in Preparative Example 15, was converted to the title compound, which was obtained as a colourless oil; $^1$H-nmr data: 1.32 (3H,s), 1.36 (3H,s), 1.96 (1H,br d), 2.06 (1H,m), 3.25 (2H,m), 3.42 (3H, s), 4.05 (1H, s), 4.56 (1H,d), 4.64 (1H,d), 5.36 (1H,s) and 7.21-7.39 (5H,m).

Example 46

(1RS,2SR,4RS,7RS)- and (1RS,2SR,4RS,7SR)-4-Benzyloxy-2-methoxy-7-vinyl-6,8-dioxabicyclo[3.2.l]octane

A mixture of anomeric methyl 2-0-benzyl-3-deoxy-4-0-methyl-6-(RS) and (SR)-C-vinyl-DL-ribo-hexopyranosides was prepared from methyl 2-0-benzyl-3-deoxy-4-0-methyl-DL-ribo-hexodialdopyranoside (see Preparative Example 10) by the procedure of Preparative Example 11 but using vinylmagnesium bromide (in THF) in place of methylmagnesium bromide. A solution of the above 6-C-vinyl derivatives (300 mg) in toluene (5 ml) containing camphorsulphonic acid (100 mg) was heated under reflux for 1 h. The resulting black solution was cooled, shaken with water, dried ($MgSO_4$) and evaporated to give a brown oil. This was subjected to a preparative layer chromatography, on silica gel eluted with ethyl acetate/hexane (1:1) to give a 2:1 mixture (7RS,exo:7SR,endo) of the title compounds as an orange oil (55 mg); $^1$H-nmr data: 1.82-2.08 (2H,m), 3.15-3.39 (2H,m), 3.40 and 3.45 (3H, 2 x s), 4.22 (0.67H,d), 4.36 (0.67H,s), 4.49-4.73 (2.66H,m), 5.14-5.34 (2H,m), 5.44 (0.33H,m), 5.50 (0.67H,m), 5.78-5.94 (1H,m) and 7.21-7.40 (5H,m).

Example 47

O-Aralkylations of (1RS,2SR,4RS)-4-hydroxy-2-methoxy-6,8-dioxabicyclo[3.2.l]octane

(1RS,2SR,4RS)-4-hydroxy-2-methoxy-6,8-dioxabicyclo[3.2.l]octane (see Preparative Example 16) was 0-aralkylated with the appropriate aralkyl halide according to General Preparative Example 1, to yield the following compounds:

1 (1RS,2SR,4RS)-4-(2-fluorobenzyloxy)-2-methoxy-6,8-dioxabicyclo[3.2.l]octane, colourless oil; $^1$H-nmr data: 1.87 (1H,dt), 2.08 (1H,d), 3.20 (1H,d), 3.32(1H,d), 3.45 (3H,s), 3.74 (1H,d), 3.81 (1H,m), 4.67 (1H,m), 4.69 (2H,s), 5.45 (1H,s) and 6.96-7.54 (4H,m).

2 (1RS,2SR,4RS)-4-(2-chlorobenzyloxy)-2-methoxy-6,8-dioxabicyclo[3.2.l]octane, colourless oil; $^1$H-nmr data: 1.89 (1H,dt), 2.11 (1H,broad d), 3.21 (1H,d), 3.35 (1H,d), 3.46 (3H,s), 3.75 (1H,d), 3.82 (1H,m), 4.69 (1H,m), 4.73 (2H,s), 5.52 (1H,s) and 7.16-7.61 (4H,m).

3 (1RS,2SR,4RS)-4-(2,4-difluorobenzyloxy)-2-methoxy-6,8-dioxabicyclo[3.2.1]octane, colourless oil: $^1$H-nmr data: 1.88 (1H,dt), 2.07 (1H,m), 3.20 (1H,d), 3.32 (1H,d), 3.45 (3H,s), 3.74 (1H,d), 3.82 (1H,m), 4.64 (2H,s), 4.68 (1H,m), 5.44 (1H,s), 6.72-6.92 (2H,m) and 7.42-7.52 (1H,m).

4 (1RS,2SR,4RS)-4-(2,4-dichlorobenzyloxy)-2-methoxy-6,8-dioxabicyclo[3.2.1]octane, colourless oil; $^1$H-nmr data: 1.91 (1H,dt), 2.10 (1H,br d), 3.21 (1H,d), 3.36 (1H,d), 3.45 (3H,s), 3.75 (1H,d), 3.83 (1H,m), 4.68 (2H,s), 4.70 (1H,m), 5.50 (1H,s) and 7.21-7.58 (3H,m).

5 (1RS,2SR,4RS)-4-(2,6-difluorobenzyloxy)-2-methoxy-6,8-dioxabicyclo[3.2.1]octane, colourless oil: $^1$H-nmr data: 1.85 (1H,dt), 2.11 (1H,br d), 3.19 (1H,d), 3.34 (1H,d), 3.44 (3H,s), 3.75 (1H,d), 3.81 (1H,m), 4.64 (1H,m), 4.66 (1H,d), 4.76 (1H,d), 5.43 (1H,s), 6.84-6.95 (2H,m) and 7.21-7.36 (1H,m).

6 (1RS,2SR,4RS)-2-methoxy-4-[-2-(trifluoromethyl)benzyloxy]6,8-dioxabicyclo[3.2.l]octane, crystalline solid, m.p. 77° C; $^1$H-nmr data: 1.88 (1H,dt), 2.12 (1H, br d), 3.11 (1H,d), 3.34 (1H,d), 3.45 (3H,s), 3.76 (1H,d), 3.83 (1H,m), 4.70 (1H,d), 4.81 (2H,s), 4.50 (1H,s) and 7.32-7.84 (4H,m).

7 (1RS,2SR,4RS)-2-methoxy-4-(3-methylbenzyloxy)-6,8-dioxabicyclo[3.2.1]octane, colourless oil; $^1$H-nmr data: (400 MHZ): 1.84 (1H,dt), 2.02 (1H,br d), 2.33 (3H, s), 3.18 (1H,d), 3.28 (1H,d), 3.45 (3H,s), 3.71 (1H,d), 3.80 (1H,m), 4.53 (1H,d), 4.64 (1H,d), 4.68 (1H,m), 5.41 (1H,s) and 7.05-7.27 (4H,m).

8 (1RS,2SR,4RS)-4-(2-cyanobenzyloxy)-4-methoxy-6,8-dioxabicyclo[3.2.1]octane, colourless oil; $^1$H-nmr data: 1.90 (1H,dt), 2.16 (1H,br d), 3.20 (1H,d), 3.41 (1H,d), 3.44 (3H,s), 3.75 (1H,d), 3.80 (1H,m), 4.68 (1H,d), 4.75 (1H,d), 4.81 (1H,d), 5.52 (1H,s) and 7.32-7.70 (4H,m).

9 (1Rs,2sR,4Rs)-2-methoxy-4-(4-methylbenzyloxy)-6,8-dioxabicyclo[3.2.1]octane, colourless oil; $^1$H-nmr data: (400 MHz): 1.82 (1H,dt), 2.01 (1H,br d), 2.34 (3H,s), 3.18 (1H,d), 3.28 (1H,d), 3.44 (3H,s), 3.70 (1H,d), 3.80 (1H,m), 4.54 (1H,d), 4.64 (1H,d), 4.67 (1H,m), 5.39 (1H,br s) and 7.20-7.36 (4H,m).

10 (1RS,2SR,4RS)-2-methoxy-4-[3-(trifluoromethyl)benzyloxy]6,8-dioxabicyclo[3.2.1]octane, colourless oil; $^1$H-nmr data: (400 MHz): 1.89 (1H,dt), 2.04 (1H,m), 3.21 (1H,d), 3.32 (1H,d), 3.46 (3H,s), 3.75 (1H,d), 3.82 (1H,m), 4.63 (1H,d), 4.67 (2H,m), 4.68 (1H,d), 5.46 (1H,s) and 7.41-7.65 (4H,m).

11 (1RS,2SR,4RS)-4-(2-chloro-3-thienyl)methoxy-2-methoxy-6,8-dioxabicyclo[3.2.1]octane, colourless oil, $^1$H-nmr data (400 MHz): 1.85 (1H,dt), 2.01 (1H,br d), 3.19 (1H,d), 3.27 (1H,d), 3.47 (3H,s), 3.73 (1H,d), 3.81 (1H,m), 4.55 (1H,d), 4.60 (1H,d), 4.68 (1H,d), 5.40 (1H,s), 7.04 (1H,d) and 7.07 (1H,d).

Example 48

(1RS,2SR,4RS,7SR)-4-(2-Fluorobenzyloxy)-2-methoxy-7-methyl-6,8-dioxabicyclo[3.2.1]octane

Using the same procedure as in Example 47, except using (1RS,2SR,4RS,7SR)-4-hydroxy-2-methoxy-7-methyl-6,8-dioxabicyclo[3.2.l]octane, prepared as in Example 43, in place of (1RS,2SR,4RS)-4-hydroxy-2-methoxy-6,8-dioxabicyclo[3.2.1]-octane, the title compound (a 7-exo-isomer) was obtained as a colourless oil. This compound was obtained as a 9:1 mixture with its (1Rs,2SR,4RS,7RS)-(7-endo)-stereoisomer; $^1$H-nmr data (400 MHz): (major isomer only) 1.26 (3H,d), 1.89 (1H,dt), 2.05 (1H,m), 3.19 (1H,d), 3.28 (1H,d), 3.42 (3H,s), 4.02 (1H,q), 4.20 (1H,s), 4.69 (2H,s), 5.48 (1H,s) and 6.95-7.55 (4H,m).

Example 49

(1RS,2RS,4RS,7RS)-4-(2-Fluorobenzyloxy)-2-methoxy-l,7-dimethyl-6,8-dioxabicyclo[3.2.1]octane

A) E-1-Benzyloxy-2-methyl-but-2-ene

A solution of diisobutylaluminium hydride (1.5 M in toluene; 350 ml; 0.53 mol) was added over a period of 2 h to a stirred solution of ethyl tiglate (30 g, 0.26 mol) in THF (200 ml) keeping the temperature below 30°C. After 60 h, dilute aqueous HCl (25 ml) was added cautiously. The organic layer was decanted and the precipitated solid dissolved by addition of further dilute aqueous HCl. The aqueous layer was extracted with ethyl acetate (3 x 50 ml) and the combined organic layers were dried ($Na_2SO_4$) and concentrated to give a colourless liquid (18.5 g). This liquid was dissolved in DMSO (200 ml) and the stirred solution was treated with finely powdered potassium hydroxide (48.0 g; 0.86 mol) followed after 5 min by benzyl bromide (25 ml; 0.21 mol). Water (200 ml) was added and the mixture was extracted with ethyl acetate (3 x 100 ml). The combined organic layers were dried ($MgSO_4$) and concentrated to give a yellow liquid (36.63 ), which was purified by distillation under reduced pressure. The fraction boiling at 65°C and 2 mmHg was title compound A (13.3 g).

B) (4RS,5SR)-4-Benzyloxymethyl-2,2,4,5-tetramethyl-2,3-dioxolane

To formic acid (90% aq; 58.5 ml) stirred at 10°C was added hydrogen peroxide (30% aq; 13.5 ml), followed by title compound A (13.3 g, 77 mmol). After 20 h at room temperature, tlc indicated complete removal of starting material. The mixture was concentrated and treated with dilute aqueous NaOH (approx. 4 ml) until the pH of the solution was 9. The mixture was extracted with ethyl acetate (3 x 30 ml) and the combined organic extracts washed with saturated aqueous $NaHCO_3$, dried ($Na_2SO_4$) and concentrated to give a colourless liquid (17.15 g) which tlc indicated to be a mixture of the desired diol and its monoformate ester. This mixture was dissolved in methanol (100 ml) and treated with a catalytic amount of sodium metal. After 1 h, the solvent was removed to give a colourless liquid (12.96 g), one peak on glc. This product, acetone (100 ml), petroleum ether (b.p. 60-80°C; 100 ml), and p-toluenesulphonic acid (0.1 g) were heated at reflux in a Dean and Stark apparatus. During 2.5 d, 3 ml of water was collected. The mixture was allowed to cool, treated with anhydrous sodium acetate, filtered, and concentrated to give title compound B (15.94 g; 80%).

C) (4RS, 5SR)-4-Hydroxymethyl-2,2,4,5-tetramethyl-1,3-dioxolane

Title compound B (15.9 g; 64 mmol) in methanol (50 ml) was treated with 4 drops of acetic acid and the mixture hydrogenated at room temperature and atmospheric pressure using 10% Pd on C as catalyst. After 20 h, the mixture was filtered through silica and concentrated under reduced pressure to afford crude product (9.96 g) which was purified by chromatography on silica eluted with ethyl acetate/hexane (3:7). A colourless oil was obtained containing title compound C (2.97 g) which was used without further purification.

D) (4RS,4′RS,5′SR)-1,1-Dimethoxy-4-(2,2,4,5-tetramethyl-1,3-dioxolan-4-yl)-4-hydroxybutan-2-one

A solution of DMSO (2.9 ml, 40.8 mmol in THF (3 ml) was added dropwise to a stirred solution of oxalyl chloride (1.0 ml, 20.4 mmol) in THF (10 ml) at -60°C under argon. After 2 min, a solution of title compound C (2.97 g, 18.5 mmol) in THF (5 ml) was added followed, after 15 min, by triethylamine (13 ml, 92.7 mmol). The mixture was stirred for 5 min, warmed to room temperature, filtered under argon, cooled to -78°C, and added via a canula to a solution of the lithium enolate of 1,1-dimethoxy-propan-2-one [generated from 1,1-dimethoxypropan-2-one (2.2 ml, 18 mmol and lithium hexamethyldisilazide (1 M in hexane; 18 ml, 18 mmol)]. After stirring for 1 h at -78°C, the reaction was quenched by addition of acetic acid (1 ml) in THF (5 ml). The mixture was partitioned between saturated aqueous sodium bicarbonate and ethyl acetate and the aqueous layer further extracted with ethyl acetate (3 x 50 ml). The combined organic phase was washed with saturated aqueous sodium bicarbonate (50 ml), dried ($Na_2SO_4$) and concentrated under reduced pressure to give a brown liquid (4.96 g) which was chromatographed on silica. Elution with hexane-ethyl acetate gave title compound D as a colourless oil (1.26 g).

E) (1RS,2SR,4RS,7RS)-4-(2-Fluorobenzyloxy)-2-methoxy-l,7-dimethyl-6,8-dioxabicyclo[3.2.1]octane

Using the method described in Example 35, but substituting title compound D (1.26 g, 4.6 mmol) for (4'Rs,5sR)-5-hydroxy-2,2-dimethoxy-5-(2,2,4-trimethyl-1,3-dioxolan-4-yl)pentan-3-one in part A and 2-fluorobenzyl chloridefor benzyl bromide in part C, title compound a 7-endo-isomer was obtained as a colourless oil (30 mg); $^1$H-nmr data: 1.25 (3H,d,J 7Hz), 1.37 (3H,s), 1.96 (1H,dt,$J_1$ = l6 Hz,$J_2$ = 5 Hz), 2.13 (1H,dd,$J_1$ = l6 Hz,$J_2$ = 1 Hz), 3.02 (1H,d,J 5 Hz), 3.32 (1H,d,J 5 Hz), 3.41 (3H,s), 3.77 (1H,q,J 6.5 Hz) 4.68 (2H, br s), 5.37 (1H, br s) and 6.97-7.51 (4H,m).

## Example 50

### (1S,6S,8R)-6-Benzyloxy-8-methoxy-3,9-dioxabicyclo[3.3.l]nonane

A) 7-0-Acetyl-2,6-anhydro-5-O-benzyl-4-deoxy-3-0-methyl-D-allo-heptononitrile

1,6-Anhydro-4-0-benzyl-3-deoxy-2-0-methyl-β -D-ribo-hexopyranose (EP 0229034A, Example 3.1; 2.5 g) was acetolysed with acetic anhydride-boron trifluoride diethyl etherate (following EP 0229034A; see Example 15, first reaction). A stirred solution of the resulting diacetate in acetonitrile (20 ml) and trimethylsilyl cyanide (2 ml) at 0° C was treated with boron trifluoride etherate (0.5 ml). After 15 min the reaction mixture was evaporated and the residue was flash chromatographed to give the title compound A - (1.55 g, 44%) as white crystals, m.p. 88-89° C (recrystallised from ethanol), $[\alpha]_D$ -74°, and its altro-isomer (1.06 g, 37%) as a yellow oil, $[\alpha]_D$ +95°. $^{13}$C-nmr data, allo-isomer: 170.6 ($OCOCH_3$), 137.2, 128.5, 128.0 and 127.8 (aromatic), 116.9 (CN), 78.8, 75.7 and 70.4 (C-3, C-5 and C-6), 70.9 ($PhCH_2O$), 68.9 (C-2), 62.8 (C-7), 57.7 ($CH_3O$), 34.6 (C-4) and 20.7 ($CH_3CO$). $^{13}$C-nmr data, altro-isomer: 170.7 ($OCOCH_3$), 137.2, 128.5, 128.0 and 127.9 (aromatic), 115.0 (CN), 74.9, 73.3 and 70.6 (C-3, C-5 and C-6), 70.6 ($PhCH_2O$), 66.9 (C-2), 62.5 (C-7), 57.1 ($CH_3O$), 31.8 (C-4) and 20.6 ($CH_3CO$).

B) 7-0-Acetyl-2,6-anhydro-5-0-benzyl-3-0-methyl-D-allo-heptitol

The allo-nitrile from Part A (1.0 g) was added to a stirred mixture of pyridine (16 ml), acetic acid (8 ml), water (8 ml), sodium hypophosphite monohydrate (2.0 g), 1,2-dianilinoethane (0.84 g), and Raney nickel (ca. 5 g). After stirring the mixture for 2 h at ambient temperature the catalyst was removed by filtration. The filtrate was partitioned between chloroform and water, the organic phase was dried ($MgSO_4$) and evaporated. The yellow oil obtained was dissolved in dichloromethane (60 ml) and the stirred solution was acidified with p-toluenesulphonic acid (1.5 g) in acetone (20 ml). The crystalline precipitate was filtered off, the filtrate was neutralised with triethylamine and evaporated to a light brown syrup which was at once dissolved in methanol (20 ml) and treated at 0° C with sodium borohydride (150 mg). After stirring at 0° C for 15 min the reaction mixture was neutralised with acetic acid and evaporated to a small volume. Partitioning between water and chloroform, drying of the organic phase ($MgSO_4$) and removal of the solvent followed by flash chromatography of the crude product gave the title compound B (500 mg, 55%) as a colourless oil; $^{13}$C-nmr data: 170.9 ($OCOH_3$), 137.6. 128.4 and 127.8 (aromatic), 80.4, 78.1, 74.1 and 71.9 (C-2, C-3, C-5 and C-6), 70.7 ($PhCH_2O$), 63.7 and 62.1 (C-1 and C-7), 56.5 ($CH_3O$), 33.9 (C-4) and 20.7 ($CH_3CO$).

C) (1S,6S,8R)-6-Benzyloxy-8-methoxy-3,9-dioxabicyclo|3.3.1]nonane

To a stirred solution of the heptitol from Part B (1.0 g) in dichloromethane (5 ml) and pyridine (5 ml) at ambient temperature, p-toluenesulphonyl chloride (1.14 g) was added. After 16 h the reaction mixture was partitioned between chloroform and water. The organic extract was dried ($MgSO_4$) and evaporated to an amber oil. This was stirred in dry methanol (10 ml) containing sodium cyanide (50 mg) for 16 h at ambient temperature. The solution was filtered through silica gel (0.063-0.200 mm) and evaporated. The residue was

dissolved in dry tetrahydrofuran (15 ml) and sodium hydride (100 mg, 80% in oil) was added with stirring at 0°C under argon. After 16 h at ambient temperature and 4 h at 60°C excess sodium hydride was destroyed by addition of methanol to the cooled reaction mixture. The solvent was removed and the crude product was subjected to flash chromatography. The title compound (0.49 g, 60%) was obtained as a colourless oil which solidified after several days at -8°C, m.p. 31-33°C, [ α ]$_D$ +22°; 13C-nmr data: 138.5, 128.2, 127.5 and 127.4 (aromatic), 76.5, 73.5, 70.9 and 70.3 (C-1, C-5, C-6 and C-8), 70.2 ($PhCH_2O$), 68.0 (C-2 and C-4), 56.4 ($CH_3O$), and 28.3 (C-7).

Example 51

(1S,2S,4R)-2-Benzyloxy-4-methoxy-8-oxa-6-thiabicyclo[3.2.1]octane

To a stirred solution of (1R,2S,4R)-2-benzyloxy-4-methoxy-6,8-dioxabicyclo[3.2.1]octane (EP 0229034A, Example 3.1) (1.3 g) at 0°C under argon was slowly added phosphorous tribromide (0.5 ml). The resulting solution was stirred at 0°C for 30 min and warmed to room temperature. After 4.5 h, the reaction mixture was cooled to -5°C and triethylamine (2 ml) was added. Finely powdered sodium hydrosulfide (2.5 g) was added and the mixture allowed to stand overnight. The reaction was quenched by pouring into dilute aqueous HC1 and extracted with ether. The organic phase was washed with water, dried ($Na_2SO_4$), filtered, and concentrated under reduced pressure to give an oil (1.09 g). Purification by preparative tlc on silica eluted with ethyl acetate-hexane (1:1) gave the title compound (131 mg) as an oil; $^1$H-nmr data: 2.01 (1H,d,J 10Hz), 2.14 (1H,dt,$J_1$ =l0Hz,$J_2$=4Hz), 2.73 (1H,d,J 7Hz), 3.14 (1H,dd,$J_1$=6Hz,$J_2$=7Hz), 3.20 (2H,m), 3.47 (3H,s), 4.66 (2H,AB-system, J 6Hz), 4.88 (1H,m) 5.64 (1H,s) and 7.20-7.45 (5H,m).

Example 52

(1RS,2RS,4SR)-4-Benzyloxy-2-methoxy-8-oxa-6-thiabicyclo[3.2.1]octane

The title compound was prepared from (1RS,2SR,4RS)-4-benzyloxy-2-methoxy-6,8-dioxabicyclo[3.2.1]-octane (see Example 40) by the method described in Example 51. It was obtained as an oil; $^1$H-nmr data: 2.06 (1H,br d,J 15Hz), 2.20 (1H,dt,$J_1$=15Hz,$J_2$=5Hz), 2.83 (1H,d,J 9Hz), 3.17 (1H,m), 3.24 (1H,dd,$J_1$=6Hz, J2=11Hz), 3.37 (1H,m), 3.44 (3H,s), 4.97 (1H,d,J 6Hz), 5.62 (1H,s) and 7.28-7.48 (5H,m).

Example 53

(1S,2S,4R)-2-Benzyloxy-4-methoxy-8-oxa-6-thiabicyclo[3.2.1]octane,6-oxide

To a stirred solution of (1S,2S,4R)-2-Benzyloxy-4-methoxy-8-oxa -6-thiabicyclo[3.2.1]octane (see Example 51) (0.30 g) in dry dichloromethane (15 ml) was slowly added 3-chloroperoxybenzoic acid (0.19 g) After 2 h, further 3-chloroperoxybenzoic acid (0.13 g) was added and the mixture stirred for 1.25 h. The mixture was partitioned between saturated aqueous sodium bicarbonate and ether, the organic layer separated and washed with water, dried ($Na_2SO_4$), filtered, and concentrated under reduced pressure to yield an oily solid (0.35 g) which contained two sulphoxide isomers. When chromatographed on silica eluted with chloroform-acetonitrile (95:5) it yielded isomer A of the title compound as an oil (35 mg). When this experiment was repeated using acetic acid/acetic anhydride as the solvent a second isomer of the title compound was isolated (isomer-B). $^1$H-nmr data (isomer A): 1.21 (1H,dt,$J_2$=17Hz, $J_2$=4Hz), 2.08-2.19 (1H,m),2.85-(1H,dd,$J_1$=7Hz,$J_2$=l2Hz), 3.09 (1H,m), 3.47 (3H,s), 4.64 (2H,AB-system,J 12Hz), 5.25 (1H,br d,J 7Hz), 5.45 (1H,br s) and 7.25-7.48 (5H,m). $^1$H-nmr data (isomer-B): (inter alia) 2.37 (1H,d,J 13Hz), 3.41 (3H,s) and 4.61 (2H,AB-system,J 12Hz).

Example 54

(1S,2S,4R)-2-Benzyloxy-4-methoxy-8-oxa-6-thiabicyclo[3.2.1]octane,6,6′-dioxide

To a stirred solution of (1S,2S,4R)-2-benzyloxy-4-methox-8-8-oxa-6-thiabicyclo[3.2.1]octane (0.5 g) (see Example 51) in methanol (3 ml) was added an aqueous solution of sodium periodate (3 eq; 2 ml). After 30 min, the mixture was extracted with ether. The organic layer was dried ($Na_2SO_4$), filtered, and concentrated to give an oil (0.35 g) which was purified by preparative tlc eluted with ethyl acetate - hexane (1:1) to yield the title compound as an oil (50 mg) which crystallised on standing; $^1$H-nmr data: 2.29 (2H,m), 2.83 (1H,d,J 14Hz), 3.36 (1H,m), 3.43 (1H,m), 3.46 (3H,s), 3.68 (1H,m), 4.66 (2H,AB-system,J 12Hz), 4.82 (1H,br s), 4.96 (1H,br d,J 10Hz) and 7.24-7.40 (5H,m).

Example 55

(1R,2S,4R)-5-Bromomethyl-2-cyclohexanemethyloxy-4-methoxy-6,8-dioxabicyclo[3.2.l]octane

A solution of (1R,2S,4R)-2-benzyloxy-5-hydroxymethyl-4-methoxy-6,8-dioxabicyclo[3.2.1]octane (see Preparative Example 17) (0.8 g) in ethanol (50 ml) was stirred under hydrogen over rhodium on alumina (5%, 0.2 g) for 24 h. The catalyst was removed by filtration, and the filtrate was evaporated to a colourless syrup. This was dissolved in dichloromethane (20 ml) and treated with pyridine (0.75 ml) and trifluoromethanesulphonic anhydride (1.5 ml) at -30 °C. After stirring for 15 min the reaction mixture was warmed to ambient temperature and poured into ice-cold aqueous sodium bicarbonate (50 ml). Extraction with dichloromethane gave, after washing with water, drying ($MgSO_4$) and removal of the solvent, the crude triflate as a brown solid. It was stirred in hexamethylphosphoramide (2.5 ml) containing tetrabutylammonium bromide (1.1 g) for 1.5 h at 50 °C. The cooled solution was partitioned between ether and water, the ether extract was dried ($MgSO_4$) and evaporated to a brown oil. Flash chromatography gave the title compound - (0.50 g, 50%) as white crystals, m.p. 78-81 °C, $[\alpha]_D$ -52°; $^{13}$C-nmr data: 105.5 (C-5), 77.2, 74.4 and 73.1 (C-1, C-2 and C-4), 75.4 ($RCH_2O$), 66.6 (C-7), 57.2 ($CH_3O$), 37.6 (C-1′), 32.6 ($CH_2Br$), 30.1 29.9, 26.6, 25.7 and 25.7 (C-2′, C-3′, C-4′, C-5′ and C-6′) and 23.9 (C-3).

Example 56

(1RS,2RS,3RS,4RS)-2,3-Epoxy-4-(2-methylbenzyloxy)-6,8-dioxabicyclo[3.2.1]octane

By the same procedure as in Example 36, stages C-E, (1RS,3RS,4SR)-3,4-epoxy-6,8-dioxabicyclo-[3.2.1]octane (F Sweet and R K Brown, Can.J.Chem., 1968, 46, 2889) was converted to a mixture of epimeric epoxides which was subjected to preparative h.p.l.c. (silica, eluant 1:1 ethyl acetate/hexane) to yield the more mobile minor isomer and then the title compound as a colourless syrup (1.4 g).

Example 57

(1R,6R,8S)-6-Benzyloxy-8-methoxy-2,9-dioxabicyclo[3.3.1]nonane

Methyl 6-0-tert-butyldimethylsilyl-3-deoxy-2-0-methyl-α-D-ribo-hexopyranoside was prepared from methyl 4,6-0-benzylidene-3-deoxy-2-0-methyl-α-D-ribo-hexopyranoside as detailed in Preparative Example 7, part A of EP 0229034A for the corresponding 6-0-tert-butyldiphenylsilyl ether, but substituting tert-butyldimethylsilyl chloride for tert-butyldiphenylchlorosilane.

This tert-butyldimethylsilylated derivative (4.5 g) was alkylated following the procedure of General Preparative Example 1 using 2-fluorobenzyl bromide to give the 4-0-(2-fluorobenzyl) ether as a syrup (4.5 g), which was desilylated to the 6-hydroxy-compound by heating under reflux with tetrabutylammonium

fluoride [24 ml, 1 M in THF, dried by evaporating to dryness twice with toluene (50 ml)] in tetrahydrofuran for 10 min. The resulting solution was evaporated onto silica gel and subjected to flash chromatography to give a syrup (2.4 g) which was then dissolved in dry dichloromethane (150 ml) and heated under reflux with p-toluenesulphonyl chloride (3.45 g), triethylamine (3.8 ml) and dimethylaminopyridine (0.12 g) until the reaction was complete (4 h). Water (10 ml) was added and heating was continued until no tosyl chloride was present by tlc. Chloroform was added and the mixture washed with water, the aqueous phase was back-extracted with chloroform and the combined organic phase was washed with dilute hydrochloric acid and saturated sodium hydrogen carbonate, dried ($MgSO_4$) and evaporated to yield the crude 6-tosylate as a syrup.

This tosylate was dissolved in dry acetone (150 ml) and refluxed with sodium iodide (5.4 g) for 20 h, when saturated sodium chloride was added and the mixture extracted twice with chloroform, dried ($MgSO_4$) and evaporated. Purification by silica gel chromatography gave methyl 3,6-dideoxy-4-0-(2-fluorobenzyl)-6-iodo-2-0-methyl-α-D-ribo-hexopyranoside as white cyrstals (2.3 g).

This 6-iodide was then dissolved in dimethylformamide/acetonitrile (1:1 v/v, 100 ml) to which was added tetrabutylammonium cyanide (9.2 g) and the mixture was stirred under an inert atmosphere overnight. Water and chloroform were added and the aqueous phase back-extracted with chloroform. The combined organic phase was washed with diluted hydrochloric acid and water, dried ($MgSO_4$) and evaporated. Flash chromatographic purification gave the 6-nitrile as white crystals (1.66 g, 99% from the 6-iodide). This nitrile was treated for 3 days at room temperature with the reagent system Raney nickel/sodium hypophosphite/1,2-dianilinoethane in acetic acid/water/pyridine (1:1:2 v/v) according to the method described in J. Org. Chem., 1973, 38, 1836, and the protected aldehyde derivative was isolated by flash chromatography as a syrup (2.22 g). This product was dissolved in dry dichloromethane (100 ml), cooled to 1°C, and p-toluenesulphonic acid (5 g) in acetone (10 ml) was added. The white precipitate which formed immediately was removed by filtration and washed with dichloromethane (3 x 20 ml). The combined filtrate was washed with water (x 2), saturated hydrogen carbonate and water, then dried ($MgSO_4$) and evaporated. The resulting aldehyde was immediately dissolved in ethanol (50 ml) and reduced with sodium borohydride (0.1 g) in ethanol (10 ml). Acetone (5 ml) was added and the mixture brought to pH7 with acid resin, filtered and evaporated with toluene (2 x). The resulting alcohol was then dissolved in toluene, mixed with a catalytic amount of p-toluenesulphonic acid and kept under an inert atmosphere at 40°C. After four days, triethylamine was added until the pH became 7, and the mixture was evaporated onto silica gel. Flash chromatography gave the title compound as a colourless syrup (0.37 g, 23% from the 6-iodide); $^{13}$C-nmr data: 160.5 (d, $J_{C,F}$ 246.2 Hz, C-F in $C_6H_4F$), 130.1 (d, $J_{C,F}$ 4.3 Hz), 129.2 (d, $J_{C,F}$ 8.1 Hz) and 124.0 (d, $J_{C,F}$ 3.6 Hz) (CH in $C_6H_4F$), 125.2 (d, $J_{C,F}$ 14.4 Hz, q-C in $C_6H_4F$), 115.0 (d, $J_{C,F}$ 21.3 Hz, CHCF in $C_6H_4F$), 95.5 (C-1), 76.7, 76.3 and 67.4 (C-5, C-6 and C-8), 64.2 (d, $J_{C,F}$ 6.6 Hz, Ar$CH_2$O), 64.1 (C-3), 56.6 ($OCH_3$), 31.1 and 29.9 (C-4 and C-7).

Composition Example 1

Granule formulation of (1R,2S,4R)-2-cyclohexanemethyloxy-4-methoxy-6,8-dioxabicyclo[3.2.1]octane

Granules are prepared by blending china clay with "Polyfon" H and "Synperonic" NP8 and extruding the mixture to form granules. The remaining constituents are dissolved in methanol, the solution applied to the granules and allowed to evaporate to give granules of the following composition:

| | Amount (grams) |
|---|---|
| Active ingredient | 10 |
| "Topanol" O | 10 |
| "Waxoline" Black | 10 |
| "Synperonic" NP8 | 2.4 |
| China Clay | to 1 kg |

The materials represented by the various trade names in the above list are as follows:

Topanol O - An anti-oxidant comprising di-tertiary-butyl-p-hydroxytoluene

Waxoline Black 5 BP - A mixture of azo and other solvent-soluble dyes acting as an absorbent for ultra-violet light
Synperonic NP 8 - a Surfactant comprising a condensate of eight molar proportions of ethylene oxide with p-nonyl-phenol
Polyron H - A dispersing agent comprising sodium lignosulphonate

Composition Example 2

Emulsifiable concentrate of (1R,2S,4R)-2-cyclohexane-methyloxy-4-methoxy-6,8-dioxabicyclo[3.2.1]octane

A concentrate is prepared by agitating the constituents together until a homogeneous solution is obtained of the following compositions.

| | Amount (grams) |
|---|---|
| Active ingredient | 100 |
| "Waxoline" Black | 10 |
| Calcium dodecylbenzene-Sulphonate (in butanol solution) | 30 |
| "Synperonic" NP13 | 20 |
| "Topanol" O | 10 |
| Solvesso 150 | to 1 litre |

For explanation of the trade named components Waxoline Black and Polyfon see Composition Example 1:
Synperonic NP13 - A surface-active agent comprising a condensate of p-nonylphenol with thirteen molar proportions of ethylene oxide
Solvesso 150 - An aromatic solvent comprising a mixture of alkylbenzenes

Composition Example 3

Granule formulation of (1R,2S,4R,7S)-2-benzyloxy-4-methoxy7-methyl-6,8-dioxabicyclo[3.2.1]octane

Granules are prepared by blending china clay with "Polyfon" H and "Synperonic" NP8 and extruding the mixture to form granules. The remaining constituents are dissolved in methanol, the solution applied to the granules and allowed to evaporate to give granules of the following composition:

| | Amount (grams) |
|---|---|
| Active ingredient | 10 |
| "Topanol" O | 10 |
| "Waxoline" Black | 10 |
| "Synperonic" NP8 | 2.4 |
| China Clay | to 1 kg |

The materials represented by the various trade names in the above list are as follows:
Topanol O - An antioxidant comprising di-tertiary-butyl-p-hydroxytoluene
Waxoline Black 5BP - A mixture of azo and other solvent-soluble dyes acting as an absorbent for ultra-violet light
Synperonic NP 8 - A surfactant comprising a condensate of eight molar proportions of ethylene oxide with

p-nonylphenol
Polyron H - A dispersing agent comprising sodium lignosulphonate

Composition Example 4

Emulsifiable concentrate of (1R,2S,4R,7S)-2-benzyloxy-4-methoxy-7-methyl-6,8-dioxabicyclo[3.2.1]octane

A concentrate is prepared by agitating the constituents together until a homogeneous solution is obtained of the following compositions:

| | Amount (grams) |
|---|---|
| Active ingredient | 100 |
| "Waxoline" Black | 10 |
| Calcium dodecylbenzene-sulphonate (in butanol solution) | 30 |
| "Synperonic" NP13 | 20 |
| "Topanol" O | 10 |
| Solvesso 150 | to 1 litre |

For explanation of the trade-named components Waxoline Black and Polyfon see Composition Example 1. Synperonic NP13 - A surface-active agent comprising a condensate of p-nonylphenol with thirteen molar proportions of ethylene oxide
Solvesso 150 - An aromatic solvent comprising a mixture of alkylbenzenes

Composition Example 5

Granule formulation of (1S,2R,4S)-4-benzyloxy-2-methoxy-6,8-dioxabicyclo[3.2.1]octane

Granules are prepared by blending china clay with "Polyfon" H and "Synperonic" NP8 and extruding the mixture to form granules. The remaining constituents are dissolved in methanol, the solution applied to the granules and allowed to evaporate to give granules of the following composition:

| | Amount (grams) |
|---|---|
| Active ingredient | 10 |
| "Topanol" O | 10 |
| "Waxoline" Black | 10 |
| "Synperonic" NP8 | 2.4 |
| China Clay | to 1 kg |

The materials represented by the various trade names in the above list are as follows:
Topanol O - An anti-oxidant comprising di-tertiary-butyl-p-hydroxytoluene
Waxoline Black 5 BP - A mixture of azo and other solvent-soluble dyes acting as an absorbent for ultra-violet light
Synperonic NP 8 - A surfactant comprising a condensate of eight molar proportions of ethylene oxide with p-nonylphenol
Polyron H - A dispersing agent comprising sodium lignosulphonate

Composition Example 6

Emulsifiable concentrate of (1S,2R,4S)-4-benzyloxy-2-methoxy-6,8-dioxabicyclo[3.2.1]octane

A concentrate is prepared by agitating the constituents together until a homogeneous solution is obtained of the following compositions:

| | Amount (grams) |
|---|---|
| Active ingredient | 100 |
| "Waxoline" Black | 10 |
| Calcium dodecylbenzene-sulphonate (in butanol solution) | 30 |
| "Synperonic" NP13 | 20 |
| "Topanol" O | 10 |
| Solvesso 150 | to 1 litre |

For explanation of the trade named components Waxoline Black and Polyfon see Composition Example 1: Synperonic NP13 - A surface-active agent comprising a condensate of p-nonylphenol with thirteen molar proportions of ethylene oxide
Solvesso 150 - An aromatic solvent comprising a mixture of alkylbenzenes

**Claims**

1. A compound having the formula (I)

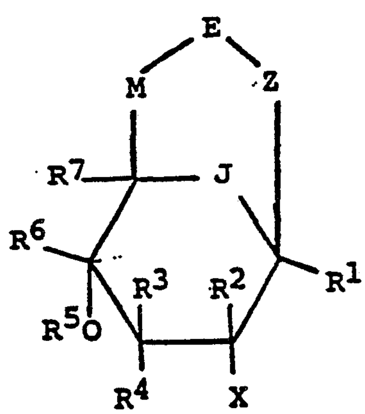

(I)

in which
$R^1$ represents a hydrogen or halgen atom, an O-, N-, P- or S-linked organic or optionally substituted hydrocarbyl group
or $R^1$, together with X, E or Z, may form an optionally substituted fused alicyclic or heterocyclic member;
$R^2$ represents a hydrogen or fluorine atom, a hydroxy group or an optionally substituted hydrocarbyl or O-linked hydrocarbyl group or $R^2$, together with X, may form a ketonic oxygen atom -O- or a group of the formula $-CH_2O-$;
$R^3$ represents a hydrogen or halogen atom, a hydroxy group or an optionally substituted hydrocarbyl or O-linked hydrocarbyl group
or $R^3$, together with $R^4$, may form an optionally substituted hydrocarbyl group;
$R^4$ represents a hydrogen or halogen atom, a hydroxyl group or an optionally substituted hydrocarbyl or O-linked hydrocarbyl group
or $R^4$, together with X, may form an ox gen atom;
$R^5$ represents an optionally substituted aralkyl, alicyclicalkyl, heterocyclicalkyl, aryl, alicyclic,

heterocyclic, alkyl, alkenyl or alkynyl group

$R^6$ represents a hydrogen atom or an optionally substituted hydrocarbyl group;

$R^7$ represents a hydrogen or halogen atom, an O-, N-, P- or S-linked organic or optionally substituted hydrocarbyl group

or together with M or E, $R^7$ may form an optionally substituted fused alicyclic or heterocyclic member;

X represents an O-, N- or S-linked organic group, an optionally substituted hydrocarbyl group, a halogen atom, a hydroxy, amino, alkoxyamino, nitro, cyano, azido, sulpho or phospho group,

M, E and Z

independently of each other represent an O or S atom, a sulphinyl, sulphonyl, amino, phosphinico or N-linked organic group, a single bond, an optionally substituted hydrocarbyl group or the group $CR^8R^9$, wherein $R^8$ represents a hydrogen or halogen atom, an O-, N-, P- or S-linked organic or optionally substituted hydrocarbyl group, a hydroxy, amino, alkoxyamino, nitro, cyano, azido, sulpho or phospho group or $R^8$, together with $R^9$, may form an optionally substituted spiroalicyclic or spiroheterocyclic member, an optionally substituted hydrocarbyl group or a double bonded oxygen atom;

and wherein $R^9$ represents a hydrogen or halogen atom, an O-, N-, P- or S-linked organic or optionally substituted hydrocarbyl group, a hydroxy, amino, alkoxyamino, nitro, cyano, azido, sulpho or phospho group or; and

wherein, adjacent M, E or Z groups may be joined by a double bond; and

wherein, two or more of M, E and Z may form an optionally substituted fused alicyclic or heterocyclic ring;

with the proviso that only one of the group M, E or Z may represent a single bond;

J represents an O or S atom, a sulphinyl, sulphonyl, amino, phosphinico or N-linked organic group or an optionally substituted hydrocarbyl group;

and an enantiomer, salt or mixture thereof;

with the following provisos:

(i) when one of M, E and Z is a single bond and J is an oxygen atom then at least one of the remainder of M, E and Z represents an O or S atom, a sulphinyl, sulphonyl, amino, phosphinico or N-linked organic group or the group $CR^8R^9$, in which at least one of $R^8$ or $R^9$ is not a hydrogen atom or O-linked organic or optionally substituted hydrocarbyl group;

(ii) when -M-E-Z- is $-OCH_2-$, J is an O atom, X and $R^5$0 are the same aralkoxy group, and $R^1$ is hydrogen, then the enantiomer thereof is not included;

(iii) when -M-E-Z- together form the group $-CH_2O-$, J is an oxygen atom, $R^7$ is a hydrogen atom, and $R^5$ is an optionally substituted aralkyl group, then

(a) $R^1$ represents an O-, N-, P- or S-linked organic group; or

(b) $R^1$, together with X, forms an optionally substituted fused alicyclic or heterocyclic member; or

(c) $R^2$ represents a fluorine atom; or

(d) $R^3$ or $R^4$ represents a halogen atom; or

(e) $R^3$, together with $R^4$, forms an optionally substituted hydrocarbyl group; or

(f) $R^4$, together with X, forms an oxygen atom; and

(iv) when -M-E-Z- is $-OCH_2-$, J is an oxygen atom $R^1$ is a hydrogen atom and X is not an optionally substituted aralkoxy group, then

(a) $R^2$ represents a fluorine atom; or

(b) $R^3$ or $R^4$ represents a halogen atom; or

(c) $R^3$, together with $R^4$, forms an optionally substituted hdyrocarbyl group; or

(d) $R^4$, together with X, forms an oxygen atom;

(v) when J is O and -M-E-Z- is $CH_2O$- then:

(a) $OR^5$, X and R are not the same substiuted alkyloxy group or all benzyloxy;

(b) when $R^3$ is a hydroxy or an O-linked organic group, X is a fluorine atom, or a hyroxy, O-linked organic, amino, azido, methyl, acetamido or benzylamino group, $R^5$ is not an unsubstituted $C_1$-$C_4$ alkyl or $C_2$-$C_4$ alkenyl or a tetrahydrofuran-1-methyl group in which each of the 2, 3 and 4 positions is substituted with an 0-linked organic or hydroxy group; or a substituted alkyl group where the substitutuent is 0-linked or contains oxygen as the only heteroatom;

(vi) when -M-E-Z- is $CR^8R^9O$-, $R^8$ is an exo-ethyl or exo-fluoro group and $R^1$, $R^7$ and $R^9$ are hydrogen, then $OR^5$, X and $R^3$ are not all benzyloxy;

(vii) when -M-E-Z- is $-CH_2O-$, J is an oxygen atom, and $R^1$, $R^2$, $R^3$, $R^6$ and $R^7$ are hydrogen atoms, then $R^5$O, $R^4$ and X are not all methoxy groups;

(viii) when -M-E-Z- is -C(phenyl)$_2$O-, J is an oxtgen atom, and $R^1$, $R^2$, $R^4$, $R^6$ and $R^7$ represent hydrogen atoms then $R^5$O, $R^3$ and X are not all methoxy; and

(ix) when -M-E-Z- is $-CH_2O-$, $R^5$ is methyl, J is an oxygen atom, $R^1$, $R^3$, $R^4$, $R^6$ and $R^7$ are hydrogen atoms then X and $R^2$ are not jointly an oxygen atom, or not both hydroxy or methoxy groups or X is not hydroxy when $R^2$ is a hydrogen atom.

2. A compound of claim 1 in which $R^3$, $R^4$, $R^6$, $R^7$, X and J are as defined in claim 1 and where

$R^1$ represents a hydrogen or halogen atom, an O-, N-, P- or S-linked organic or optionally substituted hydrocarbyl group

or $R^1$, together with X, E or Z, may form an optionally substituted fused alicyclic or heterocyclic member;

or, when -M-E-Z- together form the group $-OCR^8R^9$-and J is an oxygen atom, $R^1$ may also form an optionally substituted fused alicyclic or heterocyclic member with X or with $R^8$ or $R^9$;

$R^2$ represents a hydrogen or fluorine atom, a hydroxy group or an optionally substituted hydrocarbyl or O-linked hydrocarbyl group

or $R^2$, together with X, may form a ketonic oxygen atom -O- or a group of the formula $-CH_2O-$;

$R^3$ represents a hydrogen or halogen atom, a hydroxy group or an optionally substituted hydrocarbyl or O-linked hydrocarbyl group

or $R^3$, together with $R^4$, may form an optionally substituted hydrocarbyl group;

$R^5$ represents an optionally substituted aralkyl, alicyclicalkyl, heterocyclicalkyl, aryl, alicyclic, heterocyclic, alkyl, alkenyl or alkynyl group

M, E and Z

independently of each other represent an O or S atom, a sulphinyl, sulphonyl, amino, phosphinico or N-linked organic group, a single bond, or the group $CR^8R^9$,

wherein $R^8$ represents a hydrogen or halogen atom, an O-, N-, P- or S-linked organic or optionally substituted hydrocarbyl group, a hydroxy, amino, alkoxyamino, nitro, cyano, azido, sulpho or phospho group or $R^8$, together with $R^9$, may form an optionally substituted spiroalicyclic or spiroheterocyclic member, an optionally substituted hydrocarbyl group or a double bonded oxygen atom;

and wherein $R^9$ represents a hydrogen or halogen atom, an O-, N-, P- or S-linked organic or optionally substituted hydrocarbyl group, a hydroxy, amino, alkoxyamino, nitro, cyano, azido, sulpho or phospho group or; and

wherein, adjacent M, E or Z groups may be joined by a double bond; and

wherein, two or more of M, E and Z may form an optionally substituted fused alicyclic or heterocyclic ring.

3. A compound of claim 1 in which -M-E-Z- together form the group $OCR^8R^9$- where $R^8$ and $R^9$ are as defined in claim 1 and in which $R^2$, $R^3$, $R^4$, $R^6$ and X are as defined in claim 1, J is an oxygen atom,

$R^1$ represents a hydrogen or halogen atom, an O-, N-, P- or S-linked organic or optionally substituted hydrocarbyl group

or together with the group X, $R^1$ may form an optionally substituted fused alicyclic or heterocyclic member or together with the group $R^8$ or $R^9$, $R^1$ may form an optionally substituted fused alicyclic or heterocyclic member;

$R^5$ represents an optionally substituted aralkyl, alicyclicalkyl, heterocyclicalkyl, aryl, alicyclic, heterocyclic, alkenyl or alkynyl group; and

$R^7$ represents a hydrogen or halogen atom, an O-, N-, P- or S-linked organic or optionally substituted hydrocarbyl group.

4. A compound of claim 1 in which J represents an oxygen atom, -M-E-Z- together represent a $-CR^8R^9O-$ group, $R^2$, $R^3$, $R^4$, $R^6$, $R^8$, $R^9$ and X are as defined in claim 1 and $R^1$ represent a hydrogen atom, an O-, N-, P- or S-linked organic or optionally substituted hydrocarbyl group;

$R^5$ represents an optionally substituted aralkyl, alicyclicalkyl, heterocyclicalkyl, aryl, alicyclic, heterocyclic, alkenyl or alkynyl group; and

$R^7$ represents a hydrogen or halogen atom, an O-, N-, P- or S-linked organic or optionally substituted hydrocarbyl group

or together with the group $R^8$ or $R^9$, $R^7$ may form an optionally substituted fused alicyclic or heterocyclic member.

5. A compound of claim 1 in which J represents an oxygen atom, -M-E-Z- together represent $-CH_2O-$, $R^2$, $R^3$, $R^4$, $R^6$, $R^7$ and X are as defined in claim 1,

$R^1$ represents a hydrogen atom, an O-, N-, P- or S-linked organic or optionally substituted hydrocarbyl group;

or together with the group X, $R^1$ may form an optionally substituted fused alicyclic or heterocyclic member; and

$R^5$ represents an optionally substituted alicyclicalkyl, heterocyclicalkyl, aryl, alicyclic, heterocyclic, alkenyl or alkynyl group.

6. A compound as claimed in claim 1 wherein
$R^1$ represents a hydrogen atom or an optionally substituted hydrocarbyl group of $C_{1-3}$ carbon atoms;
$R^2$ represents a hydrogen atom;
$R^3$ represents a hydrogen atom;
$R^4$ represents a hydrogen atom;
$R^5$ represents a benzyl, methylbenzyl, benzothienyl, 2-thiophenemethyl, 3-thiophenemethyl, indan-2 yl, tetrahydropyran-2-ylmethyl or cyclohexanemethyl group optionally substituted with up to two substituents independently chosen from chlorine, bromine or fluorine atoms, a methoxy group or a $C_{1-3}$ alkyl group optionally substituted with up to three fluorineatoms;
$R^6$ represents a hydrogen atom;
$R^7$ represents a hydrogen atom or an optionally substituted hydrocarbyl group of $C_{1-6}$ carbon atoms;
M, E and Z
independently represent an O or S atom, an amino, sulphinyl, sulphonyl or $C_{1-6}$ alkylamino group, a single bond or a $C_{1-6}$ alkylene group or the group $-CR^8R^9-$ wherein;
$R^8$ represents a hydrogen, fluorine, chlorine or bromine atom, an optionally substituted hydrocarbyl group of $C_{1-6}$ carbon atoms or an alkylthio group;
$R^9$ represents a hydrogen, fluorine, chlorine or bromine atom, an optionally substituted hydrocarbyl group of $C_{1-6}$ carbon atoms, or an alkylthio group;
X represents a $C_{1-6}$ alkoxy group, an optionally substituted hydrocarbyl group of $C_{1-6}$ carbon atoms, a fluorine, chlorine or bromine atom or an azido group;
J represents an O or S atom, an amino or a $C_{1-6}$ alkylamino group or a $C_{1-6}$ alkylene group.

7. A compound as claimed in claim 1, in which
$R^5$ is ($C_{6-8}$-cycloalkyl)-methyl, 2-alkyl-alkyl or cycloalkenemethyl and $R^1$, $R^2$, $R^3$, $R^4$, $R^6$, $R^7$, $R^8$, $R^9$, X, J, M, E and Z are as defined in claim 6.

8. A compound as claimed in claim 1, wherein
$R^1$ represents a hydrogen atom or an unbranched $C_{1-6}$ alkyl group optionally substituted in the terminal position with a chlorine or methoxy group;
$R^2$ represents a hydrogen atom;
$R^3$ represents a hydrogen atom;
$R^4$ represents a hydrogen atom;
$R^5$ represents a benzyl, methylbenzyl, benzothienyl, 2-thiophenemethyl, 3-thiophenemethyl, indan-2-yl, tetrahydropyran-2-ylmethyl, cyclohexanemethyl group optionally substituted with up to two substituents independently chosen from chlorine, bromine or fluorine atoms, a methoxy group or a $C_{1-3}$ alkyl group optionally substituted with up to three fluorine atoms;
$R^6$ represents a hydrogen atom;
$R^7$ represents a hydrogen atom or an optionally substituted hydrocarbyl group of $C_{1-6}$ carbon atoms;
M, E and Z
independently represent an O or S atom, an amino or $C_{1-6}$ alkylamino group, a single bond, a $C_{1-6}$ alkylene group or the group $-CR^8R^9$; and
$R^8$ represents a hydrogen, fluorine, chlorine or bromine atom, an optionally substituted hydrocarbyl group of $C_{1-6}$ carbon atoms or an alkylthio group;
$R^9$ represents a hydrogen, fluorine, chlorine or bromine atom, an optionally substituted hydrocarbyl group of $C_{1-6}$ carbon atoms or an alkylthio group;
X represents an ethoxy, methoxy or azido group, a fluorine, chlorine or bromine atom, or an optionally substituted hydrocarbyl group of $C_{1-6}$ carbon atoms;
J represents an O or S atom, an amino or a $C_{1-6}$ alkylamino group or a $C_{1-6}$ alkylene group.

9. A compound as claimed in claim 1 wherein
$R^1$ is a halogen atom and $R^2$, $R^3$, $R^4$, $R^5$, $R^6$, $R^7$, $R^8$, $R^9$, X, J, M, E and Z are as defined in claim 8.

10. A compound as claimed in claim 1 wherein
$R^5$ is cyclooctanemethyl, 3-cyclohexenemethyl, or 2-(G-$2^{alkyl}$)-$C_{3-6}^{alkyl}$ and $R^1$, $R^2$, $R^3$, $R^4$, $R^6$, $R^7$, $R^8$, $R^9$, X, J, M, E and Z are as defined in claim 8.

11. A compound as claimed in claim 10 wherein $R^5$ is 2-(methyl or ethyl)-(propyl, butyl or pentyl).

12. A compound as claimed in claim 2, wherein
$R^1$ represent a hydrogen atom or an optionally substituted hydrocarbyl group of $C_{1-6}$ carbon atoms;
$R^2$ represents a hydrogen atom;
$R^3$ represents a hydrogen atom;
$R^4$ represents a hydrogen atom;
$R^5$ represents a benzyl, methylbenzyl, benzothienyl, 2-thiophenemethyl, 3-thiophenemethyl, indan-2-yl,

tetrahydropyran-2-ylmethyl or cyclohexanemethyl group optionally substituted with up to two substituents independently chosen from chlorine, bromine or fluorine atoms, a methoxy group or a $C_{1-3}$ alkyl group optionally substituted with up to three fluorine atoms;

$R^6$ represents a hydrogen atom;

$R^7$ represent a hydrogen atom or an optionally substituted hydrocarbyl group of $C_{1-6}$ carbon atoms;

X represents a $C_{1-6}$ alkoxy group, an optionally substituted hydrocarbyl group of $C_{-6}$ carbon atoms, a fluorine, chlorine or bromine atom or an azido group;

M represent an O or S atom, an amino or $C_{-6}$ alkylamino group, a single bond or a $C_{1-6}$ alkylene group;

Z represents an O or S atom, an amino or $C_{1-6}$ alkylamino group, a single bond or a $C_{1-6}$ alkylene group; and

J represents an O or S atom, an amino or a $C_{-6}$ alkylamino group or a $C_{1-6}$ alkylene group.

13. A compound as claimed in claim 2, wherein

$R^1$ represents a hydrogen atom or an unbranched $C_{1-6}$ alkyl group optionally substituted in the terminal position with a chlorine or methoxy group;

$R^2$ represents a hydrogen atom;

$R^3$ represents a hydrogen atom;

$R^4$ represents a hydrogen atom;

$R^5$ represents a benzyl, methylbenzyl, benzothienyl, 2-thiophenemethyl, 3-thiophenemethyl, indan-2-yl, tetrahydropyran-2-ylmethyl, cyclohexanemethyl group optionally substituted with up to two substituents independently chosen from chlorine, bromine, or fluorine atoms, a methoxy group or a $C_{1-3}$ alkyl group optionally substituted with up to three fluorine atoms;

$R^6$ represents a hydrogen atom;

$R^7$ represents a hydrogen atom or an optionally substituted hydrocarbyl group of $C_{1-6}$ carbon atoms;

X represents an ethoxy, methoxy or azido group, or a fluorine, chlorine or bromine atom;

M represents an O or S atom, an amino or $C_{1-6}$ alkylamino group, a single bond, a $C_{1-6}$ alkylene group;

E represents an O or S atom, an amino or $C_{1-6}$ alkylamino group, a single bond or a $C_{1-6}$ alkylene group;

Z represents an O or S atom, an amino or $C_{1-6}$ alkylamino group, a single bond or a $C_{1-6}$ alkylene group; and

J represents an O or S atom, an amino or a $C_{1-6}$ alkylamino group or a $C_{1-6}$ alkylene group.

14. A compound as claimed in claim 3, wherein

$R^1$ represents a hydrogen atom or optionally substituted hydrocarbyl group of $C_{1-6}$ carbon atoms;

$R^2$ represents a hydrogen atom;

$R^3$ represents a hydrogen atom;

$R^4$ represents a hydrogen atom;

$R^5$ represents a benzyl, methylbenzyl, benzothienyl, 2-thiophenemethyl, 3-thiophenemethyl, indan-2-yl, tetrahydropyran-2-ylmethyl, cyclohexanemethyl group optionally substituted with up to two substituents independently chosen from chlorine, bromine or fluorine atoms, a methoxy group or a $C_{1-3}$ alkyl group optionally substituted with up to three fluorine atoms;

$R^6$ represents a hydrogen atom;

$R^7$ represents a hydrogen atom or an optionally substituted hydrocarbyl group of $C_{1-6}$ carbon atoms;

$R^8$ represents a hydrogen, fluorine, chlorine or bromine atom, an optionally substituted hydrocarbyl group of $C_{1-6}$ carbon atoms or an alkylthio group;

$R^9$ represents a hydrogen, fluorine, chlorine or bromine atom, an optionally substituted hydrocarbyl group of $C_{1-6}$ carbon atoms or an alkylthio group; and

X represents a $C_{1-6}$ alkoxy group, an optionally substituted hydrocarbyl group of $C_{1-6}$ carbon atoms, a fluorine, chlorine or bromine atom or an azido group.

15. A compound as claimed in claim 3, wherein

$R^1$ represents a hydrogen atom or an unbranched $_{1-6}$ alkyl group optionally substituted in the terminal position with a chlorine or methoxy group;

$R^2$ represents a hydrogen atom;

$R^3$ represents a hydrogen atom;

$R^4$ represents a hydrogen atom;

$R^5$ represents a benzyl, methylbenzyl, benzothienyl, 2-thiophenemethyl, 3-thiophenemethyl, indan-2-yl, tetrahydropyran-2-ylmethyl, cyclohexanemethyl group optionally substituted with up to two substituents independently chosen from chlorine, bromine or fluorine atoms, a methoxy group or a $C_{1-3}$ alkyl group

optionally substituted with up to three fluorine atoms;
$R^6$ represents a hydrogen atom;
$R^7$ represents a hydrogen atom or an optionally substituted hydrocarbyl group of $C_{1-6}$ carbon atoms;
$R^8$ represents a hydrogen, fluorine, chlorine or bromine atom, an optionally substituted hydrocarbyl group of $C_{1-6}$ carbon atoms or an alkylthio group;
$R^9$ represents a hydrogen, fluorine, chlorine or bromine atom, an optionally substituted hydrocarbyl group of $C_{1-6}$ carbon atoms or an alkylthio group; and
X represents an ethoxy or methoxy group, an optionally substituted hydrocarbyl group of $C_{1-6}$ carbon atoms, a fluorine, chlorine or bromine atom or an azido group.

16. A compound as claimed in claim 4, wherein
$R^1$ represents a hydrogen atom or an optionally substituted hydrocarbyl group of $C_{1-6}$ carbon atoms;
$R^2$ represents a hydrogen atom;
$R^3$ represents a hydrogen atom;
$R^4$ represents a hydrogen atom;
$R^5$ represents a benzyl, methylbenzyl, benzothienyl, 2-thiophenemethyl, 3-thiophenemethyl, indan-2-yl, tetrahydropyran-2-ylmethyl or cyclohexanemethyl group optionally substituted with up to two substituents independently chosen from chlorine, bromine or fluorine atoms, a methoxy group or a $C_{1-3}$ alkyl group optionally substituted with up to three fluorine atoms;
$R^6$ represents a hydrogen atom;
$R^7$ represents a hydrogen atom or an optionally substituted hydrocarbyl group of $C_{1-6}$ carbon atoms;
$R^8$ represents a hydrogen, fluorine, chlorine or bromine atom, an optionally substituted hydrocarbyl group of $C_{1-6}$ carbon atoms or an alkylthio group;
$R^9$ represents a hydrogen, fluorine, chlorine or bromine atom, an optionally substituted hydrocarbyl group of $C_{1-6}$ carbon atoms or an alkylthio group; and
X represents a $C_{1-6}$ alkoxy group, an optionally substituted hydrocarbyl group of $C_{1-6}$ carbon atoms, a fluorine, chlorine or bromine atom or an azido group.

17. A compound as claimed in claim 4, wherein
$R^1$ represents a hydrogen atom or an unbranched $C_{1-6}$ alkyl group optionally substituted in the terminal position with a chlorine or methoxy group;
$R^2$ represents a hydrogen atom;
$R^3$ represents a hydrogen atom;
$R^4$ represents a hydrogen atom;
$R^5$ represents a benzyl, methylbenzyl, benzothienyl, 2-thiophenemethyl, 3-thiophenemethyl, indan-2-yl, tetrahydropyran-2-ylmethyl or cyclohexanemethyl group optionally substituted with up to two substituents independently chosen from chlorine, bromine or fluorine atoms, a methoxy group or a $C_{1-3}$ alkyl group optionally substituted with up to three fluorine atoms;
$R^6$ represents a hydrogen atom;
$R^7$ represents a hydrogen atom or an optionally substituted hydrocarbyl group of $C_{1-6}$ carbon atoms;
$R^8$ represents a hydrogen, fluorine, chlorine or bromine atom, an optionally substituted hydrocarbyl group of $C_{1-6}$ carbon atoms or an alkylthio group;
$R^9$ represents a hydrogen, fluorine, chlorine or bromine atom, an optionally substituted hydrocarbyl group of $C_{1-6}$ carbon atoms or an alkylthio group; and
X represents an ethoxy, methoxy or azido group, or a fluorine, chlorine or bromine atom.

18. A compound as claimed in claim 5, wherein
$R^1$ represents a hydrogen atom or optionally substituted hydrocarbyl group of $C_{1-6}$ carbon atoms;
$R^2$ represents a hydrogen atom;
$R^3$ represents a hydrogen atom;
$R^4$ represents a hydrogen atom;
$R^5$ represents an optionally substituted alicyclicalkyl or hetereocyclicalkyl group;
$R^6$ reperesents a hydrogen atom;
X represents a $C_{1-6}$ alkoxy group, an optionally substituted hydrocarbyl group of $C_{1-6}$ carbon atoms, a fluorine, chlorine or bromine atom or an azido group.

19. A compound as claimed in claim 5 wherein
$R^1$ represent a hydrogen atom or an unbranched $C_{1-6}$ alkyl group optionally substituted in the terminal position with a chlorine or methoxy group;
$R^2$ represents a hydrogen atom;
$R^3$ represents a hydrogen atom;
$R^4$ represents a hydrogen atom;

R represents an optionally substituted cyclohexanemethyl or (tetrahydropyran-2-yl)methyl group;
$R^6$ represent a hydrogen atom;
X represents an ethoxy or methoxy group, an optionally substituted hydrocarbyl group of $C_{1-6}$ carbon atoms, a fluorine, chlorine or bromine atom or an azido group.

20. A compound selected from (1R,2S,4R)-2-cyclopentanemethyloxy-4-methoxy-6,8-dioxabicyclo[3.2.1]-octane;
(1R,2S,4R)-2-cycloheptanemethyloxy-4-methoxy-6,8-dioxabicyclo[3.2.1]octane;
(1R,2S,4R)-4-methoxy-2-[(tetrahydropyran-2-yl)methyloxy]-6,8-dioxabicyclo[3.2.1]octane;
(1R,2S,4R)-4-methoxy-2-(2-methylpentyloxy)-6,8-dioxabicyclo[3.2.1]octane;
(1R,2S,4R)-4-methoxy-2-(2-methylbutyloxy)-6,8-dioxabicyclo[3.2.1]octane;
(1R,2S,4R)-2-(3-cyclohexene-1-methyloxy)-4-methoxy-6,8-dioxabicyclo[3.2.1]octane;
(1R,2S,4R)-4-methoxy-2-[(1S,2S,5S)-myrtanyloxy]-6,8-dioxabicyclo[3.2.1]octane;
(1R,2S,4R)-2-cyclohexanemethyloxy-4-ethoxy-6,8-dioxabicyclo[3.2.1]octane;
(1R,2S,4R)-2-cyclooctanemethyoxy-4-methoxy-6,8-dioxabicyclo[3.2.l]octane;
(1R,2S,4R)-4-methoxy-2-(1-methylcyclohexane-1-methyloxy)-6,8-dioxabicyclo[3.2.1]octane;
(1R,2S,4R)-2-(2-ethylbutyloxy)-4-methoxy-6,8-dioxabicyclo[3.2.1]octane;
(1R,2S,4R)-2-(-2-cyclohexylethyloxy)-4-methoxy-6,8-dioxabicyclo[3.2.1]octane;
(1R,2S,4R)-2-cyclohexyloxy-4-methoxy-6,8-dioxabicyclo[3.2.1]octane;
(1R,2S,4R)-2-cyclohexanemethyloxy-4-methoxy-6,8-dioxabicyclo[3.2.1]octane;
(1R,2S,4R)-5-butyl-2-cyclohexanemethyloxy-4-methoxy-6,8-dioxabicyclo[3.2.1]octane;
(1R,2S,4R,1′RS)-2-(1y-cyclohexyl-ethyloxy)-4-methoxy6,8-dioxabicyclo[3.2.1]octane;
(1R,2S,4R,4′RS)-4-methoxy-2-(4′-methylcyclohexane)methyloxy-6,8-dioxabicyclo[3.2.1]octane;
(1R,2S,4R,2′RS)-4-methoxy-2-(2′-methylcyclohexane)methyloxy-6,8-dioxabicyclo[3.2.1]octane;
(1R,2S,4R)-2-cyclohexanemethyloxy-4-methoxy-5-propyl-6,8-dioxabicyclo[3.2.1]octane;
(1R,2S,4R)-2-cyclohexanemethyloxy-5-ethyl-4-methoxy-6,8-dioxabicyclo[3.2.1]octane;
(1Rs,2SR,4RS)-4-cyclohexanemethyloxy-2-methoxy-6,8-dioxabicyclo[3.2.1]octane;
(1R,2S,4R)-2-cyclohexanemethyloxy-4-ethoxy-5-ethyl-6,8-dioxabicyclo[3.2.1]octane;
(1R,2S,4R)-2-cyclohexanemethyloxy-4-ethoxy-5-methyl-6,8-dioxabicyclo[3.2.1]octane;
(1R,2S,4R)-2-(3-cyclohexene-1-methyloxy)-4-methoxy-5-methyl-6,8-dioxabicyclo[3.2.1]octane;
(1R,2S,4R)-2-(3-cyclohexene-1-methyloxy)-4-methoxy-5-ethyl-6,8-dioxabicyclo[3.2.1]octane;
(1R,2S,4R)-2-(3-cyclohexene-1-methyloxy)-4-ethoxy-5-ethyl-6,8-dioxabicyclo[3.2.1]octane;
(1R,2S,4R)-2-(3-cyclohexene-1-methyloxy)-4-ethoxy-5-methyl-6,8-dioxabicyclo[3.2.1]octane;
(1R,2S,4R)-2-(3-cyclohexene-l-methyloxy)-4-ethoxy-5propyl-6,8-dioxabicyclo[3.2.1]octane;
(1R,2S,4R)-2-cyclohexanemethyloxy-4-methoxy-5-(2-propen-1-yl)-6,8-dioxabicyclo[3.2.1]octane;
(1R,2S,4R)-2-cyclohexanemethyloxy-4-ethoxy-5-(2-propen-1-yl)-6,8-dioxabicyclo[3.2.1]octane;
(1R,2S,4R)-2-Benzyloxy-4-methoxy-1-methyl-6,8-dioxabicyclo[3.2.1]octane;
(1Rs,2S,4R,7S)-2-Benzyloxy-4-methoxy-7-methyl-6,8-dioxabicyclo[3.2.1]octane;
(1RS,2S,4R,7S)-2-Benzyloxy-7-ethyl-4-methoxy-6,8-dioxabicyclo[3.2.1]octane;
(1S,2S,4R,7R)-2-Benzyloxy-4-methoxy-7-methyl-6,8 dioxabicyclo[3.2.1]octane and its (7S)-isomer;
(1S,2S,4R)-2-Benzyloxy-4-methoxy-7,7-dimethyl-6,8-dioxabicyclo[3.2.1]octane;
(1S,2S,4R,7R)-2-Benzyloxy-7-ethyl-4-methoxy-6,8-dioxabicyclo[3.2.1]octane;
(1S,2S,4R,7S)-2-Benzyloxy-4-methoxy-7-butyl-6,8-dioxabicyclo[3.2.1]octane;
(1RS,2SR,4RS,7RS)-2-Benzyloxy-4-methoxy-5,7-dimethyl-6,8-dioxabicyclc[3.2.1]octane;
(1RS,2SR,4RS)-2-Benzyloxy-4-methoxy-1,5-dimethyl-6,8-dioxabicyclo[3.2.1]octane
(1R,2S,4R)-2-Benzyloxy-1-ethyl-4-methoxy-6,8-dioxabicyclo[3.2.1]octane;
(1S,2R,4S)-4-Benzyloxy-2-methoxy-6,8-dioxabicyclo[3.2.1]octane;
(1S,2R,4S)-4-Benzyloxy-5-chloromethyl-2-methoxy-6,8-dioxabicyclo[3.2.1]octane;
(1S,2R,4S)-4-Benzyloxy-2-methoxy-5-methyl-6,8-dioxabicyclo[3.2.1]octane;
(1S,2R,4S)-4-Benzyloxy-2-methoxy-1-propyl-6,8-dioxabicyclo[3.2.1]octane;
(1Rs,2SR,4RS,7RS)-4-Benzyloxy-2-methoxy-5,7-dimethyl-6,8-dioxabicyclo[3.2.1]octane;
(1Rs,2SR,4RS)-4-Benzyloxy-2-methoxy-1,5-dimethyl-6,8-dioxabicyclo[3.2.1]octane;
(1RS,2RS,4SR,7RS)-4-Benzyloxy-7-ethyl-2-methoxy-6,8-dioxabicyclo[3.2.1]octane;
(1RS,2SR,4RS)-4-Benzyloxy-2-methoxy-6,8-dioxabicyclo[3.2.1]octane;
(1RS,2SR,4RS)-2-Methoxy-4-(2-methylbenzyloxy)-6,8-dioxabicyclo[3.2.1]octane
(1RS,2SR,4RS,7SR)-4-Benzyloxy-2-methoxy-7-methyl-6,8-dioxabicyclo[3.2.1]octane;
(1RS,2SR,4RS,7SR)-2-Methoxy-7-methyl-4-(2-methylbenzyloxy)6,8-dioxabicyclo[3.2.1]octane and
(1RS,2SR,4RS,7SR)-4-Hydroxy-2-methoxy-7-methyl-6,8-dioxabicyclo[3.2.1]octane;
(1RS,2SR,4RS,7RS)-4-Benzyloxy-2-methoxy-7-methyl-6,8-dioxabicoyclo[3.2.1]octane and its

(1RS,2SR,4RS,7SR)-stereoisomer;
(1RS,2RS,4SR)-4-Benzyloxy-2-methoxy-7,7-dimethyl-6,8-dioxabicyclo[3.2.1]octane;
(1RS,2SR,4RS}-4-(2-fluorobenzyloxy)-2-methoxy-6,8-dioxabicyclo[3.2.1]octane;
(1RS,2SR,4RS)-4-(2-chlorobenzyloxy)-2-methoxy-6,8-dioxabicyclo[3.2.1]octane;
(1RS,2SR,4RS)-4-(2,4-difluorobenzyloxy)-2-methoxy-6,8-dioxabicyclo[3.2.1]octane;
(1RS,2SR,4RS)-4-(2,4-dichlorobenzyloxy)-2-methoxy-6,8-dioxabicyclo[3.2.1]octane;
(1RS,2SR,4RS)-4-(2,6-difluorobenzyloxy)-2-methoxy-6,8-dioxabicyclo[3.2.1]octane;
(1RS,2SR,4RS)-2-methoxy-4-[2-(trifluoromethyl)benzyloxy]-6,8-dioxabicyclo[3.2.1]octane;
(1RS,2SR,4RS)-2-methoxy-4-(3-methylbenzyloxy)-6,8-dioxabicyclo[3.2.1]octane;
(1RS,2SR,4RS)-4-(2-cyanobenzyloxy)-4-methoxy-6,8-dioxabicyclo[3.2.1]octane;
(1RS,2SR,4RS)-2-methoxy-4-(4-methylbenzyloxy)-6,8-dioxabicyclo[3.2.1]octane
(1RS,2SR,4RS)-4-(2-chloro-3-thienyl)methoxy-2-methoxy-6,8-dioxabicyclo[3.2.1]octane;
(1RS,2SR,4RS,7SR)-4-(2-Fluorobenzyloxy)-2-methoxy-7-methyl-6,8-dioxabicyclo[3.2.1]octane;
(1S,6S,8R)-6-Benzyloxy-8-methoxy-3,g-dioxabicyclo[3.3.1]nonane;
(1S,2S,4R)-2-Benzyloxy-4-methoxy-8-oxa-6-thiabicyclo[3.2.1]octane;
(1S,2S,4R)-2-Benzyloxy-4-methoxy-8-oxa-6-thiabicyclo[3.2.1]octane,6-oxide;
(1S,2S,4R)-2-Benzyloxy-4-methoxy-8-oxa-6-thiabicyclo[3.2.l]octane,6,6′-dioxide;
(1RS,2RS,3RS,4RS)-2,3-Epoxy-4-(2-methylbenzyloxy)-6,8-dioxabicyclo[3.2.1l]octane.

21. A herbicidal composition comprising, together with a carrier and/or surface-active agent, an effective amount of at least one herbicidal agent selected from compounds of formula IA

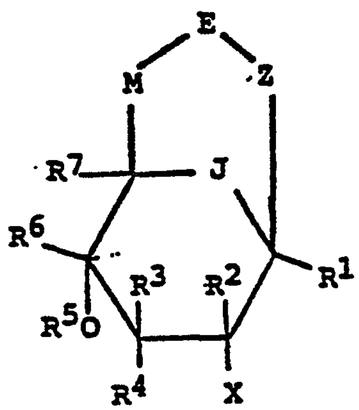

IA

in which

$R^1$ represents a hydrogen or halogen atom, an O-, N-, P- or S-linked organic or optionally substituted hydrocarbyl group
or $R^1$, together with X, E or Z, may form an optionally substituted fused alicyclic or heterocyclic member;
$R^2$ represents a hydrogen or fluorine atom, a hydroxy group or an optionally substituted hydrocarbyl or O-linked hydrocarbyl group
or $R^2$, together with X, may form a ketonic oxygen atom -O- or a group of the formula $-CH_2O-$;
$R^3$ represents a hydrogen or halogen atom, a hydroxy group or an optionally substituted hydrocarbyl or O-linked hydrocarbyl group
or $R^3$, together with $R^4$, may form an optionally substituted hydrocarbyl group;
$R^4$ represents a hydrogen or halogen atom, a hydroxy group or an optionally substituted hydrocarbyl or O-linked hydrocarbyl group
or $R^4$, together with X, may form an oxygen atom;
$R^5$ represents an optionally substituted aralkyl, alicyclicalkyl, heterocyclicalkyl, aryl, alicyclic, heterocyclic, alkyl, alkenyl or alkynyl group
$R^6$ represents a hydrogen atom or an optionally substituted hydrocarbyl group;
$R^7$ represents a hydrogen or halogen atom, an O-, N-, P- or S-linked organic or optionally substituted hydrocarbyl group
or together with M or E, $R^7$ may form an optionally substituted fused alicyclic or heterocyclic member;
X represents an O-, N- or S-linked organic group, an optionally substituted hydrocarbyl group, a halogen atom, a hydroxy, amino, alkoxyamino, nitro, cyano, azido, sulpho or phospho group,
M, E and Z
independently of each other represent an O or S atom, a sulphinyl, sulphonyl, amino, phosphinico or N-linked organic group, a single bond, an optionally substituted hydrocarbyl group or the group $CR^8R^9$, wherein $R^8$ represents a hydrogen or halogen atom, an O-, N-, P- or S-linked organic or optionally substituted hydrocarbyl group, a hydroxy, amino, alkoxyamino, nitro, cyano, azido, sulpho or phospho group or $R^8$, together with $R^9$, may form an optionally substituted spiroalicyclic or spiroheterocyclic member, an optionally substituted hydrocarbyl group or a double bonded oxygen atom;
and wherein $R^9$ represents a hydrogen or halogen atom, an O-, N-, P- or S-linked organic or optionally substituted hydrocarbyl group, a hydroxy, amino, alkoxyamino, nitro, cyano, azido, sulpho or phospho group or; and
wherein, adjacent M, E or Z groups may be joined by a double bond; and
where n, two or more of M, E and Z may form an optionally substituted fused alicyclic or heterocyclic ring; with the proviso that only one of the group M, E or Z may represent a single bond;
J represents an O or S atom, a sulphinyl, sulphonyl, amino, phosphinico or N-linked organic group or an optionally substituted hydrocarbyl group;
and the enantiomers, salts and mixtures thereof;
with the following provisos:
(i) when one of M, E and Z is a single bond and J is an oxygen atom then at least one of the remainder of M, E and Z represents an O or S atom, a sulphinyl, sulphonyl, amino, phosphinico or N-linked organic group or the group $CR^8R^9$, in which at least one of $R^8$ or $R^9$ is not a hydrogen atom or O-linked organic or optionally substituted hydrocarbyl group;
(ii) when -M-E-Z- is $-OCH_2-$, J is an O atom, X and $R^5O$ are the same aralkoxy group, and $R^1$ is hydrogen, then the enantiomer thereof is not included;
(iii) that when -M-E-Z- together form the group $-CH_2O-$, J is an oxygen atom, $R^7$ is a hydrogen atom, and $R^5$ is an optionally substituted aralkyl group, then
(a) $R^1$ represents an O-, N-, P- or S-linked organic group; or
(b) $R^1$, together with X, forms an optionally substituted fused alicyclic or heterocyclic member; or
(c) $R^2$ represents a fluorine atom; or
(d) $R^3$ or $R^4$ represents a halogen atom; or
(e) $R^3$, together with $R^4$, forms an optionally substituted hydrocarbyl group; or
(f) $R^4$, together with X, forms an oxygen atom; and
(iv) -M-E-Z- is $-CH_2O-$ and $R^1$ is a hydrogen then X is not an optionally substituted aralkoxy group.

22. A herbicidal composition as claimed in claim 21 wherein the herbicidal agent is selected from compounds as claimed in any of claims 2 to 20.

23. Plant growth regulatory compositions comprising, together with a carrier and/or surface active agent, an effective amount of at least one plant grown regulatory active agent selected from compounds of formula IA

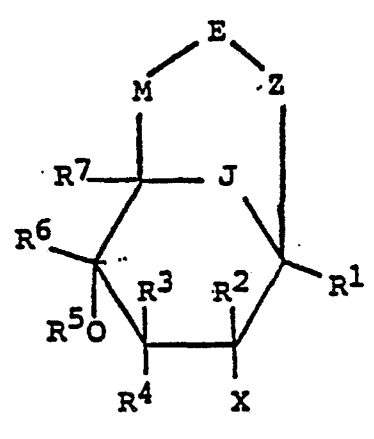

(IA)

in which
$R^1$ represents a hydrogen or halogen atom, an O-, N-, P- or S-linked organic or optionally substituted hydrocarbyl group
or $R^1$, together with X, E or Z, may form an optionally substituted fused alicyclic or heterocyclic member;
$R^2$ represents a hydrogen or fluorine atom, a hydroxy group or an optionally substituted hydrocarbyl or O-linked hydrocarbyl group
or $R^2$, together with X, may form a ketonic oxygen atom -O- or a group of the formula $-CH_2O-$;
$R^3$ represents a hydrogen or halogen atom, a hydroxy group or an optionally substituted hydrocarbyl or

O-linked hydrocarbyl group
or $R^3$, together with $R^4$, may form an optionally substituted hydrocarbyl group;
$R^4$ represents a hydrogen or halogen atom, a hydroxy group or an optionally substituted hydrocarbyl or O-linked hydrocarbyl group
or $R^4$, together with X, may form an oxygen atom;
$R^5$ represents an optionally substituted aralkyl, alicyclicalkyl, heterocyclicalkyl, aryl, alicyclic, heterocyclic, alkyl, alkenyl or alkynyl group
$R^6$ represents a hydrogen atom or an optionally substituted hydrocarbyl group;
$R^7$ represents a hydrogen or halogen atom, an O-, N-, P- or S-linked organic or optionally substituted hydrocarbyl group
or together with M or E, $R^7$ may form an optionally substituted fused alicyclic or heterocyclic member;
X represents an O-, N- or S-linked organic group, an optionally substituted hydrocarbyl group, a halogen atom, a hydroxy, amino, alkoxyamino, nitro, cyano, azido, sulpho or phospho group,
M, E and Z
independently of each other represent an O or S atom, a sulphinyl, sulphonyl, amino, phosphinico or N-linked organic group, a single bond, an optionally substituted hydroca byl group or the group $CR^8R^9$, wherein $R^8$ represents a hydrogen or halogen atom, an O-, N-, P- or S-linked organic or optionally substituted hydrocarbyl group, a hydroxy, amino, alkoxyamino, nitro, cyano, azido, sulpho or phospho group or $R^8$, together with $R^9$, may form an optionally substituted spiroalicyclic or spiroheterocyclic member, an optionally substituted hydrocarbyl group or a double bonded oxygen atom;
and wherein $R^9$ represents a hydrogen or halogen atom, an O-, N-, P- or S-linked organic or optionally substituted hydrocarbyl group, a hydroxy, amino, alkoxyamino, nitro, cyano, azido, sulpho or phospho group or; and
wherein, adjacent M, E or Z groups may be joined by a double bond; and
where n, two or more of M, E and Z may form an optionally substituted fused alicyclic or heterocyclic ring;
with the proviso that only one of the group M, E or Z may represent a single bond;
J represents an O or S atom, a sulphinyl, sulphonyl, amino, phosphinico or N-linked organic group or an optionally substituted hydrocarbyl group;
and enantiomers, salts and mixtures thereof;
with the following provisos:

(i) when one of M, E and Z is a single bond and J is an oxygen atom then at least one of the remainder of M, E and Z represents an O or S atom, a sulphinyl, sulphonyl, amino, phosphinico or N-linked organic group or the group $CR^8R^9$, in which at least one of $R^8$ or $R^9$ is not a hydrogen atom or O-linked organic or optionally substituted hydrocarbyl group;

(ii) when -M-E-Z- is $-OCH_2-$, J is an O atom, X and $R^5O$ are the same aralkoxy group, and $R^1$ is hydrogen, then the enantiomer thereof is not included;

(iii) that when -M-E-Z- together form the group $-CH_2O-$, J is an oxygen atom, $R^7$ is a hydrogen atom, and $R^5$ is an optionally substituted aralkyl group, then
(a) $R^1$ represents an O-, N-, P- or S-linked organic group; or
(b) $R^1$, together with X, forms an optionally substituted fused alicyclic or heterocyclic member; or
(c) $R^2$ represents a fluorine atom; or
(d) $R^3$ or $R^4$ represents a halogen atom; or
(e) $R^3$, together with $R^4$, forms an optionally substituted hydrocarbyl group; or
(f) $R^4$, together with X, forms an oxygen atom; and

(iv) -M-E-Z- is $-CH_2O-$ and $R^1$ is hydrogen then X is not an optionally substituted aralkoxy group.

24. A plant growth regulatory composition as claimed in claim 23 wherein the plant growth regulatory active agent is selected from compounds as claimed in any of claims 2 to 20.

25. A method of controlling unwanted plants comprising applying to the plants a herbicidally effective amount of a compound of formula IA as defined in claim 21.

26. A method of regulating plant growth comprising applying a plant growth regulating amount of a compound of formula IA as defined in claim 23.

27. A process for producing a compound of the formula (I)

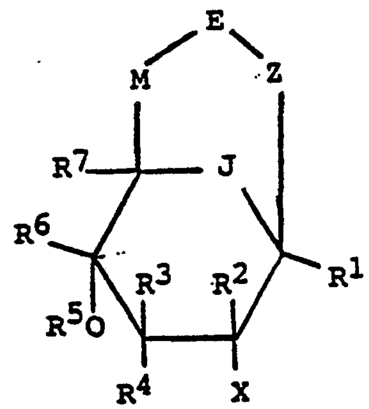

(I)

in which

$R^1$ represents a hydrogen or halogen atom, an O-, N-, P- or S-linked organic or optionally substituted hydrocarbyl group
or $R^1$, together with X, E or Z, may form an optionally substituted fused alicyclic or heterocyclic member;
$R^2$ represents a hydrogen or fluorine atom, a hydroxy group or an optionally substituted hydrocarbyl or O-linked hydrocarbyl group
or $R^2$, together with X, may form a ketonic oxygen atom -O- or a group of the formula $-CH_2O-$;
$R^3$ represents a hydrogen or halogen atom, a hydroxy group or an optionally substituted hydrocarbyl or O-linked hydrocarbyl group or $R^3$, together with $R^4$, may form an optionally substituted hydrocarbyl group;
$R^4$ represents a hydrogen or halogen atom, a hydroxy group or an optionally substituted hydrocarbyl or O-linked hydrocarbyl group
or $R^4$, together with X, may form an oxygen atom;
$R^5$ represents an optionally substituted aralkyl, alicyclicalkyl, heterocyclicalkyl, aryl, alicyclic, heterocyclic, alkyl, alkenyl or alkynyl group
$R^6$ represents a hydrogen atom or an optionally substituted hydrocarbyl group;
$R^7$ represents a hydrogen or halogen atom, an O-, N-, P- or S-linked organic or optionally substituted hydrocarbyl group or together with M or E, $R^7$ may form an optionally substituted fused alicyclic or heterocyclic member;
X represents an O-, N- or S-linked organic group, an optionally substituted hydrocarbyl group, a halogen atom, a hydroxy, amino, alkoxyamino, nitro, cyano, azido, sulpho or phospho group,
M, E and Z
independently of each other represent an O or S atom, a sulphinyl, sulphonyl, amino, phosphinico or N-linked organic group, a single bond, or the group $CR^8R^9$,
wherein $R^8$ represents a hydrogen or halogen atom, an O-, N-, P- or S-linked organic or optionally substituted hydrocarbyl group, a hydroxy, amino, alkoxyamino, nitro, cyano, azido, sulpho or phospho group or $R^8$, together with $R^9$, may form an optionally substituted spiroalicyclic or spiroheterocyclic member, an optionally substituted hydrocarbyl group or a double bonded oxygen atom;
and wherein $R^9$ represents a hydrogen or halogen atom, an O-, N-, P- or S-linked organic or optionally substituted hydrocarbyl group, a hydroxy, amino, alkoxyamino, nitro, cyano, azido, sulpho or phospho group or; and
wherein, adjacent M, E or Z groups may be joined by a double bond; and
wherein, two or more of M, E and Z may form an optionally substituted fused alicyclic or heterocyclic ring;
with the proviso that only one of the group M, E or Z may represent a single bond;
J represents an O or S atom, a sulphinyl, sulphonyl, amino, phosphinico or N-linked organic group or an optionally substituted hydrocarbyl group;
and the enantiomers, salts and mixtures thereof;
with the following provisos:
(i) when one of M, E and Z is a single bond and J is an oxygen atom then at least one of the remainder of M, E and Z represents an O or S atom, a sulphinyl, sulphonyl, amino, phosphinico or N-linked organic group or the group $CR^8R^9$, in which at least one of $R^8$ or $R^9$ is not a hydrogen atom or O-linked organic or optionally substituted hydrocarbyl group;
(ii) when -M-E-Z- is $-OCH_2-$, J is an O atom, X and $R^5O$ are the same aralkoxy group, and $R^1$ is hydrogen, then the enantiomer thereof is not included;
(iii) when -M-E-Z- together form the group $-CH_2O-$, J is an oxygen atom, $R^7$ is a hydrogen atom, and $R^5$

is an optionally substituted aralkyl group, then
(a) $R^1$ represents an O-, N-, P- or S-linked organic group; or
(b) $R^1$, together with X, forms an optionally substituted fused alicyclic or heterocyclic member; or
(c) $R^2$ represents a fluorine atom; or
(d) $R^3$ or $R^4$ represents a halogen atom; or
(e) $R^3$, together with $R^4$, forms an optionally substituted hydrocarbyl group; or
(f) $R^4$, together with X, forms an oxygen atom;
(iv) when -M-E-Z- is $-OCH_2-$, J is an oxygen atom, $R^1$ is a hydrogen atom and X is an optionally substituted aralkoxy group, then
(a) $R^2$ represents a fluorine atom; or
(b) $R^3$ or $R^4$ represents a halogen atom; or
(c) $R^3$, together with $R^4$, forms an optionally substituted hydrocarbyl group; or
(d) $R^4$, together with X, forms an oxygen atom;
(v) when J is O, -M-E-Z- is $-CH_2O-$ and $R^1$ and $R^7$ both represent a hydrogen atom then:
(a) $OR^5$, X and $R^3$ are not the same substituted alkyloxy group;
(b) when $R^3$ is a hydroxy or O-linked organic group, X is a fluorine atom, or a hydroxy, O-linked organic, amino, azido, methyl, acetamido or benzylamino group, $R^5$ is not an unsubstituted $C_1$-$C_4$ alkyl or $C_2$-$C_4$ alkenyl or a tetrahydrofuran-1-methyl group in which each of the 2, 3 and 4 positions is substituted with an O-linked organic or hydroxy group; or a substituted alkyl group where the substituent is 0-linked or contains oxygen as the only heteroatom; and
(vi) when -M-E-Z- is $-CR^8R^9O-$, $R^8$ is an exo-ethyl or exo-fluoro group and $R^1$, $R^7$ and $R^9$ are hydrogen, then $OR^5$, X and $R^3$ are not all benzyloxy;
(vii) when -M-E-Z- is $-CH_2O-$, J is an oxygen atom, and $R^1$, $R^2$, $R^3$, $R^6$ and $R^7$ are hydrogen atoms, then $R^5O$, $R^4$-and X are not all methoxy groups;
(viii) when -M-E-Z- is $-C(phenyl)_2O-$, J is an oxygen atom, and $R^1$, $R^2$, $R^4$, $R^6$ and $R^7$ represent hydrogen atoms then $R^5O$, $R^3$ and X are not all methoxy;
(ix) when -M-E-Z- is $-CH_2O-$, $R^5$ is methyl, J is an oxygen atom, $R^1$, $R^3$, $R^4$, $R^6$ and $R^7$ are hydrogen atoms then X and $R^2$ are not jointly an oxygen atom, or not both hydroxy or methoxy groups or X is not hydroxy when $R^2$ is a hydrogen atom;
comprising one or more of the following reactions:
(a) cyclising a compound of formula (II), (IIb), (IIc), (IId), (IIIa), (IIIb), (IIIc) and (IIID)

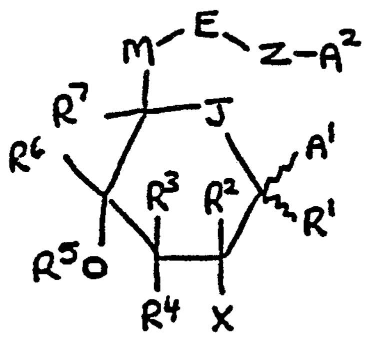

(IIa)

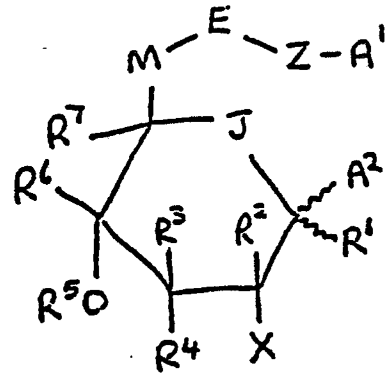

(IIb)

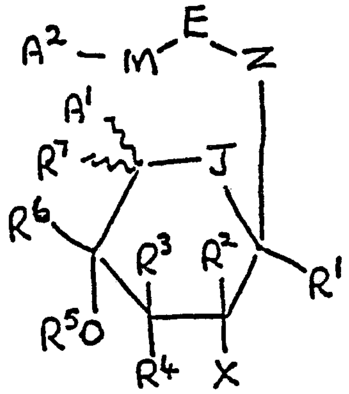

(IIc)

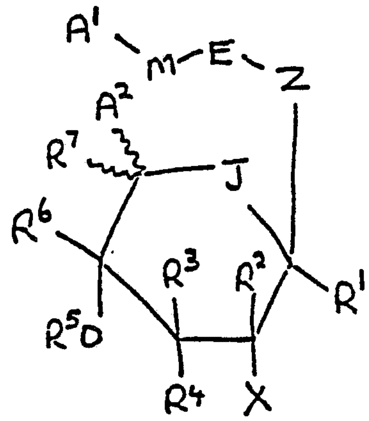

(IId)

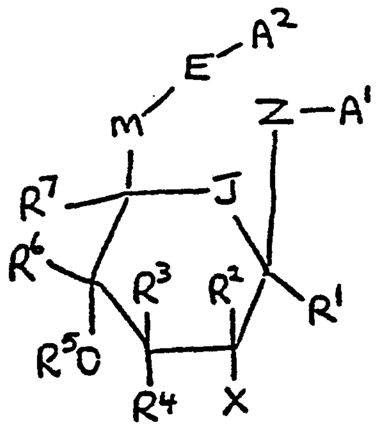

(IIIa)

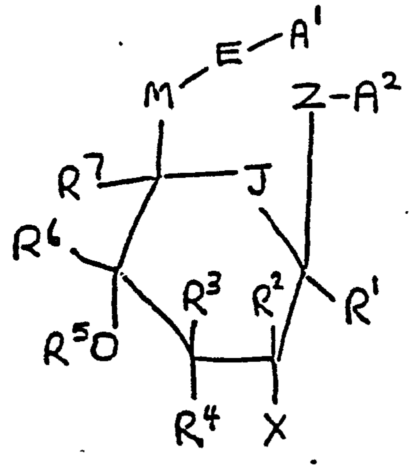

(IIIb)

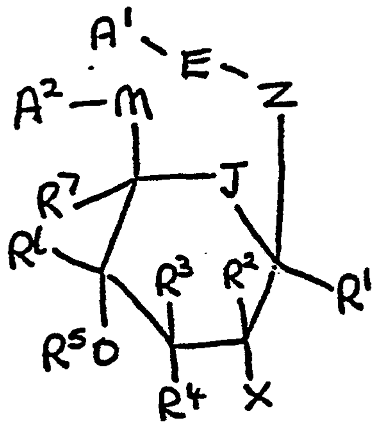

(IIIc)

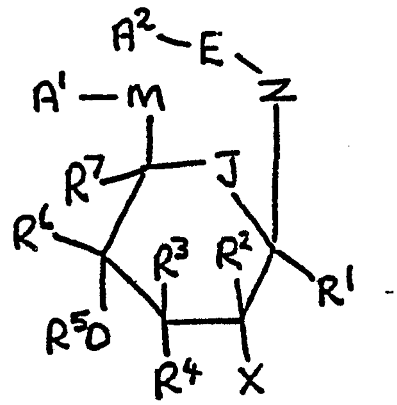

(IIId)

(in which X, $R^1$, $R^2$, $R^3$, $R^4$, $R^5$, $R^6$, $R^7$, M, J, E and Z are as defined above, or represent protected forms thereof,

$A^2$ is a group or atom capable of being split off to leave the atom or group to which it was joined with a nefative charge,

and $A^1$ is a group or atom capable of being split off to leave the group or atom to which it was joined with a positive charge)

or an enantiomer thereof,

and optionally subsequently splitting off or converting any protective groups present to produce the desired compounds;

(b) reacting a compound of formula (VII)

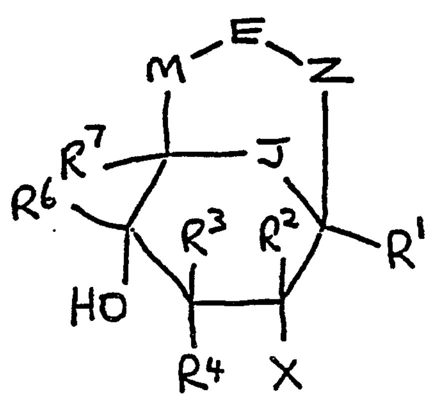

(VII)

or a corresponding alkali metal alkoxide

(wherein X, $R^1$, $R^2$, $R^3$, $R^4$, $R^6$, $R^7$, J, M, E and Z are as defined above or represent protected forms thereof)

or an enantiomer thereof,

with a compound serving to introduce the group $R^5$ in place of the hydroxy hydrogen atom

and optionally subsequently splitting off or converting any protective groups present to produce the desired compound;

(c) for the preparation of compounds wherein X represents an O-linked organic group and/or wherein $R^3$ and/or $R^4$ represent an optionally substituted O-linked hydrocarbyl group and $R^1$, $R^2$, $R^5$, $R^6$, $R^7$, J, M, E and Z and the remainder f X, $R^3$ and $R^4$ are as defined above:
reacting a corresponding compound of formula (I) wherein X and/or $R^3$ and/or $R^4$ represent a hydroxy group, and the remainder of X, $R^1$, $R^2$, $R^3$, $R^4$, $R^5$, $R^6$, $R^7$, J, M, E and Z are as defined above or represent protected forms thereof,
or an enantiomer thereof,
or a corresponding alkali metal alkoxide thereof,
with a compound serving to introduce respectively an O-linked organic group at X and/or an optionally substituted O-linked hydrocarbyl group at $R^3$ and/or $R^4$
and optionally subsequently splitting off or converting any protective groups present, to produce the desired compound;

(d) for the preparation of compounds wherein X represents a hydroxy group and $R^2$ represents a hydrogen atom:
reducing a compound of formula (I) wherein X together with the group $R^2$ represents a ketonic oxygen atom, and $R^1$, $R^3$, $R^4$, $R^5$, $R^6$, $R^7$, J, M, E and Z are as defined above or represent protected forms thereof,
or an enantiomer thereof,
and optionally subsequently splitting off or converting any protective groups present, to produce the desired compound;

(e) for the preparation of compounds wherein X together with the group $R^2$ represents a ketonic oxygen atom and the groups $R^3$ and $R^4$ represent hydrogen atoms and $R^5$ is defined as above:
reaction of an enone of formula (IX)

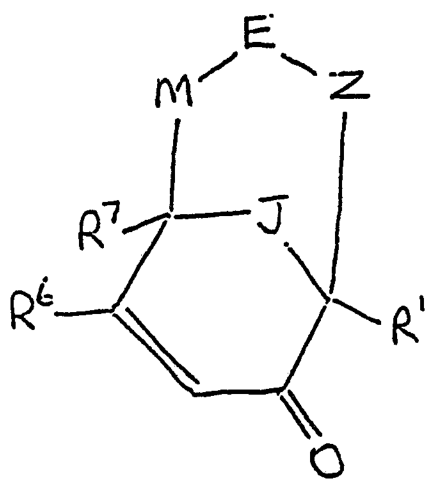

(IX)

with an alcohol of formula (X)

$R^5OH$ (X)

(wherein $R^5$ is defined as above)

f) for the preparation of compounds wherein X, $R^1$, $R^2$, $R^5$, $R^6$, $R^7$, J, M, E and Z are as defined above and $R^3$ and $R^4$ represent hydrogen atoms:
reacting a compound of formula (XII)

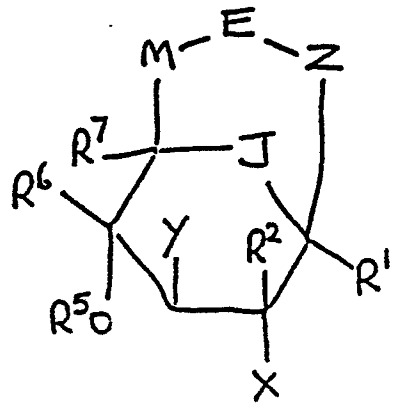

(XII)

(in which X, $R^1$, $R^2$, $R^5$, $R^6$, $R^7$, J, M, E and Z are defined as above, or represent protected forms thereof, and Y represents the group OC(S)Q; wherein Q may be, for example, thiomethyl, phenoxy, imidazol-1-yl or other suitable moiety or represents a protected form thereof),
or an enantiomer thereof,
with trialkyltin hydride, (eg, tributyltin hydride)
and optionally subsequently splitting off or converting any protective groups to produce the desired compounds;

(g) for the preparation of compounds wherein $R^1$, $R^5$, $R^7$, J, M, E and Z are as defined above, $R^2$, $R^4$ and $R^6$ represent hydrogen atoms, $R^3$ represents a hydroxy group and X is the same as the group $OR^5$: diether formation involving the 1- and 3-oxygen atoms of the 1,2,3-triol (XIII)

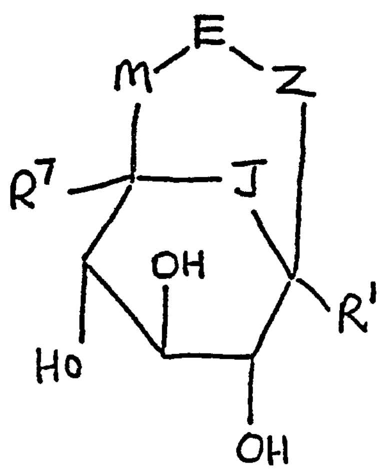

(XIII)

;

(h) for the preparation of compounds wherein $R^3$ represents a hydroxy group, X represents an O-linked organic group, an optionally substituted hydrocarbyl group, a halogen atom, or a hydroxy, amino, cyano or azido group, $R^2$ and $R^4$ represent hydrogen atoms, and $R^1$, $R^5$, $R^6$, $R^7$, M, E, Z and J are as defined above:
reacting an epoxide of formula (XIV)

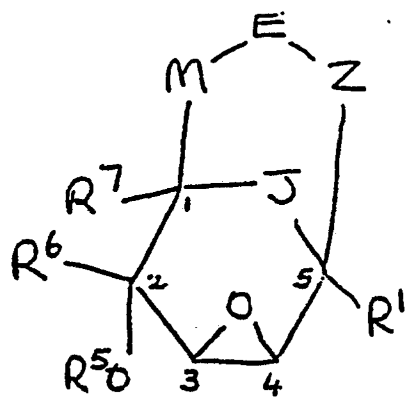

(XIV)

(wherein $R^1$, $R^5$, $R^6$, $R^7$, J, M, E and Z are as defined for formula (I) or represent protected forms thereof) or an enantiomer thereof,
with a compound serving to selectively introduce the group X as defined above at the 4-position,
and optionally subsequently splitting off or converting any protective groups present to produce the desired compound;

(i) for the preparation of compounds wherein $R^2$ represents a hydrogen atom and X represents an S-linked organic group or an azido group and $R^1$, $R^3$, $R^4$, $R^5$, $R^6$, $R^7$, M, E, Z and J are as defined above:
reacting a compound of formula (XVI)

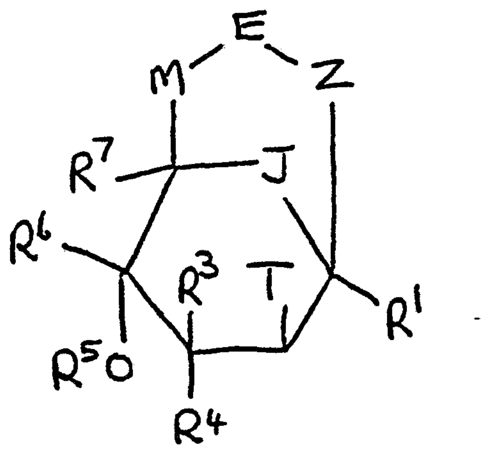

(XVI)

(wherein $R^1$, $R^3$, $R^4$, $R^5$, $R^6$, $R^7$, J, M, E and Z are as defined above or represent protected forms thereof,
and T represents a nucleophilically exchangeable group,
with an organic thiol of formula (XVIII)

$R^1SH$ (XVII)

(wherein $R^1$ represents an organic group)
or a reactive derivative thereof,
or with sodium azide,
and optionally subsequently splitting off or converting protective groups to produce the desired compound;

(j) for the preparation of compounds wherein X represents a mono- or di-alkylamino, mono- or di-aralkylamino, trialkylammonium, carboxamido or dicarboximido group and $R^1$, $R^2$, $R^3$, $R^4$, $R^5$, $R^6$, $R^7$, J, M, E and Z are defined as above:
reacting a compound of formula (I), wherein X represents an amino group and $R^1$, $R^2$, $R^3$, $R^4$, $R^5$, $R^6$, $R^7$, J, M, E and Z are defined as above or represent protected forms thereof, with a compound capable of converting said amino group to a mono- or dialkylamino, mono- or di-aralkylamino, trialkylammonium, carboxamido or dicarboximido group,
and optionally subsequently splitting off or converting any protective groups present to produce the desired compound;

(k) for the preparation of compounds wherein X represents a carboxamido or dicarboxamido group and $R^1$, $R^2$, $R^3$, $R^4$, $R^5$, $R^6$, $R^7$, J, M, E and Z are defined as above:
reacting a compound of formula (I) wherein X represents an azido group and $R^1$, $R^2$, $R^3$, $R^4$, $R^5$, $R^6$, $R^7$, J, M, E and Z are defined as above or represent protected forms thereof,
or an activated derivative thereof,
with an anhydride reagent corresponding to the desired carboxamido or dicarboximido group,
and optionally subsequently splitting off or converting any protective groups to produce the desired compound;

(l) for the preparation of compounds wherein X represents an amino group and $R^1$, $R^2$, $R^3$, $R^4$, $R^5$, $R^6$, $R^7$, J, M, E and Z are defined as above:
reducing a corresponding azido compound of formula (I) wherein X represents an azido group, and $R^1$, $R^2$, $R^3$, $R^4$, $R^5$, $R^6$, $R^7$, J, M, E and Z are as defined above
or represent protected forms thereof,
and optionally subsequently splitting off or converting any protective groups to produce the desired compound;

(m) for the preparation of compounds wherein X together with the group $R^2$ represents a group of formula $-CH_2O-$ and $R^1$, $R^3$, $R^4$, $R^5$, $R^6$, $R^7$, J, M, E and Z are defined as above:
reacting a corresponding compound of formula (I) wherein X together with a group $R^2$ represents a ketonic oxygen atom and $R^1$, $R^3$, $R^4$, $R^5$, $R^6$, $R^7$, J, M, E and Z as above or represent protected forms thereof,
with diazomethane,
and optionally subsequently splitting off or converting any protective groups to produce the desired compound;

(n) for the preparation of compounds wherein $R^3$ is a hydrogen atom and $R^4$ represents a hydroxy group, and X, $R^1$, $R^2$, $R^5$, $R^6$, $R^7$, J, M, E and Z are defined as above:
reducing a compound of formula (XVIII)

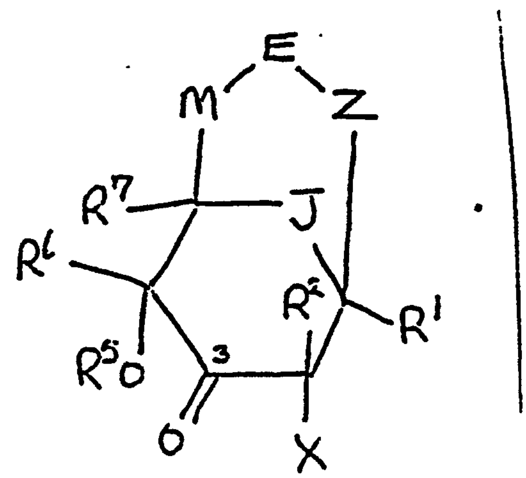

(XVIII)

(wherein X, $R^1$, $R^2$, $R^5$, $R^6$, $R^7$, J, M, E and Z are defined as above or represent protected forms thereof)
with a reducing agent capable of selectively forming the desired reduction product with the appropriate overall inversion of stereochemistry at C-3,
and optionally subsequently splitting off or converting any protective groups to produce the desired compound;

(o) for the preparation of compounds wherein $R^1$ represents an optionally substituted hydrocarbyl group, Z and J independently represent an O or S atom, an amino or an N-linked organic group and X, $R^2$, $R^3$, $R^4$, $R^5$, $R^6$, $R^7$, M and E are defined as above:
ring-closing a -JH and -ZH protected form of a compound of formula (XIX)

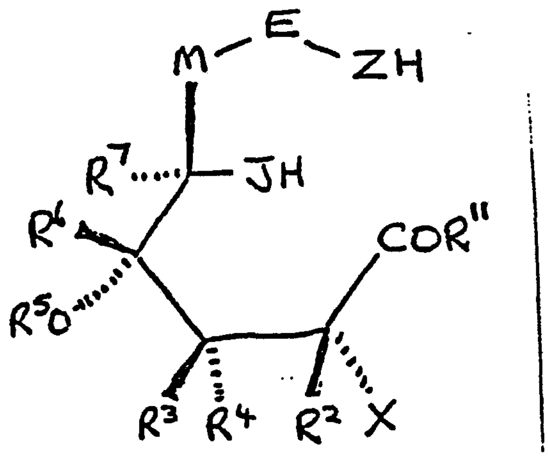

(XIX)

(wherein Z and J independently represent an O or S atom, an amino or an N-linked organic group and X, $R^2$, $R^3$, $R^4$, $R^5$, $R^6$, $R^7$, M and E are defined as above or represent protected forms thereof, and $R''$ represents an optionally substituted hydrocarbyl group)
and optionally subsequently splitting off or converting any protective groups present to produce the desired compound;

(p) for the preparation of compounds wherein $R^7$ represents an optionally substituted hydrocarbyl group, M and J independently represent an O or S atom, an amino or an N-linked organic group and X, $R^1$, $R^2$, $R^3$, $R^4$, $R^5$, $R^6$, Z and E are defined as above:
ring-closing a -JH and -MH protected form of a compound of formula (XXIV)

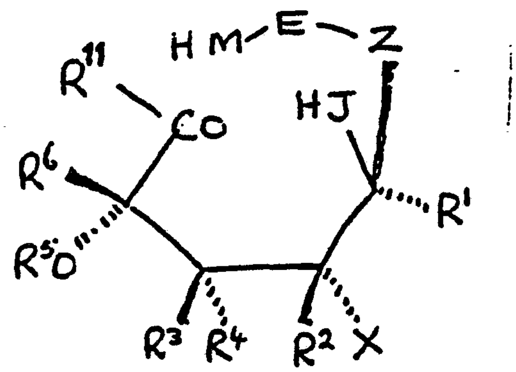

(XXIV)

(wherein M and J independently represent an O or S atom, an amino or an N-linked organic group and X, $R^1$, $R^2$, $R^3$, $R^4$, $R^5$, $R^6$, Z and E are defined as above or represent protected forms thereof, and $R''$ represents an optionally substituted hydrocarbyl group)
and optionally subsequently splitting off or converting any protective groups present to produce the desired compound;

(q) for the preparation of compounds of formula (I) wherein X represents a hydroxy group, $R^2$, $R^3$ and $R^4$ represent hydrogen atoms and $R^1$, $R^5$, $R^6$, $R^7$, J, M, E and Z are as defined above:
reacting a compound of formula (XXIX)

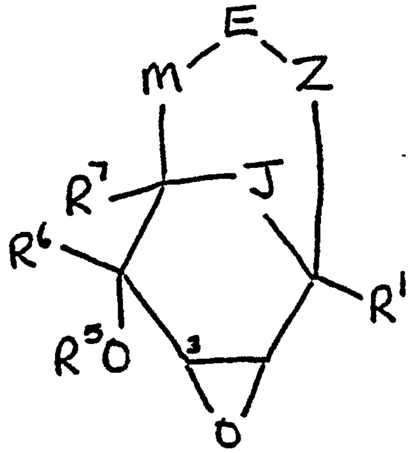

(XXIX)

(wherein $R^1$, $R^5$, $R^6$, $R^7$, J, M, E and Z are as defined above) with a reducing agent (eg, lithium aluminium hydride) capable of specifically or selectively cleaving the epoxide at the 3-position;

(r) for the preparation of compounds of formula (I) wherein X and $R^4$ together represent an oxygen atom, $R^2$ and $R^3$ represent hydrogen atoms and $R^1$, $R^5$, $R^6$, $R^7$, J, M, E and Z are as defined above: reacting an allylic alcohol of formula (XXX)

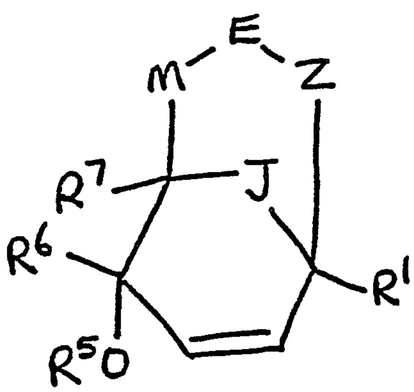

(XXX)

(wherein $R^1$, $R^5$, $R^6$, $R^7$, J, M, E and Z are defined as above) with a reagent capable of stereoselectively epoxidizing the double bond;

(s) for the preparation of compounds of formula (I) wherein $R^1$ represents a hydrogen atom, -M-E-Z- represents an $-OCH_2-$ group and X, $R^2$, $R^3$, $R^4$, $R^5$, $R^6$ and $R^7$ are as defined above: ring-closing of a vic-dihydroxy compound of formula (XXXII)

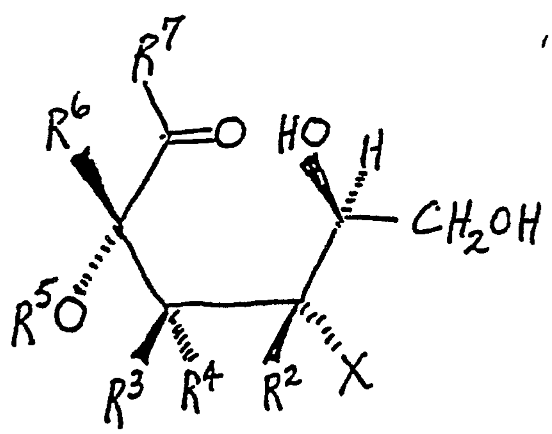

(XXXII)

(wherein X, $R^2$, $R^3$, $R^4$, $R^5$, $R^6$ and $R^7$ are as defined above or represent protected forms thereof) and optionally subsequently splitting off or converting any protective group present to produce the desired compound.

(t) ring closing of a compound of the formula (XXXVIII) or formula (XXXVIIIa)

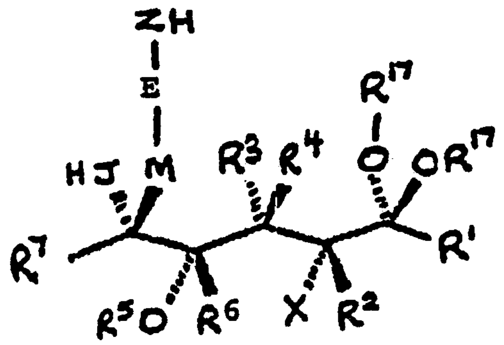

XXXVIII

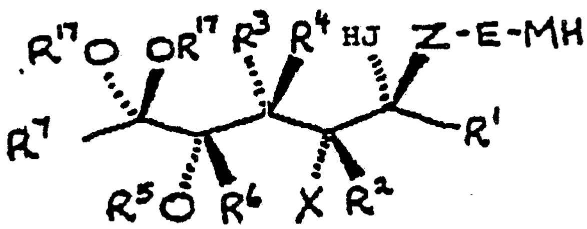

XXXVIII a

(wherein $R^1$, $R^2$, $R^3$, $R^4$, $R^5$, $R^6$, $R^7$, X, J, M, E and Z are as defined under formula (I) and $R^{17}$ is an alkyl group) or a protected form thereof.

(u) for the preparation of copounds wherein $R^1$ represents an optionally substituted hydrocarbyl group:
selectively reacting a compound of the formula (XXXXI)

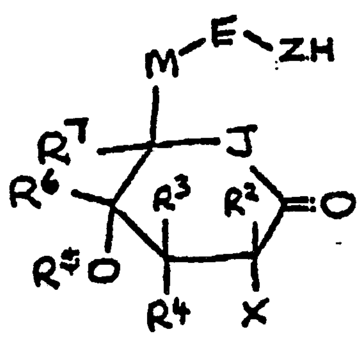

(XXXXI)

(wherein $R^2$, $R^3$, $R^4$, $R^5$, $R^6$, $R^7$, J, X, M, E and Z are as defined under formula I) or a protected form thereof with one mole equivalent of an organometallic reagent, and simultaneously or subsequently ring closing the compound thus obtained and then optionally splitting off or converting any protective groups present;

(v) for the preparation of compounds wherein $R^1$ together with X, Z or E represents a fused alicyclic or heterocyclic member:
covalently linking pre-existing groups for $R^1$ and X, Z or E in a similar manner to cyclisation of compounds of formula IIa-IIId described above, the atoms being linked having substituents $A_1$ and $A_2$ as defined above;

(w) for the preparation of compounds wherein $R^3$ is a fluorine atom:
reacting a corresponding compound of formula (I) in which $R^3$ is hydrogen and $R^4$ is a group or atom capable of being split off to leave the group or atom to which it was joined with a positive charge, e.g. where $R^3$ together with $R^4$ is an epoxide,
with a suitable fluoride source;

(x) for the preparation of compounds in which -M-E-Z- is a bridge containing two or more carbon atoms:
ring expanding a corresponding compound of formula (I) containing one less carbon by conventional ring expansion methods;

(y) for the preparation of compounds wherein $R^1$ is an optionally substituted hydrocarbyl group, J is O, Z is $-CR^8R^9$ , -M-E- or -M- is an O or S atom, or a sulphinyl, sulphonyl, amino, phosphinico or N-linked organic group:
ring closing of a compound of formula (XXXXII)

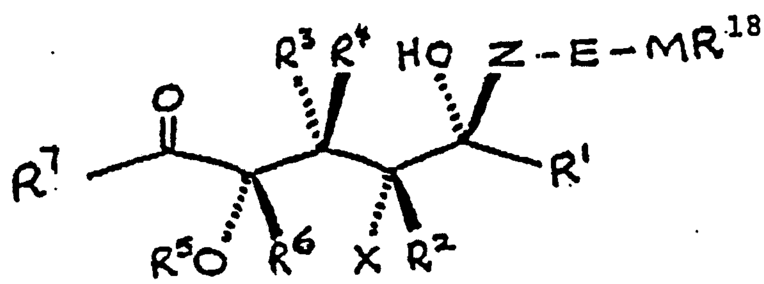

(XXXXII)

(wherein $R^2$, $R^3$, $R^4$, $R^5$, $R^6$, $R^7$ and X are as defined under formula (I), $R^1$ is an optionally substituted hydrocarbyl group, J is an oxygen atom, Z is $-CR^8R^9-$, -M-E- or -M- is an O or S atom, or a sulphinyl, sulphonyl amino, phosphinico or N-linked organic group)
or a protected form thereof;

(z) for the preparation of salts of compounds of formula (I) and enantiomers thereof:
reacting a compound of formula (I) or enantiomer thereof, initially obtained, with an acid or base, optionally in the presence of a suitable solvent, to form the desired salt.

28. A process as claimed in claim 27 wherein the compound of formula (I) is as defined in any of claims 2 to 20.

Claims for the following Contracting State: ES

1. A process for producing a compound of the formula (I)

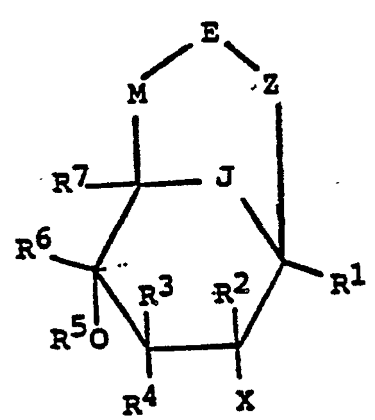

(I)

in which

$R^1$ represents a hydrogen or halogen atom, an O-, N-, P- or S-linked organic or optionally substituted

hydrocarbyl group

or $R^1$, together with X, E or Z, may form an optionally substituted fused alicyclic or heterocyclic member;

$R^2$ represents a hydrogen or fluorine atom, a hydroxy group or an optionally substituted hydrocarbyl or O-linked hydrocarbyl group

or $R^2$, together with X, may form a ketonic oxygen atom -O- or a group of the formula $-CH_2O-$;

$R^3$ represents a hydrogen or halogen atom, a hydroxy group or an optionally substituted hydrocarbyl or O-linked hydrocarbyl group

or $R^3$, together with $R^4$, may form an optionally substituted hydrocarbyl group;

$R^4$ represents a hydrogen or halogen atom, a hydroxy group or an optionally substituted hydrocarbyl or O-linked hydrocarbyl group

or $R^4$, together with X, may form an oxygen atom;

$R^5$ represents an optionally substituted aralkyl, alicyclicalkyl, heterocyclicalkyl, aryl, alicyclic, heterocyclic, alkyl, alkenyl or alkynyl group

$R^6$ represents a hydrogen atom or an optionally substituted hydrocarbyl group;

$R^7$ represents a hydrogen or halogen atom, an O-, N-, P- or S-linked organic or optionally substituted hydrocarbyl group or together with M or E, $R^7$ may form an optionally substituted fused alicyclic or heterocyclic member;

X represents an O- N- or S-linked organic group, an optionally substituted hydrocarbyl group, a halogen atom, a hydroxy, amino, aloxyamino, nitro, cyano, azido, sulpho or phospho group,

M, E and Z

independently of each other represent an O or S atom, a sulphinyl, sulphonyl, amino, phosphinico or N-linked organic group, a single bond, or the group $CR^8R^9$,

wherein $R^8$ represents a hydrogen or halogen atom, an O-, N-, P- or S-linked organic or optionally substituted hydrocarbyl group, a hydroxy, amino, alkoxyamino, nitro, cyano, azido, sulpho or phospho group or $R^8$, together with $R^9$, may form an optionally substituted spiroalicyclic or spiroheterocyclic member, an optionally substituted hydrocarbyl group or a double bonded oxygen atom;

and wherein $R^9$ represents a hydrogen or halogen atom, an O-, N-, P- or S-linked organic or optionally substituted hydrocarbyl group, a hydroxy, amino, alkoxyamino, nitro, cyano, azido, sulpho or phospho group or; and

wherein, adjacent M, E or Z groups may be joined by a double bond; and

wherein, two or more of M, E and Z may form an optionally substituted fused alicyclic or heterocyclic ring;

with the proviso that only one of the group M, E or Z may represent a single bond;

J represents an O or S atom, a sulphinyl, sulphonyl, amino, phosphinico or N-linked organic group or an optionally substituted hydrocarbyl group;

and the enantiomers, salts and mixtures thereof;

with the following provisos:

(i) when one of M, E and Z is a single bond and J is an oxygen atom then at least one of the remainder of M, E and Z represents an O or S atom, a sulphinyl, sulphonyl, amino, phosphinico or N-linked organic group or the group $CR^8R^9$, in which at least one of $R^8$ or $R^9$ is not a hydrogen atom or O-linked organic or optionally substituted hydrocarbyl group;

(ii) when -M-E-Z- is $-OCH_2-$, J is an O atom, X and $R^5O$ are the same aralkoxy group, and $R^1$ is hydrogen, then the enantiomer thereof is not included;

(iii) when -M-E-Z- together form the group $-CH_2O-$, J is an oxygen atom, $R^7$ is a hydrogen atom, and $R^5$ is an optionally substituted aralkyl group, then

(a) $R^1$ represents an O-, N-, P- or S-linked organic group; or

(b) $R^1$, together with X, forms an optionally substituted fused alicyclic or heterocyclic member; or

(c) $R^2$ represents a fluorine atom; or

(d) $R^3$ or $R^4$ represents a halogen atom; or

(e) $R^3$, together with $R^4$, forms an optionally substituted hydrocarbyl group; or

(f) $R^4$, together with X, forms an oxygen atom;

(iv) when -M-E-Z- is $-OCH_2-$, J is an oxygen atom, $R^1$ is a hydrogen atom and X is an optionally substituted aralkoxy group, then

(a) $R^2$ represents a fluorine atom; or

(b) $R^3$ or $R^4$ represents a halogen atom; or

(c) $R^3$, together with $R^4$, forms an optionally substituted hydrocarbyl group; or

(d) $R^4$, together with X, forms an oxygen atom;

(v) when J is O, -M-E-Z- is $-CH_2O-$ and $R^1$ and $R^7$ both represent a hydrogen atom then:

(a) $OR^5$, X and $R^3$ are not the same substituted alkyloxy group;

(b) when $R^3$ is a hydroxy or O-linked organic group, X is a fluorine atom, or a hydroxy, O-linked organic, amino, azido, methyl, acetamido or benzylamino group, $R^5$ is not an unsubstituted $C_1$-$C_4$ alkyl or $C_2$-$C_4$ alkenyl or a tetrahydrofuran-1-methyl group in which each of the 2, 3 and 4 positions is substituted with an O-linked organic or hydroxy group; or a substituted alkyl group where the substituent is O-linked or contains oxygen as the only heteroatom; and

(vi) when -M-E-Z- is $-CR^8R^9O-$, $R^8$ is an exo-ethyl or exo-fluoro group and $R^1$, $R^7$ and $R^9$ are hydrogen, then $OR^5$, X and $R^3$ are not all benzyloxy;

(vii) when -M-E-Z- is $-CH_2O-$, J is an oxygen atom, and $R^1$, $R^2$, $R^3$, $R^6$ and $R^7$ are hydrogen atoms, then $R^5O$, $R^4$ and X are not all methoxy groups;

(viii) when -M-E-Z- is $-C(phenyl)_2O-$, J is an oxygen atom, and $R^1$, $R^2$, $R^4$, $R^6$ and $R^7$ represent hydrogen atoms then $R^5O$, $R^3$ and X are not all methoxy;

(ix) when -M-E-Z- is $-CH_2O-$, $R^5$ is methyl, J is an oxygen atom, $R^1$, $R^3$, $R^4$, $R^6$ and $R^7$ are hydrogen atoms then X and $R^2$ are not jointly an oxygen atom, or not both hydroxy or methoxy groups or X is not hydroxy when $R^2$ is a hydrogen atom;

comprising one or more of the following reactions:

(a) cyclising a compound of formula (IIa), (IIb), (IIc), (IId), (IIIa), (IIIb), (IIIc) and (IIId)

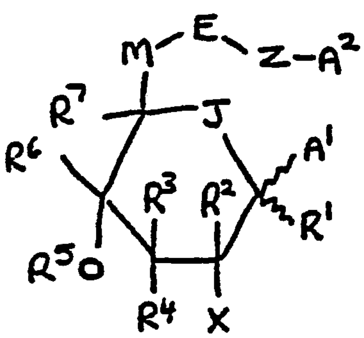

(IIa)

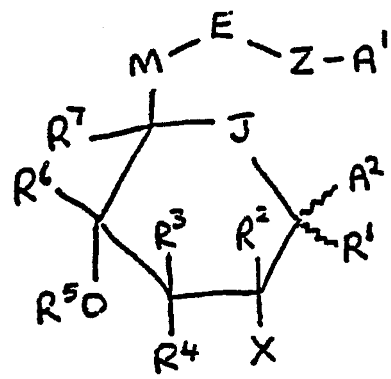

(IIb)

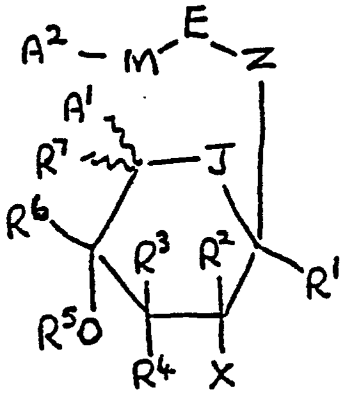

(IIc)

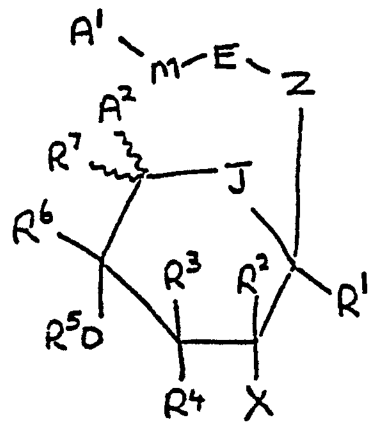

(IId)

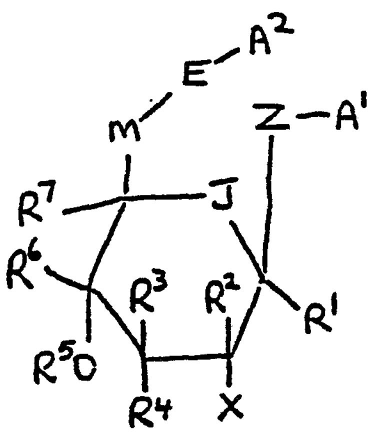

(IIIa)

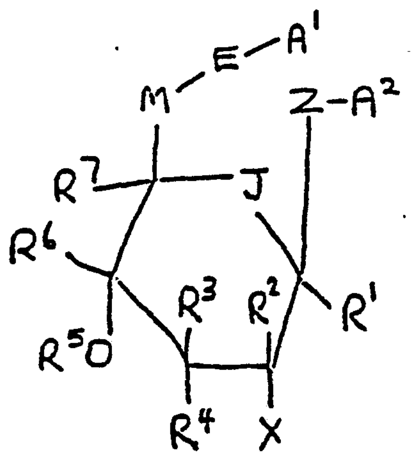

(IIIb)

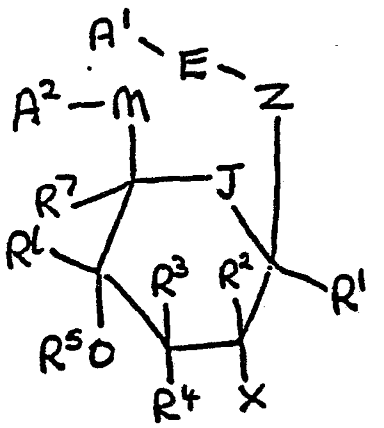

(IIIc)

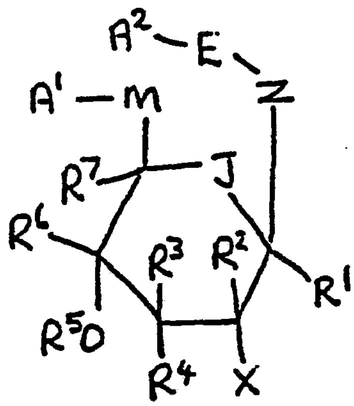

(IIId)

(in which X, $R^1$, $R^2$, $R^3$, $R^4$, $R^5$, $R^6$, $R^7$, M, J, E and Z are as defined above, or represent protected forms thereof,
$A^2$ is a group or atom capable of being split off to leave the atom or group to which it was joined with a negative charge,
and $A^1$ is a group or atom capable of being split off to leave the group or atom to which it was joined with a positive charge)
or an enantiomer thereof,
and optionally subsequently splitting off or converting any protective groups present to produce red compounds;

(b) reacting a compound of formula (VII)

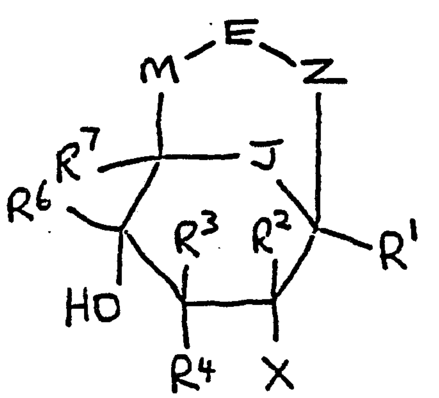

(VII)

or a corresponding alkali metal alkoxide
(wherein X, $R^1$, $R^2$, $R^3$, $R^4$, $R^6$, $R^7$, J, M, E and Z are as defined above or represent protected forms thereof)
or an enantiomer thereof,
with a compound serving to introduce the group $R^5$ in place of the hydroxy hydrogen atom

and optionally subsequently splitting off or converting any protective groups present to produce the desired compound;

(c) for the preparation of compounds wherein X represents an O-linked organic group and/or wherein $R^3$ and/or $R^4$ represent an optionally substituted O-linked hydrocarbyl group and $R^1$, $R^2$, $R^5$, $R^6$, $R^7$, J, M, E and Z and the remainder of X, $R^3$ and $R^4$ are as defined above:
reacting a corresponding compound of formula (I) wherein X and/or $R^3$ and/or $R^4$ represent a hydroxy group, and the remainder of X, $R^1$, $R^2$, $R^3$, $R^4$, $R^5$, $R^6$, $R^7$, J, M, E and Z are as defined above or represent protected forms thereof,
or an enantiomer thereof,
or a corresponding alkali metal alkoxide thereof,
with a compound serving to introduce respectively an O-linked organic group at X and/or an optionally substituted O-linked hydrocarbyl group at $R^3$ and/or $R^4$
and optionally subsequently splitting off or converting any protective groups present, to produce the desired compound;

(d) for the preparation of compounds wherein X represents a hydroxy group and $R^2$ represents a hydrogen atom:
reducing a compound of formula (I) wherein X together with the group $R^2$ represents a ketonic oxygen atom, and $R^1$, $R^3$, $R^4$, $R^5$, $R^6$, $R^7$, J, M, E and Z are as defined above or represent protected forms thereof,
or an enantiomer thereof,
and optionally subsequently splitting off or converting any protective groups present, to produce the desired compound;

(e) for the preparation of compounds wherein X together with the group $R^2$ represents a ketonic oxygen atom and the groups $R^3$ and $R^4$ represent hydrogen atoms and $R^5$ is defined as above:
reaction of an enone of formula (IX)

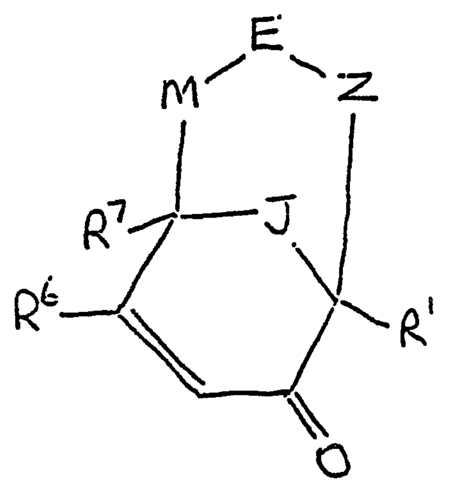

(IX)

with an alcohol of formula (X)

$R^5OH$ (X)

(wherein $R^5$ is defined as above)

f) for the preparation of compounds wherein X, $R^1$, $R^2$, $R^5$, $R^6$, $R^7$, J, M, E and Z are as defined above and $R^3$ and $R^4$ represent hydrogen atoms:
reacting a compound of formula (XII)

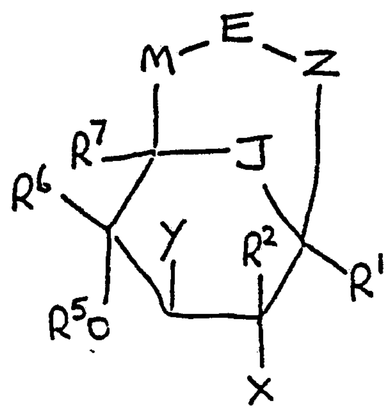

(XII)

(in which X, $R^1$, $R^2$, $R^5$, $R^6$, $R^7$, J, M, E and Z are defined as above, or represent protected forms thereof, and Y represents the group OC(S)Q; wherein Q may be, for example, thiomethyl, phenoxy, imidazol-1-yl or other suitable moiety or represents a protected form thereof),
or an enantiomer thereof,
with trialkyltin hydride, (eg, tributyltin hydride)
and optionally subsequently splitting off or converting any protective groups to produce the desired compounds;

(g) for the preparation of compounds wherein $R^1$, $R^5$, $R^7$, J, M, E and Z are as defined above, $R^2$, $R^4$ and $R^6$ represent hydrogen atoms, $R^3$ represents a hydroxy group and X is the same as the group $OR^5$: diether formation involving the 1- and 3-oxygen atoms of the 1,2,3-triol (XIII)

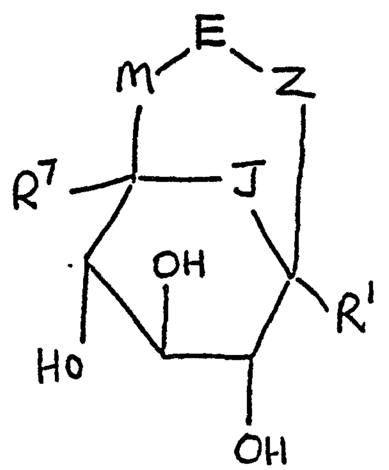

(XIII)

;

(h) for the preparation of compounds wherein $R^3$ represents a hydroxy group, X represents an O-linked organic group, an optionally substituted hydrocarbyl group, a halogen atom, or a hydroxy, amino, cyano or azido group, $R^2$ and $R^4$ represent hydrogen atoms, and $R^1$, $R^5$, $R^6$, $R^7$, M, E, Z and J are as defined above:
reacting an epoxide of formula (XIV)

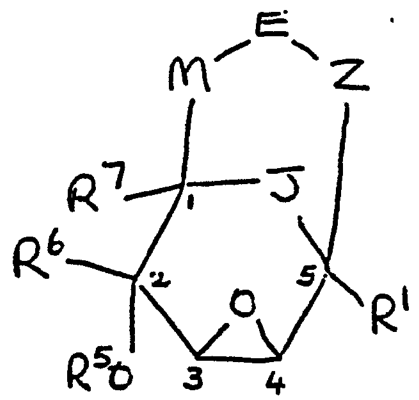

(XIV)

(wherein $R^1$, $R^5$, $R^6$, $R^7$, J, M, E and Z are as defined for formula (I) or represent protected forms thereof) or an enantiomer thereof,
with a compound serving to selectively introduce the group X as defined above at the 4-position,
and optionally subsequently splitting off or converting any protective groups present to produce the desired compound;

(i) for the preparation of compounds wherein $R^2$ represents a hydrogen atom and X represents an S-linked organic group or an azido group and $R^1$, $R^3$, $R^4$, $R^5$, $R^6$, $R^7$, M, E, Z and J are as defined above:
reacting a compound of formula (XVI)

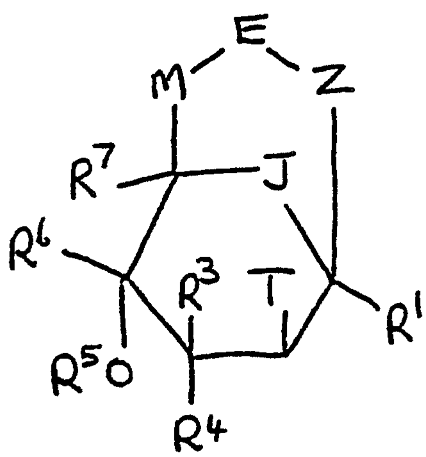

(XVI)

(wherein $R^1$, $R^3$, $R^4$, $R^5$, $R^6$, $R^7$, J, M, E and Z are as defined above or represent protected forms thereof, and T represents a nucleophilically exchangeable group,
with an organic thiol of formula (XVIII)

$R^1SH$ (XVII)

(wherein $R^1$ represents an organic group)
or a reactive derivative thereof,
or with sodium azide,
and optionally subsequently splitting off or converting protective groups to produce the desired compound;

(j) for the preparation of compounds wherein X represents a mono- or di-alkylamino, mono- or di-aralkylamino, trialkylammonium, carboxamido or dicarboximido group and $R^1$, $R^2$, $R^3$, $R^4$, $R^5$, $R^6$, $R^7$, J, M, E and Z are defined as above:
reacting a compound of formula (I), wherein X represents an amino group and $R^1$, $R^2$, $R^3$, $R^4$, $R^5$, $R^6$, $R^7$, J, M, E and Z are defined as above or represent protected forms thereof, with a compound capable of converting said amino group to a mono- or dialkylamino, mono- or di-aralkylamino, trialkylammonium, carboxamido or dicarboximido group,
and optionally subsequently splitting off or converting any protective groups present to produce the desired compound;

(k) for the preparation of compounds wherein X represents a carboxamido or dicarboxamido group and $R^1$, $R^2$, $R^3$, $R^4$, $R^5$, $R^6$, $R^7$, J, M, E and Z are defined as above:
reacting a compound of formula (I) wherein X represents an azido group and $R^1$, $R^2$, $R^3$, $R^4$, $R^5$, $R^6$, $R^7$, J, M, E and Z are defined as above or represent protected forms thereof,
or an activated derivative thereof,
with an anhydride reagent corresponding to the desired carboxamido or dicarboximido group,
and optionally subsequently splitting off or converting any protective groups to produce the desired compound;

(l) for the preparation of compounds wherein X represents an amino group and $R^1$, $R^2$, $R^3$, $R^4$, $R^5$, $R^6$, $R^7$, J, M, E and Z are defined as above:
reducing a corresponding azido compound of formula (I) wherein X represents an azido group, and $R^1$, $R^2$, $R^3$, $R^4$, $R^5$, $R^6$, $R^7$, J, M, E and Z are as defined above
or represent protected forms thereof,
and optionally subsequently splitting off or converting any protective groups to produce the desired compound;

(m) for the preparation of compounds wherein X together with the group $R^2$ represents a group of formula $-CH_2O-$ and $R^1$, $R^3$, $R^4$, $R^5$, $R^6$, $R^7$, J, M, E and Z are defined as above:
reacting a corresponding compound of formula (I) wherein X together with a group $R^2$ represents a ketonic oxygen atom and $R^1$, $R^3$, $R^4$, $R^5$, $R^6$, $R^7$, J, M, E and Z as above or represent protected forms thereof, with diazomethane,
and optionally subsequently splitting off or converting any protective groups to produce the desired compound;

(n) for the preparation of compounds wherein $R^3$ is a hydrogen atom and $R^4$ represents a hydroxy group, and X, $R^1$, $R^2$, $R^5$, $R^6$, $R^7$, J, M, E and Z are defined as above:
reducing a compound of formula (XVIII)

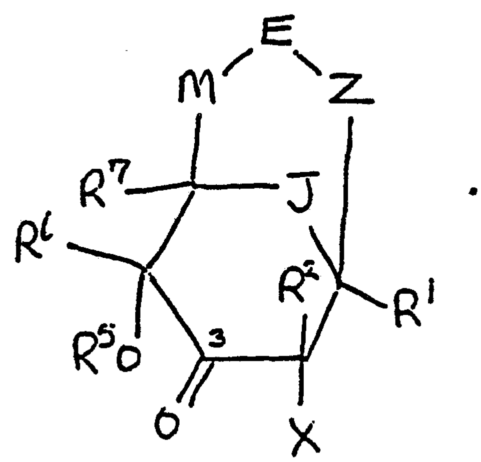
(XVIII)

wherein X, $R^1$, $R^2$, $R^5$, $R^6$, $R^7$, J, M, E and Z are defined as above or represent protected forms thereof)
with a reducing agent capable of selectively forming the desired reduction product with the appropriate overall inversion of stereochemistry at C-3,
and optionally subsequently splitting off or converting any protective groups to produce the desired compound;

(o) for the preparation of compounds wherein $R^1$ represents an optionally substituted hydrocarbyl group, Z and J independently represent an O or S atom, an amino or an N-linked organic group and X, $R^2$, $R^3$, $R^4$, $R^5$, $R^6$, $R^7$, M and E are defined as above:
ring-closing a -JH and -ZH protected form of a compound of formula (XIX)

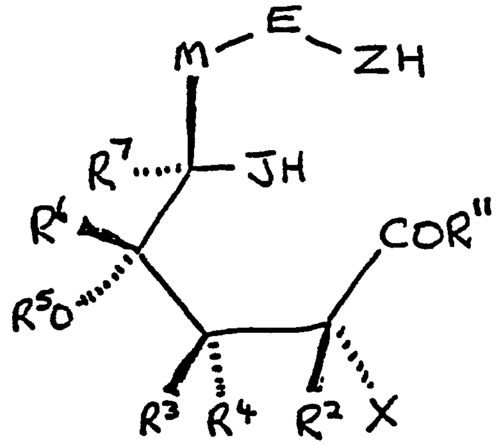

(XIX)

(wherein Z and J independently represent an O or S atom, an amino or an N-linked organic group and X, $R^2$, $R^3$, $R^4$, $R^5$, $R^6$, $R^7$, M and E are defined as above or represent protected forms thereof, and $R''$ represents an optionally substituted hydrocarbyl group)
and optionally subsequently splitting off or converting any protective groups present to produce the desired compound;

(p) for the preparation of compounds wherein $R^7$ represents an optionally substituted hydrocarbyl group, M and J independently represent an O or S atom, an amino or an N-linked organic group and X, $R^1$, $R^2$, $R^3$, $R^4$, $R^5$, $R^6$, Z and E are defined as above:
ring-closing a -JH and -MH protected form of a compound of formula (XXIV)

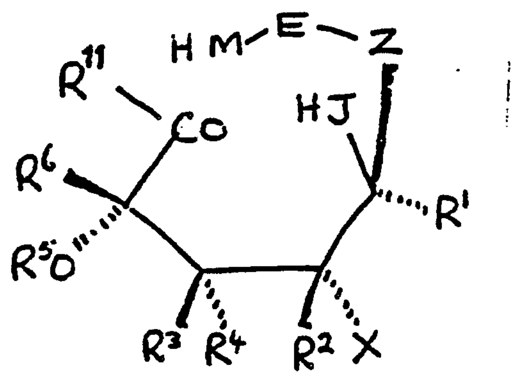

(XXIV)

(wherein M and J independently represent an O or S atom, an amino or an N-linked organic group and X, $R^1$, $R^2$, $R^3$, $R^4$, $R^5$, $R^6$, Z and E are defined as above or represent protected forms thereof, and $R''$ represents an optionally substituted hydrocarbyl group)
and optionally subsequently splitting off or converting any protective groups present to produce the desired compound;

(q) for the preparation of compounds of formula (I) wherein X represents a hydroxy group, $R^2$, $R^3$ and $R^4$ represent hydrogen atoms and $R^1$, $R^5$, $R^6$, $R^7$, J, M, E and Z are as defined above:
reacting a compound of formula (XXIX)

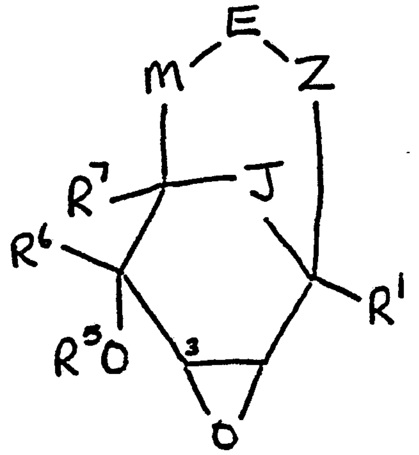

(XXIX)

(wherein $R^1$, $R^5$, $R^6$, $R^7$, J, M, E and Z are as defined above) with a reducing agent (eg, lithium aluminium hydride) capable of specifically or selectively cleaving the epoxide at the 3-position;

(r) for the preparation of compounds of formula (I) wherein X and $R^4$ together represent an oxygen atom, $R^2$ and $R^3$ represent hydrogen atoms and $R^1$, $R^5$, $R^6$, $R^7$, J, M, E and Z are as defined above: reacting an allylic alcohol of formula (XXX)

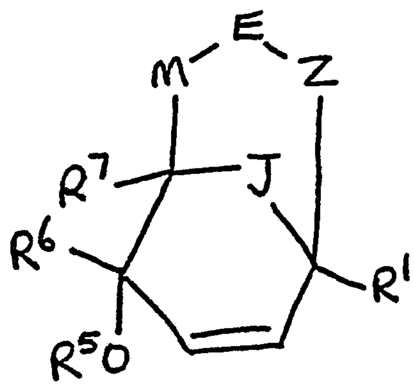

(XXX)

(wherein $R^1$, $R^5$, $R^6$, $R^7$, J, M, E and Z are defined as above) with a reagent capable of stereoselectively epoxidizing the double bond;

(s) for the preparation of compounds of formula (I) wherein $R^1$ represents a hydrogen atom, -M-E-Z- represents an $-OCH_2-$ group and X, $R^2$, $R^3$, $R^4$, $R^5$, $R^6$ and $R^7$ are as defined above: ring-closing of a vic-dihydroxy compound of formula (XXXII)

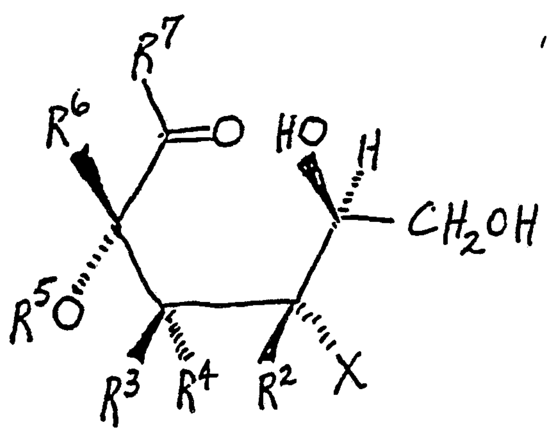

(XXXII)

(wherein X, $R^2$, $R^3$, $R^4$, $R^5$, $R^6$ and $R^7$ are as defined above or represent protected forms thereof) and optionally subsequently splitting off or converting any protective group present to produce the desired compound.

(t) ring closing of a compound of the formula (XXXVIII) or formula (XXXVIIIa)

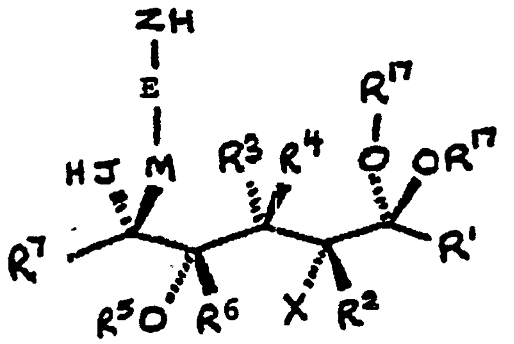

XXXVIII

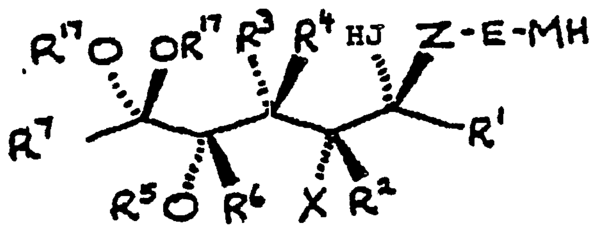

XXXVIIIa

(wherein $R^1$, $R^2$, $R^3$, $R^4$, $R^5$, $R^6$, $R^7$, X, J, M, E and Z are as defined under formula (I) and $R^{17}$ is an alkyl group)
or a protected form thereof.

(u) for the preparation of copounds wherein $R^1$ represents an optionally substituted hydrocarbyl group:
selectively reacting a compound of the formula (XXXXI)

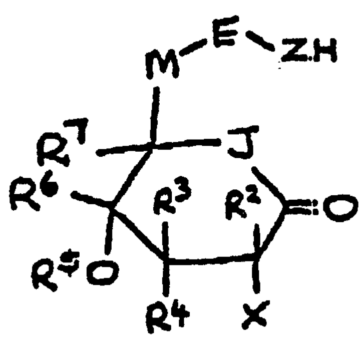

(XXXXI)

(wherein $R^2$, $R^3$, $R^4$, $R^5$, $R^6$, $R^7$, J, X, M, E and Z are as defined under formula I)
or a protected form thereof
with one mole equivalent of an organometallic reagent, and simultaneously or subsequently ring closing the compound thus obtained and then optionally splitting off or converting any protective groups present;

(v) for the preparation of compounds wherein $R^1$ together with X, Z or E represents a fused alicyclic or heterocyclic member:
covalently linking pre-existing groups for $R^1$ and X, Z or E in a similar manner to cyclisation of compounds of formula IIa-IIId described above, the atoms being linked having substituents $A_1$ and $A_2$ as defined above;

(w) for the preparation of compounds wherein $R^3$ is a fluorine atom:
reacting a corresponding compound of formula (I) in which $R^3$ is hydrogen and $R^4$ is a group or atom capable of being split off to leave the group or atom to which it was joined with a positive charge, e.g. where $R^3$ together with $R^4$ is an epoxide,
with a suitable fluoride source;

(x) for the preparation of compounds in which -M-E-Z- is a bridge containing two or more carbon atoms:
ring expanding a corresponding compound of formula (I) containing one less carbon by conventional ring expansion methods;

(y) for the preparation of compounds wherein $R^1$ is an optionally substituted hydrocarbyl group, J is O, Z is $-CR^8R^9-$, -M-E- or -M- is an O or S atom, or a sulphinyl, sulphonyl, amino, phosphinico or N-linked organic group:
ring closing of a compound of formula (XXXXII)

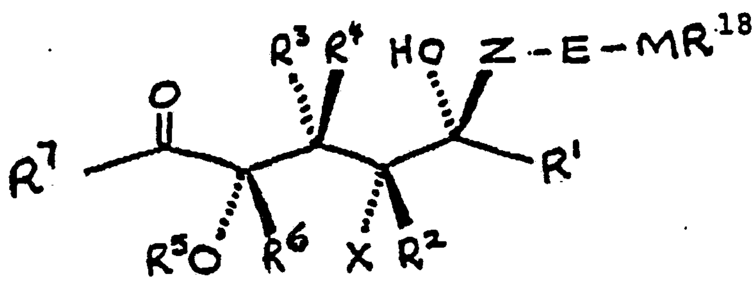

(XXXXII)

(wherein $R^2$, $R^3$, $R^4$, $R^5$, $R^6$, $R^7$ and X are as defined under formula (I), $R^1$ is an optionally substituted hydrocarbyl group, J is an oxygen atom, Z is $-CR^8R^9-$, -M-E- or -M- is an O or S atom, or a sulphinyl, sulphonyl amino, phosphinico or N-linked organic group)
or a protected form thereof;

(z) for the preparation of salts of compounds of formula (I) and enantiomers thereof:
reacting a compound of formula (I) or enantiomer thereof, initially obtained, with an acid or base, optionally in the presence of a suitable solvent, to form the desired salt.

2. A process of claim 1 in which $R^3$, $R^4$, $R^6$, $R^7$, X and J are as defined in claim 1 and where
$R^1$ represents a hydrogen or halogen atom, an O-, N-, P- or S-linked organic or optionally substituted hydrocarbyl group
or $R^1$, together with X, E or Z, may form an optionally substituted fused alicyclic or heterocyclic member;
or, when -M-E-Z- together form the group $-OCR^8R^9-$ and J is an oxygen atom, $R^1$ may also form an optionally substituted fused alicyclic or heterocyclic member with X or with $R^8$ or $R^9$;
$R^2$ represents a hydrogen or fluorine atom, a hydroxy group or an optionally substituted hydrocarbyl or O-linked hydrocarbyl group
or $R^2$, together with X, may form a ketonic oxygen atom -O- or a group of the formula $-CH_2O-$;
$R^3$ represents a hydrogen or halogen atom, a hydroxy group or an optionally substituted hydrocarbyl or O-linked hydrocarbyl group
or $R^3$, together with $R^4$, may form an optionally substituted hydrocarbyl group;
$R^5$ represents an optionally substituted aralkyl, alicyclicalkyl, heterocyclicalkyl, aryl, alicyclic, heterocyclic, alkyl, alkenyl or alkynyl group
M, E and Z
independently of each other represent an O or S atom, a sulphinyl, sulphonyl, amino, phosphinico or N-linked organic group, a single bond, or the group $CR^8R^9$,
wherein $R^8$ represents a hydrogen or halogen atom, an O-, N-, P- or S-linked organic or optionally substituted hydrocarbyl group, a hydroxy, amino, alkoxyamino, nitro, cyano, azido, sulpho or phospho group or $R^8$, together with $R^9$, may form an optionally substituted spiroalicyclic or spiroheterocyclic member, an optionally substituted hydrocarbyl group or a double bonded oxygen atom;
and wherein $R^9$ represents a hydrogen or halogen atom, an O-, N-, P- or S-linked organic or optionally substituted hydrocarbyl group, a hydroxy, amino, alkoxyamino, nitro, cyano, azido, sulpho or phospho group

or; and
wherein, adjacent M, E or Z groups may be joined by a double bond; and
wherein, two or more of M, E and Z may form an optionally substituted fused alicyclic or heterocyclic ring.

3. A process of claim 1 in which -M-E-Z- together form the group $OCR^8R^9$- where $R^8$ and $R^9$ are as defined in claim 1 and in which $R^2$, $R^3$, $R^4$, $R^6$ and X are as defined in claim 1, J is an oxygen atom,
$R^1$ represents a hydrogen or halogen atom, an O-, N-, P- or S-linked organic or optionally substituted hydrocarbyl group
or together with the group X, $R^1$ may form an optionally substituted fused alicyclic or heterocyclic member
or together with the group $R^8$ or $R^9$, $R^1$ may form an optionally substituted fused alicyclic or heterocyclic member;
$R^5$ represents an optionally substituted aralkyl, alicyclicalkyl, heterocyclicalkyl, aryl, alicyclic, heterocyclic, alkenyl or alkynyl group; and
$R^7$ represents a hydrogen or halogen atom, an O-, N-, P- or S-linked organic or optionally substituted hydrocarbyl group.

4. A process of claim 1 in which J represents an oxygen atom, -M-E-Z- together represent a $-CR^8R^9O$-group, $R^2$, $R^3$, $R^4$, $R^6$, $R^8$, $R^9$ and X are as defined in claim 1 and
$R^1$ represent a hydrogen atom, an O-, N-, P- or S-linked organic or optionally substituted hydrocarbyl group;
$R^5$ represents an optionally substituted aralkyl, alicyclicalkyl, heterocyclicalkyl, aryl, alicyclic, heterocyclic, alkenyl or alkynyl group; and
$R^7$ represents a hydrogen or halogen atom, an O-, N-, P- or S-linked organic or optionally substituted hydrocarbyl group
or together with the group $R^8$ or $R^9$, $R^7$ may form an optionally substituted fused alicyclic or heterocyclic member.

5. A process of claim 1 in which J represents an oxygen atom, -M-E-Z- together represent $-CH_2O$-, $R^2$, $R^3$, $R^4$, $R^6$, $R^7$ and X are as defined in claim 1,
$R^1$ represents a hydrogen atom, an O-, N-, P- or S-linked organic or optionally substituted hydrocarbyl group;
or together with the group X, $R^1$ may form an optionally substituted fused alicyclic or heterocyclic member; and
$R^5$ represents an optionally substituted alicyclicalkyl, heterocyclicalkyl, aryl, alicyclic, heterocyclic, alkenyl or alkynyl group.

6. A process as claimed in claim 1 wherein
$R^1$ represents a hydrogen atom or an optionally substituted hydrocarbyl group of $C_{1-6}$ carbon atoms;
$R^2$ represents a hydrogen atom;
$R^3$ represents a hydrogen atom;
$R^4$ represents a hydrogen atom;
$R^5$ represents a benzyl, methylbenzyl, benzothienyl, 2-thiophenemethyl, 3-thiophenemethyl, indan-2 yl, tetrahydropyran-2-ylmethyl or cyclohexanemethyl group optionally substituted with up to two substituents independently chosen from chlorine, bromine or fluorine atoms, a methoxy group or a $C_{1-3}$ alkyl group optionally substituted with up to three fluorineatoms;
$R^6$ represents a hydrogen atom;
$R^7$ represents a hydrogen atom or an optionally substituted hydrocarbyl group of $C_{1-6}$ carbon atoms;
M, E and Z
independently represent an O or S atom, an amino, sulphinyl, sulphonyl or $C_{1-6}$ alkylamino group, a single bond or a $C_{1-6}$ alkylene group or the group $-CR^8R^9$- wherein;
$R^8$ represents a hydrogen, fluorine, chlorine or bromine atom, an optionally substituted hydrocarbyl group of $C_{1-6}$ carbon atoms or an alkylthio group;
$R^9$ represents a hydrogen, fluorine, chlorine or bromine atom, an optionally substituted hydrocarbyl group of $C_{1-6}$ carbon atoms, or an alkylthio group;
X represents a $C_{1-6}$ alkoxy group, an optionally substituted hydrocarbyl group of $C_{1-6}$ carbon atoms, a fluorine, chlorine or bromine atom or an azido group;
J represents an O or S atom, an amino or a $C_{1-6}$ alkylamino group or a $C_{1-6}$ alkylene group.

7. A process as claimed in claim 1, in which
$R^5$ is ($C_{6-8}$-cycloalkyl)-methyl, 2-alkyl-alkyl or cycloalkenemethyl and $R^1$, $R^2$, $R^3$, $R^4$, $R^6$, $R^7$, $R^8$, $R^9$, X, J, M, E and Z are as defined in claim 6.

8. A process as claimed in claim 1, wherein

$R^1$ represents a hydrogen atom or an unbranched $C_{1-6}$ alkyl group optionally substituted in the terminal position with a chlorine or methoxy group;

$R^2$ represents a hydrogen atom;

$R^3$ represents a hydrogen atom;

$R^4$ represents a hydrogen atom;

$R^5$ represents a benzyl, methylbenzyl, benzothienyl, 2-thiophenemethyl, 3-thiophenemethyl, indan-2-yl, tetrahydropyran-2-ylmethyl, cyclohexanemethyl group optionally substituted with up to two substituents independently chosen from chlorine, bromine or fluorine atoms, a methoxy group or a $C_{1-3}$ alkyl group optionally substituted with up to three fluorine atoms;

$R^6$ represents a hydrogen atom;

$R^7$ represents a hydrogen atom or an optionally substituted hydrocarbyl group of $C_{1-6}$ carbon atoms;

M, E and Z

independently represent an O or S atom, an amino or $C_{1-6}$ alkylamino group, a single bond, a $C_{1-6}$ alkylene group or the group $-CR^8R^9$; and

$R^8$ represents a hydrogen, fluorine, chlorine or bromine atom, an optionally substituted hydrocarbyl group of $C_{1-6}$ carbon atoms or an alkylthio group;

$R^9$ represents a hydrogen, fluorine, chlorine or bromine atom, an optionally substituted hydrocarbyl group of $C_{1-6}$ carbon atoms or an alkylthio group;

X represents an ethoxy, methoxy or azido group, a fluorine, chlorine or bromine atom, or an optionally substituted hydrocarbyl group of $C_{1-6}$ carbon atoms;

J represents an O or S atom, an amino or a $C_{1-6}$ alkylamino group or a $C_{1-6}$ alkylene group.

9. A process as claimed in claim 1 wherein

$R^1$ is a halogen atom and $R^2$, $R^3$, $R^4$, $R^5$, $R^6$, $R^7$, $R^8$, $R^9$, X, J, M, E and Z are as defined in claim 8.

10. A process as claimed in claim 1 wherein

$R^5$ is cyclooctanemethyl, 3-cyclohexenemethyl, or 2-(G-$2^{alkyl}$)-$C_{3-6}{}^{alkyl}$ and $R^1$, $R^2$, $R^3$, $R^4$, $R^6$, $R^7$, $R^8$, $R^9$, X, J, M, E and Z are as defined in claim 8.

11. A process as claimed in claim 10 wherein

$R^5$ is 2-(methyl or ethyl)-(propyl, butyl or pentyl).

12. A process as claimed in claim 2, wherein

$R^1$ represent a hydrogen atom or an optionally substituted hydrocarbyl group of $C_{1-6}$ carbon atoms;

$R^2$ represents a hydrogen atom;

$R^3$ represents a hydrogen atom;

$R^4$ represents a hydrogen atom;

$R^5$ represents a benzyl, methylbenzyl, benzothienyl, 2-thiophenemethyl, 3-thiophenemethyl, indan-2-yl, tetrahydropyran-2-ylmethyl or cyclohexanemethyl group optionally substituted with up to two substituents independently chosen from chlorine, bromine or fluorine atoms, a methoxy group or a $C_{1-3}$ alkyl group optionally substituted with up to three fluorine atoms;

$R^6$ represents a hydrogen atom;

$R^7$ represent a hydrogen atom or an optionally substituted hydrocarbyl group of $C_{1-6}$ carbon atoms;

X represents a $C_{1-6}$ alkoxy group, an optionally substituted hydrocarbyl group of $C_{1-6}$ carbon atoms, a fluorine, chlorine or bromine atom or an azido group;

M represent an O or S atom, an amino or $C_{1-6}$ alkylamino group, a single bond or a $C_{1-6}$ alkylene group;

Z represents an O or S atom, an amino or $C_{1-6}$ alkylamino group, a single bond or a $C_{1-6}$ alkylene group; and

J represents an O or S atom, an amino or a $C_{1-6}$ alkylamino group or a $C_{1-6}$ alkylene group.

13. A process as claimed in claim 2, wherein

$R^1$ represents a hydrogen atom or an unbranched $C_{1-6}$ alkyl group optionally substituted in the terminal position with a chlorine or methoxy group;

$R^2$ represents a hydrogen atom;

$R^3$ represents a hydrogen atom;

$R^4$ represents a hydrogen atom;

$R^5$ represents a benzyl, methylbenzyl, benzothienyl, 2-thiophenemethyl, 3-thiophenemethyl, indan-2-yl, tetrahydropyran-2-ylmethyl, cyclohexanemethyl group optionally substituted with up to two substituents independently chosen from chlorine, bromine, or fluorine atoms, a methoxy group or a $C_{1-3}$ alkyl group optionally substituted with up to three fluorine atoms;

$R^6$ represents a hydrogen atom;

$R^7$ represents a hydrogen atom or an optionally substituted hydrocarbyl group of $C_{1-6}$ carbon atoms;
X represents an ethoxy, methoxy or azido group, or a fluorine, chlorine or bromine atom;
M represents an O or S atom, an amino or $C_{1-6}$ alkylamino group, a single bond, a $C_{1-6}$ alkylene group;
E represents an O or S atom, an amino or $C_{1-6}$ alkylamino group, a single bond or a $C_{1-6}$ alkylene group;
Z represents an O or S atom, an amino or $C_{1-6}$ alkylamino group, a single bond or a $C_{1-6}$ alkylene group; and
J represents an O or S atom, an amino or a $C_{1-6}$ alkylamino group or a $C_{1-6}$ alkylene group.

14. A process as claimed in claim 3, wherein
$R^1$ represents a hydrogen atom or optionally substituted hydrocarbyl group of $C_{1-6}$ carbon atoms;
$R^2$ represents a hydrogen atom;
$R^3$ represents a hydrogen atom;
$R^4$ represents a hydrogen atom;
$R^5$ represents a benzyl, methylbenzyl, benzothienyl, 2-thiophenemethyl, 3-thiophenemethyl, indan-2-yl, tetrahydropyran-2-ylmethyl, cyclohexanemethyl group optionally substituted with up to two substituents independently chosen from chlorine, bromine or fluorine atoms, a methoxy group or a $C_{1-3}$ alkyl group optionally substituted with up to three fluorine atoms;
$R^6$ represents a hydrogen atom;
$R^7$ represents a hydrogen atom or an optionally substituted hydrocarbyl group of $C_{1-6}$ carbon atoms;
$R^8$ represents a hydrogen, fluorine, chlorine or bromine atom, an optionally substituted hydrocarbyl group of $C_{1-6}$ carbon atoms or an alkylthio group;
$R^9$ represents a hydrogen, fluorine, chlorine or bromine atom, an optionally substituted hydrocarbyl group of $C_{1-6}$ carbon atoms or an alkylthio group; and
X represents a $C_{1-6}$ alkoxy group, an optionally substituted hydrocarbyl group of $C_{1-6}$ carbon atoms, a fluorine, chlorine or bromine atom or an azido group.

15. A process as claimed in claim 3, wherein
$R^1$ represents a hydrogen atom or an unbranched $C_{1-6}$ alkyl group optionally substituted in the terminal position with a chlorine or methoxy group;
$R^2$ represents a hydrogen atom;
$R^3$ represents a hydrogen atom;
$R^4$ represents a hydrogen atom;
$R^5$ represents a benzyl, methylbenzyl, benzothienyl, 2-thiophenemethyl, 3-thiophenemethyl, indan-2-yl, tetrahydropyran-2-ylmethyl, cyclohexanemethyl group optionally substituted with up to two substituents independently chosen from chlorine, bromine or fluorine atoms, a methoxy group or a $C_{1-3}$ alkyl group optionally substituted with up to three fluorine atoms;
$R^6$ represents a hydrogen atom;
$R^7$ represents a hydrogen atom or an optionally substituted hydrocarbyl group of $C_{1-6}$ carbon atoms;
$R^8$ represents a hydrogen, fluorine, chlorine or bromine atom, an optionally substituted hydrocarbyl group of $C_{1-6}$ carbon atoms or an alkylthio group;
$R^9$ represents a hydrogen, fluorine, chlorine or bromine atom, an optionally substituted hydrocarbyl group of $C_{1-6}$ carbon atoms or an alkylthio group; and
X represents an ethoxy or methoxy group, an optionally substituted hydrocarbyl group of $C_{1-6}$ carbon atoms, a fluorine, chlorine or bromine atom or an azido group.

16. A process as claimed in claim 4, wherein
$R^1$ represents a hydrogen atom or an optionally substituted hydrocarbyl group of $C_{1-6}$ carbon atoms;
$R^2$ represents a hydrogen atom;
$R^3$ represents a hydrogen atom;
$R^4$ represents a hydrogen atom;
$R^5$ represents a benzyl, methylbenzyl, benzothienyl, 2-thiophenemethyl, 3-thiophenemethyl, indan-2-yl, tetrahydropyran-2-ylmethyl or cyclohexanemethyl group optionally substituted with up to two substituents independently chosen from chlorine, bromine or fluorine atoms, a methoxy group or a $C_{1-3}$ alkyl group optionally substituted with up to three fluorine atoms;
$R^6$ represents a hydrogen atom;
$R^7$ represents a hydrogen atom or an optionally substituted hydrocarbyl group of $C_{1-6}$ carbon atoms;
$R^8$ represents a hydrogen, fluorine, chlorine or bromine atom, an optionally substituted hydrocarbyl group of $C_{1-6}$ carbon atoms or an alkylthio group;
$R^9$ represents a hydrogen, fluorine, chlorine or bromine atom, an optionally substituted hydrocarbyl

group of $C_{1-6}$ carbon atoms or an alkylthio group; and

X represents a $C_{1-6}$ alkoxy group, an optionally substituted hydrocarbyl group of $C_{1-6}$ carbon atoms, a fluorine, chlorine or bromine atom or an azido group.

17. A process as claimed in claim 4, wherein

$R^1$ represents a hydrogen atom or an unbranched $C_{1-6}$ alkyl group optionally substituted in the terminal position with a chlorine or methoxy group;

$R^2$ represents a hydrogen atom;

$R^3$ represents a hydrogen atom;

$R^4$ represents a hydrogen atom;

$R^5$ represents a benzyl, methylbenzyl, benzothienyl, 2-thiophenemethyl, 3-thiophenemethyl, indan-2-yl, tetrahydropyran-2-ylmethyl or cyclohexanemethyl group optionally substituted with up to two substituents independently chosen from chlorine, bromine or fluorine atoms, a methoxy group or a $C_{1-3}$ alkyl group optionally substituted with up to three fluorine atoms;

$R^6$ represents a hydrogen atom;

$R^7$ represents a hydrogen atom or an optionally substituted hydrocarbyl group of $C_{1-6}$ carbon atoms;

$R^8$ represents a hydrogen, fluorine, chlorine or bromine atom, an optionally substituted hydrocarbyl group of $C_{1-6}$ carbon atoms or an alkylthio group;

$R^9$ represents a hydrogen, fluorine, chlorine or bromine atom, an optionally substituted hydrocarbyl group of $C_{1-6}$ carbon atoms or an alkylthio group; and

X represents an ethoxy, methoxy or azido group, or a fluorine, chlorine or bromine atom.

18. A process as claimed in claim 5, wherein

$R^1$ represents a hydrogen atom or optionally substituted hydrocarbyl group of $C_{1-6}$ carbon atoms;

$R^2$ represents a hydrogen atom;

$R^3$ represents a hydrogen atom;

$R^4$ represents a hydrogen atom;

$R^5$ represents an optionally substituted alicyclicalkyl or hetereocyclicalkyl group;

$R^6$ reperesents a hydrogen atom;

X represents a $C_{1-6}$ alkoxy group, an optionally substituted hydrocarbyl group of $C_{1-6}$ carbon atoms, a fluorine, chlorine or bromine atom or an azido group.

19. A process as claimed in claim 5 wherein

$R^1$ represent a hydrogen atom or an unbranched $C_{1-6}$ alkyl group optionally substituted in the terminal position with a chlorine or methoxy group;

$R^2$ represents a hydrogen atom;

$R^3$ represents a hydrogen atom;

$R^4$ represents a hydrogen atom;

$R^5$ represents an optionally substituted cyclohexanemethyl or (tetrahydropyran-2-yl)methyl group;

$R^6$ represent a hydrogen atom;

X represents an ethoxy or methoxy group, an optionally substituted hydrocarbyl group of $C_{1-6}$ carbon atoms, a fluorine, chlorine or bromine atom or an azido group.

20. A process as claimed in claim 1 wherein the compound of formula (I) is selected from:

(1R,2S,4R)-2-cyclopentanemethyloxy-4-methoxy-6,8-dioxabicyclo[3.2.1]octane;
(1R,2S,4R)-2-cycloheptanemethyloxy-4-methoxy-6,8-dioxabicyclo[3.2.1]octane;
(1R,2S,4R)-4-methoxy-2-[(tetrahydropyran-2-yl)methyloxy]-6,8-dioxabicyclo[3.2.1]octane;
(1R,2S,4R)-4-methoxy-2-(2-methylpentyloxy)-6,8-dioxabicyclo[3.2.1]octane;
(1R,2S,4R)-4-methoxy-2-(2-methylbutyloxy)-6,8-dioxabicyclo[3.2.1]octane;
(1R,2S,4R)-2-(3-cyclohexene-1-methyloxy)-4-methoxy-6,8-dioxabicyclo[3.2.1]octane;
(1R,2S,4R)-4-methoxy-2-[(1S,2S,5S)-myrtanyloxy]-6,8-dioxabicyclo[3.2.1]octane;
(1R,2S,4R)-2-cyclohexanemethyloxy-4-ethoxy-6,8-dioxabicyclo[3.2.1]octane;
(1R,2S,4R)-2-cyclooctanemethyloxy-4-methoxy-6,8-dioxabicyclo[3.2.1]octane;
(1R,2S,4R)-4-methoxy-2-(1-methylcyclohexane-1-methyloxy)-6,8-dioxabicyclo[3.2.1]octane;
(1R,2S,4R)-2-(2-ethylbutyloxy)-4-methoxy-6,8-dioxabicyclo[3.2.1]octane;
(1R,2S,4R)-2-(-2-cyclohexylethyloxy)-4-methoxy-6,8-dioxabicyclo[3.2.1]octane;
(1R,2S,4R)-2-cyclohexyloxy-4-methoxy-6,8-dioxabicyclo[3.2.1]octane;
(1R,2S,4R)-2-cyclohexanemethyloxy-4-methoxy-6,8-dioxabicyclo[3.2.1]octane;
(1R,2S,4R)-5-butyl-2-cyclohexanemethyloxy-4-methoxy-6,8-dioxabicyclo[3.2.1]octane;
(1R,2S,4R,1′RS)-2-(1′-cyclohexyl-ethyloxy)-4-methoxy-6,8-dioxabicyclo[3.2.1]octane;
(1R,2S,4R,4′RS)-4-methoxy-2-(4′-methylcyclohexane)methyloxy-6,8-dioxabicyclo[3.2.1]octane;
(1R,2S,4R,2yRS)-4-methoxy-2-(2′-methylcyclohexane)methyloxy-6,8-dioxabicyclo[3.2.1]octane;

(1R,2S,4R)-2-cyclohexanemethyloxy-4-methoxy-5-propyl-6,8-dioxabicyclo[3.2.1]octane;
(1R,2S,4R)-2-cyclohexanemethyloxy-5-ethyl-4-methoxy-6,8-dioxabicyclo[3.2.1]octane;
(1RS,2SR,4RS)-4-cyclohexanemethyloxy-2-methoxy-6,8-dioxabicyclo[3.2.1]octane;
(1R,2S,4R)-2-cyclohexanemethyloxy-4-ethoxy-5-ethyl-6,8-dioxabicyclo[3.2.1]octane;
(1R,2S,4R)-2-cyclohexanemethyloxy-4-ethoxy-5-methyl-6,8-dioxabicyclo[3.2.1]octane;
(1R,2S,4R)-2-(3-cyclohexene-1-methyloxy)-4-methoxy-5-methyl-6,8-dioxabicyclo[3.2.1]octane;
(1R,2S,4R)-2-(3-cyclohexene-1-methyloxy)-4-methoxy-5-ethyl-6,8-dioxabicyclo[3.2.1]octane;
(1R,2S,4R)-2-(3-cyclohexene-1-methyloxy)-4-ethoxy-5-ethyl-6,8-dioxabicyclo[3.2.1]octane;
(1R,2S,4R)-2-(3-cyclohexene-1-methyloxy)-4-ethoxy-5-methyl-6,8-dioxabicyclo[3.2.1]octane;
(1R,2S,4R)-2-(3-cyclohexene-1-methyloxy)-4-ethoxy-5-propyl-6,8-dioxabicyclo[3.2.1]octane;
(1R,2S,4R)-2-cyclohexanemethyloxy-4-methoxy-5-(2-propen-1-yl)-6,8-dioxabicyclo[3.2.1]octane;
(1R,2S,4R)-2-cyclohexanemethyloxy-4-ethoxy-5-(2-propen-1-yl)-6,8-dioxabicyclo[3.2.1]octane;
(1R,2S,4R)-2-Benzyloxy-4-methoxy-1-methyl-6,8-dioxabicyclo[3.2.1]octane;
(1RS,2S,4R,7S)-2-Benzyloxy-4-methoxy-7-methyl-6,8-dioxabicyclo[3.2.1]octane;
(1RS,2S,4R,7S)-2-Benzyloxy-7-ethyl-4-methoxy-6,8-dioxabicyclo[3.2.1]octane;
(1S,2S,4R,7R)-2-Benzyloxy-4-methoxy-7-methyl-6,8 dioxabicyclo[3.2.1]octane and its (7S)-isomer;
(1S,2S,4R)-2-Benzyloxy-4-methoxy-7,7dimethyl-6,8-dioxabicyclo[3.2.1]octane;
(1S,2S,4R,7R)-2-Benzyloxy-7-ethyl-4-methoxy-6,8-dioxabicyclo[3.2.1]octane;
(1S,2S,4R,7S)-2-Benzyloxy-4-methoxy-7-butyl-6,8-dioxabicyclo[3.2.1]octane;
(1RS,2SR,4RS;7RS)-2-Benzyloxy-4-methoxy-5,7-dimethyl-6,8-dioxabicyclo[3.2.1]octane;
(1RS,2SR,4RS)-2-Benzyloxy-4-methoxy-1,5-dimethyl-6,8-dioxabicyclo[3.2.1]octane
(1R,2S,4R)-2-Benzyloxy-1-ethyl-4-methoxy-6,8-dioxabicyclo[3.2.1]octane;
(1S,2R,4S)-4-Benzyloxy-2-methoxy-6,8-dioxabicyclo[3.2.1]octane;
(1S,2R,4S)-4-Benzyloxy-5-chloromethyl-2-methoxy-6,8-dioxabicyclo[3.2.1]octane;
(1S,2R,4S)-4-Benzyloxy-2-methoxy-5-methyl-6,8-dioxabicyclo[3.2.1]octane;
(1S,2R,4S)-4-Benzyloxy-2-methoxy-1-propyl-6,8-dioxabicyclo[3.2.1]octane;
(1RS,2SR,4RS,7RS)-4-Benzyloxy-2-methoxy-5,7-dimethyl-6,8-dioxabicyclo[3.2.1]octane;
(1RS,2SR,4RS)-4-Benzyloxy-2-methoxy-1,5-dimethyl-6,8-dioxabicyclo[3.2.1]octane;
(1RS,2RS,4SR,7RS)-4-Benzyloxy-7-ethyl-2-methoxy-6,8-dioxabicyclo[3.2.1]octane;
(1RS,2SR,4RS)-4-Benzyloxy-2-methoxy-6,8-dioxabicyclo[3.2.1]octane;
(1RS,2SR,4RS)-2-Methoxy-4-(2-methylbenzyloxy)-6,8-dioxabicyclo[3.2.1]octane
(1RS,2SR,4RS,7SR)-4-Benzyloxy-2-methoxy-7-methyl-6,8-dioxabicyclo[3.2.1]octane;
(1RS, 2SR,4RS,7SR)-2-Methoxy-7-methyl-4-(2-methylbenzyloxy)-6,8-dioxabicyclo[3.2.1]octane and
(1RS,2SR,4RS,7SR)-4-Hydroxy-2-methoxy-7-methyl-6,8-dioxabicyclo[3.2.1.]octane;
(1RS,2SR,4RS,7RS)-4-Benzyloxy-2-methoxy-7-methyl-6,8-dioxabicyclo[3.2.1]octane and its (1RS, 2SR,4RS,7SR)stereoisomer;
(1RS,2RS,4SR)-4-Benzyloxy-2-methoxy-7,7-dimethyl-6,8-dioxabicyclo[3.2.1]octane;
(1RS,2SR,4RS)-4-(2-fluorobenzyloxy)-2-methoxy-6,8-dioxabicyclo[3.2.1]octane;
(1RS,2SR,4RS)-4-(2-chlorobenzyloxy)-2-methoxy-6,8-dioxabicyclo[3.2.1]octane;
(1RS,2SR,4RS)-4-(2,4-difluorobenzyloxy)-2-methoxy-6,8-dioxabicyclo[3.2.1]octane;
(1RS,2SR,4RS)-4-(2,4-dichlorobenzyloxy)-2-methoxy-6,8-dioxabicyclo[3.2.1]octane;
(1RS,2SR,4RS)-4-(2,6-difluorobenzyloxy)-2-methoxy-6,8-dioxabicyclo[3.2.1]octane;
(1RS,2SR,4RS)-2-methoxy-4-[2-(trifluoromethyl)benzyloxy]-6,8-dioxabicyclo[3.2.1]octane;
(1RS,2SR,4RS)-2-methoxy-4-(3-methylbenzyloxy)-6,8-dioxabicyclo[3.2.1]octane;
(1RS,2SR,4RS)-4-(2-cyanobenzyloxy)-4-methoxy-6,8-dioxabicyclo[3.2.1]octane;
(1RS,2SR,4RS)-2-methoxy-4-(4-methylbenzyloxy)-6,8-dioxabicyclo[3.2.1]octane
(1RS,2SR,4RS)-4-(2-chloro-3-thienyl)methoxy-2-methoxy-6,8-dioxabicyclo[3.2.1]octane;
(1RS,2SR,4RS,7SR)-4-(2-Fluorobenzyloxy)-2-methoxy-7-methyl-6,8-dioxabicyclo[3.2.1]octane;
(1S,6S,8R)-6-Benzyloxy-8-methoxy-3,9-dioxabicyclo[3.3.1]nonane;
(1S,2S,4R)-2-Benzyloxy-4-methoxy-8-oxa-6-thiabicyclo[3.2.1]octane;
(1S,2S,4R)-2-Benzyloxy-4-methoxy-8-oxa-6-thiabicyclo[3.2.1]octane,6-oxide;
(1S,2S,4R)-2-Benzyloxy-4-methoxy-8-oxa-6-thiabicyclo[3.2.1]octane,6,6′-dioxide;
(1RS,2RS,3RS,4RS)-2,3-Epoxy-4-(2-methylbenzyloxy)-6,8-dioxabicyclo[3.2.1]octane.

21. A process for making a herbicidal composition comprising mixing a herbicidally effective amount of a compound of formula IA

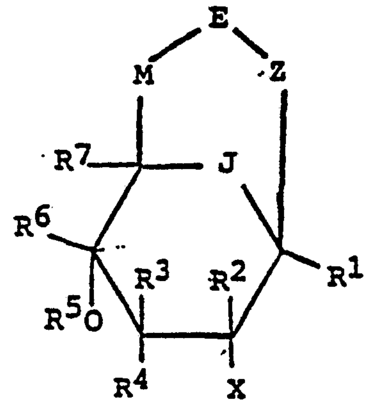

wherein $R^1$, $R^2$, $R^3$, $R^4$, $R^5$, $R^6$, $R^7$, M, E, Z, J and X are as defined in claim 1 for formula I but not including provisos (v) to (ix), with a carrier and/or surface active agent.

22. A process as claimed in claim 21 wherein the compound of formula IA is a compound of formula I obtained by a process as claimed in any of claims 1 to 20.

23. A process for making a plant regulating composition comprising mixing an effective plant growth regulating amount of a compound of formula IA as defined in claim 21 with a carrier and/or surface active agent.

24. A process as claimed in claim 23 wherein the compound of formula IA is a compound of formula I obtained by a process as claimed in any of claims 1 to 20.

25. A method of controlling unwanted plants comprising applying to the plants a herbicidally effective amount of a compound of formula IA as defined in claim 21.

26. A method of regulating plant growth comprising applying a plant growth regulating amount of a compound of formula IA as defined in claim 21.